# EUROPEAN PATENT APPLICATION

(11) **EP 4 495 113 A1**
(43) Date of publication of application: **22.01.2025**
(21) Application number: 23769679.4
(22) Date of filing: 10.03.2023
(51) Int. Cl.: C07D 405/14, C07D 401/14, C07D 401/12, C07D 413/14, C07D 491/044, A61K 31/4427, A61P 35/00

(54) **KIF18A INHIBITOR**

(30) Priority: 17.03.2022 CN 202210266719
(71) Applicant: Wigen Biomedicine Technology (Shanghai) Co., Ltd., Shanghai 201203 (CN)
(72) Inventor: XIE, Yuli, Shanghai 201203 (CN); WU, Yingming, Shanghai 201203 (CN); QIAN, Lihui, Shanghai 201203 (CN)
(74) Representative: Willett, Christopher David
(86) International application number: PCT/CN2023/080775
(87) International publication number: WO 2023/174175

(57) **Abstract**

Disclosed in the present invention is a class of KIF18A inhibitors. Specifically, the present invention relates to a compound of general formula (1), a method for preparing same, and use of the compound of general formula (1) or an isomer thereof, a crystalline form thereof, a pharmaceutically acceptable salt thereof, a hydrate thereof, or a solvate thereof as a KIF18A inhibitor in preparing an anti-tumor medicament.

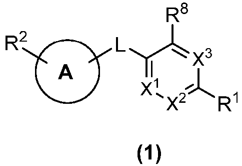

## Description

The present application claims priority to Chinese Patent Application No. 202210266719.4 filed on March 17, 2022, which is incorporated herein by reference in its entirety.

### TECHNICAL FIELD

The present invention relates to the field of pharmaceutical chemistry, and particularly to a class of compounds with an inhibitory effect on KIF18A protein, a method for preparing same, and use of the class of compounds in preparing anti-tumor medicaments.

### BACKGROUND

Genomic instability is a common feature of most tumor cells. Most tumor cells exhibit abnormal gain or deletion of chromosomes. The chromosome instability of tumor cells leads to the interaction of abnormal chromosomes with mitotic spindle microtubules, which in turn causes chromosome segregation errors. Cells with chromosome instability develop increased spindle microtubule polymerization and reduced spindle microtubule-centromere contact turnover, as compared to cells with normal chromosomes. Therefore, anti-mitotic therapies directed against the microtubule backbone may be particularly effective for cells with chromosome instability.

Kinesins are a class of molecular motors that play important roles in cell division and intracellular vesicle and organelle transport. Mitotic kinesins play important roles in several aspects such as spindle assembly, chromosome segregation, centrosome segregation, and dynamics. Human kinesins are classified into 14 subfamilies based on differences in amino acid sequences of motor domains, and the ATPase activity located in the motor domains drives the proteins to move unidirectionally along the microtubules. The non-motor domains of these proteins are responsible for interacting with substrates, and a variety of different membranous organelles, signal transduction scaffold systems, and chromosomes serve as substrates with which the non-motor domains can interact. The kinesins gain energy through ATP hydrolysis, moving the substrates along polarized microtubules. Therefore, kinesins are commonly referred to as "plus-end" or "minus-end" directed motors.

KIF18A protein belongs to the kinesin-8 subfamily. KIF18A protein is overexpressed in various types of cancers, such as lung, ovarian, cervical, breast, pancreatic, prostate, colon, and bladder cancers. Studies suggest that KIF18A affects the dynamics of the plus-ends of the centromere microtubules so as to control correct chromosome positioning and spindle tension. In tumor cells with chromosome instability, abnormal microtubule dynamics make such cells particularly dependent on KIF18A protein to reduce spindle microtubule-centromere contact turnover and to limit microtubule growth (Nat Commun. 2021, 12, 1213). When KIF18A protein is deleted from tumor cells with chromosome instability, centrosomes of the cells are fragmented and mitotic progression is slowed or stopped. However, these phenomena do not occur in cells with normal chromosomes. Therefore, the activity of KIF18A protein does not have a significant effect on the proliferation of normal cells but is very critical for the growth of chromosomally unstable tumors. Accordingly, the development of KIF18A inhibitors is a new promising approach against tumors with chromosome instability.

### SUMMARY

The present invention provides a compound of general formula (1) or an isomer thereof, a crystalline form thereof, a pharmaceutically acceptable salt thereof, a hydrate thereof, or a solvate thereof: 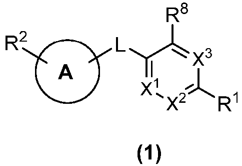 wherein in general formula (1):
X¹ is -CR⁵ = or N;
X² is -CR⁶ = or N;
X³ is -CR⁷ = or N;
ring A is 7-10 membered cycloalkylene, 7-10 membered heterocycloalkylene, 7-10 membered heteroarylene, or 10-membered arylene, wherein the 7-10 membered cycloalkylene, 7-10 membered heterocycloalkylene, 7-10 membered heteroarylene, or 10-membered arylene may be optionally substituted with 0, 1, 2, or 3 groups selected from H, halogen, -C₁₋₄ hydrocarbyl, -C₁₋₄ halohydrocarbyl, or -O-C₁₋₄ hydrocarbyl;
L is -(C=O)-NR³-**** or -NR³-(C=O)-****; **** represents attachment to a 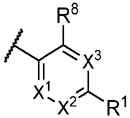 end;
R¹ is -CN or -Z-R¹⁰, wherein Z is a chemical bond, -C₀₋₄ hydrocarbylene-, -NR¹¹-, -NR¹¹SO₂-, -SO₂NR¹¹-,-NR¹¹-S(=O)(=NH)-**, -S(=O)(=NH)-, -S-, -S(=O)-, -SO₂-, -C₀₋₄ hydrocarbylene-O-**, -(C=O)-, - (C=O)NR¹¹-, -C(=N-OH)-, or -NR¹¹(C=O)-**; or the group -Z-R¹⁰ is -N=S(=O)-(R¹⁰)₂, wherein the two R¹⁰ may be combined with the sulfur atom to which they are each attached to form a saturated or partially saturated 3-, 4-, 5-, or 6-membered monocyclic ring containing 0, 1, 2, or 3 N atoms and 0, 1 or 2 atoms selected from O and S; ** represents attachment to an R¹⁰ end;
R² is halogen or a group -Y-R¹², wherein Y is a chemical bond, -C₀₋₄ hydrocarbylene-, -N(C₀₋₁ hydrocarbyl)-C₀₋₄ hydrocarbylene-***, -C(=O)NR^{a} (C₁₋₄ hydrocarbyl)-***, -O-C₀₋₄ hydrocarbylene-***, -S-, -S(=O)-, - SO₂-, -SO₂NR¹²-***, or -S(=O)(=NH)-***; *** represents attachment to an R¹² end;
R³ is H or C₁₋₆ hydrocarbyl;
R⁵ is H, halogen, C₁₋₈ alkyl, or C₁₋₄ haloalkyl;
R⁶ is H, halogen, C₁₋₈ alkyl, C₁₋₄ haloalkyl, -OH, -O-R^{6a}, or -O-R^{6b};
R⁷ is H, halogen, C₁₋₈ hydrocarbyl, or C₁₋₄ halohydrocarbyl;
R⁸ is selected from the group consisting of: 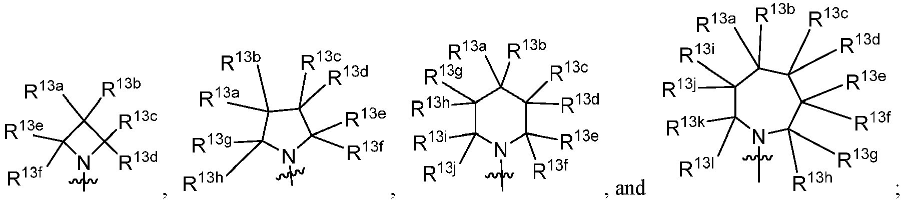
   R^{13a}, R^{13b}, R^{13c}, R^{13d}, R^{13e}, R^{13f}, R^{13g}, R^{13h}, R¹³ⁱ, R^{13j}, R^{13k}, and R^{13l} are each independently H, halogen, R^{13m}, or R¹³ⁿ; or each of the pairs of R^{13a}/R^{13b}, R^{13c}/R^{13d} R^{13e}/R^{13f}, R^{13g}/R^{13h}, R¹³ⁱ/R^{13j}, and R^{13k}/R^{13l} may independently form, with the carbon atom to which they are each attached, a saturated or partially saturated 3-, 4-, 5-, or 6-membered monocyclic ring spiro-linked to R⁸ ring, wherein the 3-, 4-, 5-, or 6-membered monocyclic ring contains 0, 1, 2, or 3 N atoms and 0, 1, or 2 atoms selected from O and S, and further, the 3-, 4-, 5-, or 6-membered monocyclic ring is substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -NR^{a}R^{a}, and oxo;
   R¹⁰ is H, R^{10a}, R^{10b}, or R^{10c};
   R¹¹ is H, R^{11a}, or R^{11b};
   R¹² is R^{12a} or R^{12b};
   R^{6a}, R^{10a}, R^{11a}, R^{12a}, or R^{13m}, in each case, is independently selected from a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0, 1 or 2 atoms selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -C(=O)R^{b}, - C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, R⁴, and oxo;
   R^{6b}, R^{10b}, R^{11b}, R^{12b}, or R¹³ⁿ, in each case, is independently selected from C₁₋₆ hydrocarbyl, wherein the hydrocarbyl may be optionally substituted with 0, 1, 2, 3, 4, or 5 groups selected from F, Cl, Br, -R^{a}, -OR^{a},-OC₁₋₄ halohydrocarbyl, and CN;
   R^{10c}, in each case, is independently selected from C₁₋₆ hydrocarbyl, wherein the hydrocarbyl may be optionally substituted with 0, 1, 2, 3, 4, or 5 groups selected from F, Cl, Br, -R^{a}, -R^{c}, -OR^{a}, -OC₁₋₄ halohydrocarbyl, and CN;
   R4, in each case, is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -C(=O)R^{b},-C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b},-N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)Rb, -C₁₋₆ hydrocarbyl C(=O)NR^{a}R^{a}, -C₁₋₆ hydrocarbyl C(=O)OR^{a}, and oxo;
   R^{a}, in each case, is independently H or R^{b};
   R^{b}, in each case, is independently C₁₋₆ hydrocarbyl, phenyl, or benzyl, wherein the hydrocarbyl may be optionally substituted with 0, 1, 2, or 3 groups selected from halogen, -OH, -OC₁₋₄ hydrocarbyl, -NH₂, -NHC₁₋₄ hydrocarbyl, -OC(=O)C₁₋₄ hydrocarbyl, or -N(C₁₋₄ hydrocarbyl) C₁₋₄ hydrocarbyl; and the phenyl and benzyl may be each independently and optionally substituted with 0, 1, 2, or 3 groups selected from halogen, C₁₋₄ hydrocarbyl, C₁₋₃ halohydrocarbyl, -OH, -OC₁₋₄ hydrocarbyl, -NH₂, -NHC₁₋₄ hydrocarbyl, -OC(=O)C₁₋₄ hydrocarbyl, or -N(C₁₋₄ hydrocarbyl) C₁₋₄ hydrocarbyl; and
   R^{c}, in each case, is independently -OC(=O)C₁₋₅ hydrocarbyl, wherein the hydrocarbyl may be optionally substituted with 1, 2, or 3 groups selected from -OH or -NH₂.

In another preferred embodiment, the general formula (1) has the following structure:

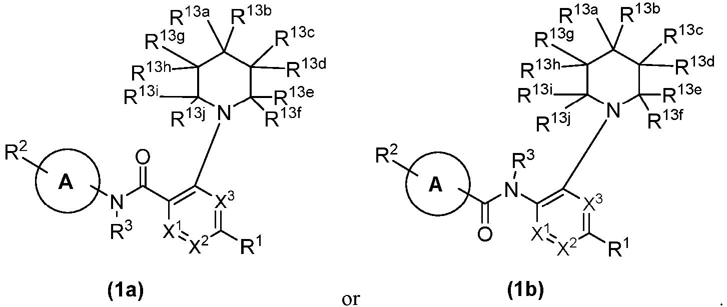

In another preferred embodiment, in the general formula (1), ring A is 9-10 membered cycloalkylene, 9-10 membered heterocycloalkylene, 9-10 membered heteroarylene, or 10-membered arylene, wherein the 9-10 membered cycloalkylene, 9-10 membered heterocycloalkylene, 9-10 membered heteroarylene, or 10-membered arylene may be optionally substituted with 0, 1, 2, or 3 groups selected from H, F, Cl, Br, -C₁₋₄ hydrocarbyl, -C₁₋₄ halohydrocarbyl, or -O-C₁₋₄ hydrocarbyl.

In another preferred embodiment, in the general formula (1), ring A is: 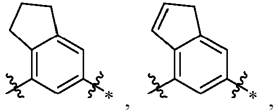 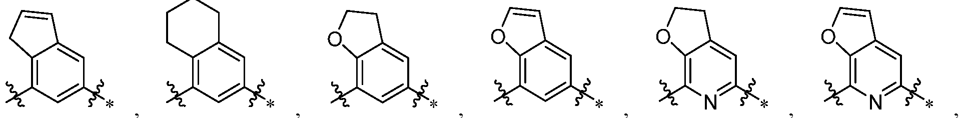 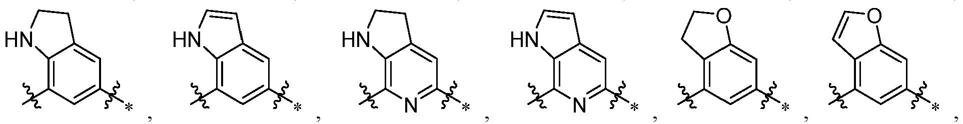 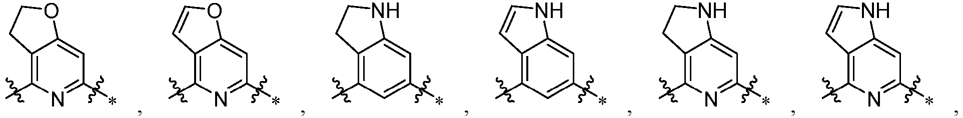 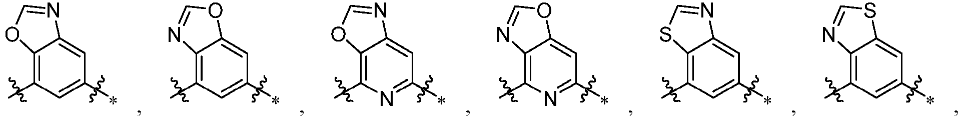 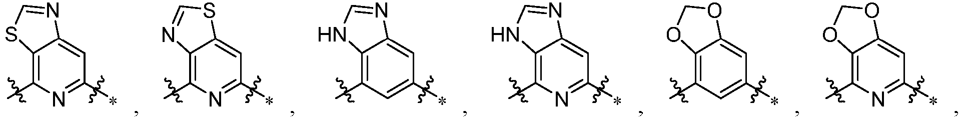 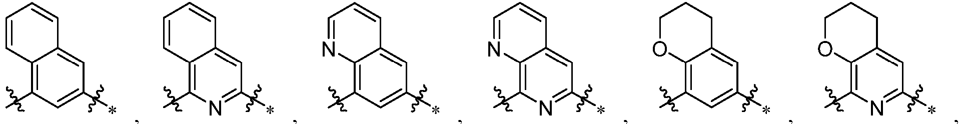 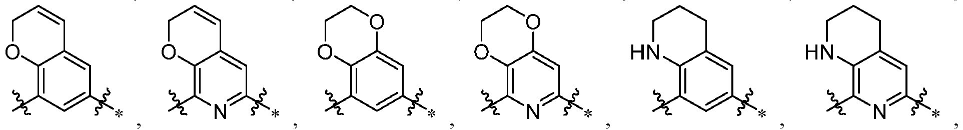 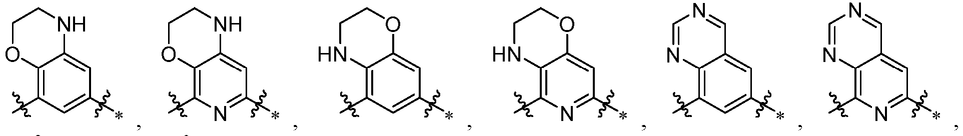 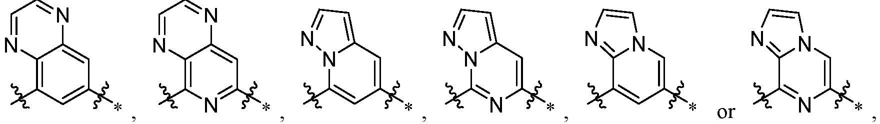 wherein
* represents attachment to an L end; and the groups described above may be optionally substituted with 0, 1, 2, or 3 groups selected from H, F, Cl, Br, -CH₃, -CH₂CH₃, -CF₃, -CH₂CF₃, -OCH₃, or -OCH₂CH₃.

In another preferred embodiment, in the general formula (1), ring A is: 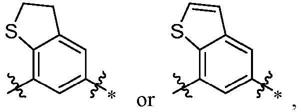 wherein * represents attachment to an L end; and the groups described above may be optionally substituted with 0, 1, 2, or 3 groups selected from H, F, Cl, Br, -CH₃, -CH₂CH₃, -CF₃, -CH₂CF₃, -OCH₃, or -OCH₂CH₃.

In another preferred embodiment, in the general formula (1), R³ is H, methyl, or ethyl, preferably H.

In another preferred embodiment, in the general formula (1), R^{13c}, R^{13d}, R^{13e}, R^{13f}, R^{13g}, R^{13h}, R¹³ⁱ, R^{13j}, R^{13k}, and R^{13l} are each independently H, halogen, C₁₋₆ hydrocarbyl, or C₁₋₄ halohydrocarbyl; and R^{13a} and R^{13b} in a pair of R^{13a}/R^{13b} may be combined with the carbon atom to which they are each attached to form a saturated 3-, 4-, or 5-membered monocyclic ring spiro-linked to R⁸ ring, wherein the monocyclic ring contains 0, 1, 2, or 3 N atoms and 0, 1, or 2 atoms selected from O and S; preferably, R^{13c}, R^{13d}, R^{13e}, R^{13f}, R^{13g}, R^{13h}, R¹³ⁱ, R^{13j}, R^{13k}, and R^{13l} are each independently H, methyl, or ethyl; and R^{13a} and R^{13b} in the pair of R^{13a}/R^{13b} may be combined with the carbon atom to which they are each attached to form a cyclopropyl, cyclobutyl, or cyclopentyl ring spiro-linked to R⁸ ring.

In another preferred embodiment, in the general formula (1), the structural unit 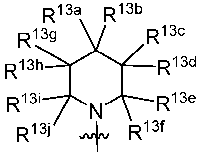 is: 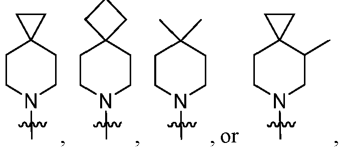 preferably

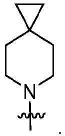

In another preferred embodiment, in the general formula (1), Z is a chemical bond, -NH-, -NHSO₂-, -SO₂NH-, -S(=O)(=NH)-, -S-, -S(=O)-, -SO₂-, -(C=O)-, -(C=O)NH-, or -NH(C=O)-.

In another preferred embodiment, in the general formula (1), R¹⁰ is selected from: (a) H; or (b) C₁₋₆ hydrocarbyl, wherein the hydrocarbyl may be optionally substituted with 0, 1, 2, or 3 groups selected from F, Cl, Br, -OH, or -OCH₃; (c) a group such that when the group -Z-R¹⁰ is -N=S(=O)-(R¹⁰)₂, the two R¹⁰ may be combined with the sulfur atom to which they are each attached to form a saturated, partially saturated, or unsaturated 3-, 4-, 5-, 6-, or 7-membered monocyclic ring containing 0, 1, 2, or 3 N atoms and 0 or 1 atom selected from O and S, wherein the monocyclic ring is substituted with 0, 1, 2, or 3 groups selected from F, Cl, Br, C₁₋₆ hydrocarbyl, C₁₋₄ halohydrocarbyl, -C₁₋₆ hydrocarbylene OH, -OH, -OCH₃, -NH₂, or oxo; or (d) C₁₋₆ hydrocarbyl, wherein the C₁₋₆ hydrocarbyl may be optionally substituted with 1, 2, or 3 groups selected from -OC(=O)C₁₋₅ hydrocarbyl, wherein the C₁₋₅ hydrocarbyl may be optionally substituted with 1 or 2 groups selected from -OH or -NH₂; and the C₁₋₆ hydrocarbyl may be optionally substituted with 0, 1, 2, or 3 groups selected from F, Cl, Br, -OH, or -OCH₃.

In another preferred embodiment, in the general formula (1), R¹ is -CN or a group -Z-R¹⁰, wherein Z is a chemical bond, -NH-, -NHSO₂-, -SO₂NH-, -S(=O)(=NH)-, -S-, -S(=O)-, -SO₂-, -(C=O)-, -(C=O)NH-, or - NH(C=O)-; and R¹⁰ is selected from:
(a) H;
(b) cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 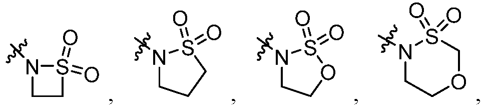 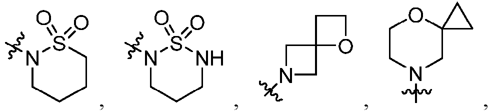 or 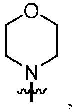 wherein each of the rings may be independently and optionally substituted with 0, 1, 2, or 3 groups selected from OH, F, methyl, -CH₂OH,-C(=O)OCH₃, -C(=O)OC(CH₃)₃, NH₂, CN, and oxo; preferably oxetanyl or cyclopropyl;
(c) C₁₋₆ hydrocarbyl substituted with 0, 1, 2, or 3 OH, F, -C(=O)OCH₃, -NH₂, -NH(CH₃), or -N(CH₃)₂; preferably C₁₋₆ hydrocarbyl substituted with 0, 1, 2, or 3 OH groups; and more preferably C₁₋₆ hydrocarbyl substituted with 1 OH group; or
(d) C₁₋₆ hydrocarbyl, wherein the C₁₋₆ hydrocarbyl may be optionally substituted with 1, 2 or 3 groups selected 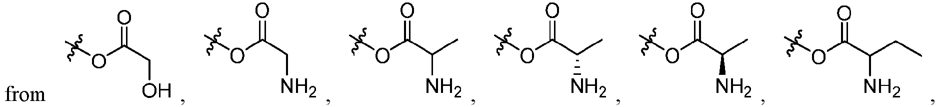 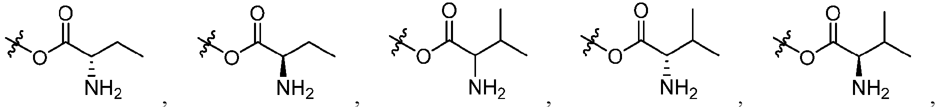 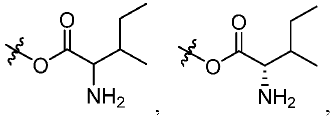 or 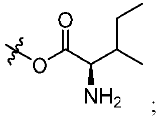 and the C₁₋₆ hydrocarbyl may be optionally
   substituted with 0, 1, 2, or 3 groups selected from F, Cl, Br, -OH, or -OCH₃.

In another preferred embodiment, in the general formula (1), the group -Z-R¹⁰ is -N=S(=O)-(R¹⁰)₂, wherein the two R¹⁰ may be combined with the sulfur atom to which they are each attached to form a saturated or partially saturated 3-, 4-, 5-, or 6-membered monocyclic ring containing 0, 1, 2, or 3 N atoms and 0, 1, or 2 atoms selected from O and S; preferably, the group -Z-R¹⁰ is selected from 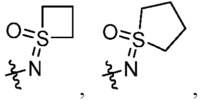 or

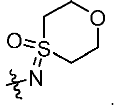

In another preferred embodiment, in the general formula (1), R¹ is a group -Z-R¹⁰, wherein Z is -NHSO₂- or - SO₂NH-; and R¹⁰ is oxetanyl or cyclopropyl; or R¹⁰ is C₁₋₆ hydrocarbyl substituted with 0, 1, 2, or 3 OH groups; or R¹⁰ is C₁₋₆ hydrocarbyl, wherein the C₁₋₆ hydrocarbyl may be optionally substituted with 1, 2 or 3 groups selected from

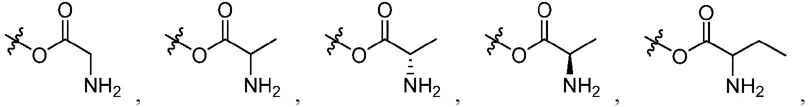

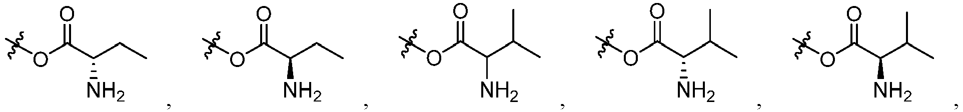

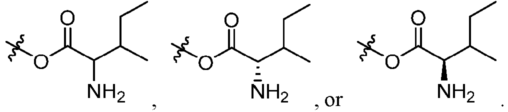

In another preferred embodiment, in the general formula (1), R¹⁰ is selected from C₁₋₆ hydrocarbyl, wherein the hydrocarbyl may be optionally substituted with 0, 1, 2, or 3 groups selected from 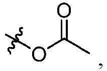 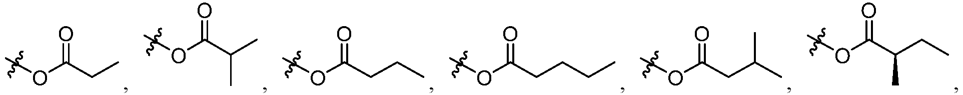 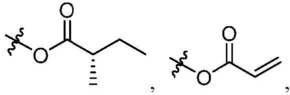 or 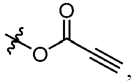 preferably 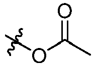 or 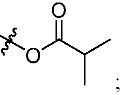 Z is -NHSO₂-
or -SO₂NH-; and Z is preferably -NHSO₂-.

In another preferred embodiment, in the general formula (1), R¹⁰ is selected from C₁₋₆ hydrocarbyl, wherein the hydrocarbyl may be optionally substituted with 1, 2, or 3 groups selected from 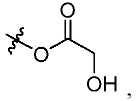 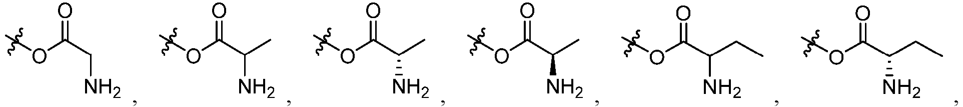 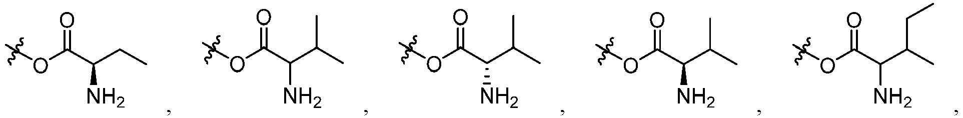 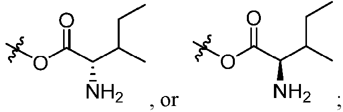 Z is -NHSO₂- or -SO₂NH-.

In another preferred embodiment, in the general formula (1), R² is halogen or a group -Y-R¹², wherein Y is a chemical bond, -NH-, -NH-(CH₂)₀₋₄-, or -O-(CH₂)₀₋₄-; and R¹² is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OH, -OC₁₋₄ halohydrocarbyl, CN, R¹⁴, and oxo; or R¹² is C₁₋₆ hydrocarbyl, wherein the hydrocarbyl may be optionally substituted with 0, 1, 2, 3, 4, or 5 groups selected from F, Cl, Br, -OH, -OC₁₋₄ halohydrocarbyl, or CN.

In another preferred embodiment, in the general formula (1), R² is a saturated 5- or 6-membered monocyclic ring, wherein each of the rings contains 0, 1, or 2 N atoms and 0 or 1 O atom, and each of the rings is substituted with 0, 1, 2, or 3 groups selected from F, Cl, Br, C₁₋₆ hydrocarbyl, C₁₋₄ halohydrocarbyl, -OH, -OC₁₋₄ halohydrocarbyl, CN, R¹⁴, and oxo.

In another preferred embodiment, in the general formula (1), R² is: (a) halogen; (b) a group -Y-R¹², wherein Y is a chemical bond; and R¹² is morpholinyl, piperidinyl, azetidinyl, pyrrolidinyl, cyclopropyl, cyclobutyl, cyclopentyl, cyclohexyl, piperazinyl, tetrahydrofuranyl, 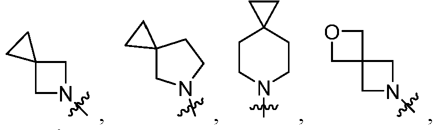 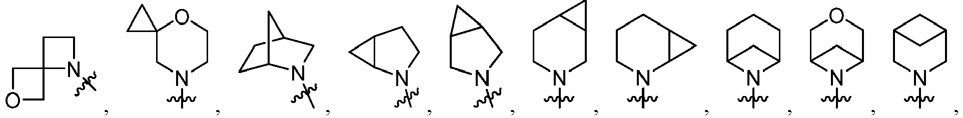 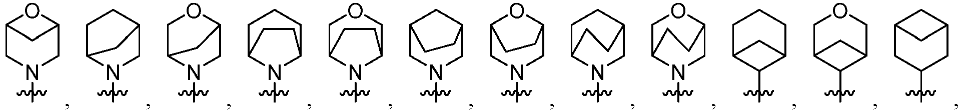 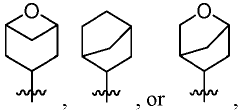 wherein each of the rings is substituted with 0, 1, 2, or 3 groups selected from F, Cl, Br, methyl, CF₃, -OH, -OCHF₂, CN, and oxo; or (c) a group -Y-R¹², wherein Y is -NH-, -O-, -O-(CH₂)-,-O-(CH₂)-(CH₂)-, or -O-(CH₂)-(CH₂)-(CH₂)-, and R¹² is 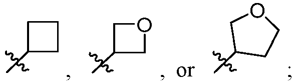 or R¹² is C₁₋₆ hydrocarbyl, wherein the hydrocarbyl may be optionally substituted with 0, 1, 2, 3, 4, or 5 groups selected from F, Cl, Br, methyl, CF3, -OH, or CN.

In another preferred embodiment, in the general formula (1), R² is morpholinyl or piperidinyl, wherein the morpholinyl and piperidinyl may be optionally substituted with 0, 1, 2, or 3 groups selected from F, Cl, Br, methyl, CF₃, -OH, -OCHF₂, and CN.

In another preferred embodiment, in the general formula (1), R² is piperidinyl substituted with 1, 2, or 3 fluorine groups.

In another preferred embodiment, in the general formula (1), R² is:

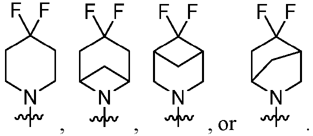

In another preferred embodiment, in the general formula (1), R² is morpholinyl substituted with 1, 2, or 3 methyl groups.

In another preferred embodiment, in the general formula (1), R² is

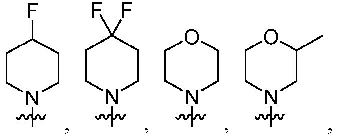

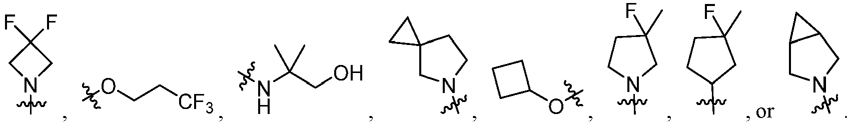

In another preferred embodiment, in the general formula (1), R¹⁰ is selected from cyclopropyl, cyclobutyl, cyclopentyl, oxetanyl, azetidinyl, tetrahydrofuranyl, and 1,3,4-oxathiazinanyl.

In another preferred embodiment, in the general formula (1), R⁵ is H or F, preferably H.

In another preferred embodiment, in the general formula (1), R⁶ is H or F, preferably H.

In another preferred embodiment, in the general formula (1), R⁷ is H.

In various different embodiments of the present invention, the compound of general formula (1) has one of the following structures:

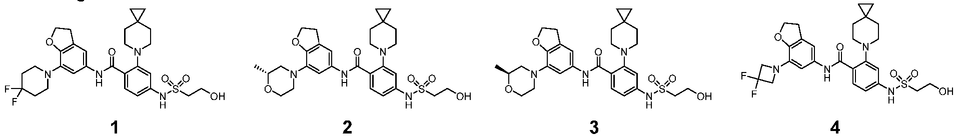

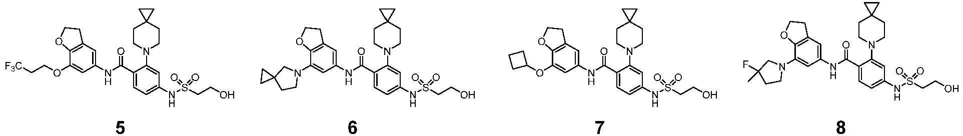

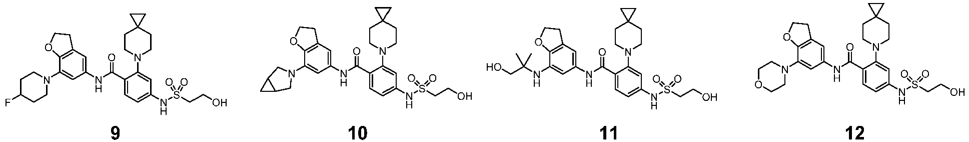

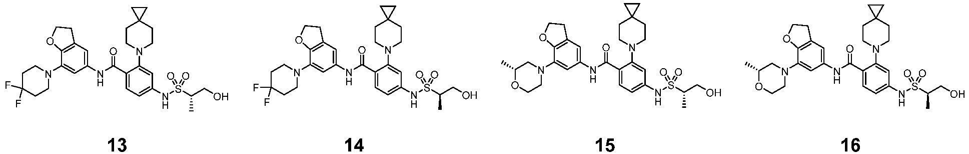

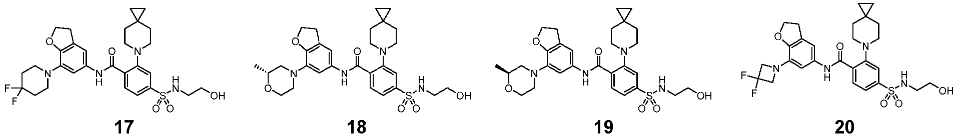

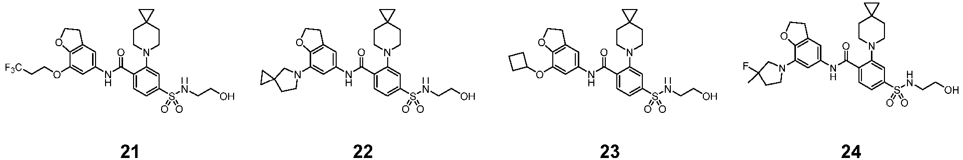

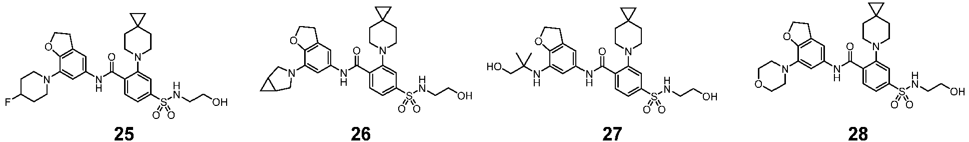

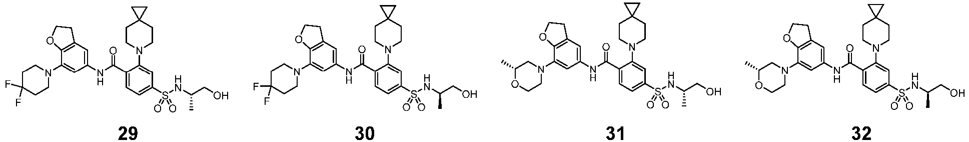

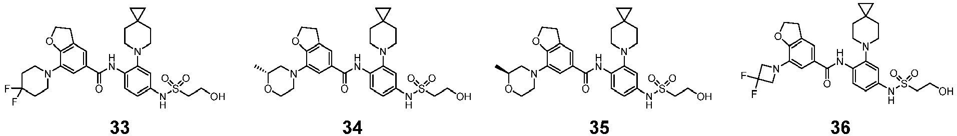

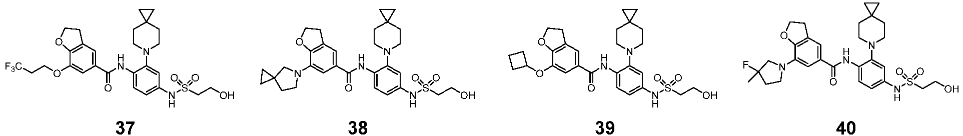

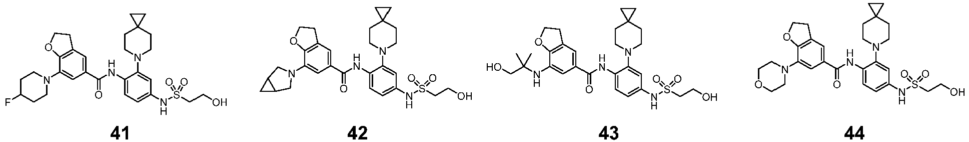

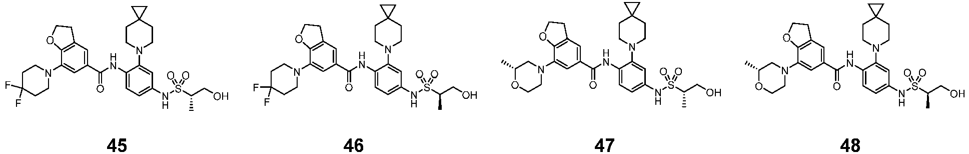

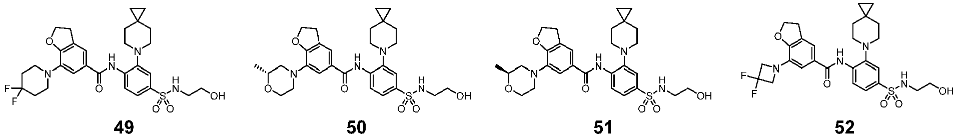

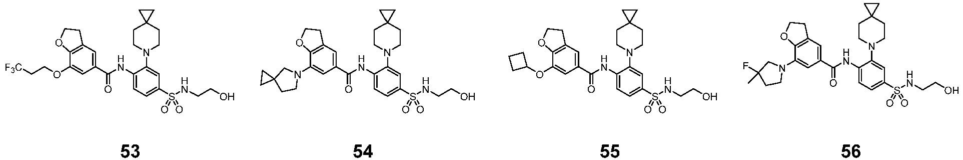

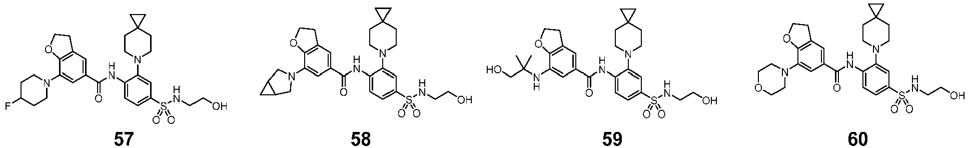

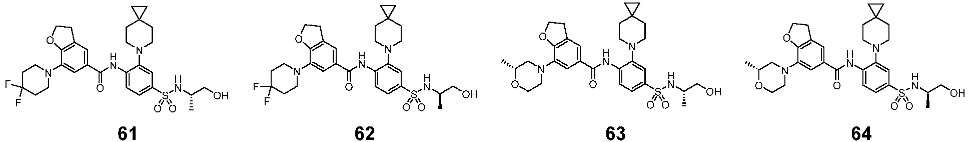

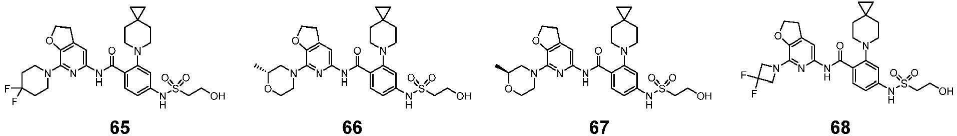

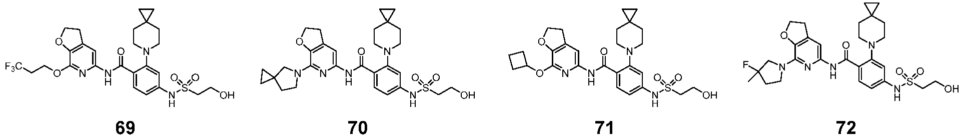

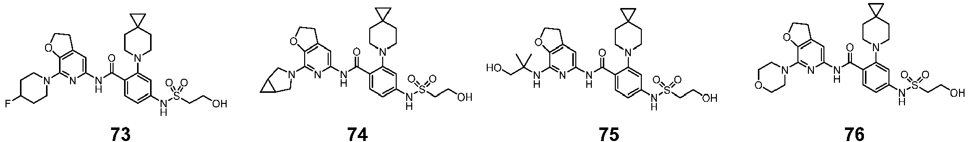

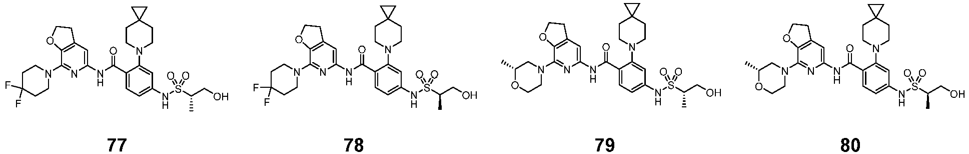

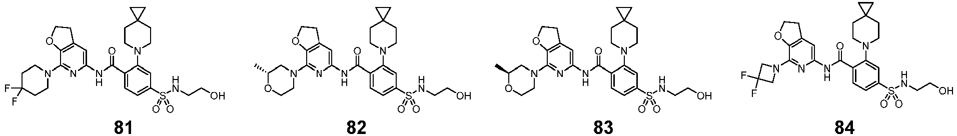

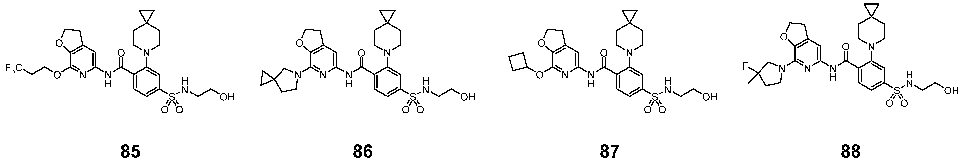

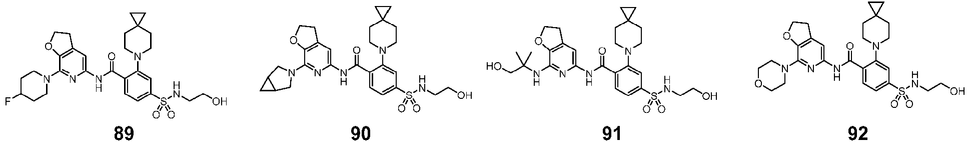

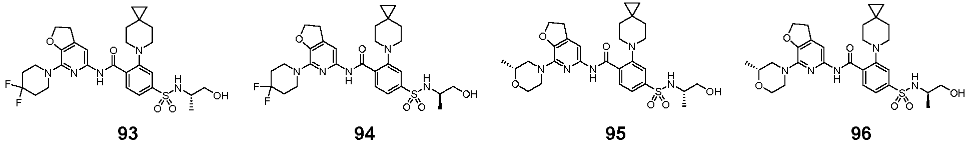

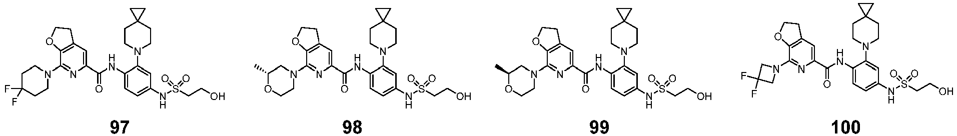

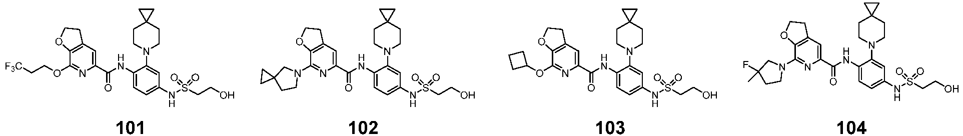

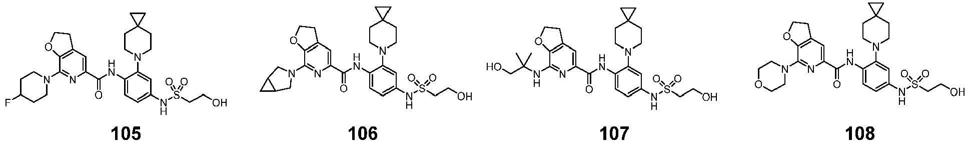

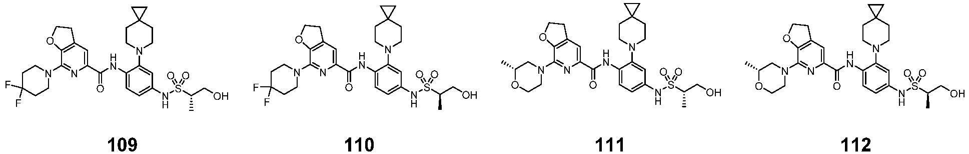

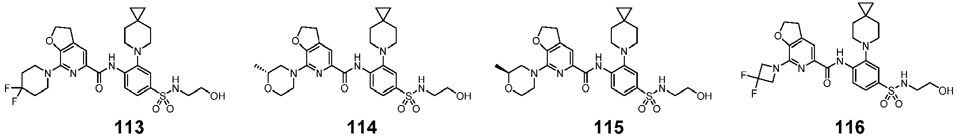

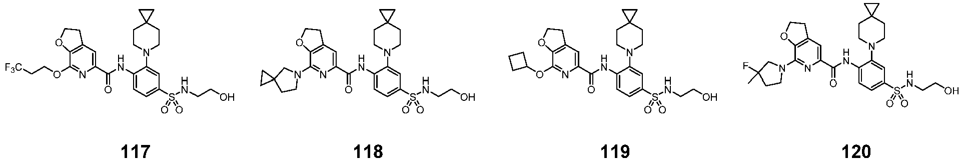

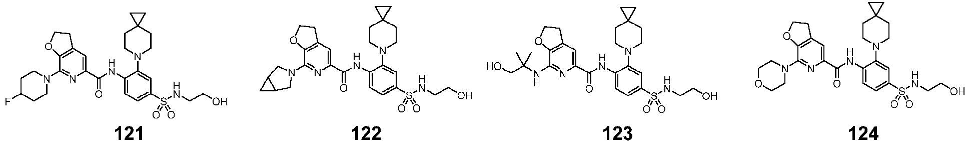

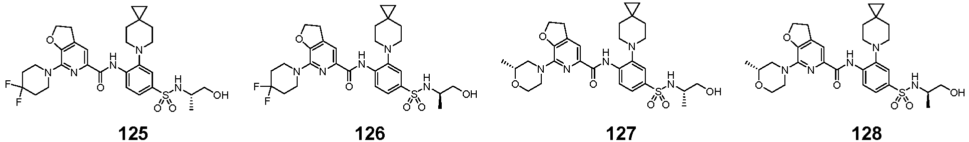

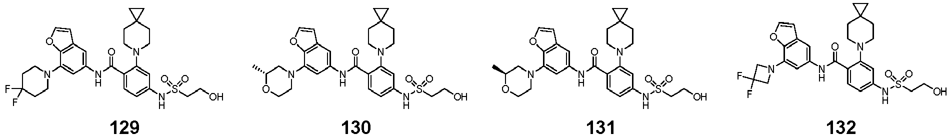

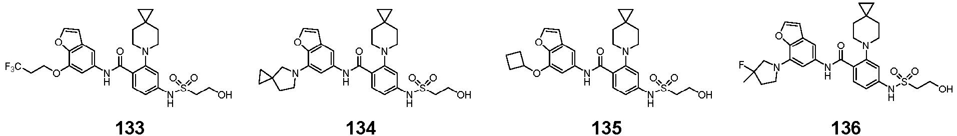

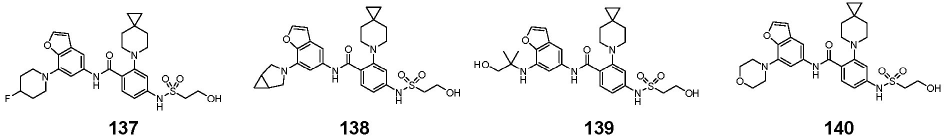

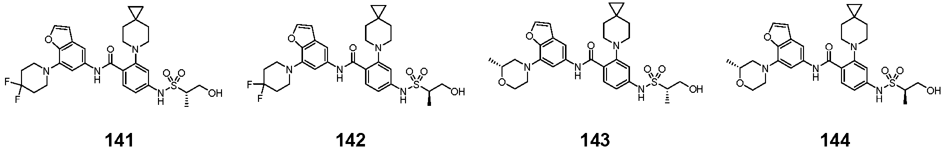

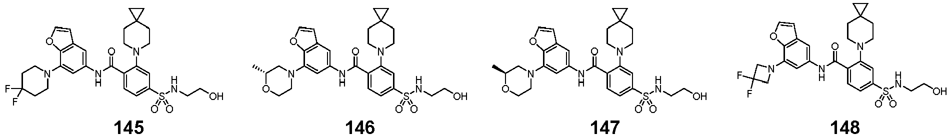

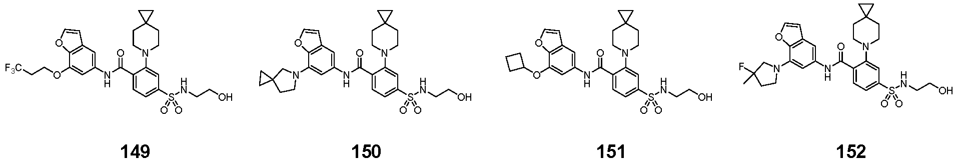

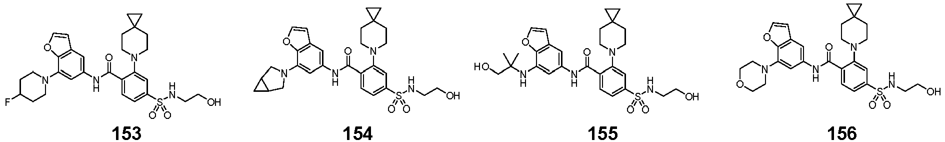

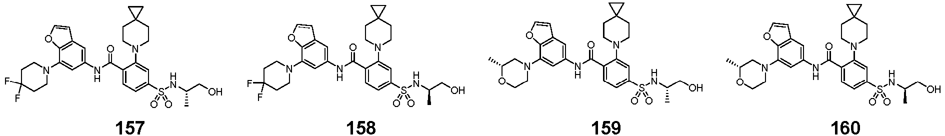

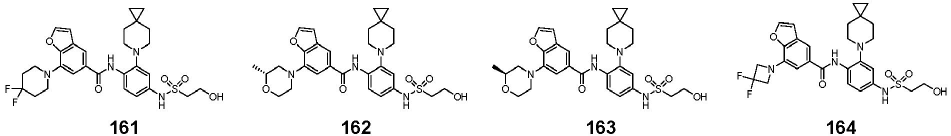

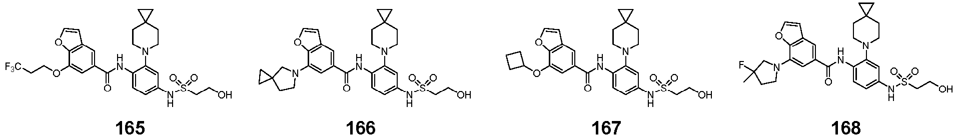

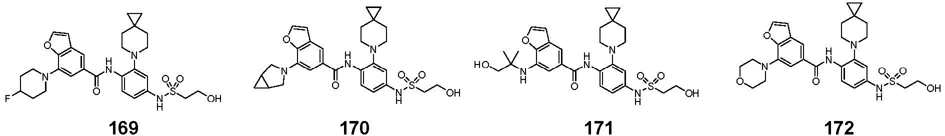

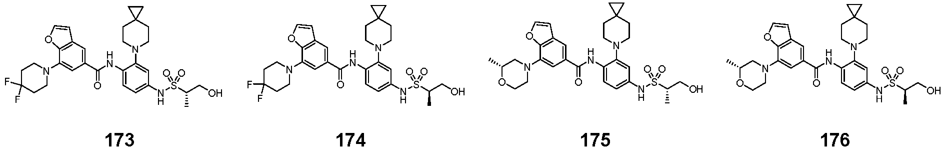

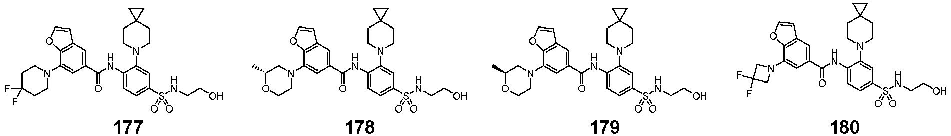

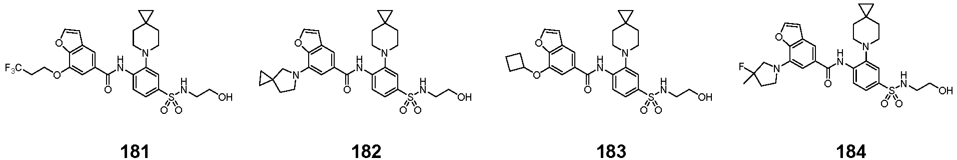

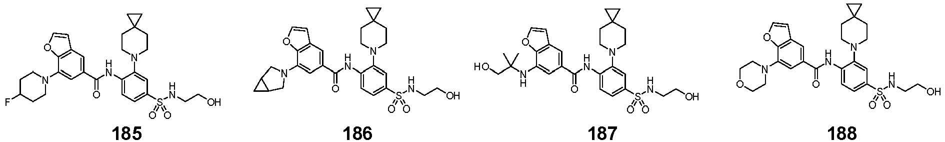

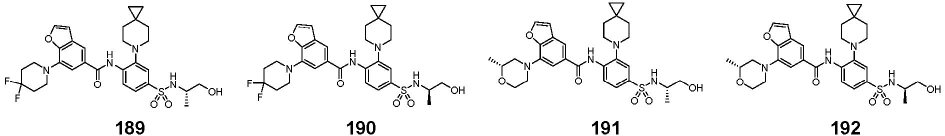

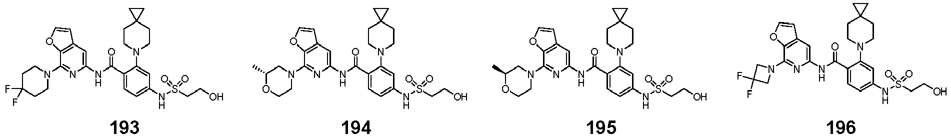

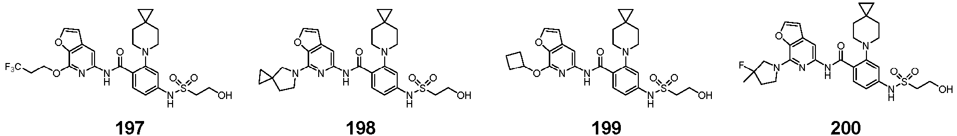

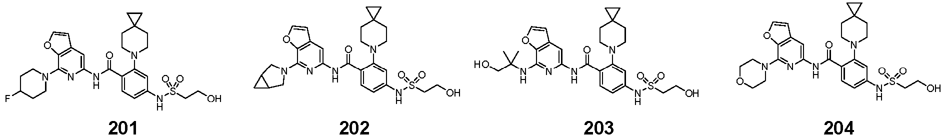

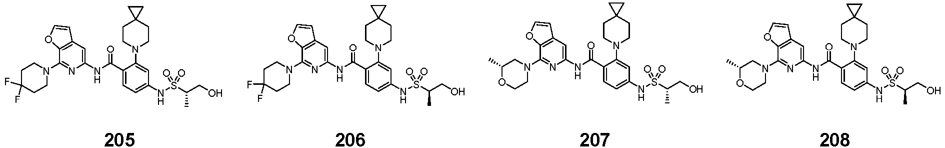

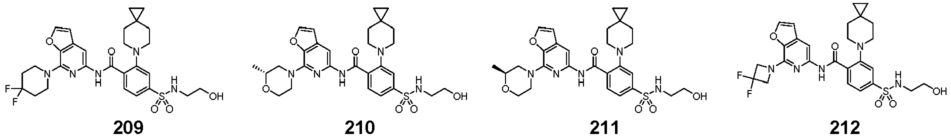

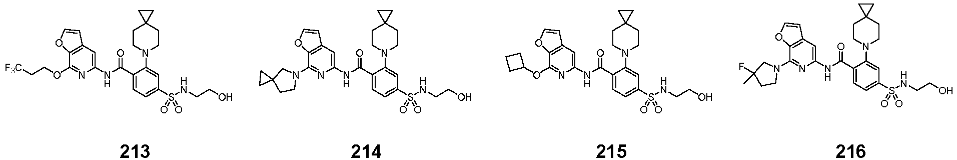

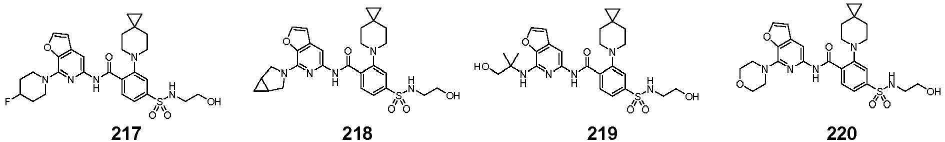

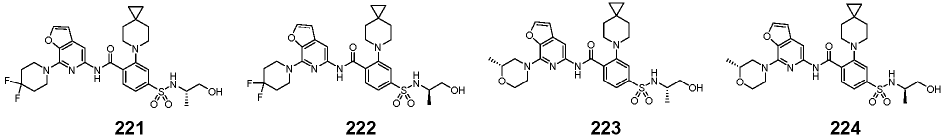

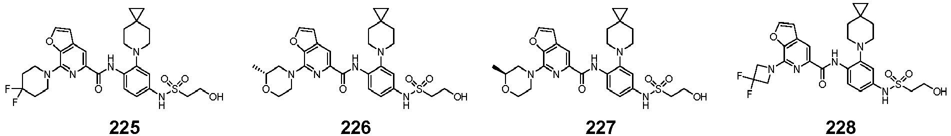

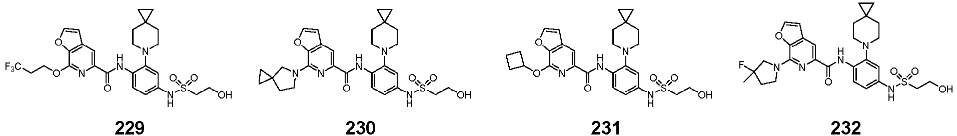

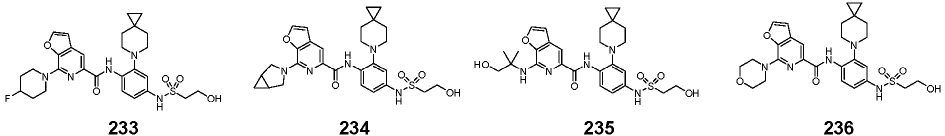

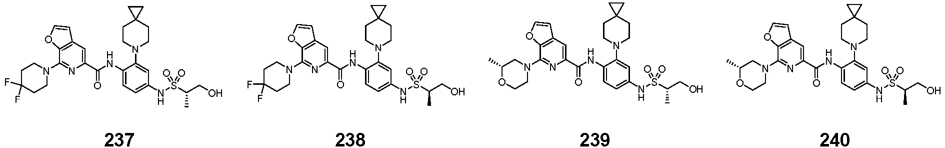

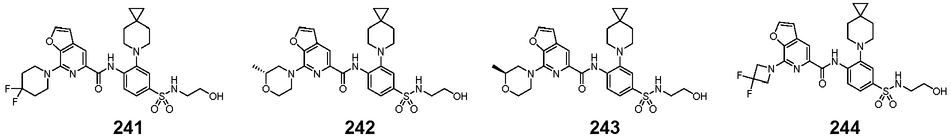

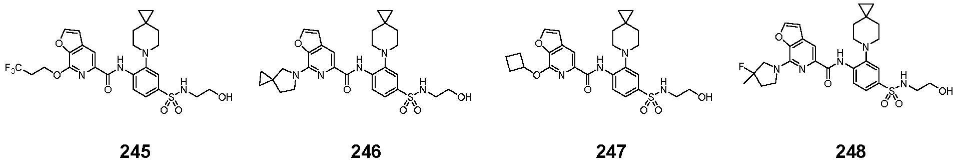

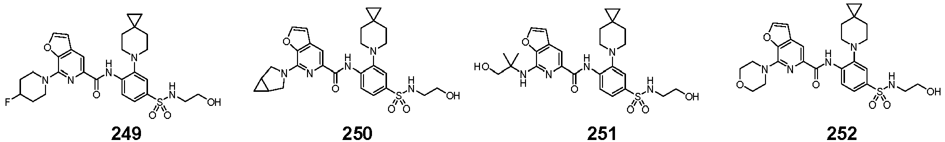

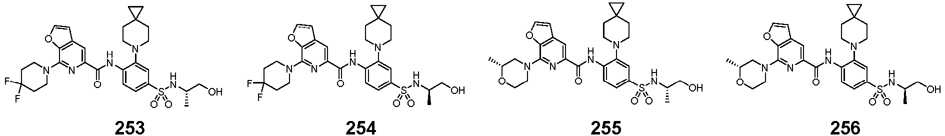

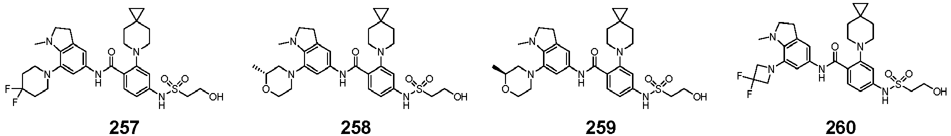

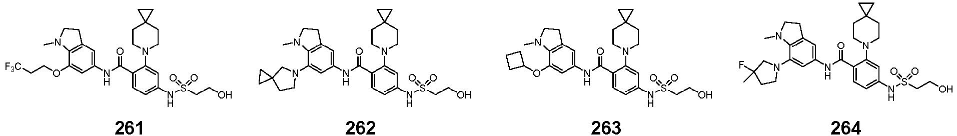

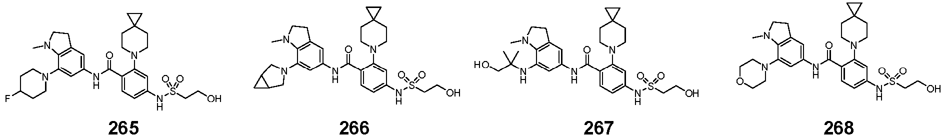

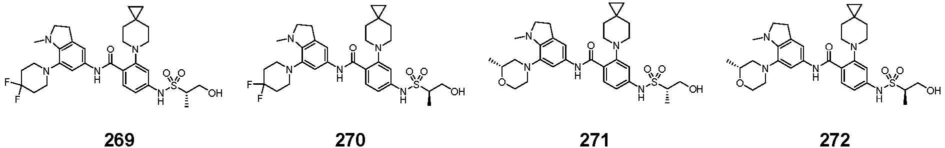

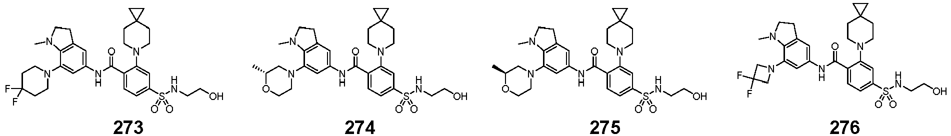

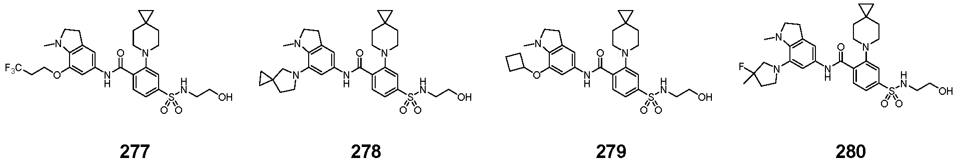

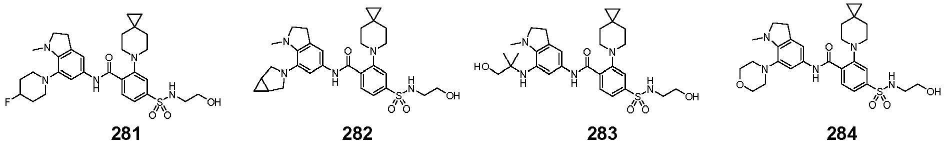

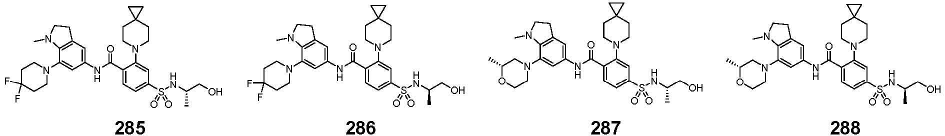

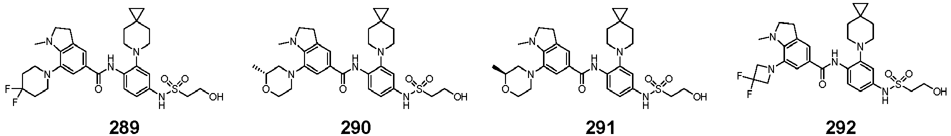

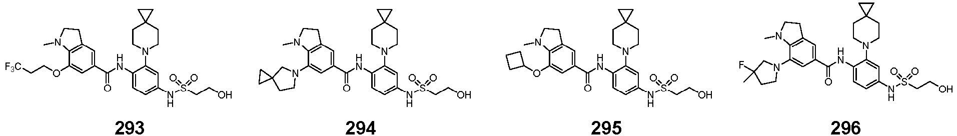

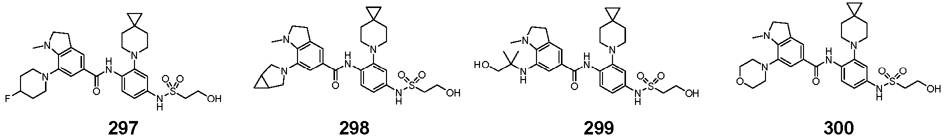

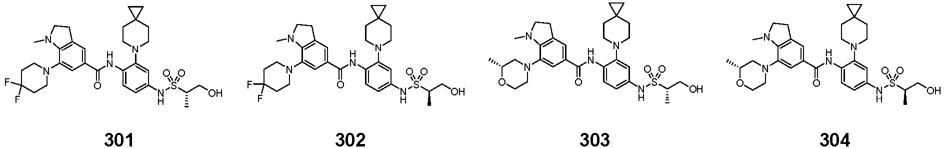

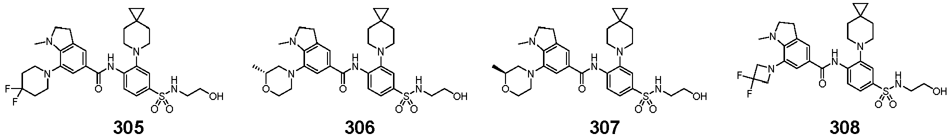

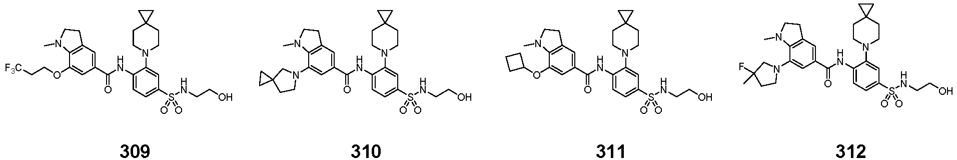

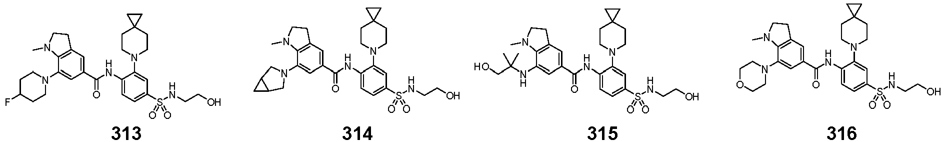

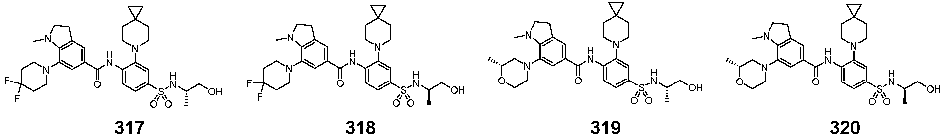

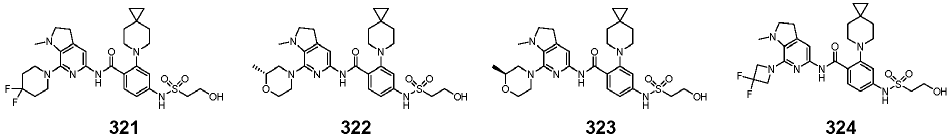

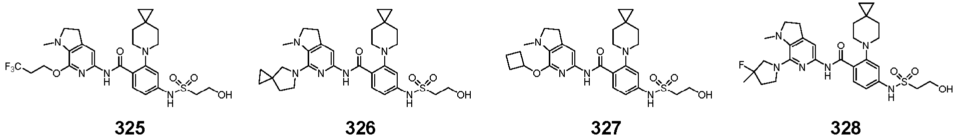

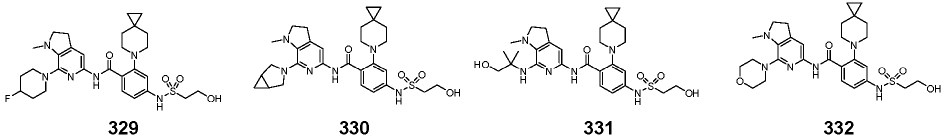

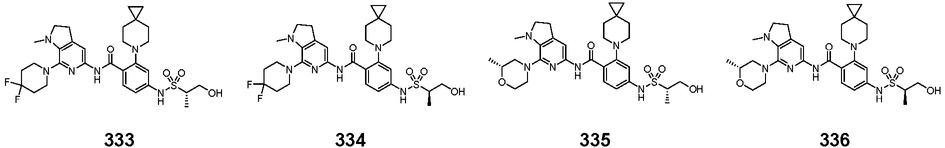

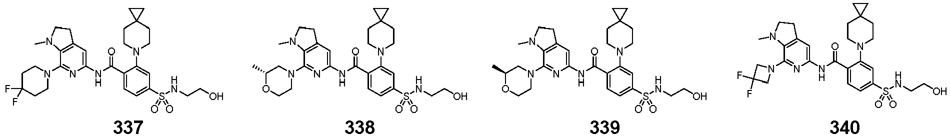

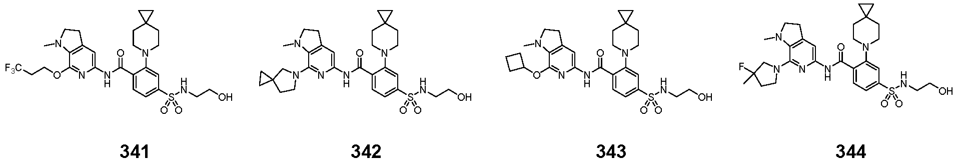

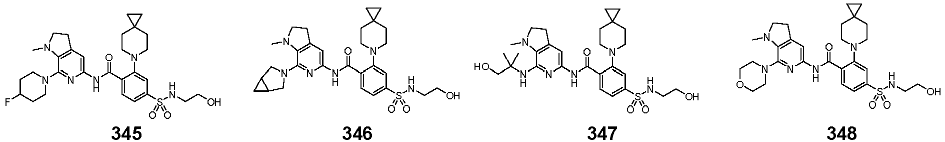

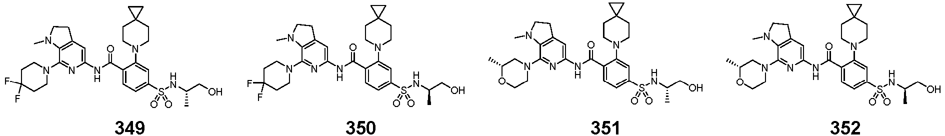

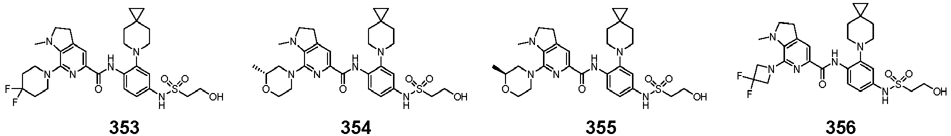

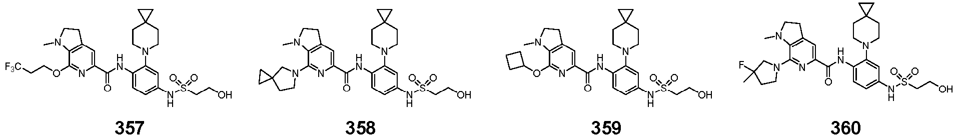

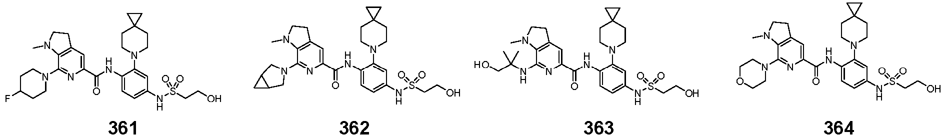

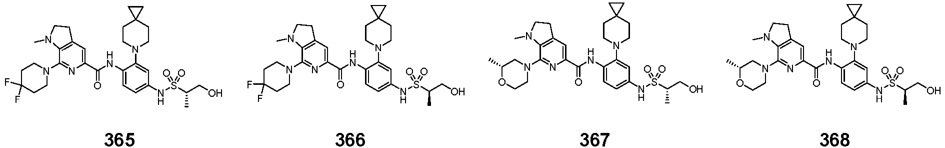

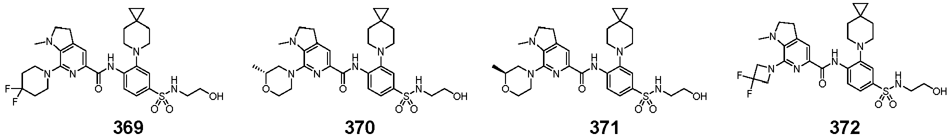

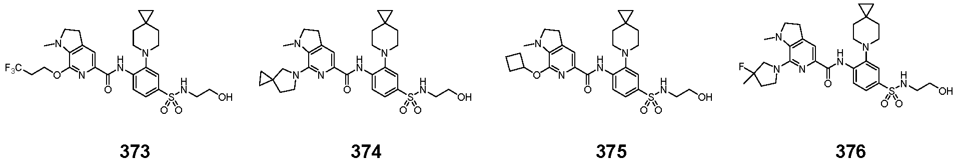

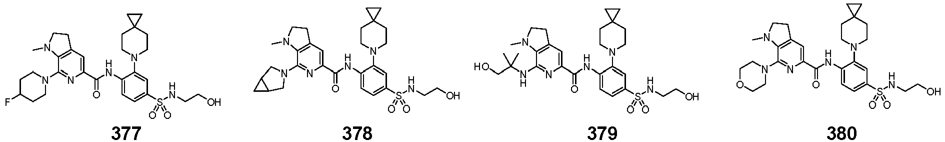

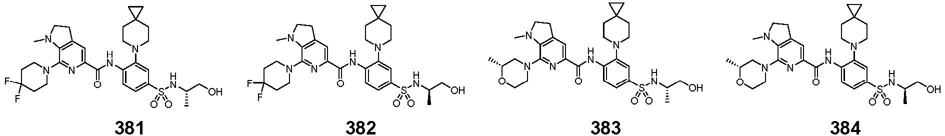

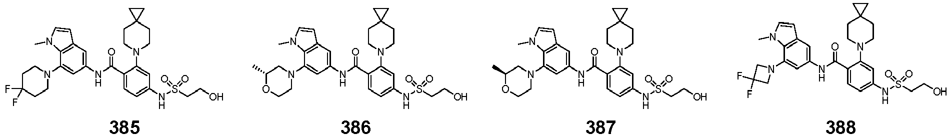

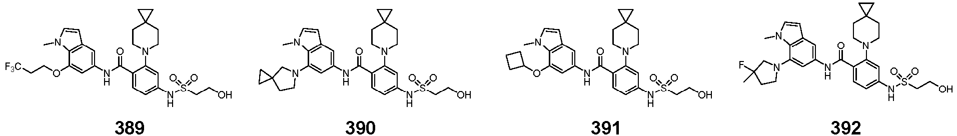

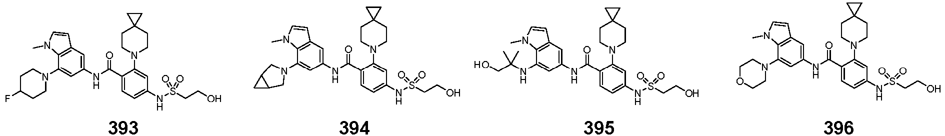

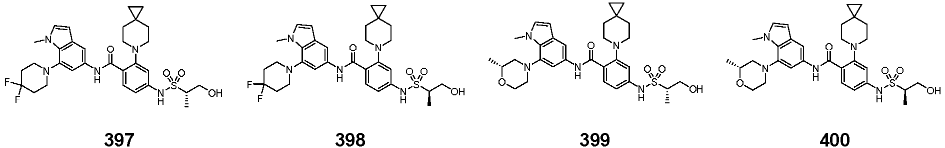

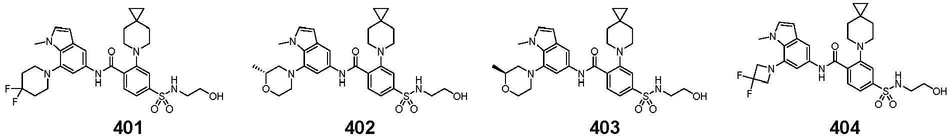

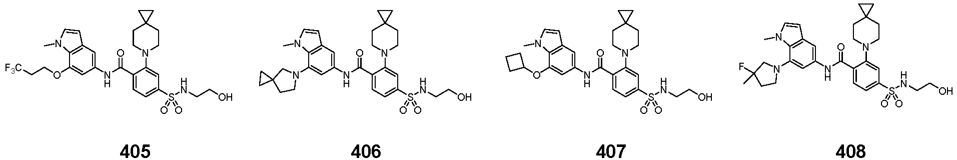

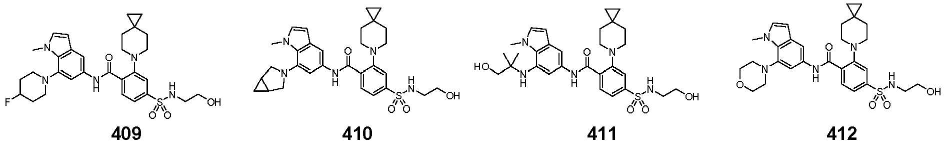

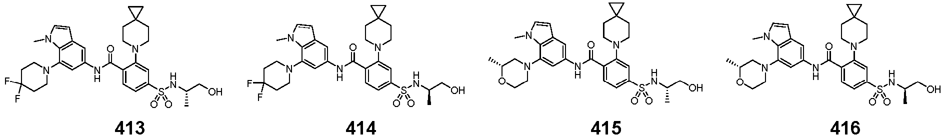

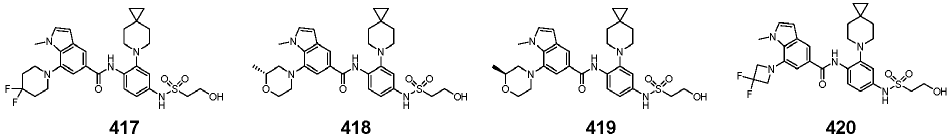

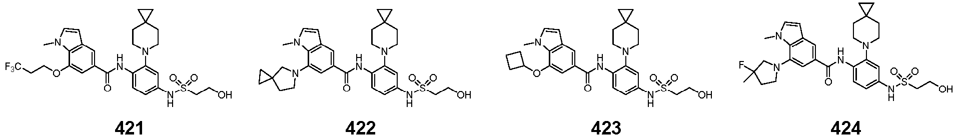

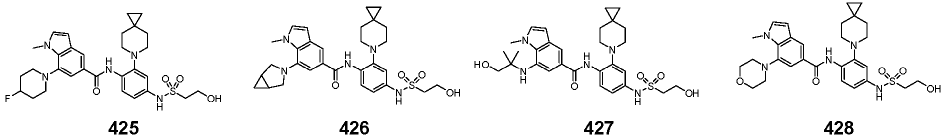

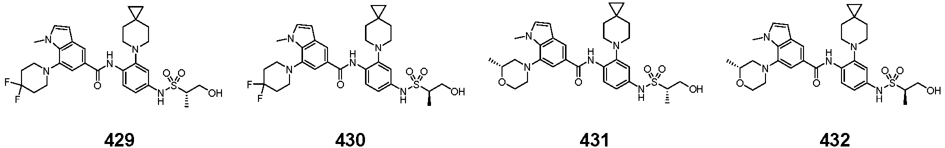

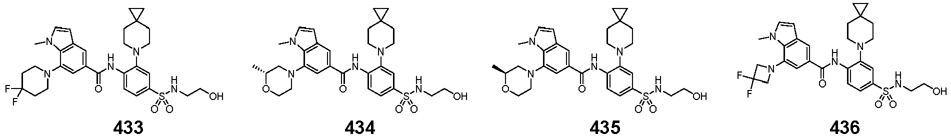

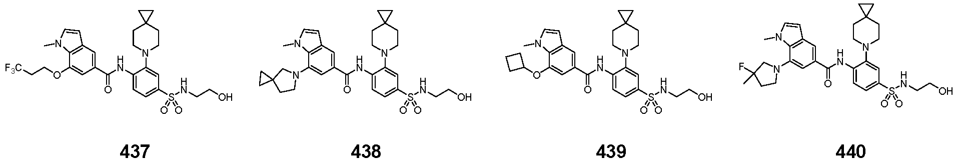

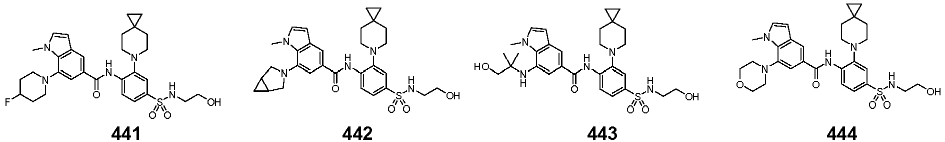

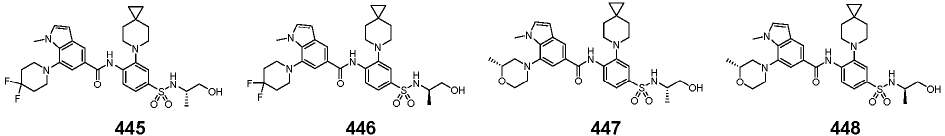

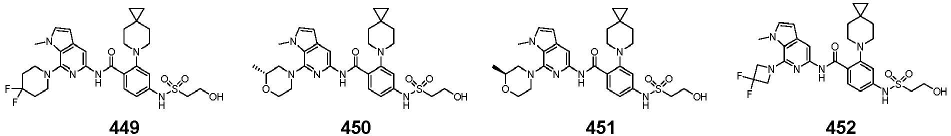

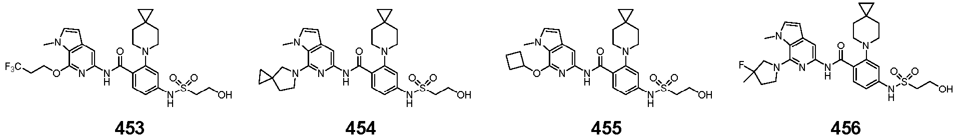

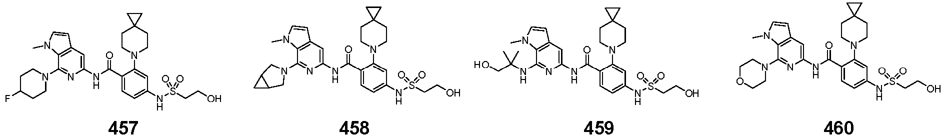

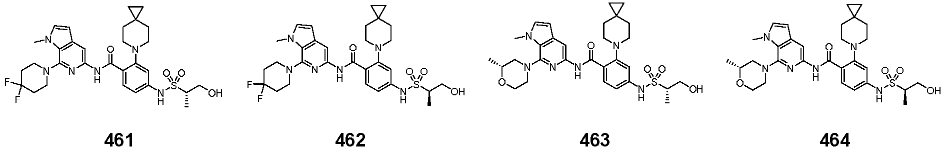

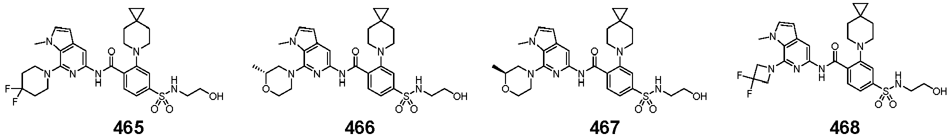

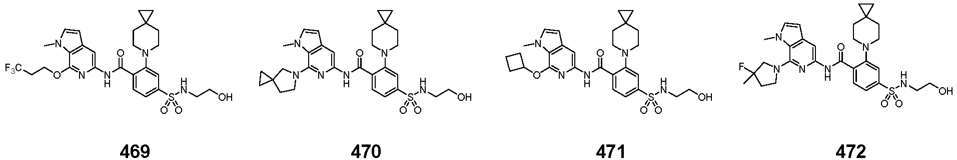

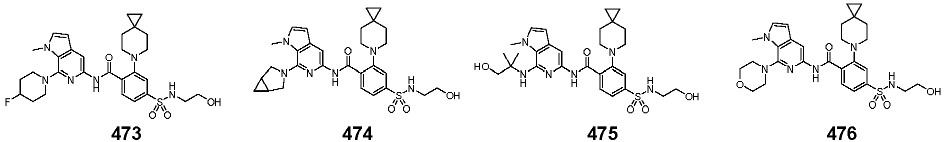

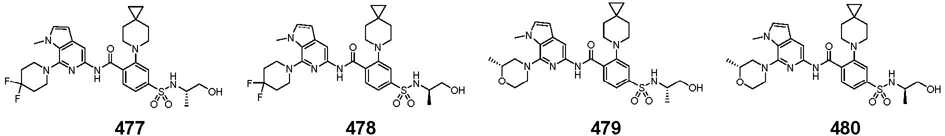

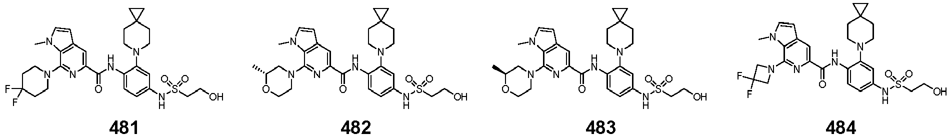

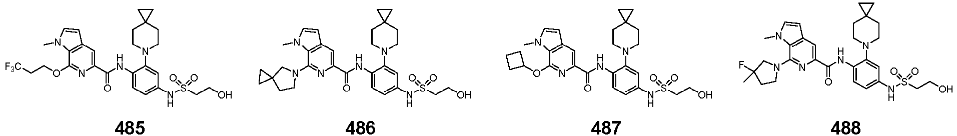

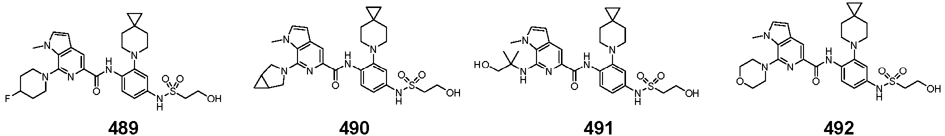

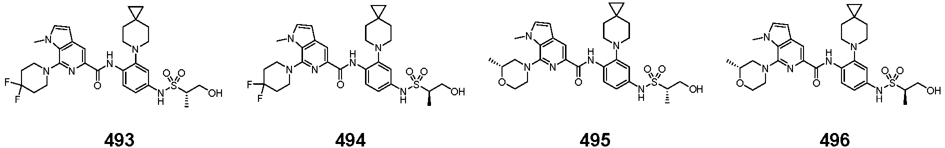

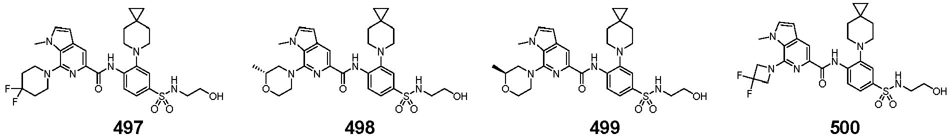

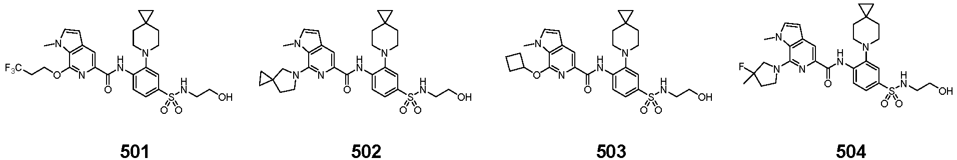

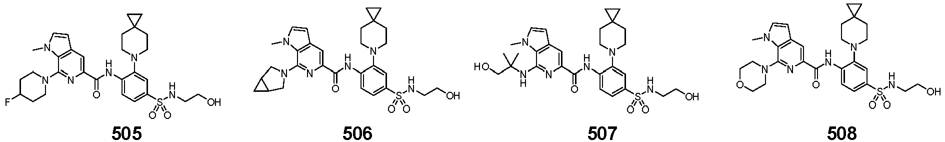

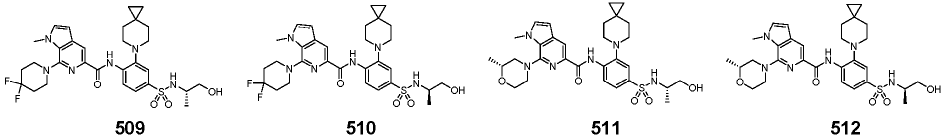

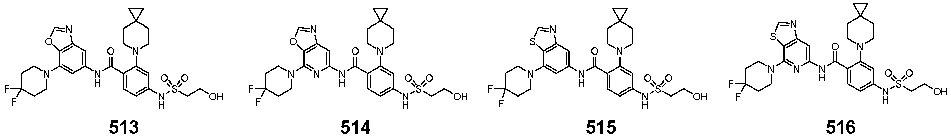

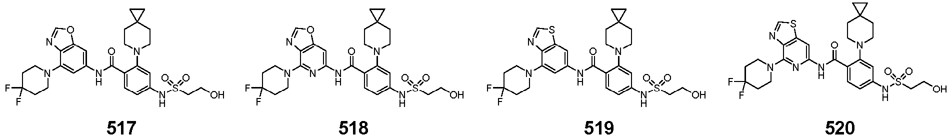

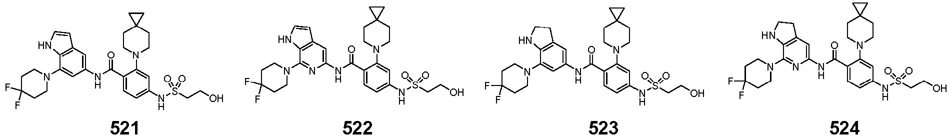

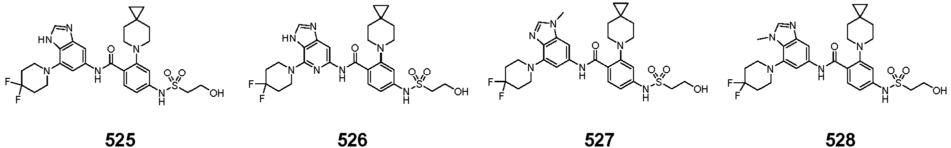

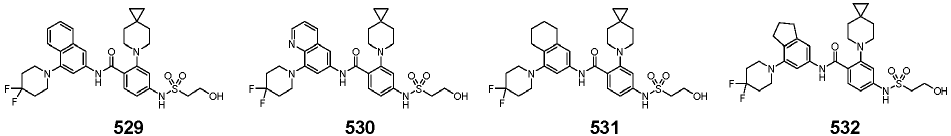

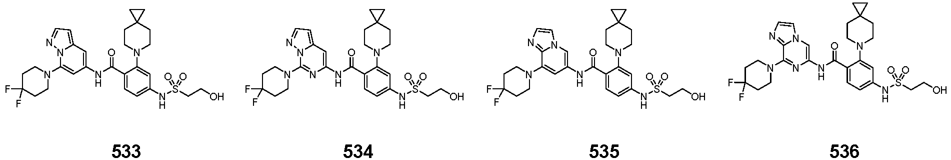

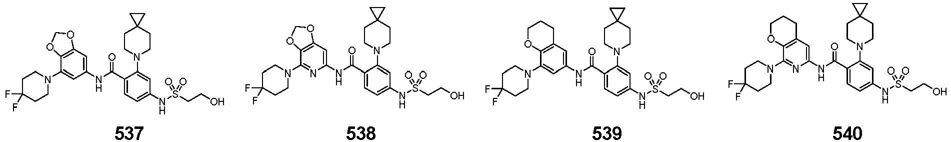

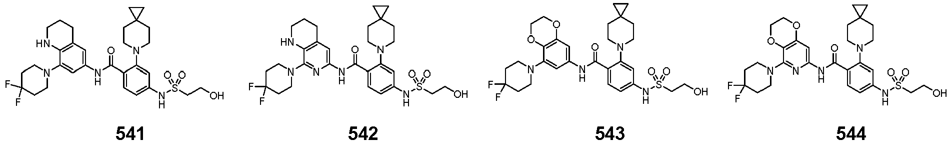

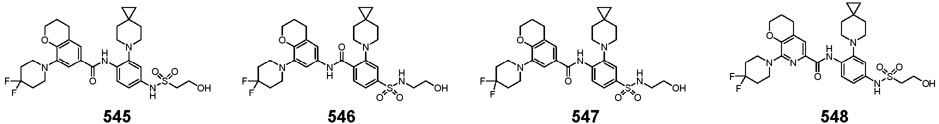

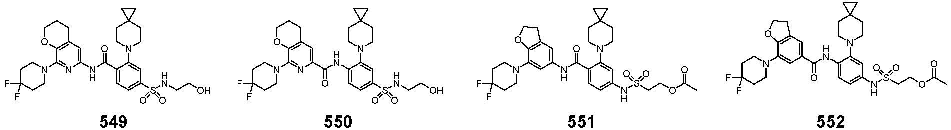

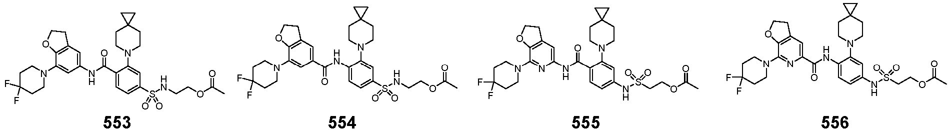

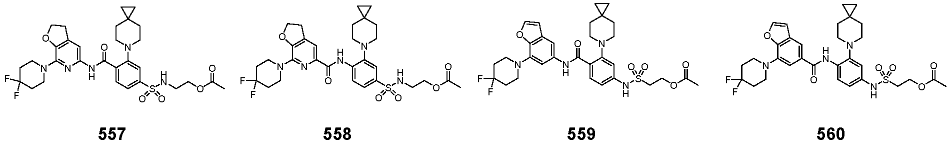

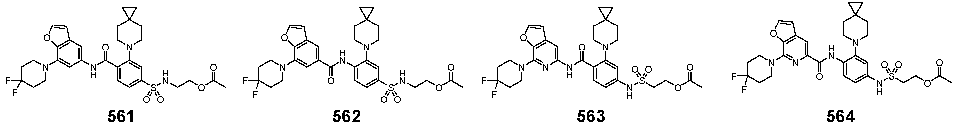

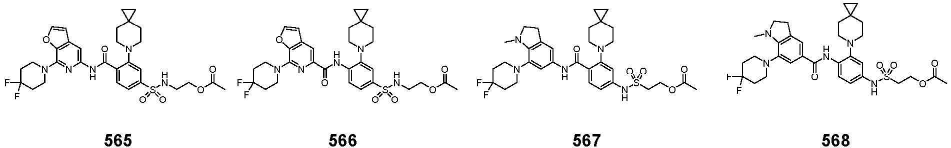

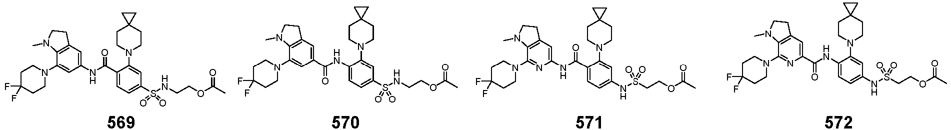

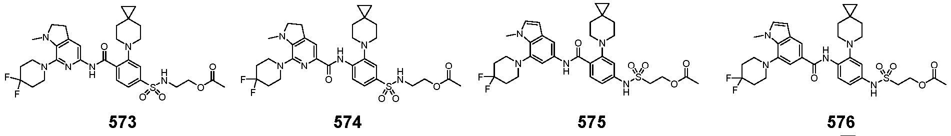

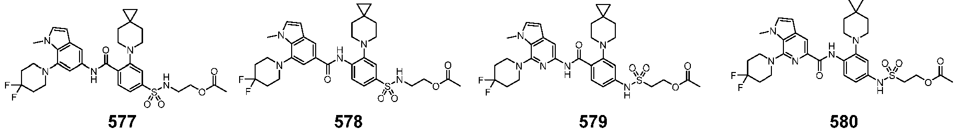

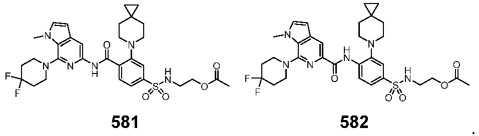

In various different embodiments of the present invention, the compound of general formula (1) also has one of the following structures:

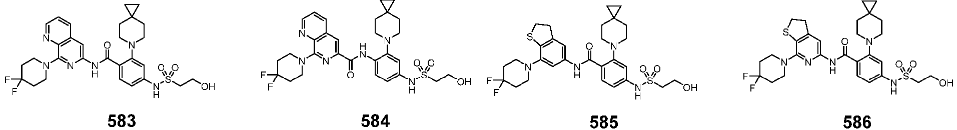

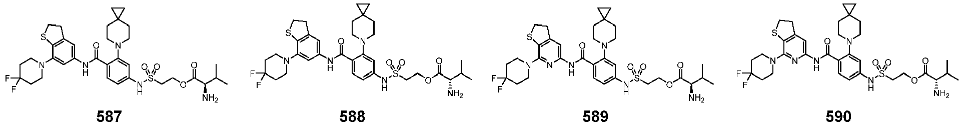

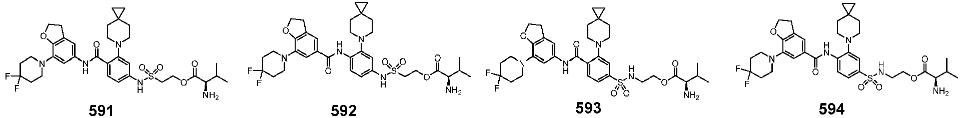

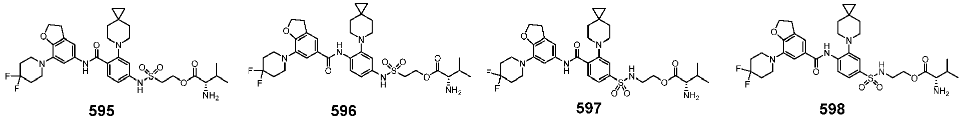

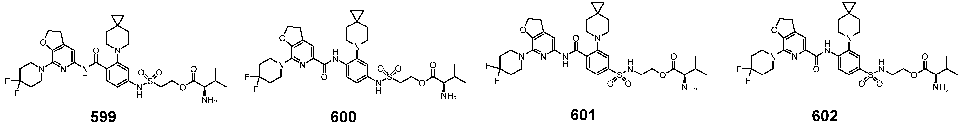

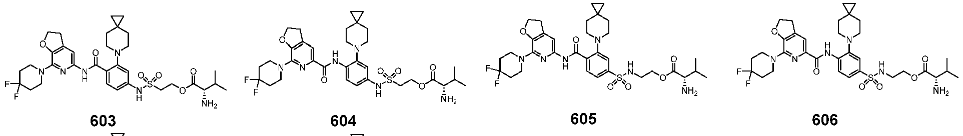

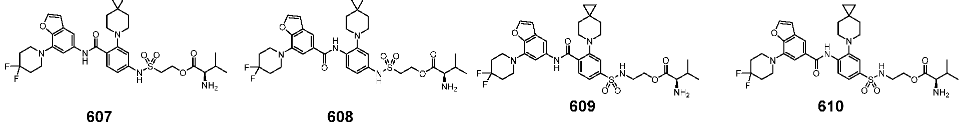

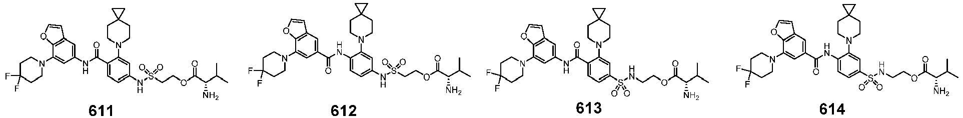

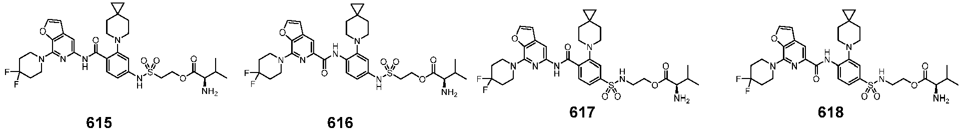

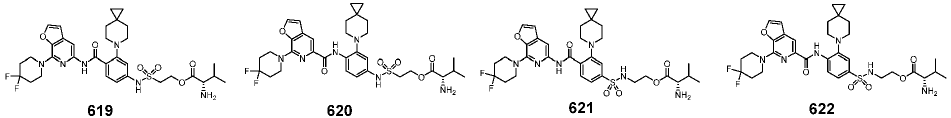

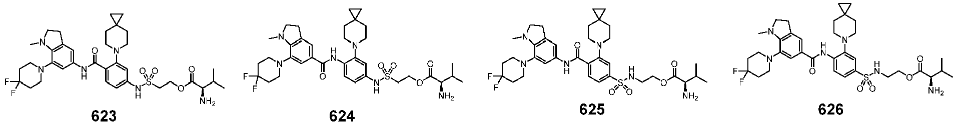

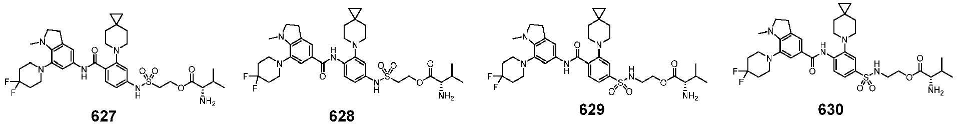

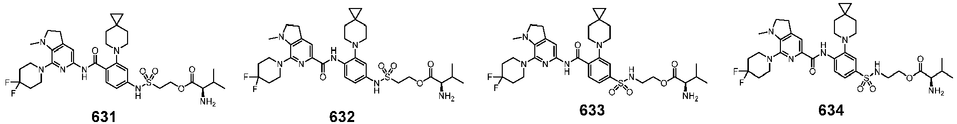

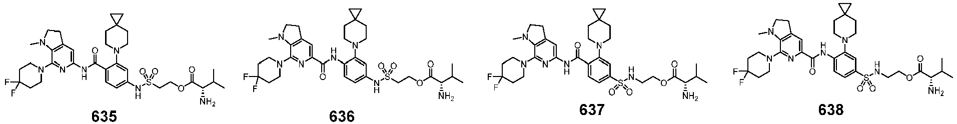

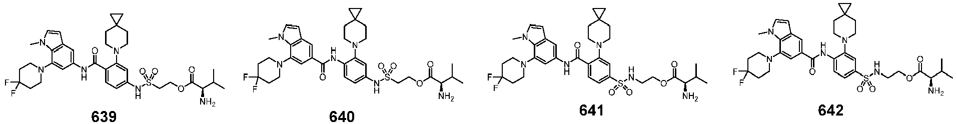

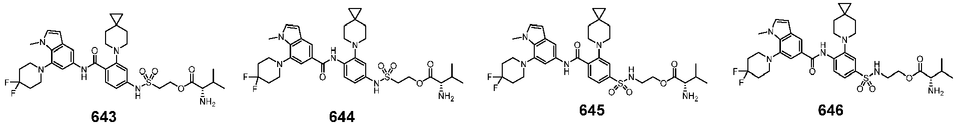

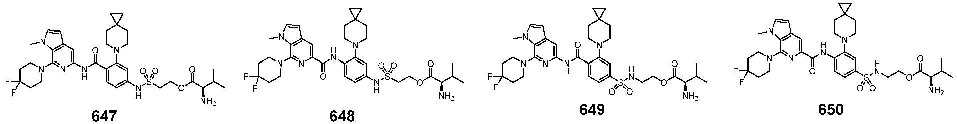

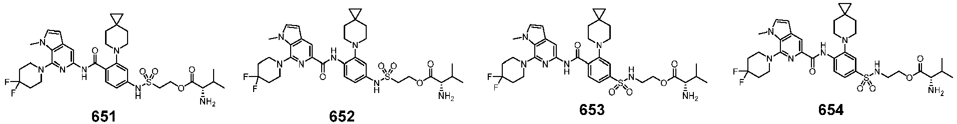

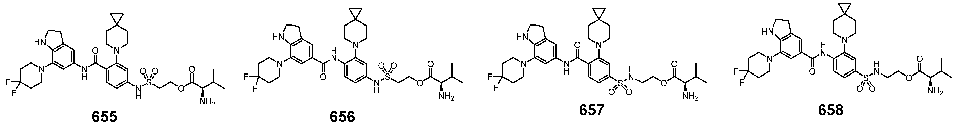

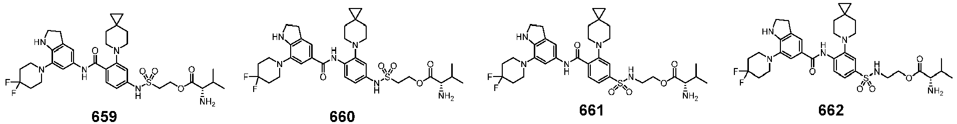

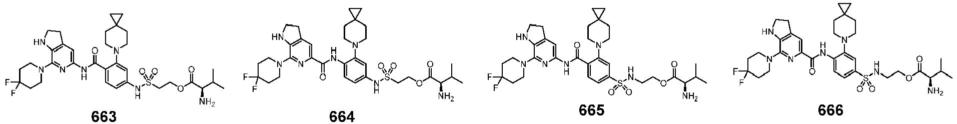

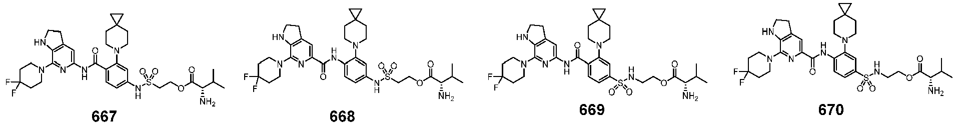

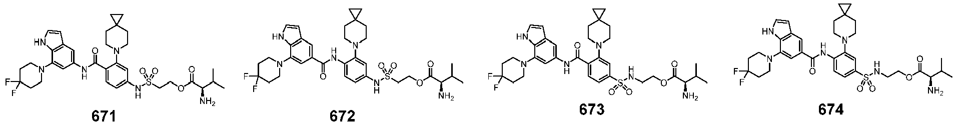

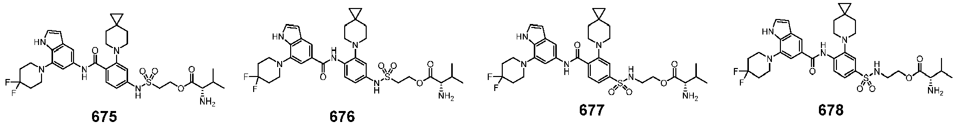

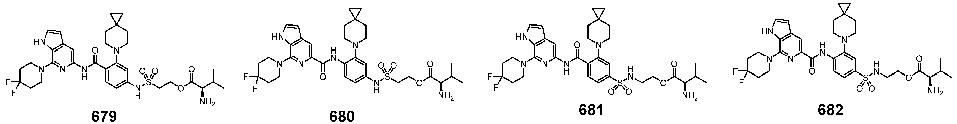

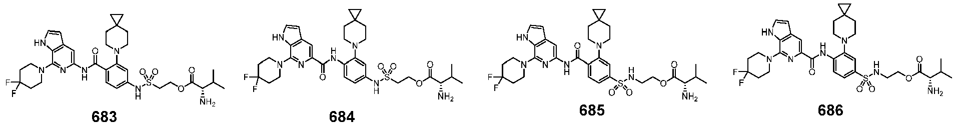

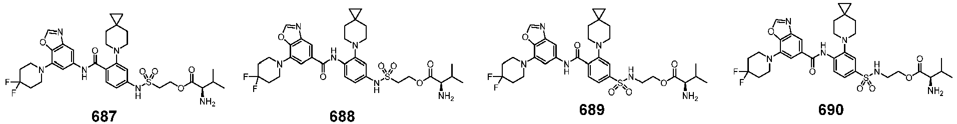

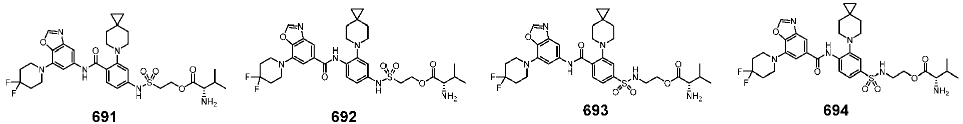

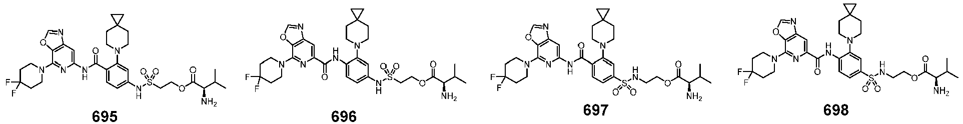

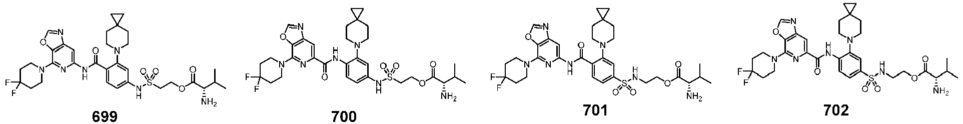

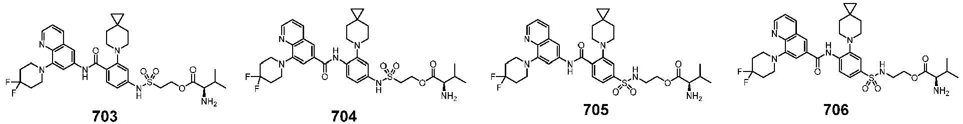

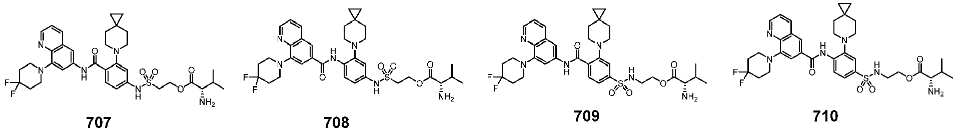

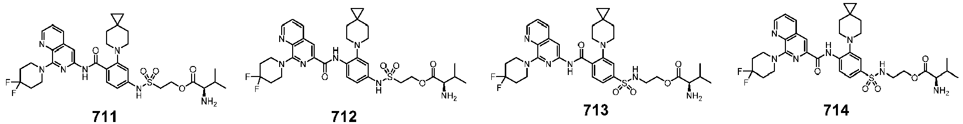

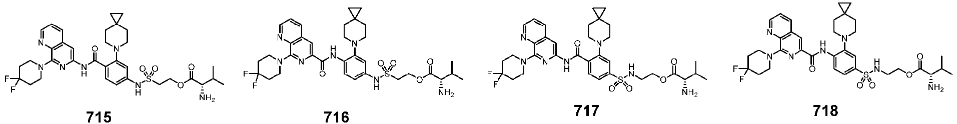

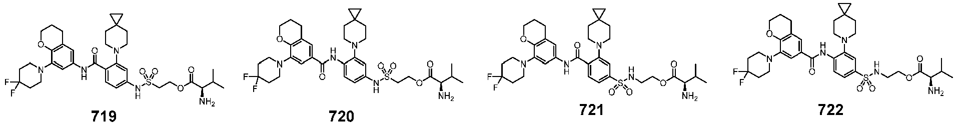

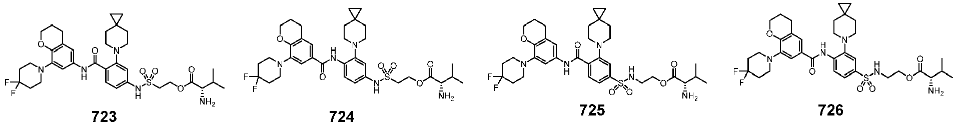

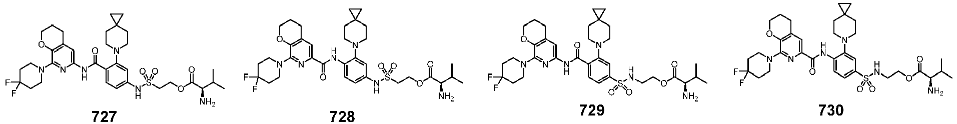

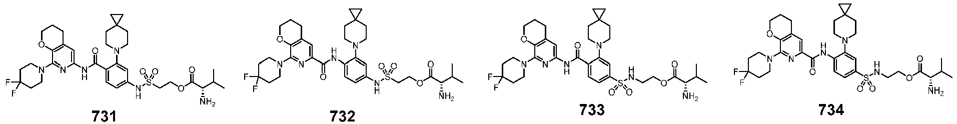

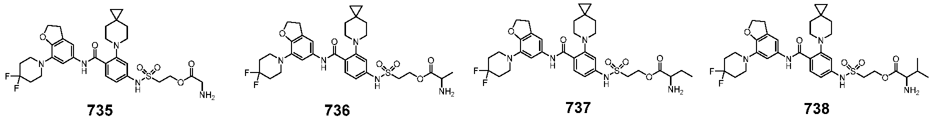

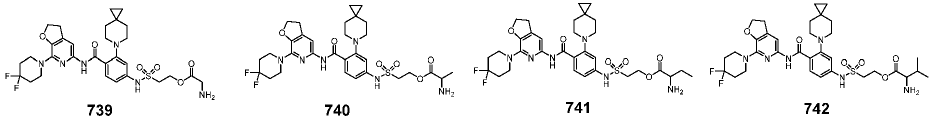

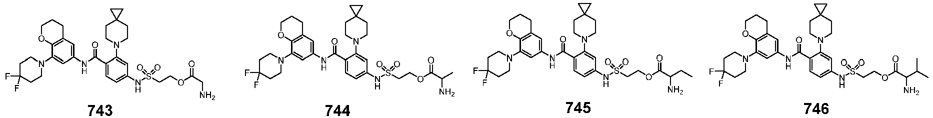

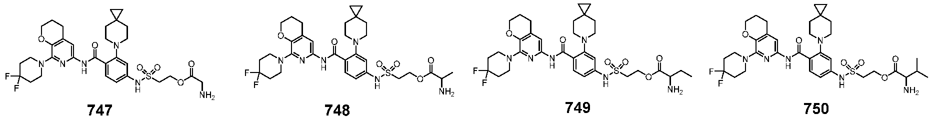

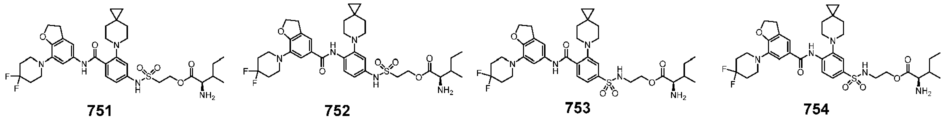

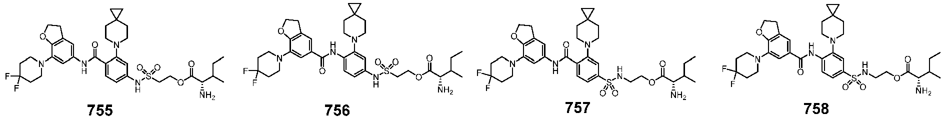

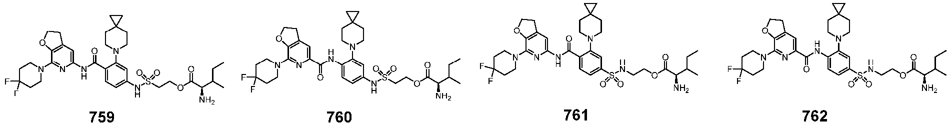

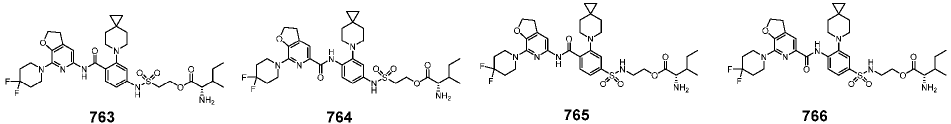

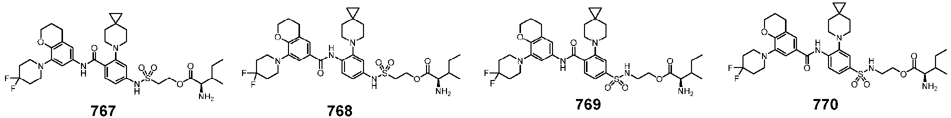

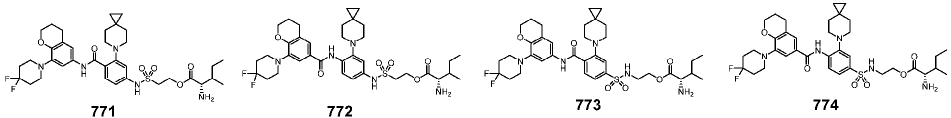

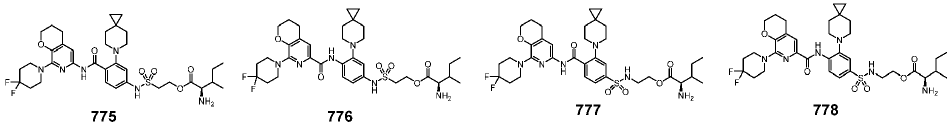

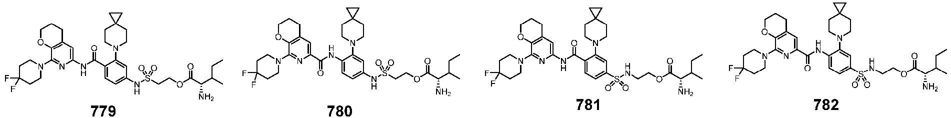

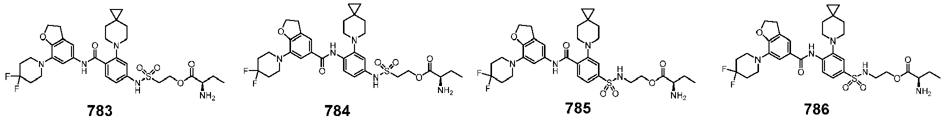

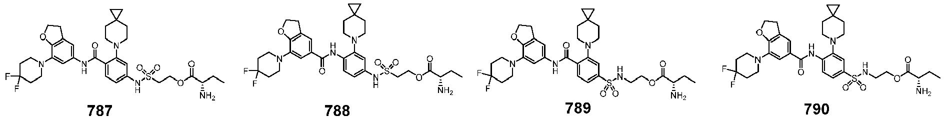

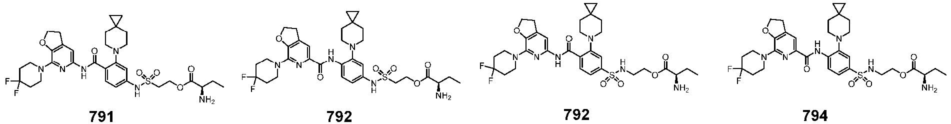

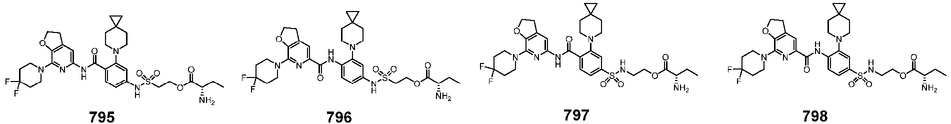

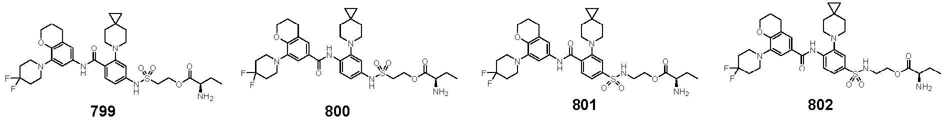

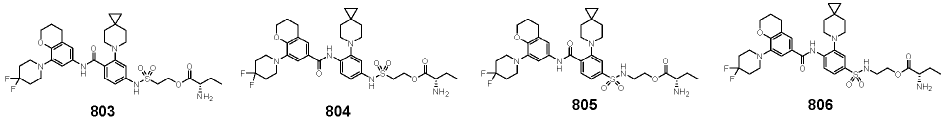

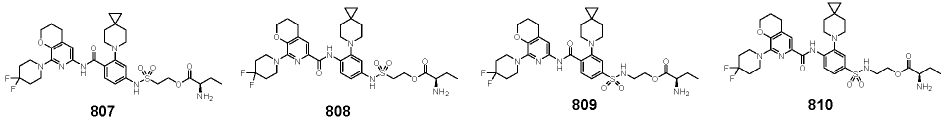

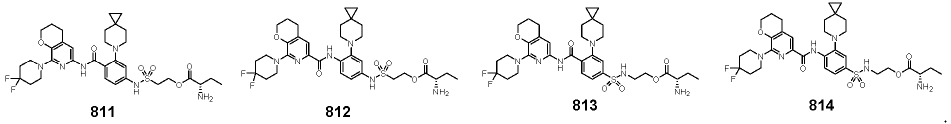

The present invention is further intended to provide a pharmaceutical composition comprising a pharmaceutically acceptable carrier, diluent, and/or excipient, as well as the compound of general formula (1) or the isomer thereof, the crystalline form thereof, the pharmaceutically acceptable salt thereof, the hydrate thereof, or the solvate thereof of the present invention as an active ingredient.

The present invention is still further intended to provide use of the compound of general formula (1) or the isomer thereof, the crystalline form thereof, the pharmaceutically acceptable salt thereof, the hydrate thereof, or the solvate thereof of the present invention or the pharmaceutical composition described above in preparing a medicament for treating, regulating, or preventing a disease related to KIF18A protein.

The present invention is even further intended to provide a method for treating, regulating, or preventing a related disease mediated by KIF18A protein, comprising administering to a subject a therapeutically effective amount of the compound of general formula (1) or the isomer thereof, the crystalline form thereof, the pharmaceutically acceptable salt thereof, the hydrate thereof, or the solvate thereof of the present invention or the pharmaceutical composition described above.

Through synthesis and careful studies of various classes of new compounds with KIF18A protein inhibitory effects, the inventors have discovered that the compound of general formula (1) has surprisingly strong KIF18A protein inhibition activity.

It should be understood that both the aforementioned general description and the following detailed description of the present invention are exemplary and explanatory, and are intended to provide further explanation of the present invention claimed.

### Synthesis of Compounds

Methods for preparing the compound of general formula (1) of the present invention are specifically described below, but these specific methods do not limit the present invention in any way.

The compound of general formula (1) described above may be synthesized using standard synthetic techniques or well-known techniques in combination with the methods described herein. In addition, the solvents, temperatures, and other reaction conditions mentioned herein may vary. Starting materials for the synthesis of the compounds may be obtained synthetically or commercially. The compounds described herein and other related compounds with different substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds may be altered by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compound used, reaction temperature, and time required for the reaction are not limited to the following explanation. The compounds of the present invention may also be conveniently prepared by optionally combining various synthetic methods described herein or known in the art, and such combinations may be easily determined by those skilled in the art to which the present invention pertains. In one aspect, the present invention further provides a method for preparing the compound of general formula (1), wherein the compound of general formula (1) may be prepared by the following general reaction scheme 1, 2, 3, or 4:

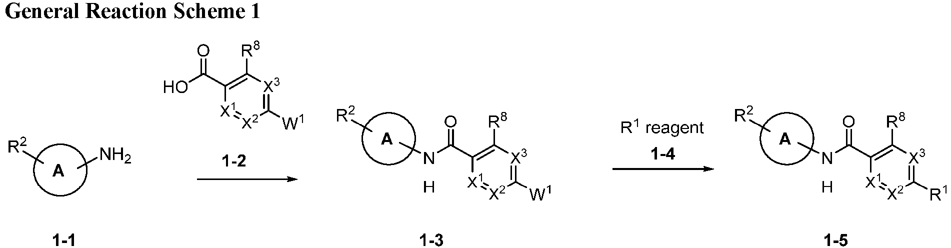

Embodiments of the compound of general formula (1) may be prepared according to general reaction scheme 1, wherein R¹, R², ring A, R⁸, X¹, X², and X³ are as defined above; W¹ represents fluorine, chlorine, bromine, or iodine; H represents hydrogen; N represents nitrogen; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol. As shown in general reaction scheme 1, compound 1-1 and compound 1-2 undergo an amidation reaction to generate compound 1-3, and compound 1-3 reacts with R¹ reagent 1-4 to generate compound 1-5.

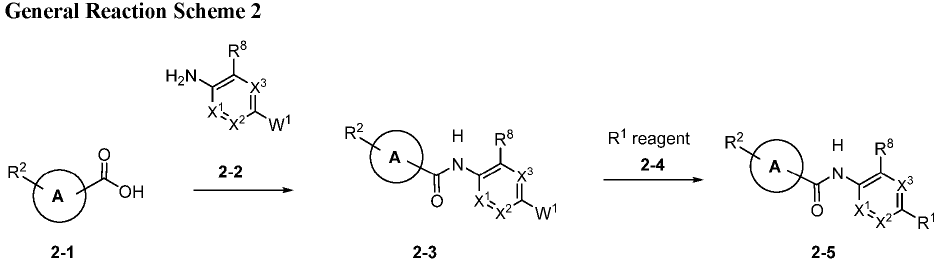

Embodiments of the compound of general formula (1) may be prepared according to general reaction scheme 2, wherein R¹, R², ring A, R⁸, X¹, X², and X³ are as defined above; W¹ represents fluorine, chlorine, bromine, or iodine; H represents hydrogen; N represents nitrogen; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol. As shown in general reaction scheme 2, compound 2-1 and compound 2-2 undergo an amidation reaction to generate compound 2-3, and compound 2-3 reacts with R¹ reagent 2-4 to generate compound 2-5.

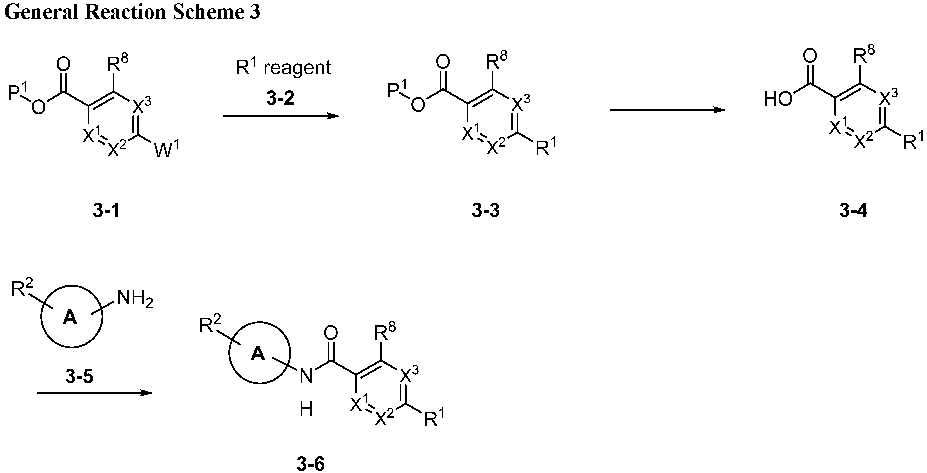

Embodiments of the compound of general formula (1) may be prepared according to general reaction scheme 3, wherein R¹, R², ring A, R⁸, X¹, X², and X³ are as defined above; W¹ represents fluorine, chlorine, bromine, or iodine; H represents hydrogen; N represents nitrogen; P¹ is an ester group-protecting group; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonamide, (7) ethyl iodoacetate, (8) 2-mercaptopropan-1-ol, (9) 2-mercapto-2-methylpropan-1-ol, (10) 2-aminoethan-1-ol, or (11) cyclopropanethiol. As shown in general reaction scheme 3, compound 3-1 reacts with R¹ reagent 3-2 to generate compound 3-3, compound 3-3 loses the ester group-protecting group P¹ to give compound 3-4, and compound 3-4 and compound 3-5 undergo an amidation reaction to generate compound 3-6.

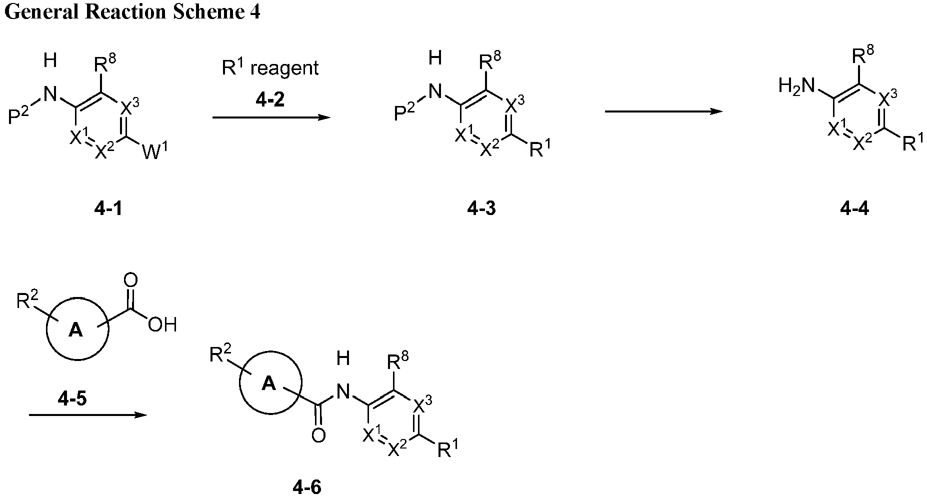

Embodiments of the compound of general formula (1) may be prepared according to general reaction scheme 4, wherein R¹, R², ring A, R⁸, X¹, X², and X³ are as defined above; W¹ represents fluorine, chlorine, bromine, or iodine; H represents hydrogen; N represents nitrogen; P² is an amino-protecting group; R¹ reagent is, for example, (1) 1-methylcyclopropane-1-sulfonamide, (2) 3-methyloxetan-3-amine, (3) tert-butyl 3-mercaptoazetidine-1-carboxylate, (4) ethyl 2-sulfamoylpropionate, (5) 2-hydroxypropane-1-sulfonamide, (6) 2-hydroxyethane-1-sulfonatnide, (7) ethyl iodoacetate, (8) 2-mercaptopropan-1-ol, (9) 2-mercapto-2-methylpropan-1-ol, (10) 2-aminoethan-1-ol, or (11) cyclopropanethiol. As shown in general reaction scheme 4, compound 4-1 reacts with R¹ reagent 4-2 to generate compound 4-3, compound 4-3 loses the amino-protecting group P² to give compound 4-4, and compound 4-4 and compound 4-5 undergo an amidation reaction to generate compound 4-6.

### Further Forms of Compounds

"Pharmaceutically acceptable" herein refers to a substance, such as a carrier or diluent, which will not lead to loss of biological activity or properties in a compound and is relatively non-toxic. For example, when an individual is given a substance, such substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed. (Wiley, 2002).

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol, or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized, and nanoparticle forms. In addition, the compound of general formula (1) includes crystalline forms, but may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability, and solubility. Different factors such as recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer, and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I), and C-14 (¹⁴C). For another example, deuterium can be used to substitute a hydrogen atom to form a deuterated compound. The bond formed by deuterium and carbon is stronger than that formed by common hydrogen and carbon, and compared with an undeuterated medicament, the deuterated medicament generally has the advantages of reduced adverse effects, increased medicament stability, enhanced efficacy, prolonged *in vivo* half-life and the like. All isotopic variations of the compound of the present invention, whether radioactive or not, are intended to be encompassed within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques, and pharmacology are used. As used herein, "or" or "and" refers to "and/or" unless otherwise stated.

Unless otherwise specified, "C_{α-β} hydrocarbyl" means a hydrocarbyl group containing a minimum of α and a maximum of β carbon atoms in a branched or linear relationship, wherein α and β represent integers. The hydrocarbyl described in this section may also contain one or two double or triple bonds. A designation of Co hydrocarbyl represents a direct bond. Examples of the C₁₋₆ hydrocarbyl include, but are not limited to, the following: 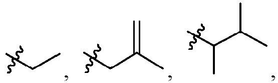 and

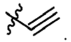

Unless otherwise specified, "C_{α-β} halohydrocarbyl" means a hydrocarbyl group, as described above, in which any number (at least one) of the hydrogen atoms attached to the hydrocarbyl chain are replaced by F, Cl, Br, or I.

Unless otherwise specified, "oxo" and "thio" represent =O (e.g., carbonyl) and =S (e.g., thiocarbonyl), respectively.

Unless otherwise specified, "halo" or "halogen" means a halogen atom selected from F, Cl, Br, and I.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, sec-butoxy, and *tert*-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO, and ^{t-}BuO.

Unless otherwise specified, "cycloalkyl" refers to a monocyclic non-aromatic hydrocarbon ring system. The ring carbon atoms of the cycloalkyl may optionally be oxidized to form an oxo or sulfido group. The cycloalkyl further includes cycloalkylene. In some embodiments, the cycloalkyl contains 0, 1, or 2 double bonds. In some embodiments, the cycloalkyl contains 1 or 2 double bonds (partially unsaturated cycloalkyl). Examples of the cycloalkyl include, but are not limited to, cyclopropyl, cyclobutyl, cyclopentyl, cyclohexyl, cycloheptyl, cyclopentenyl, cyclohexenyl, cyclohexadienyl, cycloheptatrienyl, 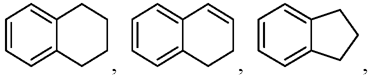 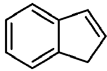 and the like.

Unless otherwise specified, "aryl" refers to an aromatic hydrocarbon group, which is monocyclic or polycyclic; for example, a monocyclic aryl ring is fused to one or more carbocyclic aromatic groups. Examples of aryl include, but are not limited to, phenyl, naphthyl, and phenanthryl.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to an aromatic group containing one or more heteroatoms (O, S, or N), and the heteroaryl is monocyclic or polycyclic. For example, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1H-pyrrolo[3,2-b]pyridinyl, 1H-pyrrolo[2,3-c]pyridinyl, 1H-pyrrolo[3,2-c]pyridinyl, 1H-pyrrolo [2,3 -b]pyridinyl,

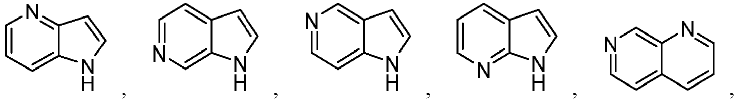

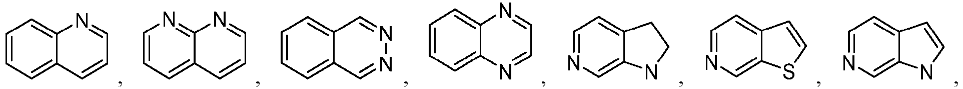

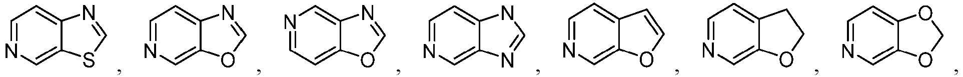

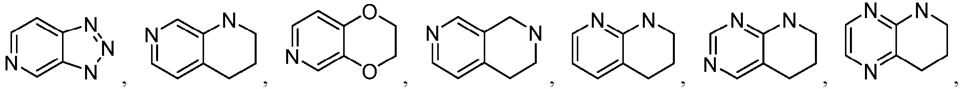

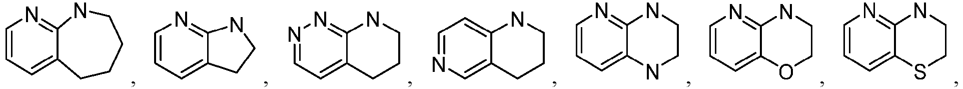

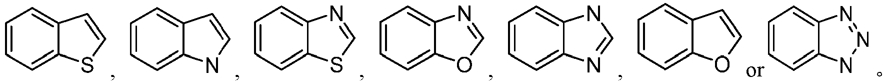

Unless otherwise specified, "heteroarylene" refers to a divalent heteroaryl as defined above.

Unless otherwise specified, "heterocycloalkyl" refers to a non-aromatic ring or ring system, which may optionally contain one or more alkenylene as part of the ring structure, having at least one heteroatom ring member independently selected from boron, phosphorus, nitrogen, sulfur, oxygen, and phosphorus. Partially unsaturated heterocycloalkyl may be referred to as "heterocycloalkenyl" if heterocycloalkyl contains at least one double bond, or "heterocycloalkynyl" if the heterocycloalkyl contains at least one triple bond.

Heterocycloalkyl may include monocyclic, bicyclic, spiro ring, or polycyclic systems (e.g., having two fused or bridged rings). In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or thio groups or other oxidized bonds (e.g., C(O), S(O), C(S) or S(O)2, N-oxides, etc.), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties (also referred to as partially unsaturated heterocyclic rings) having one or more aromatic rings fused to (i.e., sharing a bond with) the heterocycloalkyl ring, for example, benzo-derivatives of piperidine, morpholine, azepin, thienyl, or the like. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1H-imidazo[4,5-c]pyridine, N-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1H,3H)-dione, 1,4-dioxanyl, morpholinyl, thiomorpholinyl, thiomorpholinyl-S-oxide, thiomorpholinyl-S,S-oxide, piperazinyl, pyranyl, pyridonyl, 3-pyrrolinyl, thiopyranyl, pyronyl, tetrahydrothienyl, 2-azaspiro[3.3]heptanyl, indolinyl,

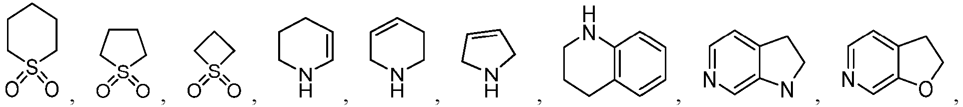

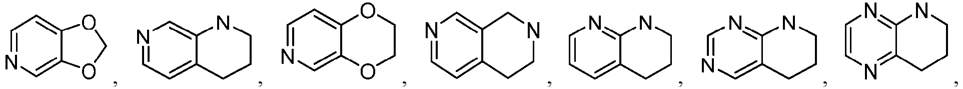

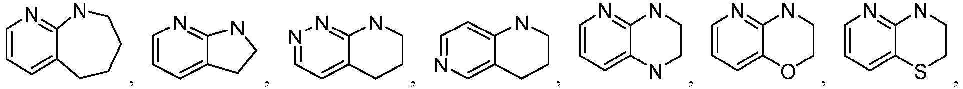

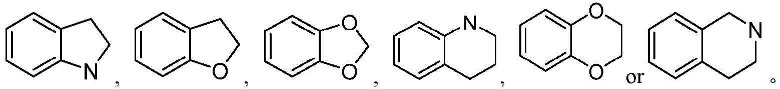

Unless otherwise specified, "heterocycloalkylidene" means a divalent heterocycloalkyl group as defined above. Unless otherwise specified, "bicyclic ring" means a group that features two connecting rings. The bicyclic ring may be a carbocyclic ring (all ring atoms are carbon atoms) or a heterocyclic ring (ring atoms include, for example, 1, 2, or 3 heteroatoms, such as N, O, or S, in addition to carbon atoms). These two rings may be aliphatic (e.g., decalin and norbornane), or may be aromatic (e.g., naphthalene), or a combination of aliphatic and aromatic (e.g., tetralin). Bicyclic rings include: (a) spirocyclic compounds, wherein the two rings share only one single atom (the spiro atom, which is typically a quaternary carbon); examples of the spirocyclic compounds include, but are not limited to: 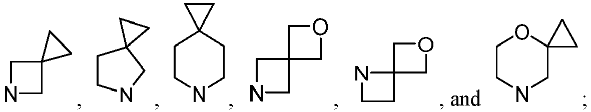 (b) fused bicyclic compounds, wherein the two rings share two adjacent atoms, that is, the rings share a covalent bond, i.e., the bridgehead atoms are directly connected (e.g., α-thujene and decalin); examples of the fused bicyclic rings include, but are not limited to: 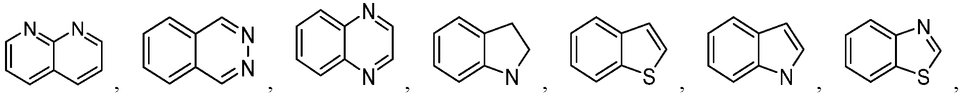 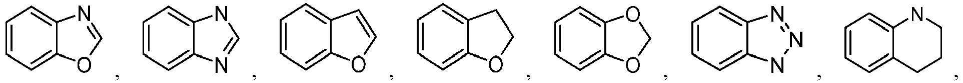 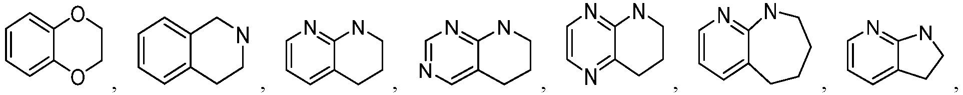 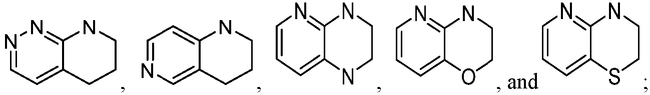 and
(c) bridged bicyclic compounds, wherein the two rings share three or more atoms and the two bridgehead atoms are separated by a bridge containing at least one atom; for example, norbornane, also known as bicyclo[2.2.1]heptane, can be thought of as a pair of cyclopentane rings, each sharing three of their five carbon atoms; examples of the bridged bicyclic rings include, but are not limited to:

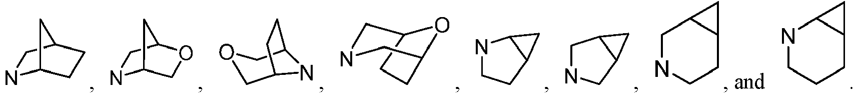

Unless otherwise specified, "carbocyclic ring" or "carbocyclic", by itself or in combination with other terms, represents a cyclic form of "C_{α-β} hydrocarbyl". Examples of the carbocyclic ring include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcamyl, bicyclo[1.1.1]pentyl, bicyclo[2.1.1]hexyl, and the like.

Unless otherwise specified, "heterocyclic ring" or "heterocyclic" means a ring containing at least one carbon atom and at least one other atom selected from N, O, and S. Examples of the heterocyclic ring that may be found in the claims include, but are not limited to, the following:

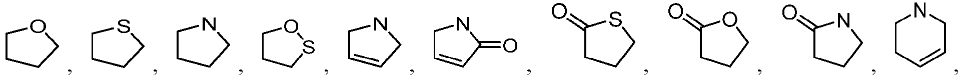

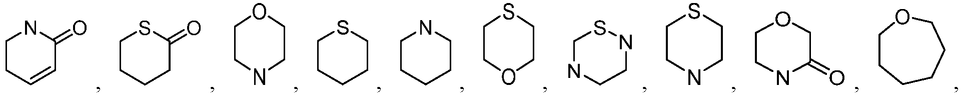

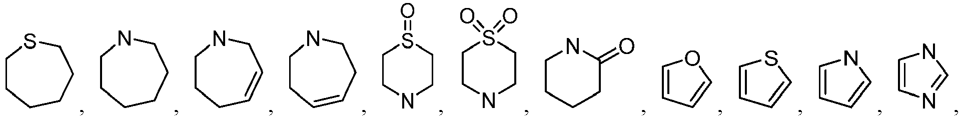

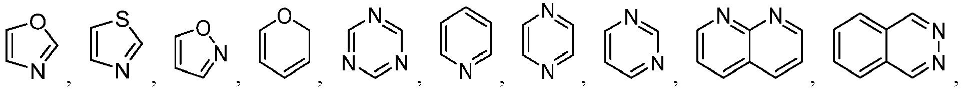

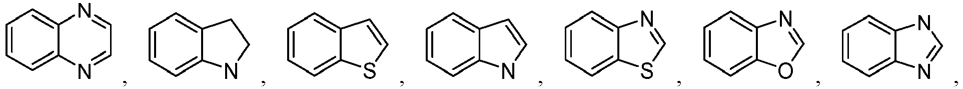

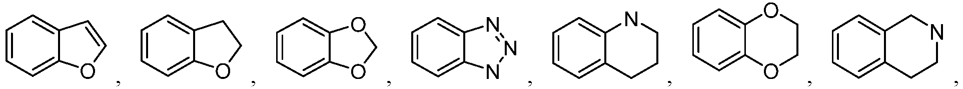

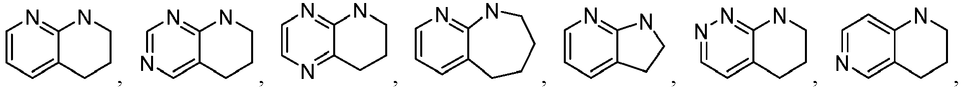

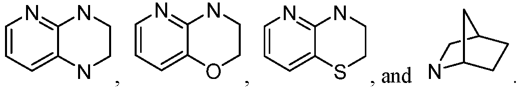

"Optional" or "optionally" means that the subsequently described event or circumstance may, but does not necessarily, occur, and the description includes instances where the event or circumstance occurs and instances where it does not.

"Saturated, partially saturated, or unsaturated" includes substituents saturated with hydrogen, substituents completely unsaturated with hydrogen, and substituents partially saturated with hydrogen.

When one of the variables is selected from a chemical bond, it means that the two groups linked by this variable are linked directly. For example, when L in X-L-Y represents a chemical bond, it means that the structure is actually X-Y.

When the number of a group is 0, such as -N(C₀ hydrocarbyl)-C₀₋₄ hydrocarbyl-, it means that the linker group is -NH-C₀₋₄ hydrocarbyl-.

When the number of a linker group is 0, such as -(CH₂)₀-, it means that the linker group is a chemical bond. Unless otherwise stated, the absolute configuration of a stereogenic center is represented by a wedged solid bond ( 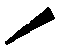 ) and a wedged dashed bond ( 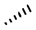 ), and the relative configuration of a stereogenic center is represented by a straight solid bond ( 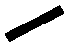 ) and a straight dashed bond ( 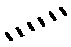 ). A wavy line ( 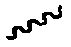 ) represents a wedged solid bond ( 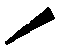 ) or a wedged dashed bond ( 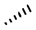 ), or a wavy line ( 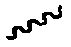 ) represents a straight solid bond ( 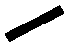 ) or a straight dashed bond ( 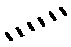 ).

Unless otherwise stated, a single bond or a double bond is represented by -̅ -̅ -̅.

### Specific Pharmaceutical and Medical Terminology

The term "acceptable", as used herein, means that a formulation component or an active ingredient does not unduly adversely affect a general therapeutic target's health.

The terms "treatment," "treatment course," and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

The "active ingredient" refers to the compound of general formula (1), and pharmaceutically acceptable inorganic or organic salts of the compound of general formula (1). The compounds of the present invention may contain one or more asymmetric centers (chiral center or axial chirality) and thus occur in the form of a racemate, racemic mixture, single enantiomer, diastereomeric compound, and single diastereomer. Asymmetric centers that may be present depend on the nature of the various substituents on the molecule. Each of these asymmetric centers will independently produce two optical isomers, and all possible optical isomers, diastereomeric mixtures, and pure or partially pure compounds are included within the scope of the present invention. The present invention is meant to include all such isomeric forms of these compounds. The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At the very least, these numerical parameters should be understood as the significant digits indicated or the numerical value obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides use of the compound of general formula (1) or the pharmaceutical composition of the present invention in inhibiting KIF18A protein, therefore, in treating one or more disorders related to the activity of KIF18A protein. Therefore, in certain embodiments, the present invention provides a method for treating a disorder mediated by KIF18A protein, the method comprising a step of administering to a patient in need thereof the compound or the pharmaceutically acceptable composition thereof of the present invention. In some embodiments, a method for treating cancer is provided, the method comprising administering to an individual in need thereof an effective amount of any of the aforementioned pharmaceutical compositions comprising the compound of structural general formula (1). In some embodiments, the cancer is mediated by KIF18A protein. In other embodiments, the cancer is a hematologic cancer and a solid tumor, preferably a tumor with chromosome instability, including, but not limited to, hematologic malignancies (leukemias, lymphomas, and myelomas including multiple myeloma, myelodysplastic syndrome and myeloproliferative family syndrome), solid tumors (carcinomas such as prostate, breast, lung, colon, pancreas, kidney, ovary and soft tissue cancers, osteosarcoma, and interstitial tumors), and the like. Leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma, or all cancer metastases are preferred; in other embodiments, the cancer is colon cancer, ovarian cancer, breast cancer, uterine cancer, cervical cancer, fallopian tube cancer, peritoneal cancer, lung cancer, liver cancer, head and neck cancer, pancreatic cancer, prostate cancer, oral cancer, esophageal cancer, and cancer metastases of these cancers; in other embodiments, the breast cancer is preferably triple negative breast cancer; in other embodiments, the ovarian cancer is preferably high-grade ovarian cancer, more preferably platinum-resistant high-grade ovarian cancer, and more preferably platinum-resistant high-grade serous ovarian cancer; in other embodiments, the peritoneal cancer is preferably primary peritoneal cancer; in other embodiments, the uterine cancer is preferably serous endometrial cancer.

### Route of Administration

The compound and the pharmaceutically acceptable salt thereof disclosed herein can be prepared into various formulations comprising a safe and effective amount of the compound or the pharmaceutically acceptable salt thereof disclosed herein, and a pharmaceutically acceptable excipient or carrier, wherein the "safe and effective amount" means that the amount of the compound is sufficient to significantly improve the condition without causing serious adverse effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" means that the components of the composition are capable of intermixing with the compound of the present invention and with each other, without significantly diminishing the pharmaceutical efficacy of the compound. Examples of pharmaceutically acceptable excipients or carriers include cellulose and its derivatives (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol, and sodium lauryl sulfate, or mixtures thereof. In the case of capsules, tablets, and pills, the dosage forms may further comprise buffers.

Solid dosage forms such as tablets, dragees, capsules, pills, and granules can be prepared using coatings and shells such as enteric coatings and other materials well known in the art. They may comprise opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups, and elixirs. In addition to the active compound, the liquid dosage form may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, and sesame oil, or mixtures of these substances.

Besides such inert diluents, the composition may further comprise adjuvants, such as wetting agents, emulsifiers, suspending agents, sweeteners, flavoring agents, and perfuming agents.

Suspensions, in addition to the active compound, may comprise suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may comprise physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof.

Dosage forms for topical administration of the compound of the present invention include ointments, pulvises, patches, sprays, and inhalants. The active ingredient is mixed under sterile conditions with a physiologically acceptable carrier and any preservatives, buffers, or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the administration dose is a pharmaceutically effective administration dose. For a human of 60 kg, the daily dose of administration is usually 1-2000 mg, preferably 50-1000 mg. In determining a specific dose, such factors as the route of administration, the health condition of the patient, and the like will also be considered, which are well known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise expressly stated, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific embodiments for reference only. After reading the description of the present invention, those skilled in the art can make various changes or modifications to the present invention, and such equivalents also fall within the scope of the present invention defined herein.

In all examples, ¹H-NMR spectra were recorded with a Varian Mercury 400 nuclear magnetic resonance spectrometer, and chemical shifts are expressed in δ (ppm); silica gel for separation was 200-300 mesh silica gel if not specified, and the ratio of the eluents was volume ratio. The following abbreviations are used in the present invention: (Boc)₂O for di-tert-butyl dicarbonate; BINAP for 2,2'-bis(diphenylphosphino)-1,1'-binaphthalene; BOPCl for bis(2-oxo-3-oxazolidinyl)phosphinic chloride; t-BuONa for sodium tert-butoxide; CDCl₃ for deuterated chloroform; Cs₂CO₃ for cesium carbonate; CuI for cuprous iodide; EtOAc for ethyl acetate; Hexane for *n*-hexane; HPLC for high-performance liquid chromatography; MeCN for acetonitrile; DCE for 1,2-dichloroethane; DCM for dichloromethane; DIPEA for diisopropylethylamine; 1,4-Dioxane for 1,4-dioxane; DMF for *N,N*-dimethylformamide; DMAPfor 4-(dimethylamino)pyridine; DMSO for dimethyl sulfoxide; h for hour; HATU for N-[(dimethylatnino)-1*H*-1,2,3-triazolo-[4,5-b]pyridin-1-methylene]-N-methylmethanaminium hexafluorophosphate-*N*-oxide; IPA for isopropanol; min for minute; K₂CO₃ for potassium carbonate; KOAc for potassium acetate; K₃PO₄ for potassium phosphate; LiBH₄ for lithium borohydride; min for minute; MeOH for methanol; MS for mass spectrometry; NMR for nuclear magnetic resonance; Pd/C for palladium on carbon; Pd(PPh₃)₄ for tetrakis(triphenylphosphine)palladium; Pd₂(dba)₃ for tris(dibenzylideneacetone)dipalladium(0); Pd(OAc)₂ for palladium acetate; PE for petroleum ether; RuPhos Pd G₃ for (2-dicyclohexylphosphino-2',6'-diisopropoxy-11'-biphenyl)[2-(2'-amino-11'-biphenyl)]palladium(II) methanesulfonate; Sarcosine for sarcosine; TFA for trifluoroacetic acid; T₃P for 1-propanephosphonic anhydride; XantPhos for 4,5-bis(diphenylphosphino)-9,9-dimethylxanthene; TLC for thin-layer chromatography; and XPhos for 2-dicyclohexylphosphonium-2',4',6'-triisopropylbiphenyl.

### Example 1. Synthesis of Compound 1

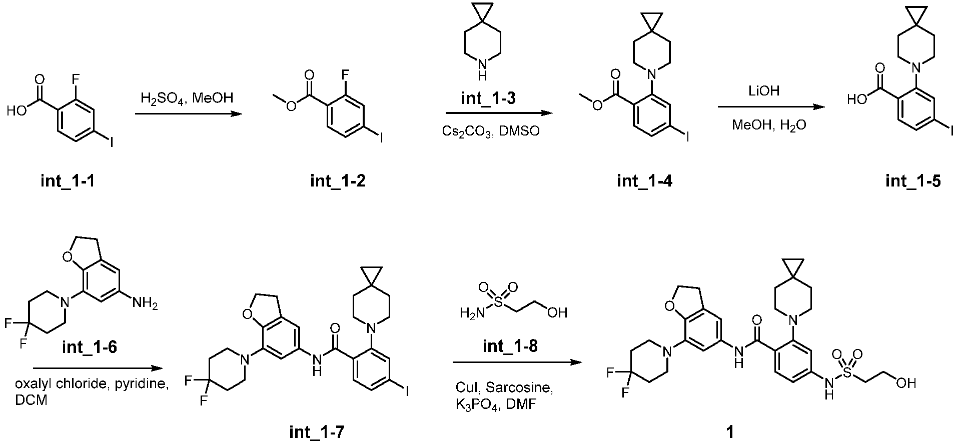

### Step 1: Synthesis of compound int_1-2:

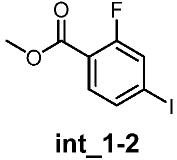

**int 1-1** (15 g, 56.3 mmol) was dissolved in methanol (150 mL), and concentrated sulfuric acid (2.5 mL) was added. The mixture was heated to 80 °C and reacted for 4 h, and LC-MS monitoring showed the reaction was complete. The reaction solution was concentrated under reduced pressure to give a crude product, and the crude product was dissolved in ethyl acetate. The organic phase was washed with a saturated sodium bicarbonate solution and then with saturated brine, dried over anhydrous sodium sulfate, filtered, and distilled under reduced pressure to give a white solid (14 g, yield: 89%). The solid was used directly in the next step. ESI-MS m/z: 281 [M+H]⁺

### Step 2: Synthesis of compound int_1-4:

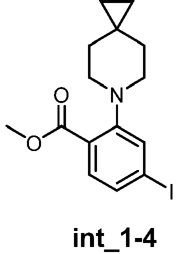

**int_1-2** (14 g, 49.9 mmol) was dissolved in DMSO (100 mL), and cesium carbonate (23.4 g, 71.7 mmol) and **int_1-3** (6.98 g, 62.8 mmol) were added. The mixture was heated to 90 °C and reacted for 24 h, and LC-MS monitoring showed the reaction was complete. The reaction solution was diluted with water (500 mL), and the aqueous phase was extracted with ethyl acetate (100 mL × 3). The organic phase was dried over anhydrous sodium sulfate, filtered, and distilled under reduced pressure to give a crude product. The crude product was purified by column chromatography (SiO₂, EtOAc:Hexane = 1:1) to give the target product (16.3 g, yield: 88%).

ESI-MS m/z: 372 [M+H]⁺.

### Step 3: Synthesis of compound int_1-5:

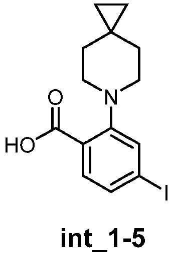

**int_1-4** (16.3 g, 43.9 mmol) was dissolved in a mixed solvent of methanol (100 mL) and water (10 mL), and lithium hydroxide (2.1 g, 87.8 mmol) was added at room temperature. The mixture was stirred at room temperature for 6 h. LC-MS monitoring showed the reaction was complete. The reaction solution was concentrated under reduced pressure to give a crude product (17 g). The crude product was directly used in the next step.

ESI-MS m/z: 358 [M+H]⁺.

### Step 4: Synthesis of compound int_1-7:

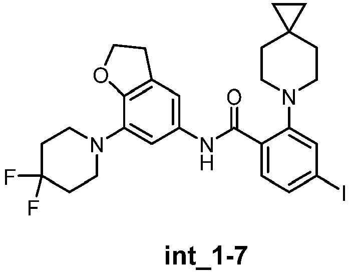

**int_1-5** (1.1 g, 3.08 mmol) was dissolved in DCM (10 mL), and oxalyl chloride (888.4 mg, 7 mmol) was added. The reaction solution was stirred at room temperature for 2 h and then concentrated under reduced pressure to remove the solvent, thus giving a solid. The solid was dissolved in DCM (10 mL), and **int_1-6** (783.2 mg, 3.08 mmol) and pyridine (730 mg, 9.24 mmol) were added. The reaction solution was stirred at 40 °C for 10 h. LC-MS monitoring showed the reaction was complete. The reaction solution was concentrated under reduced pressure to give a crude product. The crude product was purified by column chromatography (SiO₂, PE:EtOAc = 100:1) to give a solid (1.3 g, yield: 71.4%).

ESI-MS m/z: 594 [M+H]⁺.

### Step 5: Synthesis of compound 1:

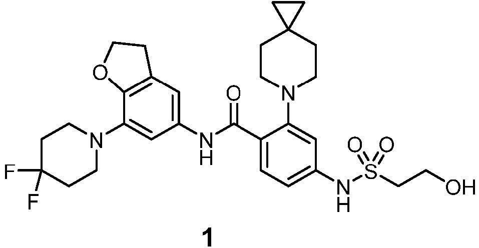

**int_1-8** (291 mg, 2.374 mmol), sarcosine (209.1 mg, 2.348 mmol), cuprous iodide (227 mg, 1.174 mmol), and potassium phosphate (1.5 g, 7.041 mmol) were dissolved in DMF (20 mL). The solution was purged with argon three times, and **int_1-7** (1.3 g, 2.19 mmol) was added. Under argon atmosphere, the reaction solution was heated to 90 °C and reacted for 3 h. LC-MS monitoring showed the reaction was complete. The reaction solution was cooled to room temperature, concentrated to dryness by rotary evaporation, and purified by column chromatography (SiO₂, EtOAc:Hexane = 1:1) to give a solid (1 g, yield: 77.5%).

¹H NMR (400 MHz, DMSO-d6) δ 11.53 (s, 1H), 7.80 (d, J = 8.5 Hz, 1H), 7.41 (d, *J* = 2.0 Hz, 1H), 7.13 (d, *J* = 2.2 Hz, 1H), 7.06 - 6.95 (m, 2H), 4.51 (t, *J* = 8.7 Hz, 2H), 3.73 (t, *J* = 6.6 Hz, 2H), 3.23 - 3.08 (m, 6H), 2.94 (t, *J* = 5.1 Hz, 4H), 2.15 - 1.96 (m, 4H), 1.53 (s, 4H), 0.33 (s, 4H).

ESI-MS m/z: 591 [M+H]⁺.

### Example 2. Synthesis of Compound 65

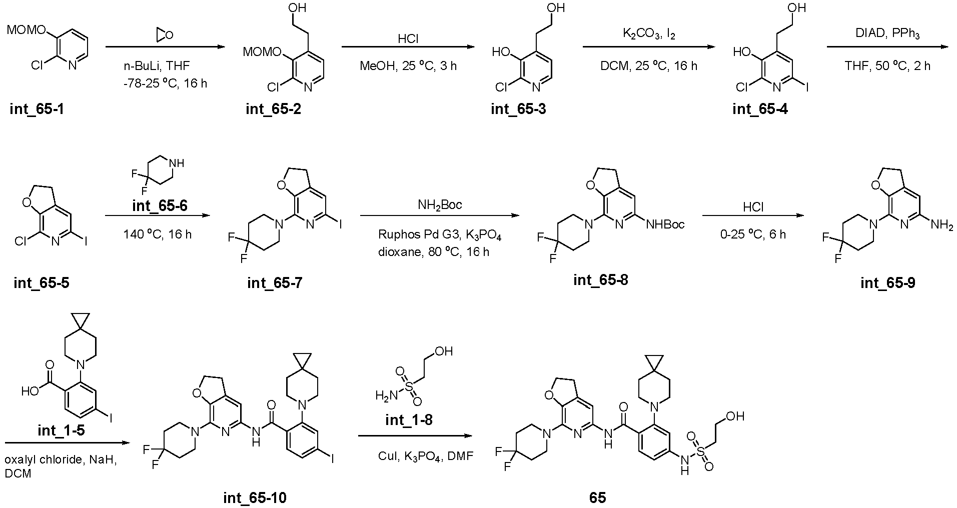

### Step 1: Synthesis of compound int_65-2:

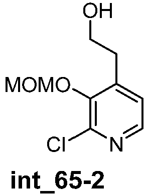

**int_65-1** (17.6 g, 101 mmol) was dissolved in THF (200 mL), and n-BuLi (2.5 M, 60.8 mL) was slowly added dropwise to the reaction solution at -78 °C under nitrogen atmosphere. After the reaction solution was reacted at -78 °C for 0.5 h, ethylene oxide (22.3 g, 506 mmol, 25.3 mL) was added dropwise to the reaction solution at -78 °C, and the reaction solution was reacted at -78 °C for 1 h, then slowly heated to room temperature, and reacted for another 1.5 h. LC-MS monitoring showed the reaction was complete. A saturated ammonium chloride solution (200 mL) was added to the reaction solution to quench the reaction. The aqueous phase was extracted with ethyl acetate (200 mL × 3), and the organic phase was dried over anhydrous sodium sulfate, filtered, and distilled under reduced pressure to give a crude product (16 g, yield: 72.5%). The crude product was directly used in the next step.

¹H NMR (400MHz, DMSO-d6) δ = 8.09 (d, J=4.9 Hz, 1H), 7.37 (d, J=4.9 Hz, 1H), 5.16 - 5.06 (m, 2H), 4.79 (t, J=5.3 Hz, 1H), 3.73 - 3.64 (m, 2H), 3.57 - 3.53 (m, 3H), 2.86 (t, J=6.7 Hz, 2H).

### Step 2: Synthesis of compound int_65-3:

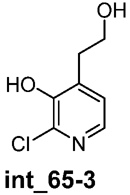

**int_65-2** (16.0 g, 73.5 mmol) was dissolved in methanol (200 mL), and a hydrochloric acid solution (12 M, 30.6 mL) was added at 0 °C. The mixture was heated to 25 °C and reacted for 3 h, and LC-MS monitoring showed the reaction was complete. At 0 °C, 1 N NaOH was added to the reaction solution to adjust the pH to 7. The aqueous phase was extracted with ethyl acetate (300 mL × 3), and the organic phase was dried over anhydrous sodium sulfate, filtered, and distilled under reduced pressure to give a crude product (10 g, yield: 78.3% ). The crude product was directly used in the next step.

¹H NMR (400MHz, DMSO-d6) δ = 8.09 (d, *J* = 4.9 Hz, 1H), 7.37 (d, *J* = 4.9 Hz, 1H), 5.16 - 5.06 (m, 2H), 4.79 (t, *J* = 5.3 Hz, 1H), 3.73 - 3.64 (m, 2H), 3.57 - 3.53 (m, 3H), 2.86 (t, *J* = 6.7 Hz, 2H).

### Step 3: Synthesis of compound int_65-4:

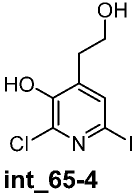

**int_1-4** (9.50 g, 54.7 mmol) was dissolved in a mixed solvent of THF (200 mL) and water (50 mL), and I₂ (13.8 g, 54.7 mmol, 11.0 mL) and K₂CO₃ (15.1 g, 109 mmol) were added at room temperature. The reaction solution was stirred at room temperature for 2 h. LC-MS monitoring showed the reaction was complete. 100 mL of water was added to the reaction solution. The aqueous phase was extracted with ethyl acetate (100 mL × 3), and the organic phase was dried over anhydrous sodium sulfate, filtered, and distilled under reduced pressure to give a crude product. The crude product was purified by column chromatography (SiO₂, PE:EtOAc = 1:1) to give a solid (13 g, yield: 79.3%).

¹H NMR (400MHz, DMSO-d6) δ = 10.10 (s, 1H), 7.58 (s, 1H), 3.63 (t, *J* = 6.3 Hz, 2H), 2.75 (t, *J* = 6.3 Hz, 2H).

### Step 4: Synthesis of compound int_65-5:

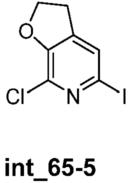

**int_65-4** (7.10 g, 23.7 mmol) and PPh₃ (9.33 g, 35.5 mmol) were dissolved in THF (100 mL), and DIAD (7.19 g, 35.5 mmol, 6.91 mL) was slowly added dropwise to the reaction solution at 0 °C under nitrogen atmosphere. The reaction solution was heated to 70 °C and stirred for 1 h. LC-MS monitoring showed the reaction was complete. 100 mL of water was added to the reaction solution. The aqueous phase was extracted with ethyl acetate (100 mL × 3), and the organic phase was dried over anhydrous sodium sulfate, filtered, and distilled under reduced pressure to give a crude product. The crude product was purified by column chromatography (SiO₂, PE:EtOAc = 3:1) to give a solid (11 g, yield: 65.9%).

¹H NMR (400MHz, CHLOROFORM-d) δ = 7.42 (s, 1H), 4.64 (t, *J* = 8.9 Hz, 2H), 3.31 - 3.19 (m, 2H).

### Step 5: Synthesis of compound int_65-7:

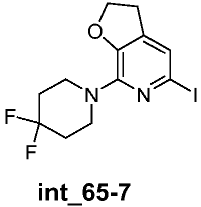

**int_65-5** (1.19 g, 4.26 mmol) and **int_65-6** (5.16 g, 42.6 mmol) were mixed, and the reaction solution was heated to 140 °C and stirred for 16 h under nitrogen atmosphere. LC-MS monitoring showed the reaction was complete. The reaction solution was diluted with water (30 mL), and the aqueous phase was extracted with ethyl acetate (30 mL × 3). The organic phase was dried over anhydrous sodium sulfate, filtered, and distilled under reduced pressure to give a crude product. The crude product was purified by column chromatography (SiO₂, EtOAc:Hexane = 5:1) to give the target product (0.35 g, yield: 22.4%).

¹H NMR (400MHz, CHLOROFORM-d) δ = 7.08 (s, 1H), 4.58 (t, *J* = 8.9 Hz, 2H), 3.77 - 3.68 (m, 4H), 3.15 (t, *J* = 8.9 Hz, 2H), 2.03 - 1.89 (m, 4H).

### Step 6: Synthesis of compound int_65-8:

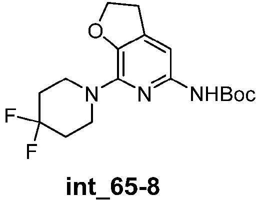

**int_65-7** (0.35 g, 955 µmol), NH₂Boc (223 mg, 1.91 mmol), RuPhos Pd G3 (79.9 mg, 95.5 µmol), and K₃PO₄ (405 mg, 1.91 mmol) were dissolved in 1,4-dioxane (5 mL), and the reaction solution was heated to 80 °C and stirred for 16 h under nitrogen atmosphere. LC-MS monitoring showed the reaction was complete. The reaction solution was diluted with water (30 mL), and the aqueous phase was extracted with ethyl acetate (30 mL × 3). The organic phase was dried over anhydrous sodium sulfate, filtered, and distilled under reduced pressure to give a crude product (0.2 g, yield: 58.8% ). The crude product was directly used in the next step.

¹H NMR (400MHz, CHLOROFORM-d) δ = 7.08 (s, 1H), 4.58 (t, *J* = 8.9 Hz, 2H), 3.77 - 3.68 (m, 4H), 3.15 (t, *J* = 8.9 Hz, 2H), 2.03 - 1.89 (m, 4H).

### Step 7: Synthesis of compound int_65-9:

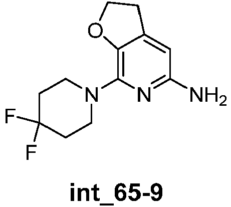

**int_65-8** (0.20 g, 562 µmol) was dissolved in dichloromethane (5 mL), and a hydrochloric acid/dioxane solution (4 M, 5 mL) was added at 0 °C. The mixture was heated to 25 °C and reacted for 2 h, and LC-MS monitoring showed the reaction was complete. Ammonia water was added to the reaction solution to adjust the pH to 9 at 0 °C. The aqueous phase was extracted with ethyl acetate (30 mL × 3), and the organic phase was dried over anhydrous sodium sulfate, filtered, and distilled under reduced pressure to give a crude product. The crude product was purified by column chromatography (SiO₂, PE:EtOAc = 1:1) to give a solid (108 mg, yield: 75.1%).

¹H NMR (400MHz, CHLOROFORM-d) δ = 5.89 (s, 1H), 4.40 (t, *J* = 8.7 Hz, 2H), 3.64 - 3.54 (m, 4H), 2.99 (t, *J* = 8.6 Hz, 2H), 2.03 - 1.86 (m, 4H).

### Step 8: Synthesis of compound int_65-10:

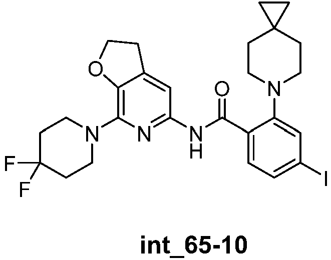

**int_1-5** (70 mg, 0.196 mmol) was dissolved in DCM (3 mL), and oxalyl chloride (253.8 mg, 2 mmol) was added. The reaction solution was stirred at room temperature for 2 h and then concentrated under reduced pressure to remove the solvent, thus giving an acyl chloride solid. **int_65-9** (50 mg, 0.196 mmol) was dissolved in THF (5 mL), and sodium hydride (30 mg, 0.75 mmol, 60% purity) was added at 0 °C under nitrogen atmosphere. The reaction solution was heated to room temperature and reacted for 1 h, and then the reaction solution was cooled to 0 °C. The acyl chloride prepared above was slowly added to the reaction solution, and the reaction solution was heated to 45 °C and stirred for 12 h. LC-MS monitoring showed the reaction was complete. The reaction solution was concentrated under reduced pressure to give a crude product. The crude product was purified by column chromatography to give a solid (92 mg, yield: 79%).

ESI-MS m/z: 595 [M+H]⁺.

### Step 9: Synthesis of compound 65:

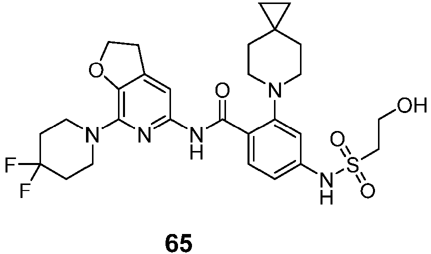

**int_1-8** (39 mg, 0.309 mmol), dimethylcyclohexane-1,2-diamine (11 mg, 0.077 mmol), cuprous iodide (15 mg, 0.077 mmol), and potassium phosphate (98 mg, 0.464 mmol) were dissolved in DMF (5 mL). The solution was purged with argon three times, and **int_65-10** (92 mg, 0.155 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (50 mg, yield: 55%).

¹H NMR (400 MHz, DMSO-d6) δ 12.74 (s, 1H), 8.02 (d, J = 8.6 Hz, 1H), 7.74 (s, 1H), 7.21 (d, *J* = 2.1 Hz, 1H), 7.07 (dd, *J* = 8.6, 2.1 Hz, 1H), 4.54 (t, *J =* 8.8 Hz, 2H), 3.73 (t, *J* = 6.5 Hz, 2H), 3.66 (t, *J* = 5.8 Hz, 4H), 3.17 (t, *J* = 8.8 Hz, 2H), 2.94 (t, *J* = 5.3 Hz, 4H), 2.16 - 1.93 (m, 4H), 1.69 (s, 4H), 0.36 (s, 4H).

ESI-MS m/z: 592 [M+H]⁺.

### Example 3. Synthesis of Compound 129

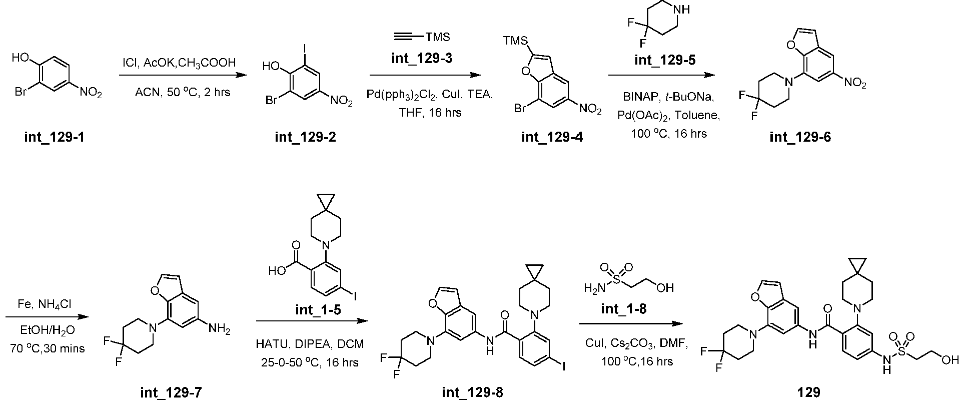

### Step 1: Synthesis of compound int_129-2:

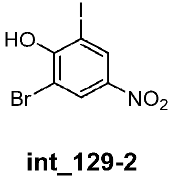

**int_129-1** (28.7 g, 131 mmol) was dissolved in acetonitrile (50 mL), and potassium acetate (29.1 g, 296 mmol), ICl (21.4 g, 131 mmol, 6.71 mL), and CH₃COOH (60 mL) were added. The mixture was heated to 50 °C and reacted for 42 h, and TLC monitoring showed the reaction was complete. 500 mL of water was added to the reaction solution. The aqueous phase was extracted with ethyl acetate (500 mL × 3). The organic phase was dried over anhydrous sodium sulfate, filtered, and distilled under reduced pressure to give a white solid (42 g, yield: 92.8%). The solid was directly used in the next step.

### Step 2: Synthesis of compound int_129-4:

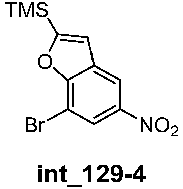

**int_129-2** (37.0 g, 107 mmol) was dissolved in acetonitrile (500 mL), and **int_129-3** (11.1 g, 112 mmol), CuI (409 mg, 2.15 mmolq), TEA (21.8 g, 215 mmol), and Pd(PPh₃)₂Cl₂ (755 mg, 1.08 mmol) were added. The reaction solution was reacted at 20 °C for 3 h under nitrogen atmosphere, and then heated to 80 °C and reacted for 8 h. LC-MS monitoring showed the reaction was complete. The reaction solution was diluted with water (500 mL), and the aqueous phase was extracted with ethyl acetate (500 mL × 3). The organic phase was dried over anhydrous sodium sulfate, filtered, and distilled under reduced pressure to give a crude product. The crude product was purified by column chromatography (SiO₂, EtOAc:Hexane = 1:10 to 3:1) to give the target product (12 g, yield: 35.5%).

¹H NMR: (400 MHz, CHLOROFORM-d) δ 8.19 (d, *J* = 17.4 Hz, 2H), 6.91 (s, 1H), 0.19 (s, 9H).

### Step 3: Synthesis of compound int_129-6:

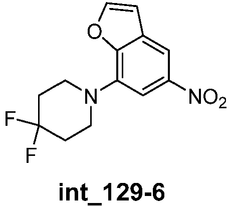

**int_129-4** (11 g, 35.0 mmol) was dissolved in methylbenzene (300 mL), and **int_129-5** (4.40 g, 36.3 mmol), BINAP (4.36 g, 7.00 mmol), t-BuONa (6.73 g, 70.0 mmol), and Pd(OAc)₂ (785 mg, 3.50 mmol) were added at room temperature. The reaction solution was heated to 110 °C and stirred for 16 h under nitrogen atmosphere. LC-MS monitoring showed the reaction was complete. Methylbenzene was removed under reduced pressure, and the reaction solution was diluted with water (200 mL). The aqueous phase was extracted with ethyl acetate (200 mL × 3), and the organic phase was dried over anhydrous sodium sulfate, filtered, and distilled under reduced pressure to give a crude product. The crude product was purified by column chromatography (SiO₂, EtOAc:Hexane = 1:10 to 3:1) to give the target product (6.4 g, yield: 64.8%).

¹H NMR (400 MHz, CHLOROFORM-d) δ 8.08 (d, *J* = 2.0 Hz, 1H), 7.71 - 7.46 (m, 2H), 6.84 (d, *J* = 2.1 Hz, 1H), 3.54 - 3.47 (m, 2H), 3.45 - 3.44 (m, 1H), 3.56 - 3.36 (m, 1H), 2.20 - 2.09 (m, 4H).

ESI-MS m/z: 283 [M+H]⁺.

### Step 4: Synthesis of compound int 129-7:

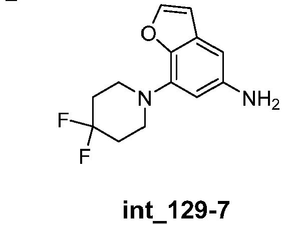

**int_129-6** (4.50 g, 15.9 mmol) was dissolved in ethanol (50 mL), and Fe (8.90 g, 159 mmol) and an ammonium chloride solution (8.53 g, 159 mmol, 3.5 M) were added at room temperature. The reaction solution was heated to 70 °C and stirred for 0.5 h under nitrogen atmosphere. LC-MS monitoring showed the reaction was complete. The reaction solution was filtered to give a filtrate, and the filtrate was concentrated under reduced pressure to give a crude product (3 g, yield: 74.6%). The crude product was directly used in the next step.

ESI-MS m/z: 253 [M+H]⁺.

### Step 5: Synthesis of compound int_129-8:

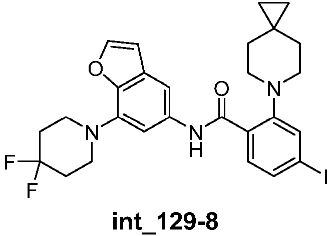

int_1-5 (3.50 g, 9.80 mmol) was dissolved in DCM (50 mL), and HATU (6.71 g, 17.6 mmol) and DIPEA (4.56 g, 35.3 mmol) were added. The reaction solution was stirred at room temperature for 0.5 h, and int_129-7 (4.20 g, 11.76 mmol) was then added. The reaction solution was heated to 50 °C and stirred for 16 h. LC-MS monitoring showed the reaction was complete. The reaction solution was concentrated under reduced pressure to give a crude product. The crude product was purified by column chromatography (SiO₂, PE:EtOAC = 10:1 to 3:1) to give a solid (5 g, yield: 71.9%).

¹H NMR (400 MHz, CHLOROFORM-d) δ 12.32 (s, 1H), 8.03 - 7.86 (m, 1H), 7.72 - 7.47 (m, 4H), 7.18 (d, *J* = 10.6 Hz, 2H), 6.70 (d, *J* = 1.4 Hz, 1H), 5.23 (s, 2H), 3.47 (br t, *J* = 5.3 Hz, 4H), 3.02 (br t, *J* = 5.0 Hz, 4H), 2.31 - 2.02 (m, 4H), 1.75 - 1.45 (m, 5H).

ESI-MS m/z: 592 [M+H]⁺.

### Step 6: Synthesis of compound 129:

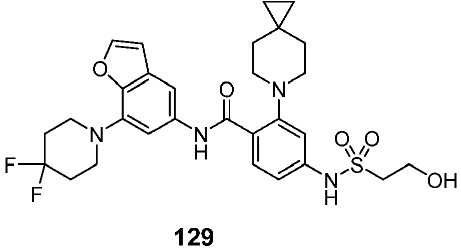

**int_1-8** (1.03 g, 8.24 mmol), dimethylcyclohexane-1,2-diamine (781 mg, 5.50 mmol), cuprous iodide (1.05 g, 5.50 mmol), and Cs₂CO₃ (3.58 g, 11.0 mmol) were dissolved in DMF (70 mL). The solution was purged with argon three times, and **int_129-8** (3.25 g, 5.50 mmol) was added. The reaction solution was heated to 110 °C and reacted for 16 h under argon atmosphere. LC-MS monitoring showed the reaction was complete. The reaction solution was cooled to room temperature, concentrated to dryness by rotary evaporation, and purified by column chromatography (SiO₂, DCM:MeOH = 10:1 to 1:1) to give a solid (1.5 g, yield: 46.4%).

¹H NMR (400 MHz, DMSO-d6) δ 11.70 (s, 2H), 7.94 (d, *J* = 2.1 Hz, 1H), 7.86 - 7.76 (m, 2H), 7.14 (d, *J* = 2.1 Hz, 1H), 7.07 (d, *J =* 1.9 Hz, 1H), 7.01 (dd, *J =* 8.5, 2.0 Hz, 1H), 6.93 (d, *J* = 2.1 Hz, 1H), 3.74 (t, *J =* 6.6 Hz, 2H), 3.44 (t, *J* = 5.7 Hz, 4H), 3.29 (d, *J* = 6.6 Hz, 2H), 2.96 (t, *J* = 5.2 Hz, 4H), 2.24 - 2.07 (m, 4H), 1.55 (s, 4H), 0.33 (s, 4H).

ESI-MS m/z: 589 [M+H]⁺.

### Example 4. Synthesis of Compound 385

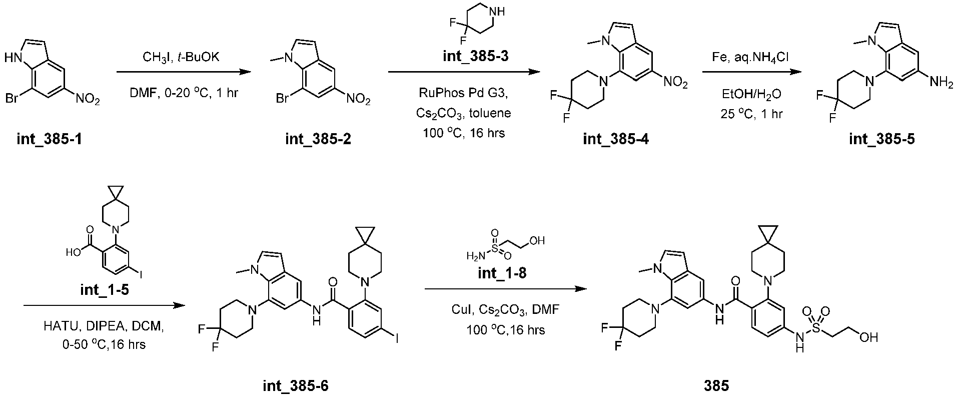

### Step 1: Synthesis of compound int_385-2:

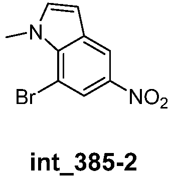

**int_385-1** (5.00 g, 20.7 mmol) was dissolved in DMF (50 mL), and t-BuOK (2.44 g, 21.8 mmol) was added at 0 °C under nitrogen atmosphere. After 0.5 h of reaction, CH₃I (5.41 g, 37.3 mmol, 98% purity) was added at 0 °C. The reaction solution was heated to 25 °C and reacted for 1 h, and TLC monitoring showed the reaction was complete. 50 mL of water was added to the reaction solution, and a precipitate precipitated out. The mixture was filtered to give a filter cake, and the filter cake was washed with water (100 mL × 2) and dried to give a yellow solid (5 g, crude product). The solid was used directly in the next step.

¹H NMR: (400 MHz, CHLOROFORM-d) δ 4.14 (s, 3 H) 6.59 (d, *J* = 3.25 Hz, 1 H) 7.08 (d, *J* = 3.25 Hz, 1 H) 8.19 (d, *J* = 2.13 Hz, 1 H) 8.40 (d, *J* = 2.13 Hz, 1 H).

### Step 2: Synthesis of compound int_385-4:

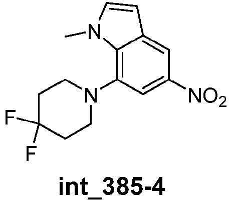

**int_385-2** (4.80 g, 18.8 mmol) was dissolved in methylbenzene (50 mL), and RuPhos Pd G3 (1.57 g, 1.88 mmol), Cs₂CO₃ (12.3 g, 37.6 mmol), and int_385-3 (4.56 g, 37.6 mmol) were added. The reaction solution was heated to 100 °C and reacted for 16 h under nitrogen atmosphere, and TLC monitoring showed the reaction was complete. The reaction solution was diluted with water (100 mL), and the aqueous phase was extracted with ethyl acetate (100 mL × 3). The organic phase was dried over anhydrous sodium sulfate, filtered, and distilled under reduced pressure to give a crude product. The crude product was purified by column chromatography (SiO₂, EtOAc:Hexane = 1:30 to 1:3) to give the target product (3.4 g, yield: 61.2%).

### Step 3: Synthesis of compound int 385-5:

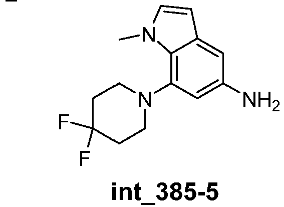

**int_385-4** (3.40 g, 11.5 mmol) was dissolved in ethanol (35 mL), and Fe (6.43 g, 115 mmol, 10.0 eq) and an ammonium chloride solution (6.16 g, 115 mmol, 3.4 M) were added at room temperature. The reaction solution was stirred at 70 °C for 0.5 h. TLC monitoring showed the reaction was complete. The reaction solution was diluted with water (20 mL), and the aqueous phase was extracted with ethyl acetate (20 mL × 3). The organic phase was dried over anhydrous sodium sulfate, filtered, and distilled under reduced pressure to give a crude product (3.7 g). The crude product was directly used in the next step.

¹H NMR: (400 MHz, CHLOROFORM-d) δ 6.82 (d, *J* = 2.9 Hz, 1H), 6.75 (s, 1H), 6.46 (s, 1H), 6.22 (d, *J =* 2.9 Hz, 1H), 3.98 (s, 3H), 3.18 (br d, *J* = 10.9 Hz, 2H), 2.96 - 2.89 (m, 2H), 2.19 - 2.01 (m, 6H).

### Step 4: Synthesis of compound int_385-6:

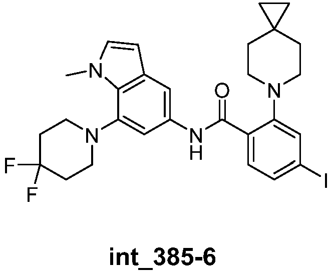

**int_1-5** (3.50 g, 9.80 mmol) was dissolved in DCM (50 mL), and HATU (5.59 g, 14.7 mmol) and DIPEA (3.80 g, 29.4 mmol) were added. The reaction solution was stirred at room temperature for 0.5 h, and int_385-5 (2.60 g, 9.80 mmol) was then added. The reaction solution was heated to 50 °C and stirred for 16 h. LC-MS monitoring showed the reaction was complete. The reaction solution was concentrated under reduced pressure to give a crude product. The crude product was purified by column chromatography (SiO₂, PE:EtOAc = 1:0 to 3:1) to give a solid (1.25 g, yield: 21.1%).

ESI-MS m/z: 605 [M+H]⁺.

### Step 5: Synthesis of compound 385:

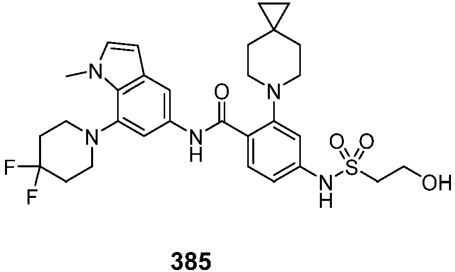

**int_385-6** (1.25 g, 2.07 mmol), dimethylcyclohexane-1,2-diamine (29.4 mg, 206 µmol), cuprous iodide (393 mg, 2.07 mmol), and Cs₂CO₃ (1.35 g, 4.14 mmol) were dissolved in DMF (25 mL). The solution was purged with argon three times, and **int_1-8** (388 mg, 3.10 mmol) was added. The reaction solution was heated to 90 °C and reacted for 3 h under argon atmosphere. LC-MS monitoring showed the reaction was complete. The reaction solution was cooled to room temperature, concentrated to dryness by rotary evaporation, and purified by column chromatography (SiO₂, EtOAc:Hexane = 1:20 to 1:1) to give a solid (0.53 g, yield: 42.8%).

¹H NMR: (400 MHz, DMSO-d6) δ 11.76 (s, 1H), 10.06 (s, 1H), 7.94 - 7.85 (m, 2H), 7.22 - 7.20 (m, 1H), 7.19 (d, *J* = 1.8 Hz, 2H), 7.06 (dd, *J =* 2.1, 8.6 Hz, 1H), 6.39 (d, *J* = 3.0 Hz, 1H), 4.94 (t, *J =* 5.6 Hz, 1H), 4.08 (s, 3H), 3.77 (q, *J* = 6.3 Hz, 2H), 3.42 (br s, 4H), 3.03 - 2.85 (m, 6H), 2.35 - 2.13 (m, 4H), 1.61 (br s, 4H), 0.37 (s, 4H).

ESI-MS m/z: 602 [M+H]⁺.

### Example 5. Synthesis of Compound 513

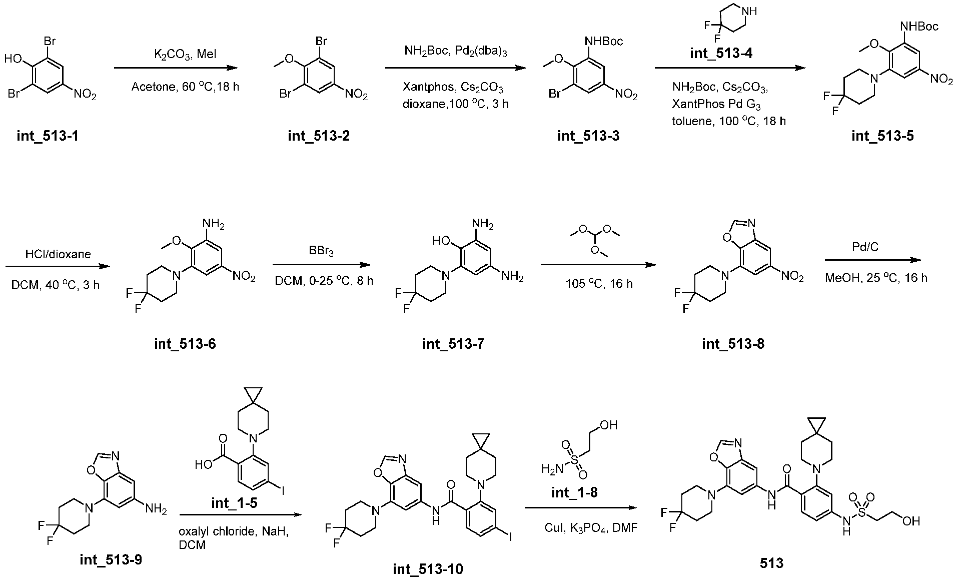

### Step 1: Synthesis of compound int_513-2:

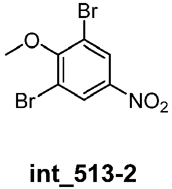

**int_513-1** (25.0 g, 84.2 mmol), K₂CO₃ (34.9 g, 252 mmol), and MeI (119 g, 842 mmol, 52.4 mL) were dissolved in acetone (250 mL), and the reaction solution was heated to 60 °C and reacted for 18 h under nitrogen atmosphere. TLC monitoring showed the reaction was complete. Water (200 mL) was added to the reaction solution to quench the reaction. The aqueous phase was extracted with dichloromethane (200 mL × 3), and the organic phase was dried over anhydrous sodium sulfate, filtered, and distilled under reduced pressure to give a crude product. The crude product was purified by column chromatography (ISCO^{®}; 120 g SepaFlash^{®} Silica Flash Column, Eluent of 0-7% Ethyl acetate/Petroleum ether gradient @ 80 mL/min) to give a solid (23 g, yield: 87.8%).

¹H NMR (400 MHz, DMSO-d6)δ= 8.49 (m, 2 H) 3.91 (s, 3 H).

### Step 2: Synthesis of compound int_513-3:

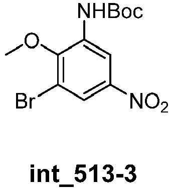

**int_513-2** (12.0 g, 38.5 mmol), NH₂Boc (4.52 g, 38.5 mmol), Cs₂CO₃ (25.1 g, 77.1 mmol), and XantPhos Pd G3 (1.83 g, 1.93 mmol) were dissolved in methylbenzene (120 mL). The reaction solution was heated to 100 °C and reacted for 3 h under nitrogen atmosphere, and LC-MS monitoring showed the reaction was complete. Water (500 mL) was added to the reaction solution. The aqueous phase was extracted with dichloromethane (500 mL × 3), and the organic phase was dried over anhydrous sodium sulfate, filtered, and distilled under reduced pressure to give a crude product. The crude product was purified by column chromatography (ISCO^{®}; 200 g SepaFlash^{®} Silica Flash Column, Eluent of 0-10% Ethyl acetate/Petroleum ether gradient @ 80 mL/min) to give a solid (9 g, yield: 67.1%).

¹H NMR (400 MHz, DMSO-d6)δ= 9.16 (s, 1 H) 8.77 (d, *J* = 2.81 Hz, 1 H) 8.12 (d, *J* = 2.81 Hz, 1 H) 3.84 (s, 3 H) 1.51 (s, 9 H).

### Step 3: Synthesis of compound int_513-5:

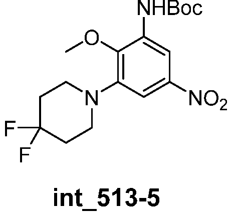

**int_513-3** (4 g, 11.52 mmol), int_513-4 (2.79 g, 23.0 mmol), Pd₂(dba)₃ (1.06 g, 1.15 mmol, 0.10 eq), Xantphos (1.33 g, 2.30 mmol, 0.20 eq), and Cs₂CO₃ (7.51 g, 23.0 mmol, 2.00 eq) were dissolved in methylbenzene (100 mL), and the reaction solution was heated to 100 °C and stirred for 3 h under nitrogen atmosphere. LC-MS monitoring showed the reaction was complete. 50 mL of water was added to the reaction solution. The aqueous phase was extracted with dichloromethane (50 mL × 3), and the organic phase was dried over anhydrous sodium sulfate, filtered, and distilled under reduced pressure to give a crude product. The crude product was purified by column chromatography (ISCO^{®}; 40 g SepaFlash^{®} Silica Flash Column, Eluent of 0-20% Ethyl acetate/Petroleum ether gradient @ 50 mL/min) to give a solid (1.78 g, yield: 39.8%).

¹H NMR (400 MHz, DMSO-d6)δ= 8.71 (s, 1 H) 8.46 (d, *J* = 2.50 Hz, 1 H) 7.51 (d, *J* = 2.75 Hz, 1 H) 3.87 (m, 3 H) 3.19 (br t, *J* = 5.19 Hz, 4 H) 2.16 (m, 4 H) 1.49 (s, 9 H).

### Step 4: Synthesis of compound int_513-6:

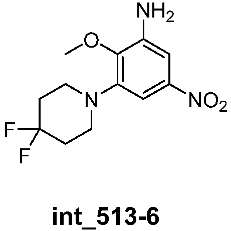

**int_513-5** (1.78 g, 4.60 mmol) was dissolved in ethyl acetate (10 mL), and a HCl/ethyl acetate solution (2.50 M, 2.00 mL) was slowly added dropwise to the reaction solution under nitrogen atmosphere. The reaction solution was heated to 40 °C and stirred for 3 h. LC-MS monitoring showed the reaction was complete. The reaction solution was distilled under reduced pressure to give a crude product (1.65 g, yield: 99.8%). The crude product was directly used in the next step.

ESI-MS m/z: 288 [M+H]⁺

### Step 5: Synthesis of compound int_513-7:

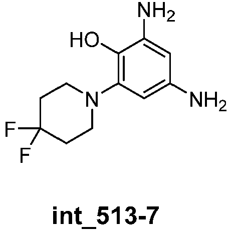

**int_513-6** (798 mg, 2.78 mmol) was dissolved in DCM (10 mL), and BBr₃ (2.79 g, 11.1 mmol, 1.07 mL) was added to the reaction solution at 0 °C under nitrogen atmosphere. The reaction solution was slowly heated to room temperature and reacted for 16 h. LC-MS monitoring showed the reaction was complete. The reaction solution was diluted with water (50 mL), and the aqueous phase was extracted with dichloromethane (50 mL × 3). The organic phase was dried over anhydrous sodium sulfate, filtered, and distilled under reduced pressure to give a crude product. The crude product was purified by column chromatography (ISCO^{®};40 g SepaFlash^{®} Silica Flash Column, Eluent of 0-30% Ethyl acetate/Petroleum ether gradient @ 50 mL/min) to give the target product (0.52 g, yield: 68.4%).

¹H NMR (400 MHz, DMSO-d6)δ= 7.35 (d, *J* = 2.69 Hz, 1 H) 7.25 (d, 7 = 2.69 Hz, 1 H) 4.09 (br s, 1 H) 3.17 (s, 2 H) 2.94 (br t, *J* = 5.20 Hz, 4 H) 2.18 (m, 4 H).

### Step 6: Synthesis of compound int_513-8:

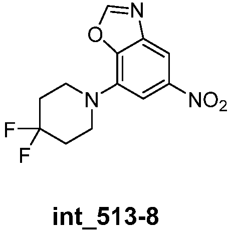

**int_513-7** (0.52 g, 1.90 mmol) was dissolved in trimethyl orthoformate (50.3 g, 474 mmol, 52.0 mL), and the reaction solution was heated to 105 °C and stirred for 18 h under nitrogen atmosphere. LC-MS monitoring showed the reaction was complete. The reaction solution was distilled under reduced pressure to give a crude product (0.5 g). The crude product was directly used in the next step.

ESI-MS m/z: 284 [M+H]⁺.

### Step 7: Synthesis of compound int_513-9:

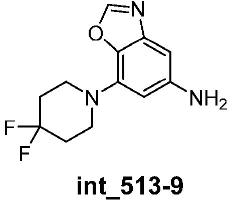

**int_513-8** (0.50 g, 1.77 mmol) and Pd/C (0.05 g, 1.77 mmol, 10% purity) were dissolved in methanol (5 mL). The reaction solution was reacted at 25 °C for 18 h under hydrogen atmosphere, and LC-MS monitoring showed the reaction was complete. The reaction solution was filtered, and the filtrate was distilled under reduced pressure to give a crude product. The crude product was purified by column chromatography (ISCO^{®}; 80 g SepaFlash^{®} Silica Flash Column, Eluent of 30% Ethyl acetate/Petroleum ether gradient @ 80 mL/min) to give a solid (0.3 g, yield: 68.2%).

¹H NMR (400 MHz, DMSO-d6)δ=7.35 (d, *J* = 2.69 Hz, 1 H) 7.25 (d, *J* = 2.69 Hz, 1 H) 4.09 (br s, 1 H) 3.17 (s, 2 H) 2.94 (br t, *J =* 5.20 Hz, 4 H) 2.18 (m, 4 H).

### Step 8: Synthesis of compound int_513-10:

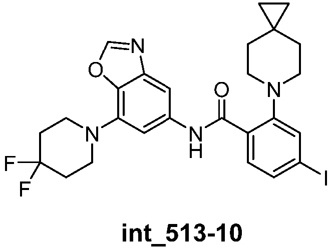

**int_1-5** (124 mg, 0.3 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_513-9** (76 mg, 0.3 mmol) was dissolved in THF (5 mL), and sodium hydride (72 mg, 1.8 mmol, 60% purity) was added at 0 °C under nitrogen atmosphere. The reaction solution was heated to room temperature and reacted for 1 h, and then the reaction solution was cooled to 0 °C. The acyl chloride prepared above was slowly added to the reaction solution, and the reaction solution was heated to 40 °C and stirred for 12 h. LC-MS monitoring showed the reaction was complete. The reaction solution was concentrated under reduced pressure to give a crude product. The crude product was purified by column chromatography to give a solid (100 mg, yield: 56.2%).

ESI-MS m/z: 593 [M+H]⁺.

### Step 9: Synthesis of compound 513:

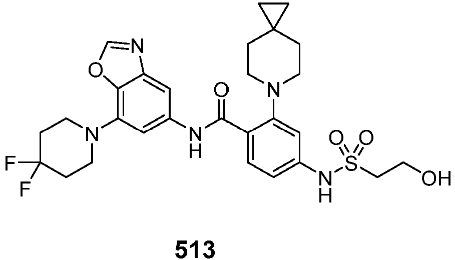

int_1-8 (63 mg, 0.51 mmol), dimethylcyclohexane-1,2-diamine (12 mg, 0.085 mmol), cuprous iodide (16 mg, 0.085 mmol), and potassium phosphate (108 mg, 0.51 mmol) were dissolved in DMF (5 mL). The solution was purged with argon three times, and **int_513-10** (100 mg, 0.17 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (33 mg, yield: 33%).

¹H NMR (400 MHz, DMSO-d6) δ 11.71 (s, 1H), 10.09 (s, 1H), 8.67 (s, 1H), 7.87 (d, *J* = 1.7 Hz, 1H), 7.82 (d, *J* = 8.5 Hz, 1H), 7.21 (d, *J* = 1.8 Hz, 1H), 7.15 (d, *J* = 2.1 Hz, 1H), 7.01 (dd, *J* = 8.5, 2.1 Hz, 1H), 3.74 (t, *J =* 6.6 Hz, 2H), 3.50 (t, *J* = 5.8 Hz, 4H), 2.96 (t, *J* = 5.3 Hz, 4H), 2.15 (tt, *J* = 13.4, 5.6 Hz, 4H), 1.53 (s, 4H), 0.32 (s, 4H).

ESI-MS m/z: 590 [M+H]⁺.

### Example 6. Synthesis of Compound 522

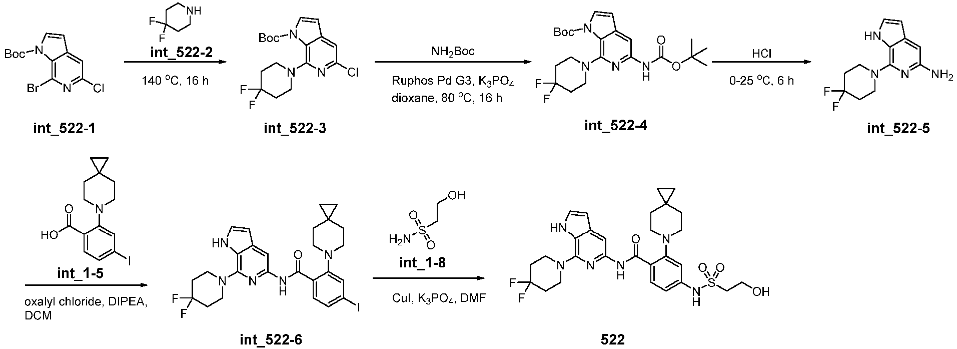

### Step 1: Synthesis of compound int_522-2:

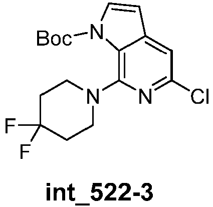

**int_522-1** (620 mg, 1.87 mmol) and **int_522-2** (226 mg, 1.87 mmol) were dissolved in 1,4-dioxane (15 mL), and cesium carbonate (913 mg, 2.8 mmol), Pd₂(dba)₃ (171 mg, 0.187 mmol), and XantPhOS (108 mg, 0.187 mmol) were added to the reaction solution. The reaction solution was heated to 100 °C and stirred for 18 h under nitrogen atmosphere. LC-MS monitoring showed the reaction was complete. The reaction solution was diluted with water (30 mL), and the aqueous phase was extracted with ethyl acetate (30 mL × 3). The organic phase was dried over anhydrous sodium sulfate, filtered, and distilled under reduced pressure to give a crude product, and the crude product was purified by column chromatography (SiO₂, EtOAc:Hexane = 1:25) to give the target product (486 mg, yield: 70%).

ESI-MS m/z: 372 [M+H]⁺.

### Step 2: Synthesis of compound int_522-4:

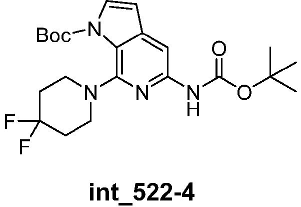

**int_522-3** (486 mg, 1.309 mmol), NH₂Boc (460 mg, 3.8 mmol), Pd₂(dba)₃ (24 mg, 0.026 mmol), X-PhOS (18 mg, 0.039 mmol), and cesium carbonate (633 mg, 1.95 mmol) were dissolved in 1,4-dioxane (5 mL), and the reaction solution was heated to 100 °C and stirred for 16 h under nitrogen atmosphere. LC-MS monitoring showed the reaction was complete. The reaction solution was diluted with water (30 mL), and the aqueous phase was extracted with ethyl acetate (30 mL × 3). The organic phase was dried over anhydrous sodium sulfate, filtered, and distilled under reduced pressure to give a crude product, and the crude product was purified by column chromatography (SiO₂, EtOAc:Hexane = 1:10) to give the target product (406 mg, yield: 68.3%).

ESI-MS m/z: 453 [M+H]⁺.

### Step 3: Synthesis of compound int_522-5:

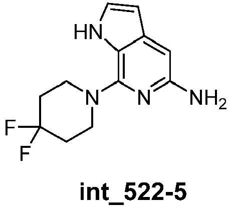

**int_522-4** (400 mg, 0.878 mmol) was dissolved in dichloromethane (10 mL), and trifluoroacetic acid (4 mL) was added at 0 °C. The mixture was heated to 25 °C and reacted for 2 h, and LC-MS monitoring showed the reaction was complete. A saturated sodium bicarbonate solution was added to the reaction solution at 0 °C to adjust the pH to 7. The aqueous phase was extracted with dichloromethane (30 mL × 3), and the organic phase was dried over anhydrous sodium sulfate, filtered, and distilled under reduced pressure to give a crude product. The crude product was purified by column chromatography to give a solid (220 mg, yield: 100%).

ESI-MS m/z: 253 [M+H]⁺.

### Step 4: Synthesis of compound int_522-6:

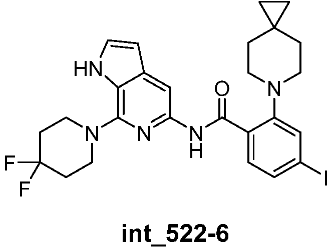

**int_1-5** (140 mg, 0.396 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_522-5** (100 mg, 0.396 mmol) was dissolved in DCM (10 mL), and DIPEA (255 mg, 1.98 mmol) was added under nitrogen atmosphere. Then the reaction solution was cooled to 0 °C, and the acyl chloride prepared above was slowly added to the reaction solution. The reaction solution was heated to room temperature and stirred for 1 h. LC-MS monitoring showed the reaction was complete. The reaction solution was concentrated under reduced pressure to give a crude product. The crude product was purified by column chromatography to give a solid (106 mg, yield: 45.2%).

ESI-MS m/z: 592 [M+H]⁺.

### Step 5: Synthesis of compound 522:

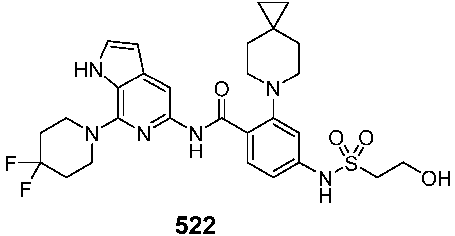

int_1-8 (45 mg, 0.358 mmol), dimethylcyclohexane-1,2-diatnine (13 mg, 0.0895 mmol), cuprous iodide (17 mg, 0.0895 mmol), and potassium phosphate (114 mg, 0.537 mmol) were dissolved in DMF (10 mL). The solution was purged with argon three times, and int_522-6 (106 mg, 0.179 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (10 mg, yield: 9.5%).

¹H NMR (400 MHz, DMSO-d6) δ 12.69 (s, 1H), 11.33 (s, 1H), 8.12 - 7.99 (m, 2H), 7.44 (t, *J* = 2.8 Hz, 1H), 7.21 (d, *J =* 2.1 Hz, 1H), 7.07 (dd, *J =* 8.5, 2.1 Hz, 1H), 6.44 (d, *J =* 2.9 Hz, 1H), 3.73 (t, *J* = 6.5 Hz, 2H), 3.52 (t, *J* = 5.7 Hz, 4H), 2.96 (s, 4H), 2.30 - 2.11 (m, 4H), 1.67 (s, 4H), 0.37 (s, 4H).

ESI-MS m/z: 589 [M+H]⁺.

### Example 7. Synthesis of Compound 530

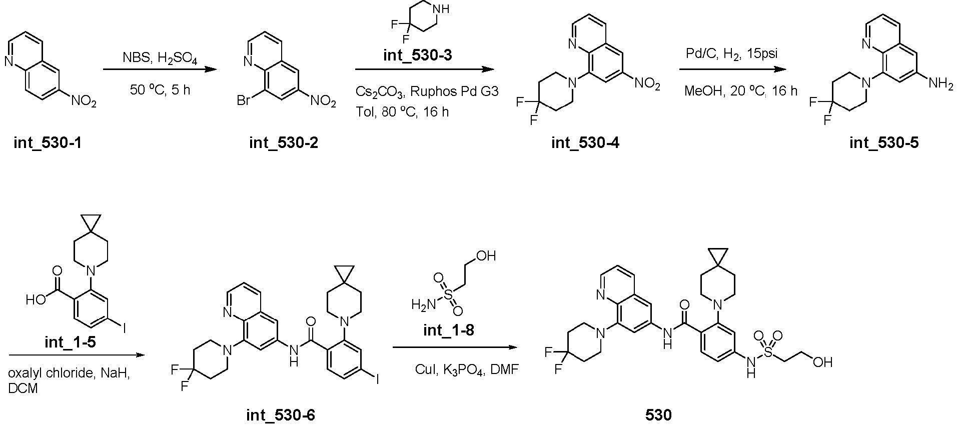

### Step 1: Synthesis of compound int_530-2:

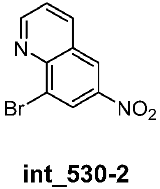

**int_530-1** (7.40 g, 42.5 mmol) was dissolved in concentrated sulfuric acid (50 mL), and NBS (8.32 g, 46.74 mmol) was added to the reaction solution. The reaction solution was heated to 50 °C and stirred for 5 h under nitrogen atmosphere. LC-MS monitoring showed the reaction was complete. The reaction solution was slowly added to ice water (200 mL). The aqueous phase was extracted with ethyl acetate (200 mL × 3), and the organic phase was dried over anhydrous sodium sulfate, filtered, and distilled under reduced pressure to give a crude product. The crude product was purified by column chromatography to give the target product (9 g, yield: 83.7%).

¹H NMR (400 MHz, CHLOROFORM-d) δ = 9.26 (dd, *J* = 1.6, 4.1 Hz, 1H), 8.89 (d, *J* = 2.5 Hz, 1H), 8.82 (d, *J =* 2.5 Hz, 1H), 8.46 - 8.40 (m, 1H), 7.69 (dd, *J =* 4.3, 8.3 Hz, 1H).

### Step 2: Synthesis of compound int_530-4:

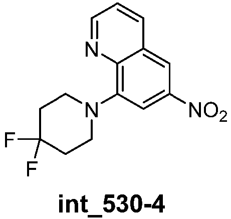

**int_522-2** (9.00 g, 35.6 mmol), **int_522-3** (6.46 g, 53.6 mmol), Cs₂CO₃ (23.1 g, 71.1 mmol), and RuPhos Pd G3 (2.97 g, 3.56 mmol) were dissolved in methylbenzene (100 mL), and the reaction solution was heated to 80 °C and stirred for 16 h under nitrogen atmosphere. LC-MS monitoring showed the reaction was complete. The reaction solution was diluted with water (200 mL), and the aqueous phase was extracted with ethyl acetate (200 mL × 3). The organic phase was dried over anhydrous sodium sulfate, filtered, and distilled under reduced pressure to give a crude product. The crude product was purified by column chromatography to give the target product (4 g, yield: 38.4%).

ESI-MS m/z: 294 [M+H]⁺.

### Step 3: Synthesis of compound int_530-5:

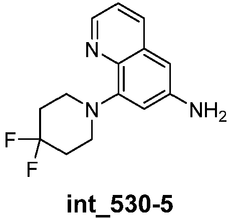

**int_530-4** (2.00 g, 6.82 mmol) was dissolved in methanol (30 mL), and Pd/C (1.00 g, 10% purity) was added. The reaction solution was reacted at 25 °C for 16 h under hydrogen atmosphere, and LC-MS monitoring showed the reaction was complete. The reaction solution was filtered, and the filtrate was distilled under reduced pressure to give a crude product. The crude product was purified by column chromatography to give a solid (2.2 g, yield: 61.3%).

¹H NMR (400 MHz, CHLOROFORM-d) δ = 8.57 (br d, *J* = 2.5 Hz, 1H), 7.79 (br d, *J* = 8.1 Hz, 1H), 7.23 - 7.13 (m, 2H), 6.60 - 6.46 (m, 2H), 4.05 - 3.63 (m, 2H), 3.39 (br t, *J* = 4.8 Hz, 4H), 2.35 - 2.14 (m, 4H).

### Step 4: Synthesis of compound int 530-6:

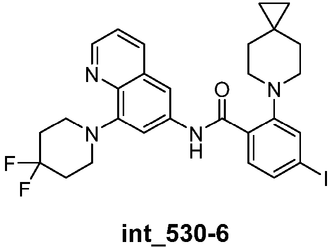

**int_1-5** (124 mg, 0.3 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_530-5** (80 mg, 0.3 mmol) was dissolved in DCM (10 mL), and NaH (72 mg, 1.8 mmol, 60% purity) was added under nitrogen atmosphere. Then the reaction was cooled to 0 °C, and the acyl chloride prepared above was slowly added to the reaction solution. The reaction solution was heated to 40 °C and stirred for 12 h. LC-MS monitoring showed the reaction was complete. The reaction solution was concentrated under reduced pressure to give a crude product. The crude product was purified by column chromatography to give a solid (58 mg, yield: 32.2%).

ESI-MS m/z: 603 [M+H]⁺

### Step 5: Synthesis of compound 530:

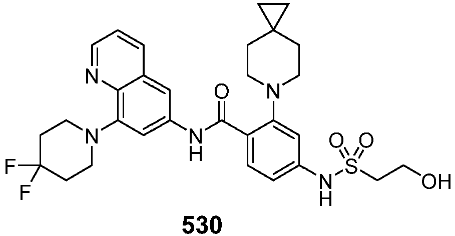

int_1-8 (19 mg, 0.15 mmol), dimethylcyclohexane-1,2-diamine (7 mg, 0.05 mmol), cuprous iodide (10 mg, 0.05 mmol), and potassium phosphate (63 mg, 0.3 mmol) were dissolved in DMF (5 mL). The solution was purged with argon three times, and **int_530-6** (58 mg, 0.1 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (14 mg, yield: 98.1%).

¹H NMR (400 MHz, CHLOROFORM-d) δ 12.84 (s, 1H), 8.85 - 8.75 (m, 1H), 8.27 (s, 1H), 8.21 (s, 1H), 8.13 (d, *J* = 8.2 Hz, 1H), 7.45 - 7.29 (m, 2H), 7.04 (d, *J* = 32.6 Hz, 2H), 4.15 (s, 2H), 3.55 (d, *J* = 6.4 Hz, 4H), 3.34 (s, 2H), 3.12 (s, 4H), 2.32 (d, *J* = 14.4 Hz, 4H), 1.71 (s, 4H), 0.45 (s, 4H).

ESI-MS m/z: 600 [M+H]⁺.

### Example 10. Synthesis of Compound 537

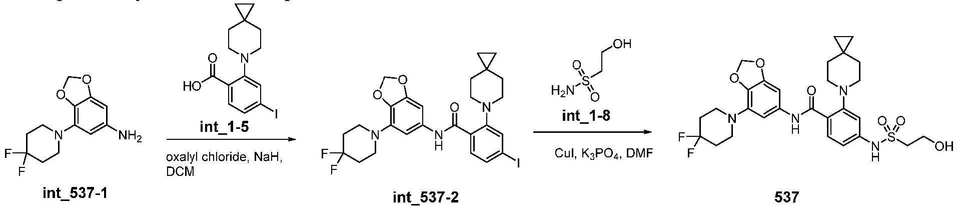

### Step 1: Synthesis of compound int_537-2:

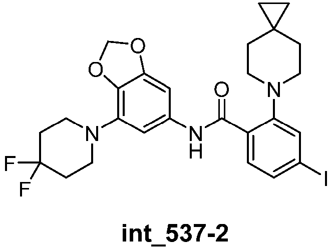

**int_1-5** (100 mg, 0.28 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_537-1** (71.7 mg, 0.28 mmol) was dissolved in DCM (10 mL), and NaH (112 mg, 2.8 mmol, 60% purity) was added under nitrogen atmosphere. Then the reaction solution was cooled to 0 °C, and the acyl chloride prepared above was slowly added to the reaction solution. The reaction solution was heated to 45 °C and stirred for 3 h. LC-MS monitoring showed the reaction was complete. The reaction solution was concentrated under reduced pressure to give a crude product. The crude product was purified by column chromatography to give a solid (127 mg, yield: 76.1%). ESI-MS m/z: 596 [M+H]⁺.

### Step 2: Synthesis of compound 537:

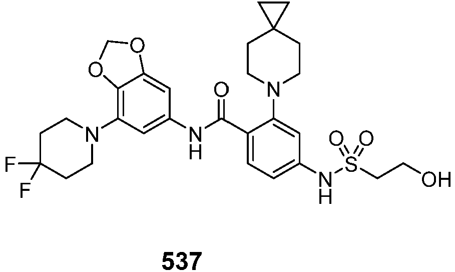

**int_1-8** (25 mg, 0.2 mmol), dimethylcyclohexane-1,2-diatnine (18 mg, 0.125 mmol), cuprous iodide (26 mg, 0.125 mmol), and potassium phosphate (164 mg, 0.747 mmol) were dissolved in DMF (5 mL). The solution was purged with argon three times, and **int_537-2** (120 mg, 0.2 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (76 mg, yield: 64.1%).

ESI-MS m/z: 593 [M+H]⁺.

### Example 9. Synthesis of Compound 539

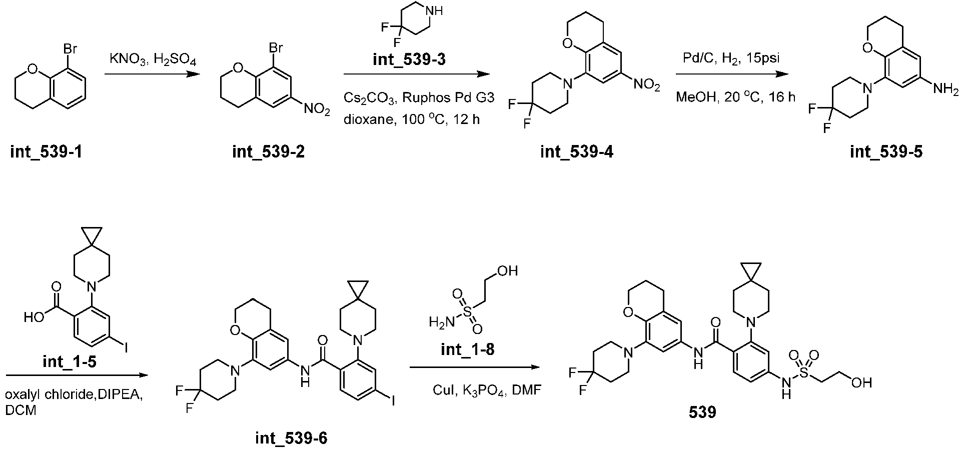

### Step 1: Synthesis of compound int_539-2:

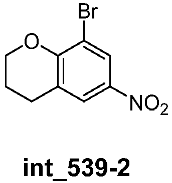

**int_539-1** (500 mg, 2.34 mmol) was dissolved in concentrated sulfuric acid (6 mL), and KNOs (356 mg, 3.51 mmol) was added to the reaction solution. The reaction solution was heated to room temperature and stirred for 2 h under nitrogen atmosphere. LC-MS monitoring showed the reaction was complete. The reaction solution was slowly added to ice water (50 mL). The aqueous phase was extracted with dichloromethane (50 mL × 3), and the organic phase was dried over anhydrous sodium sulfate, filtered, and distilled under reduced pressure to give a crude product. The crude product was purified by column chromatography to give the target product (395 mg, yield: 65.2%).

ESI-MS m/z: 258 [M+H]⁺.

### Step 2: Synthesis of compound int_539-4:

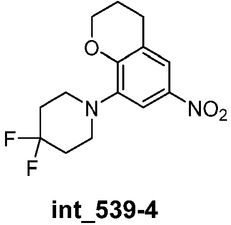

**int_539-2** (300 mg, 1.16 mmol), **int_539-3** (155 mg, 1.27 mmol), Cs₂CO₃ (756 mg, 2.32 mmol), and RuPhos Pd G3 (97 mg, 0.116 mmol) were dissolved in 1,4-dioxane (20 mL), and the reaction solution was heated to 100 °C and stirred for 12 h under nitrogen atmosphere. LC-MS monitoring showed the reaction was complete. The reaction solution was diluted with water (20 mL), and the aqueous phase was extracted with ethyl acetate (20 mL × 3). The organic phase was dried over anhydrous sodium sulfate, filtered, and distilled under reduced pressure to give a crude product. The crude product was purified by column chromatography to give the target product (200 mg, yield: 44.4%).

ESI-MS m/z: 299 [M+H]⁺.

### Step 3: Synthesis of compound int_539-5:

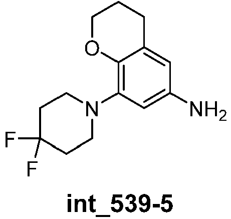

**int_530-4** (200 mg, 0.67 mmol) was dissolved in methanol (15 mL), and Pd/C (200 mg, 10% purity) was added. The reaction solution was reacted at 25 °C for 16 h under hydrogen atmosphere, and LC-MS monitoring showed the reaction was complete. The reaction solution was filtered, and the filtrate was distilled under reduced pressure to give a crude product. The crude product was purified by column chromatography to give a solid (140 mg, yield: 78.2%).

ESI-MS m/z: 269 [M+H]⁺.

### Step 4: Synthesis of compound int_539-6:

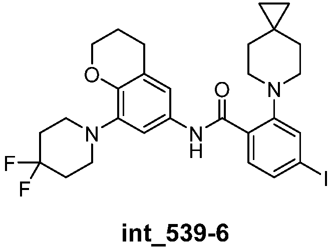

**int_1-5** (200 mg, 0.559 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_539-5** (120 mg, 0.447 mmol) was dissolved in DCM (10 mL), and DIPEA (173 mg, 1.34 mmol) was added under nitrogen atmosphere. Then the reaction solution was cooled to 0 °C, and the acyl chloride prepared above was slowly added to the reaction solution. The reaction solution was heated to 40 °C and stirred at room temperature for 1 h. LC-MS monitoring showed the reaction was complete. The reaction solution was concentrated under reduced pressure to give a crude product. The crude product was purified by column chromatography to give a solid (235 mg, yield: 74.3%).

ESI-MS m/z: 608 [M+H]⁺.

### Step 5: Synthesis of compound 539:

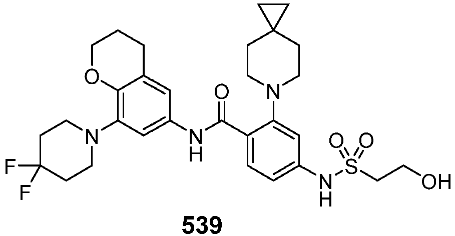

int_1-8 (97 mg, 0.773 mmol), dimethylcyclohexane-1,2-diamine (27 mg, 0.193 mmol), cuprous iodide (37 mg, 0.193 mmol), and potassium phosphate (246 mg, 1.158 mmol) were dissolved in DMF (15 mL). The solution was purged with argon three times, and **int_539-6** (235 mg, 0.386 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (160 mg, yield: 68.6%).

¹H NMR (400 MHz, DMSO-d6) δ 11.51 (s, 1H), 7.80 (d, *J* = 8.5 Hz, 1H), 7.28 (d, *J* = 2.3 Hz, 1H), 7.13 (d, *J* = 2.1 Hz, 1H), 7.03 (d, *J* = 2.4 Hz, 1H), 7.00 (dd, *J* = 8.5, 2.1 Hz, 1H), 4.17 - 4.10 (m, 2H), 3.73 (t, *J* = 6.6 Hz, 2H), 3.31 (s, 2H), 3.05 (t, *J* = 5.5 Hz, 4H), 2.93 (t, *J* = 5.3 Hz, 4H), 2.72 (t, *J* = 6.5 Hz, 2H), 2.06 (q, *J* = 6.0 Hz, 4H), 1.90 (p, *J* = 6.1 Hz, 2H), 1.53 (t, *J* = 5.3 Hz, 4H), 0.33 (s, 4H).

ESI-MS m/z: 605 [M+H]⁺.

### Example 10. Synthesis of Compound 583

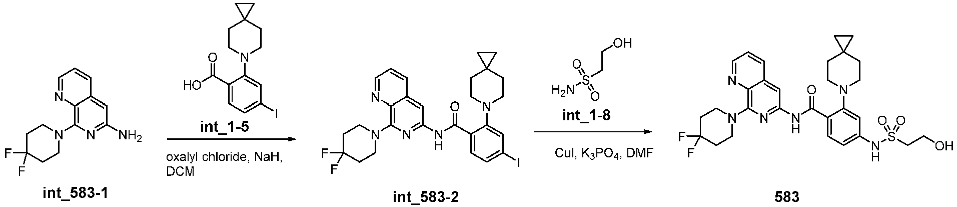

### Step 1: Synthesis of compound int_583-2:

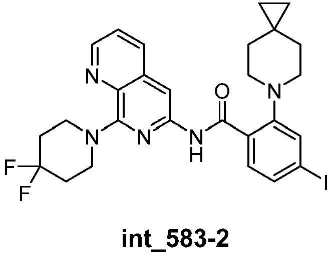

**int_1-5** (100 mg, 0.28 mmol) was dissolved in DCM (10 mL), and oxalyl chloride (253.8 mg, 2 mmol) was added. The reaction solution was stirred at room temperature for 2 h and then concentrated under reduced pressure to remove the solvent, thus giving an acyl chloride solid. **int_530-5** (74 mg, 0.28 mmol) was dissolved in DCM (10 mL), and NaH (112 mg, 2.8 mmol, 60% purity) was added under nitrogen atmosphere. Then the reaction solution was cooled to 0 °C, and the acyl chloride prepared above was slowly added to the reaction solution. The reaction solution was heated to 45 °C and stirred for 3 h. LC-MS monitoring showed the reaction was complete. The reaction solution was concentrated under reduced pressure to give a crude product. The crude product was purified by column chromatography to give a solid (150 mg, yield: 88.8%).

ESI-MS m/z: 604 [M+H]⁺.

### Step 2: Synthesis of compound 583:

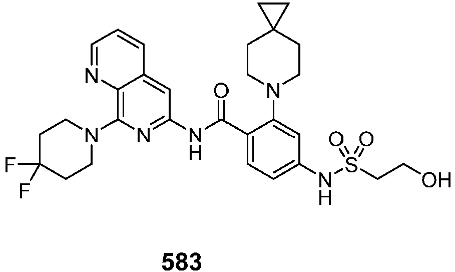

**int_1-8** (34 mg, 0.249 mmol), dimethylcyclohexane-1,2-diatnine (18 mg, 0.125 mmol), cuprous iodide (26 mg, 0.125 mmol), and potassium phosphate (164 mg, 0.747 mmol) were dissolved in DMF (5 mL). The solution was purged with argon three times, and **int_583-2** (150 mg, 0.249 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (57 mg, yield: 38%).

¹H NMR (400 MHz, DMSO-d6) δ 12.93 (s, 1H), 8.70 (dd, *J* = 4.2, 1.8 Hz, 1H), 8.22 (dd, *J* = 8.5, 1.8 Hz, 1H), 8.09 (s, 1H), 8.05 (d, *J* = 8.6 Hz, 1H), 7.59 (dd, *J* = 8.4, 4.1 Hz, 1H), 7.21 (d, *J* = 2.1 Hz, 1H), 7.07 (dd, *J =* 8.7, 2.1 Hz, 1H), 4.17 (t, *J* = 5.3 Hz, 4H), 3.73 (t, *J* = 6.5 Hz, 2H), 2.99 (d, *J* = 6.7 Hz, 4H), 2.15 (dq, *J* = 14.0, 7.2, 5.8 Hz, 4H), 1.73 (s, 4H), 0.39 (s, 4H).

ESI-MS m/z: 601 [M+H]⁺.

### Example 11. Synthesis of Compound 584

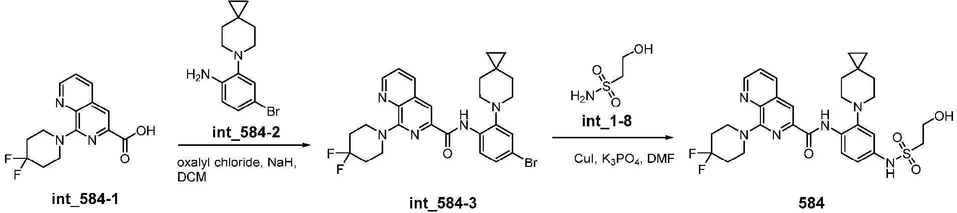

### Step 1: Synthesis of compound int_584-3:

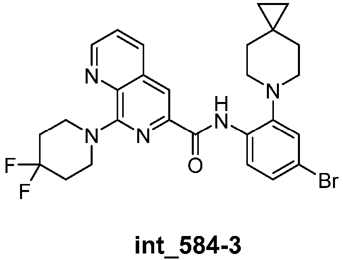

**int_584-1** (100 mg, 0.341 mmol) was dissolved in DCM (5 mL), and oxalyl chloride (253.8 mg, 2 mmol) was added. The reaction solution was stirred at room temperature for 2 h and then concentrated under reduced pressure to remove the solvent, thus giving an acyl chloride solid. **int_584-2** (90 mg, 0.321 mmol) was dissolved in THF (5 mL), and NaH (112 mg, 2.8 mmol, 60% purity) was added under nitrogen atmosphere. Then the reaction solution was cooled to 0 °C, and the acyl chloride prepared above was slowly added to the reaction solution. The reaction solution was heated to 45 °C and stirred for 12 h. LC-MS monitoring showed the reaction was complete. The reaction solution was concentrated under reduced pressure to give a crude product. The crude product was purified by column chromatography to give a solid (150 mg, yield: 83.8%).

ESI-MS m/z: 556 [M+H]⁺.

### Step 2: Synthesis of compound 584:

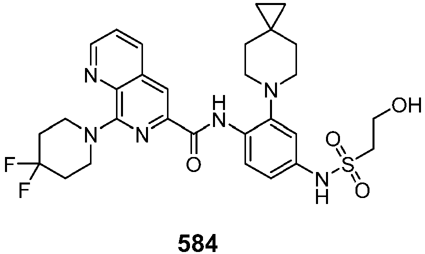

**int_1-8** (84 mg, 0.674 mmol), dimethylcyclohexane-1,2-diatnine (32 mg, 0.225 mmol), cuprous iodide (43 mg, 0.225 mmol), and potassium phosphate (286 mg, 1.348 mmol) were dissolved in DMF (10 mL). The solution was purged with argon three times, and **int_584-3** (150 mg, 0.270 mmol) was added. Under argon atmosphere, the reaction solution was heated to 100 °C and reacted for 16 h. LC-MS monitoring showed the reaction was complete. The reaction solution was cooled to room temperature, concentrated to dryness by rotary evaporation, and purified by column chromatography to give a solid (50 mg, yield: 30.9%).

¹H NMR (400 MHz, DMSO-d6) δ 9.67 (s, 1H), 8.97 (dt, *J =* 4.1, 2.1 Hz, 1H), 8.47 (dd, *J =* 8.3, 1.9 Hz, 1H), 8.18 (d, *J* = 2.0 Hz, 1H), 7.98 (d, *J* = 8.6 Hz, 1H), 7.72 - 7.58 (m, 2H), 7.11 (d, *J =* 2.4 Hz, 1H), 6.98 (dd, *J =* 8.6, 2.3 Hz, 1H), 3.73 (t, *J* = 6.8 Hz, 2H), 3.55 (t, *J* = 5.5 Hz, 4H), 3.22 (t, *J* = 6.7 Hz, 2H), 2.86 (t, *J* = 5.2 Hz, 4H), 2.24 (q, *J* = 12.2 Hz, 4H), 1.51 (s, 4H), 0.32 (d, *J* = 2.3 Hz, 4H).

### Example 12. Synthesis of Compound 595

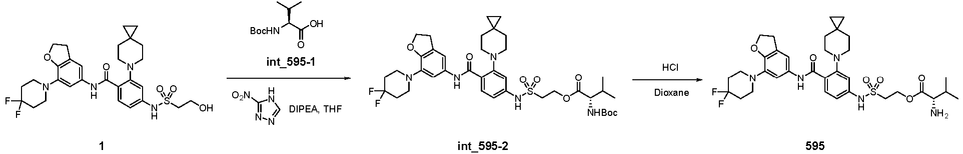

### Step 1: Synthesis of compound int_595-2

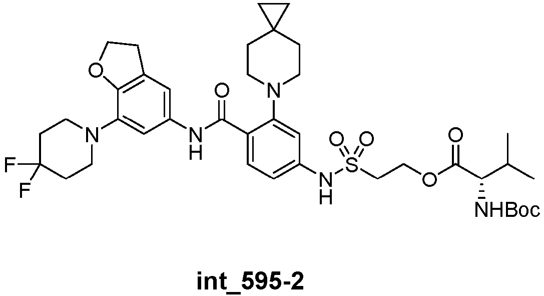

1 (0.50 g, 846 µmol) was dissolved in tetrahydrofuran (10 mL), and **int_595-1** (919 mg, 4.23 mmol), BOPCl (538 mg, 2.12 mmol), 3-nitro-4H-1,2,4-triazole (241 mg, 2.12 mmol), and DIPEA (547 mg, 4.23 mmol) were added. The mixture was reacted at room temperature for 4 h, and LC-MS monitoring showed the reaction was complete. The reaction solution was filtered to give a filtrate, and the filtrate was concentrated under reduced pressure to give a crude product. The crude product was purified by column chromatography (SiO₂, PE/EtOAc = 10/0 to 1/1) to give the target product (0.6 g, yield: 89.7%).

ESI-MS m/z: 790 [M+H]⁺.

### Step 2: Synthesis of compound 595

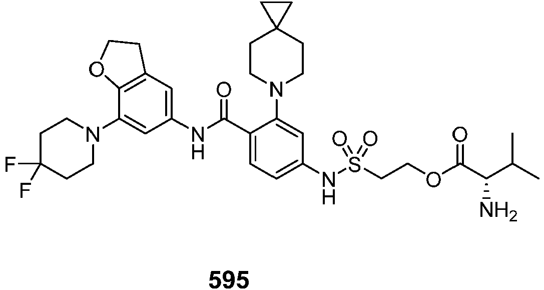

**int_595-2** (0.60 g, 759 µmol) was dissolved in a HCl/dioxane solution (4 M, 6 mL). The solution was reacted at room temperature for 2 h, and LC-MS monitoring showed the reaction was complete. The reaction solution was concentrated under reduced pressure to give a crude product, and a saturated NaHCOs solution (6 mL) was added to the crude product to adjust the pH to 7-8. The aqueous phase was extracted with dichloromethane (6 mL × 3), and the organic phases were combined, dried over anhydrous sodium sulfate, filtered, and distilled under reduced pressure to give the target product (490 mg, yield: 90.3%).

¹H NMR (400 MHz, CHLOROFORM-d) δ 12.13 (s, 1H), 8.17 (d, *J* = 8.5 Hz, 1H), 7.31 (s, 1H), 7.26 (d, *J* = 1.9 Hz, 1H), 7.06 (s, 1H), 6.98 (dd, *J* = 1.9, 8.5 Hz, 1H), 4.60 - 4.46 (m, 4H), 3.43 - 3.35 (m, 2H), 3.27 - 3.20 (m, 5H), 3.17 (t, *J* = 8.8 Hz, 2H), 3.00 (br t, *J* = 5.1 Hz, 4H), 2.15 - 1.92 (m, 5H), 1.58 (br s, 4H), 0.90 (dd, *J* = 6.9, 11.5 Hz, 6H), 0.35 (s, 4H).

ESI-MS m/z: 690 [M+H]⁺.

### Example 13. Synthesis of Compound 611

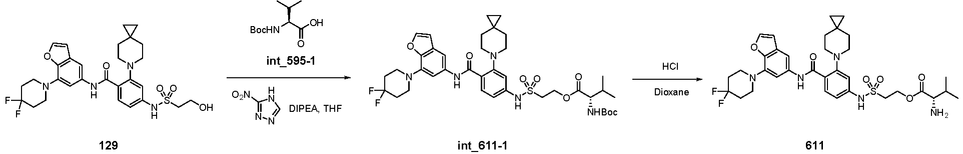

### Step 1: Synthesis of compound int_611-1

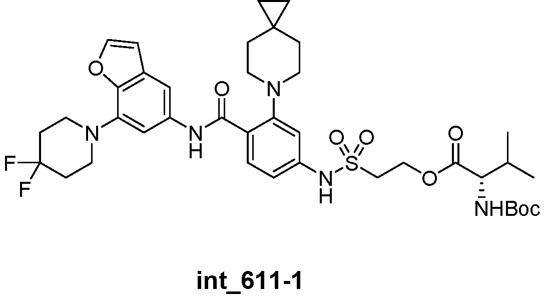

**385** (0.58 g, 985 µmol) was dissolved in tetrahydrofuran (10 mL), and **int_595-1** (1.07 g, 4.93 mmol), BOPCl (627 mg, 2.46 mmol), 3-nitro-4H-1,2,4-triazole (281 mg, 2.46 mmol), and DIPEA (637 mg, 4.93 mmol) were added. The mixture was reacted at room temperature for 4 h, and LC-MS monitoring showed the reaction was complete. The reaction solution was filtered to give a filtrate, and the filtrate was concentrated under reduced pressure to give a crude product. The crude product was purified by column chromatography (SiO₂, PE/EtOAc = 5/1 to 3/1) to give the target product (0.17 g, yield: 38.5%).

ESI-MS m/z: 788 [M+H]⁺.

### Step 2: Synthesis of compound 611

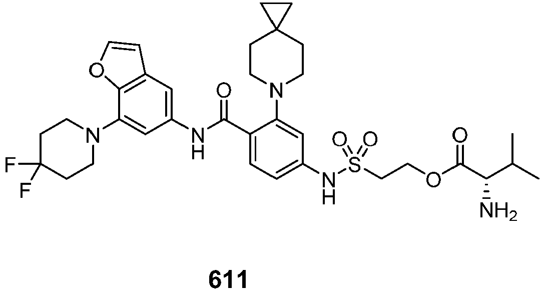

**int_611-1** (0.93 g, 1.18 mmol) was dissolved in a HCl/dioxane solution (4 M, 2.95 mL). The solution was reacted at room temperature for 0.5 h, and LC-MS monitoring showed the reaction was complete. The reaction solution was concentrated under reduced pressure to give a crude product, and a saturated NaHCOs solution (10 mL) was added to the crude product to adjust the pH to 7-8. The aqueous phase was extracted with dichloromethane (10 mL × 3), and the organic phases were combined, dried over anhydrous sodium sulfate, filtered, and distilled under reduced pressure to give the target product (130 mg, yield: 28.5%).

¹H NMR (400 MHz, DMSO-d6) δ 12.29 (s, 1H), 8.20 - 8.18 (d, *J* = 8.4 Hz, 1H), 7.62 (s, 1H), 7.54 (s, 1H), 7.28 (d, *J* = 2.0 Hz, 1H), 7.19 - 7.14 (m, 1H), 7.08 (d, *J* = 2.1 Hz, 1H), 6.69 (d, *J* = 2.0 Hz, 1H), 4.51 (t, *J* = 5.8 Hz, 2H), 3.45 (m, 4H), 3.39 (m, 2H), 3.25 - 3.16 (m, 1H), 3.12 (br s, 4H), 2.39 - 2.22 (m, 4H), 2.01 - 1.83 (m, 1H), 1.81 - 1.64 (m, 4H), 0.97 - 0.87 (m, 6H), 0.48 (s, 4H).

ESI-MS m/z: 688 [M+H]⁺.

### Example 14. Synthesis of Compound 643

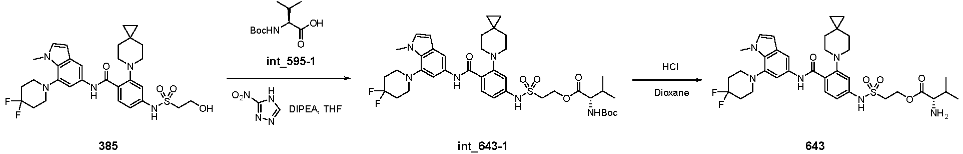

### Step 1: Synthesis of compound int_643-1

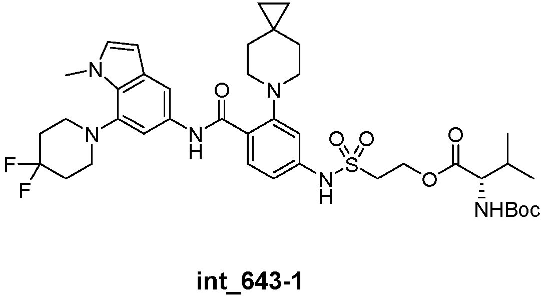

**385** (0.50 g, 831 µmol) was dissolved in tetrahydrofuran (10 mL), and **int_595-1** (902 mg, 4.15 mmol), BOPCl (528 mg, 2.08 mmol), 3-nitro-4*H*-1,2,4-triazole (237 mg, 2.08 mmol), and DIPEA (537 mg, 4.15 mmol) were added. The mixture was reacted at room temperature for 4 h, and LC-MS monitoring showed the reaction was complete. The reaction solution was filtered to give a filtrate, and the filtrate was concentrated under reduced pressure to give a crude product. The crude product was purified by column chromatography (SiO₂, PE/EtOAc = 10/0 to 1/1) to give the target product (0.5 g, yield: 75.1%).

ESI-MS m/z: 801 [M+H]⁺.

### Step 2: Synthesis of compound 643

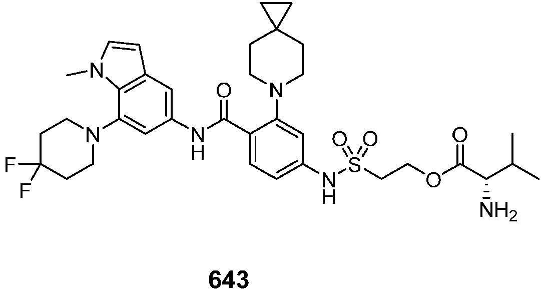

**int_643-1** (0.50 g, 624.3 µmol) was dissolved in a HCl/dioxane solution (4 M, 5 mL). The solution was reacted at room temperature for 2 h, and LC-MS monitoring showed the reaction was complete. The reaction solution was concentrated under reduced pressure to give a crude product, and a saturated NaHCOs solution (5 mL) was added to the crude product to adjust the pH to 7-8. The aqueous phase was extracted with dichloromethane (5 mL × 3), and the organic phases were combined, dried over anhydrous sodium sulfate, filtered, and distilled under reduced pressure to give the target product (130 mg, yield: 28.5%).

¹H NMR (400 MHz, CHLOROFORM-d) δ 12.27 (s, 1H), 8.29 (d, *J* = 8.5 Hz, 1H), 7.86 (d, *J* = 1.6 Hz, 1H), 7.44 (d, *J =* 1.6 Hz, 1H), 7.37 (d, *J =* 1.6 Hz, 1H), 7.11 (br d, *J* = 7.0 Hz, 1H), 7.01 - 6.97 (m, 1H), 6.51 - 6.47 (m, 1H), 4.73 - 4.50 (m, 2H), 4.18 - 4.09 (m, 3H), 3.57 - 3.47 (m, 2H), 3.35 (br d, *J* = 10.4 Hz, 2H), 3.11 (br t, *J* = 5.2 Hz, 7H), 2.13 (br s, 5H), 1.65 (br s, 4H), 1.06 - 0.96 (m, 6H), 0.46 - 0.37 (m, 4H).

ESI-MS m/z: 701 [M+H]⁺.

The target compounds **2-64, 66-128, 130-384, 386-512, 514-521, 523-529, 531-536, 538, 540-582, 585-594, 596-610, 612-642, and 644-814** in **Table 1** were obtained by using the above synthesis methods with different starting materials.

**Table 1**

| **Compound** | **Compound structure** | **MS (M+H)⁺** | **Compound** | **Compound structure** | **MS (M+H)⁺** |
|---|---|---|---|---|---|
| **2** | 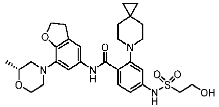 | 571 | **3** | 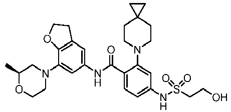 | 571 |
| **4** | 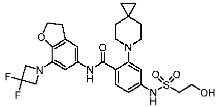 | 563 | **5** | 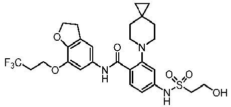 | 584 |
| **6** | 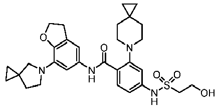 | 567 | **7** | 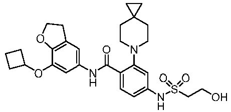 | 542 |
| **8** | 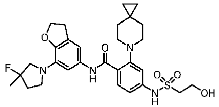 | 573 | **9** | 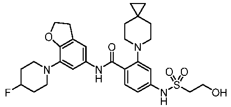 | 573 |
| **10** | 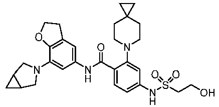 | 553 | **11** | 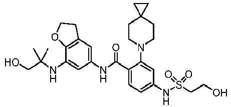 | 559 |
| **12** | 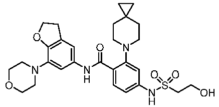 | 557 | **13** | 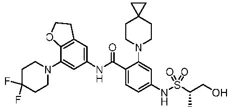 | 605 |
| **14** | 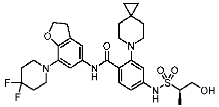 | 605 | **15** | 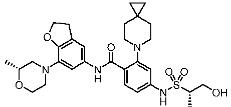 | 585 |
| **16** | 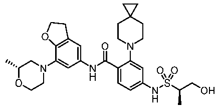 | 585 | **17** | 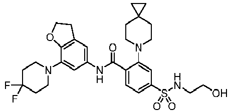 | 591 |
| **18** | 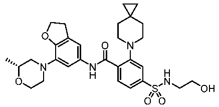 | 571 | **19** | 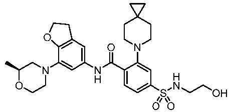 | 571 |
| **20** | 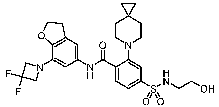 | 563 | **21** | 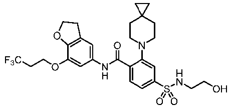 | 584 |
| **22** | 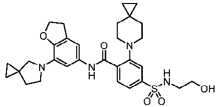 | 567 | **23** | 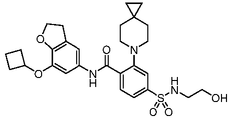 | 542 |
| **24** | 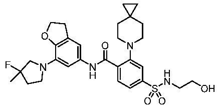 | 573 | **25** | 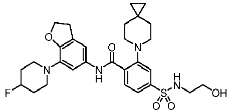 | 573 |
| **26** | 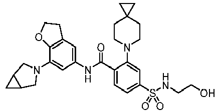 | 553 | **27** | 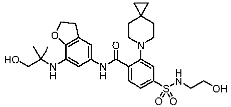 | 559 |
| **28** | 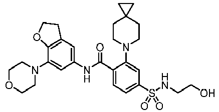 | 557 | **29** | 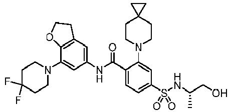 | 605 |
| **30** | 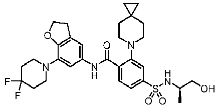 | 605 | **31** | 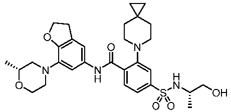 | 585 |
| **32** | 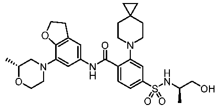 | 585 | **33** | 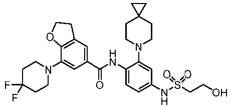 | 591 |
| **34** | 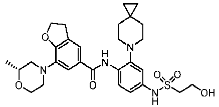 | 571 | **35** | 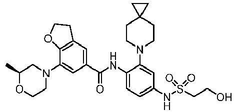 | 571 |
| **36** | 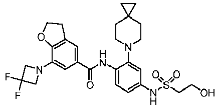 | 563 | **37** | 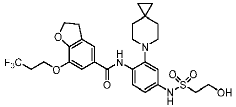 | 584 |
| **38** | 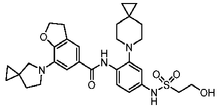 | 567 | **39** | 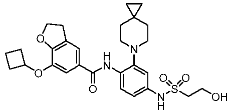 | 542 |
| **40** | 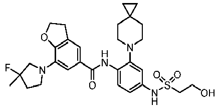 | 573 | **41** | 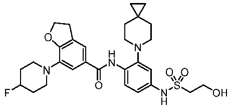 | 573 |
| **42** | 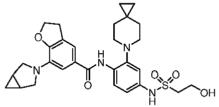 | 553 | **43** | 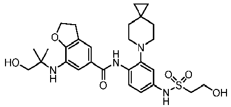 | 559 |
| **44** | 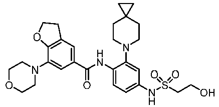 | 557 | **45** | 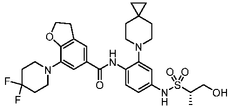 | 605 |
| **46** | 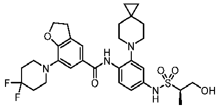 | 605 | **47** | 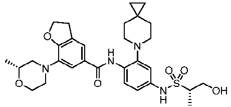 | 585 |
| **48** | 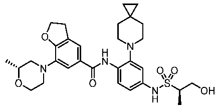 | 585 | **49** | 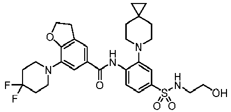 | 591 |
| **50** | 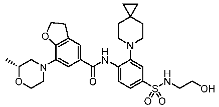 | 571 | **51** | 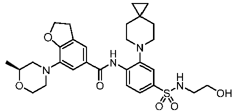 | 571 |
| **52** | 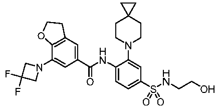 | 563 | **53** | 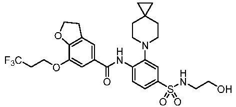 | 584 |
| **54** | 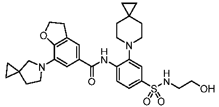 | 567 | **55** | 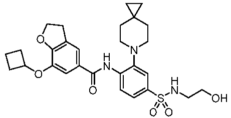 | 542 |
| **56** | 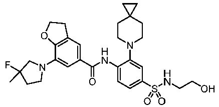 | 573 | **57** | 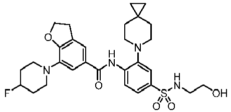 | 574 |
| **58** | 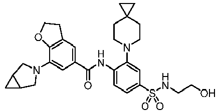 | 553 | **59** | 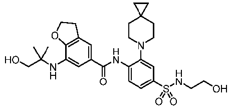 | 559 |
| **60** | 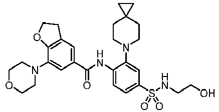 | 557 | **61** | 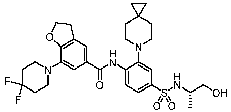 | 605 |
| **62** | 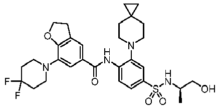 | 605 | **63** | 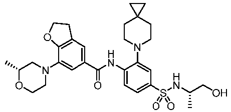 | 585 |
| **64** | 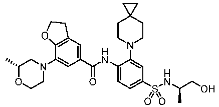 | 585 | **65** | 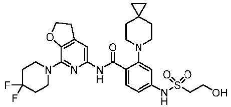 | 592 |
| **66** | 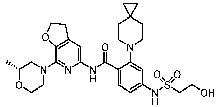 | 572 | **67** | 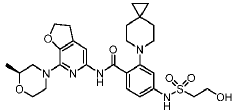 | 572 |
| **68** | 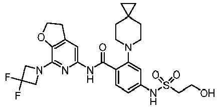 | 564 | **69** | 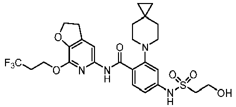 | 585 |
| **70** | 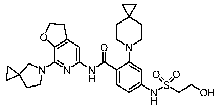 | 568 | **71** | 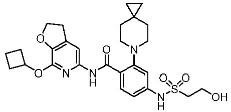 | 543 |
| **72** | 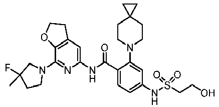 | 574 | **73** | 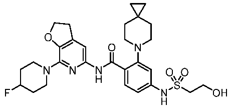 | 574 |
| **74** | 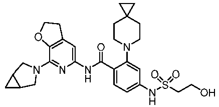 | 554 | **75** | 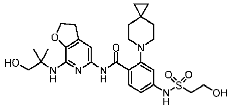 | 560 |
| **76** | 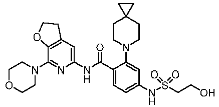 | 558 | **77** | 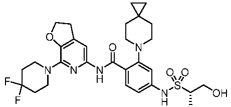 | 606 |
| **78** | 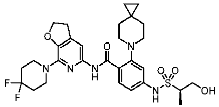 | 606 | **79** | 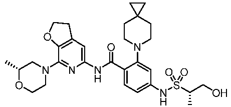 | 586 |
| **80** | 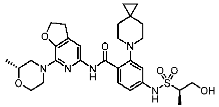 | 586 | **81** | 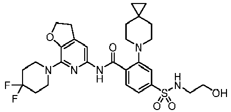 | 592 |
| **82** | 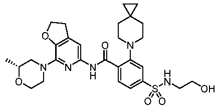 | 572 | **83** | 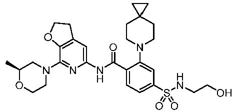 | 572 |
| **84** | 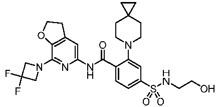 | 564 | **85** | 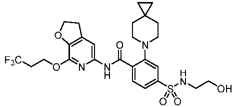 | 585 |
| **86** | 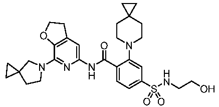 | 568 | **87** | 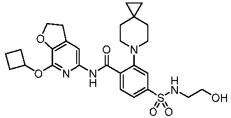 | 543 |
| **88** | 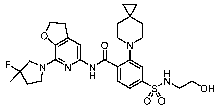 | 574 | **89** | 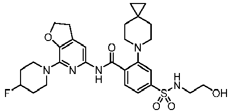 | 574 |
| **90** | 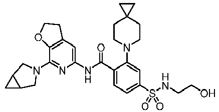 | 554 | **91** | 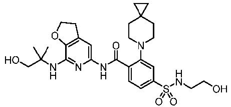 | 560 |
| **92** | 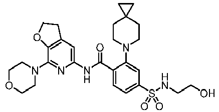 | 558 | **93** | 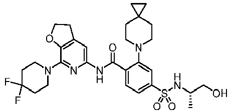 | 606 |
| **94** | 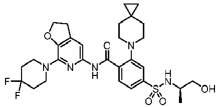 | 606 | **95** | 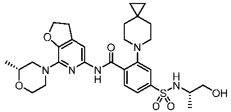 | 586 |
| **96** | 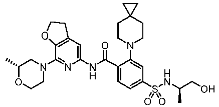 | 586 | **97** | 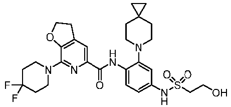 | 592 |
| **98** | 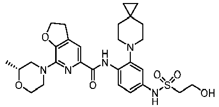 | 572 | **99** | 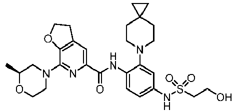 | 572 |
| **100** | 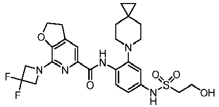 | 564 | **101** | 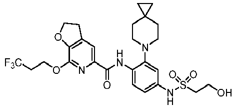 | 585 |
| **102** | 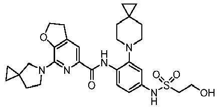 | 568 | **103** | 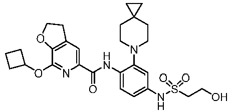 | 543 |
| **104** | 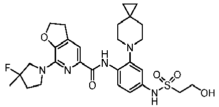 | 574 | **105** | 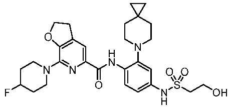 | 574 |
| **106** | 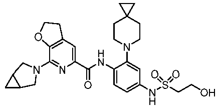 | 554 | **107** | 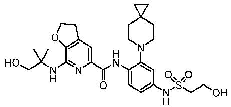 | 560 |
| **108** | 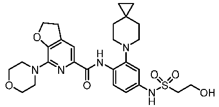 | 558 | **109** | 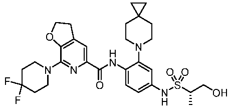 | 606 |
| **110** | 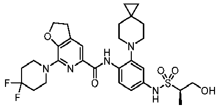 | 606 | **111** | 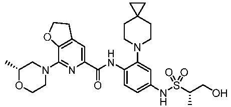 | 586 |
| **112** | 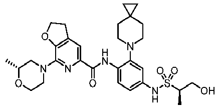 | 586 | **113** | 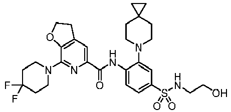 | 592 |
| **114** | 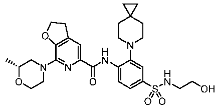 | 572 | **115** | 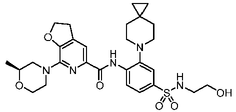 | 572 |
| **116** | 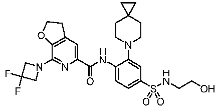 | 564 | **117** | 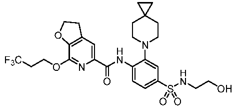 | 585 |
| **118** | 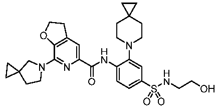 | 568 | **119** | 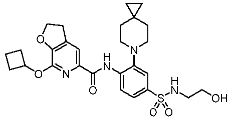 | 543 |
| **120** | 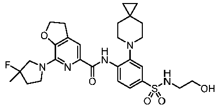 | 574 | **121** | 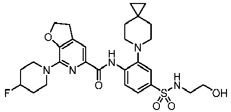 | 574 |
| **122** | 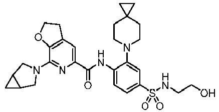 | 554 | **123** | 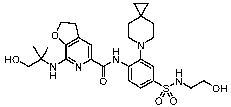 | 560 |
| **124** | 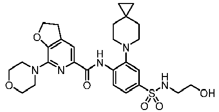 | 558 | **125** | 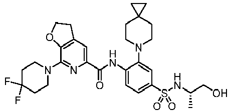 | 606 |
| **126** | 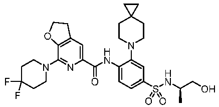 | 606 | **127** | 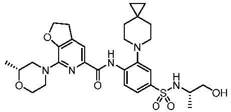 | 586 |
| **128** | 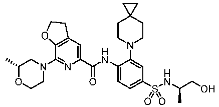 | 586 | **129** | 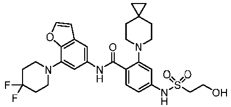 | 589 |
| **130** | 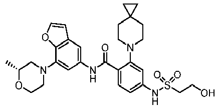 | 569 | **131** | 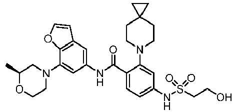 | 569 |
| **132** | 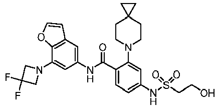 | 561 | **133** | 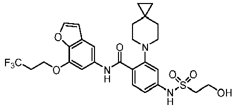 | 582 |
| **134** | 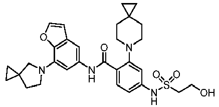 | 565 | **135** | 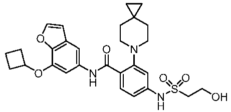 | 540 |
| **136** | 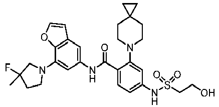 | 571 | **137** | 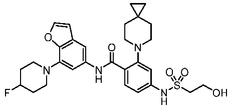 | 571 |
| **138** | 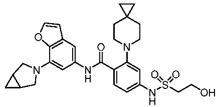 | 551 | **139** | 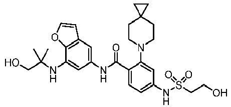 | 557 |
| **140** | 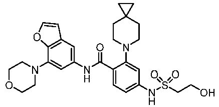 | 555 | **141** | 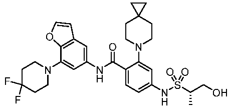 | 603 |
| **142** | 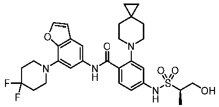 | 603 | **143** | 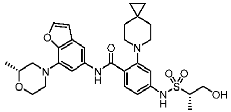 | 583 |
| **144** | 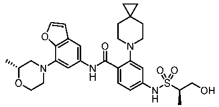 | 583 | **145** | 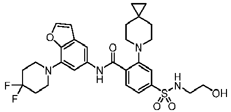 | 589 |
| **146** | 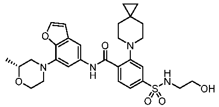 | 569 | **147** | 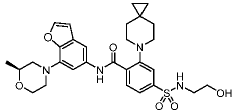 | 569 |
| **148** | 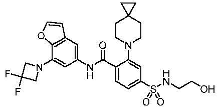 | 561 | **149** | 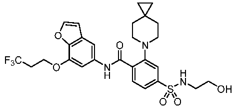 | 582 |
| **150** | 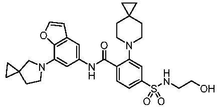 | 565 | **151** | 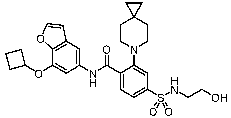 | 540 |
| **152** | 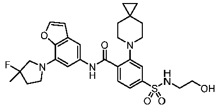 | 571 | **153** | 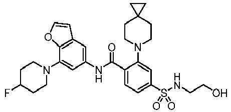 | 571 |
| **154** | 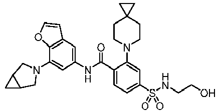 | 551 | **155** | 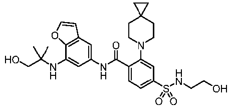 | 557 |
| **156** | 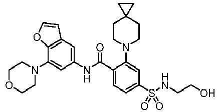 | 555 | **157** | 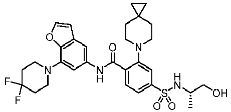 | 603 |
| **158** | 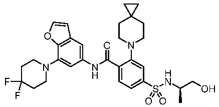 | 603 | **159** | 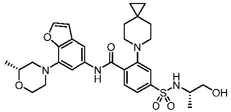 | 583 |
| **160** | 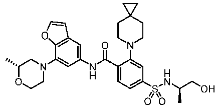 | 583 | **161** | 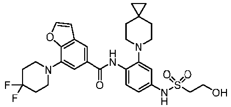 | 589 |
| **162** | 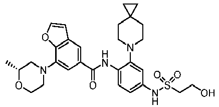 | 569 | **163** | 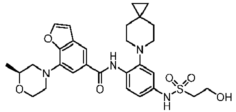 | 569 |
| **164** | 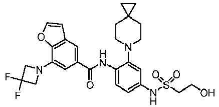 | 561 | **165** | 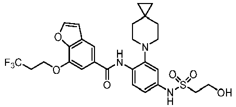 | 582 |
| **166** | 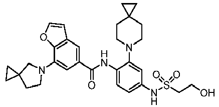 | 565 | **167** | 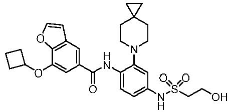 | 540 |
| **168** | 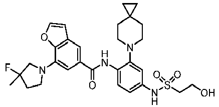 | 571 | **169** | 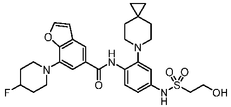 | 571 |
| **170** | 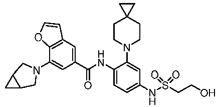 | 551 | **171** | 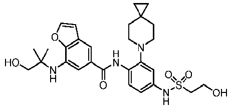 | 557 |
| **172** | 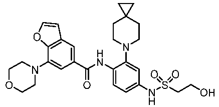 | 555 | **173** | 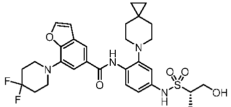 | 603 |
| **174** | 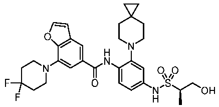 | 603 | **175** | 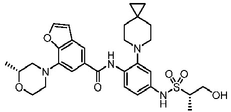 | 583 |
| **176** | 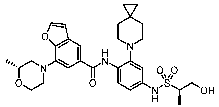 | 583 | **177** | 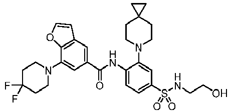 | 589 |
| **178** | 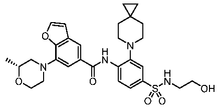 | 569 | **179** | 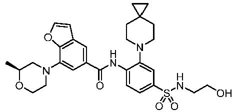 | 569 |
| **180** | 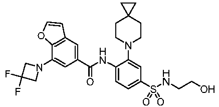 | 561 | **181** | 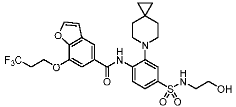 | 582 |
| **182** | 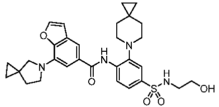 | 565 | **183** | 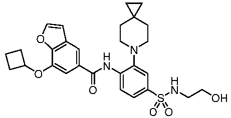 | 540 |
| **184** | 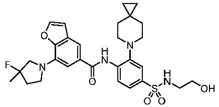 | 571 | **185** | 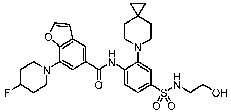 | 571 |
| **186** | 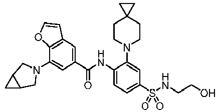 | 551 | **187** | 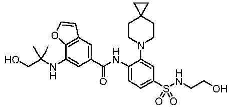 | 557 |
| **188** | 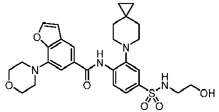 | 555 | **189** | 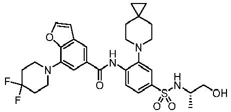 | 603 |
| **190** | 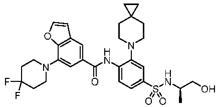 | 603 | **191** | 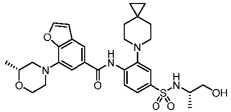 | 583 |
| **192** | 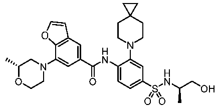 | 583 | **193** | 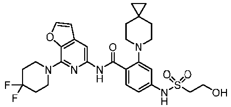 | 590 |
| **194** | 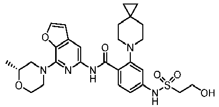 | 570 | **195** | 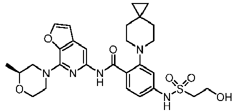 | 570 |
| **196** | 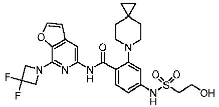 | 562 | **197** | 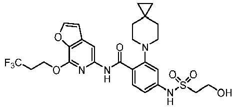 | 583 |
| **198** | 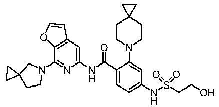 | 566 | **199** | 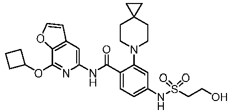 | 541 |
| **200** | 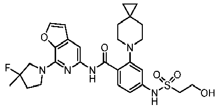 | 572 | **201** | 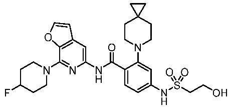 | 572 |
| **202** | 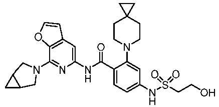 | 552 | **203** | 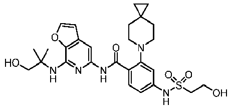 | 558 |
| **204** | 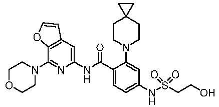 | 556 | **205** | 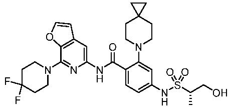 | 604 |
| **206** | 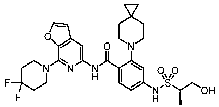 | 604 | **207** | 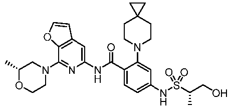 | 584 |
| **208** | 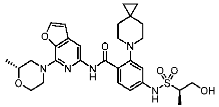 | 584 | **209** | 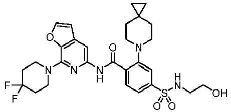 | 590 |
| **210** | 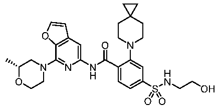 | 570 | **211** | 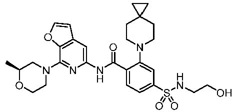 | 570 |
| **212** | 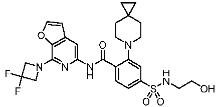 | 562 | **213** | 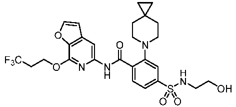 | 583 |
| **214** | 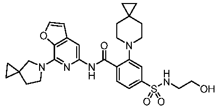 | 566 | **215** | 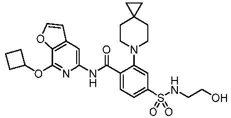 | 541 |
| **216** | 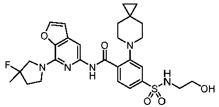 | 572 | **217** | 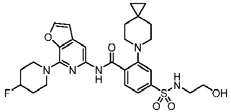 | 572 |
| **218** | 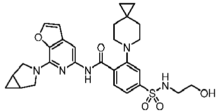 | 552 | **219** | 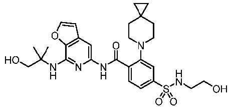 | 558 |
| **220** | 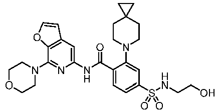 | 556 | **221** | 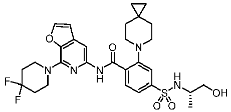 | 604 |
| **222** | 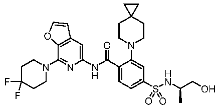 | 604 | **223** | 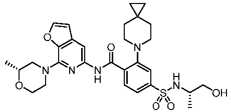 | 584 |
| **224** | 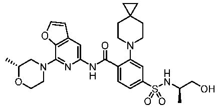 | 584 | **225** | 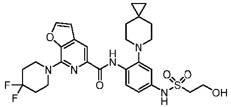 | 590 |
| **226** | 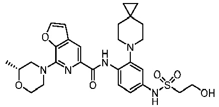 | 570 | **227** | 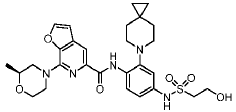 | 570 |
| **228** | 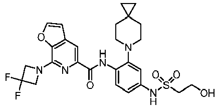 | 562 | **229** | 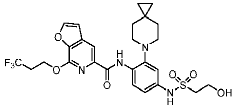 | 583 |
| **230** | 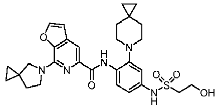 | 566 | **231** | 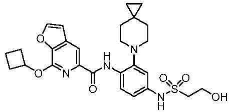 | 541 |
| **232** | 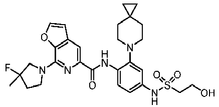 | 572 | **233** | 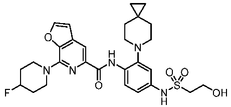 | 572 |
| **234** | 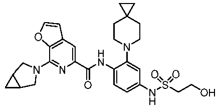 | 552 | **235** | 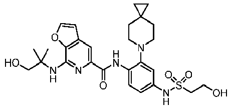 | 558 |
| **236** | 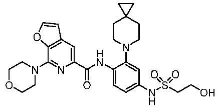 | 556 | **237** | 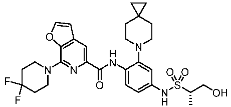 | 604 |
| **238** | 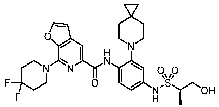 | 604 | **239** | 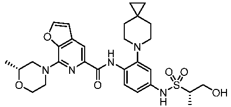 | 584 |
| **240** | 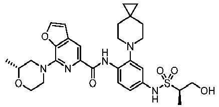 | 584 | **241** | 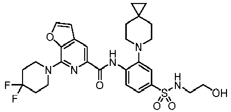 | 590 |
| **242** | 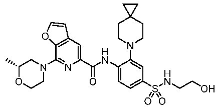 | 570 | **243** | 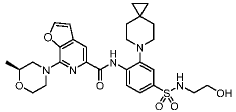 | 570 |
| **244** | 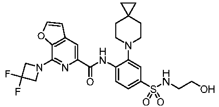 | 562 | **245** | 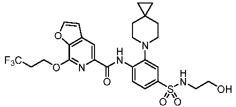 | 583 |
| **246** | 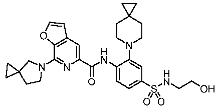 | 566 | **247** | 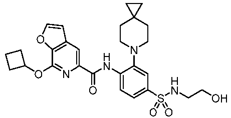 | 541 |
| **248** | 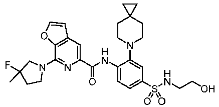 | 572 | **249** | 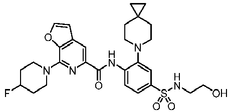 | 572 |
| **250** | 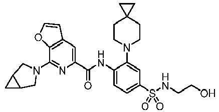 | 552 | **251** | 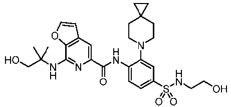 | 558 |
| **252** | 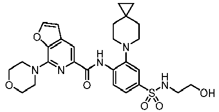 | 556 | **253** | 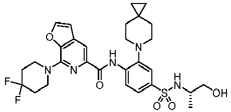 | 604 |
| **254** | 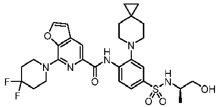 | 604 | **255** | 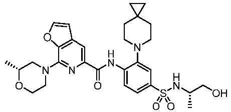 | 584 |
| **256** | 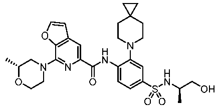 | 584 | **257** | 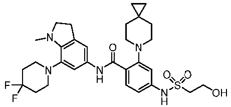 | 604 |
| **258** | 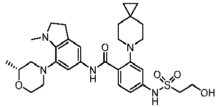 | 584 | **259** | 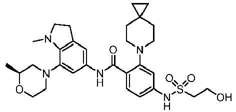 | 584 |
| **260** | 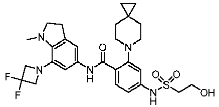 | 576 | **261** | 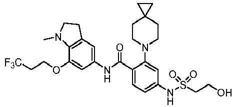 | 597 |
| **262** | 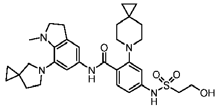 | 580 | **263** | 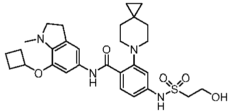 | 555 |
| **264** | 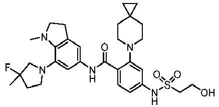 | 586 | **265** | 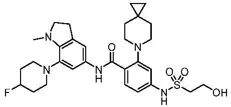 | 586 |
| **266** | 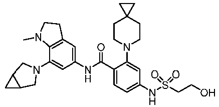 | 566 | **267** | 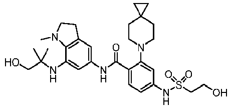 | 572 |
| **268** | 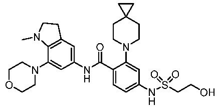 | 570 | **269** | 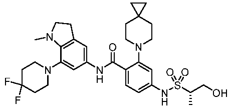 | 618 |
| **270** | 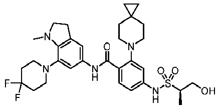 | 618 | **271** | 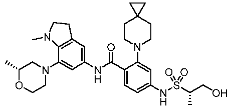 | 598 |
| **272** | 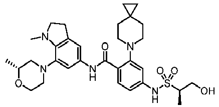 | 598 | **273** | 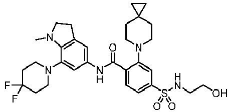 | 604 |
| **274** | 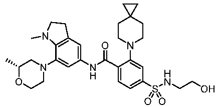 | 584 | **275** | 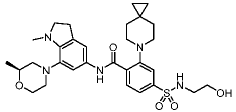 | 584 |
| **276** | 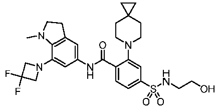 | 576 | **277** | 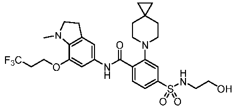 | 597 |
| **278** | 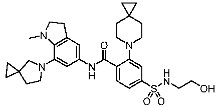 | 580 | **279** | 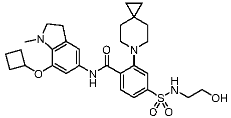 | 555 |
| **280** | 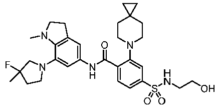 | 586 | **281** | 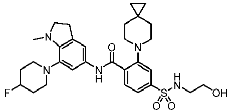 | 586 |
| **282** | 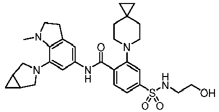 | 566 | **283** | 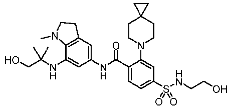 | 572 |
| **284** | 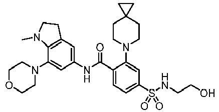 | 570 | **285** | 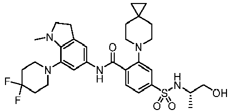 | 618 |
| **286** | 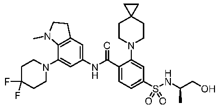 | 618 | **287** | 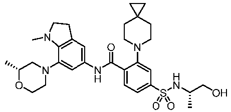 | 598 |
| **288** | 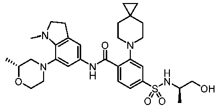 | 598 | **289** | 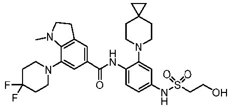 | 604 |
| **290** | 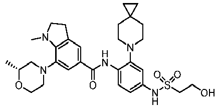 | 584 | **291** | 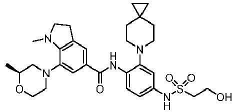 | 584 |
| **292** | 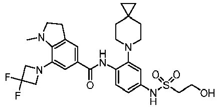 | 576 | **293** | 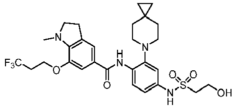 | 597 |
| **294** | 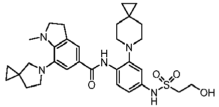 | 580 | **295** | 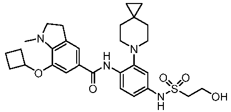 | 555 |
| **296** | 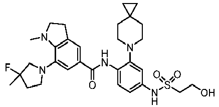 | 586 | **297** | 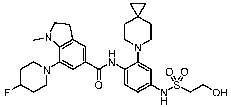 | 586 |
| **298** | 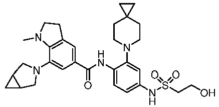 | 566 | **299** | 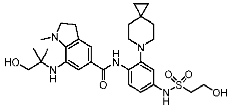 | 572 |
| **300** | 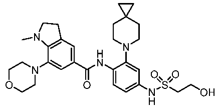 | 570 | **301** | 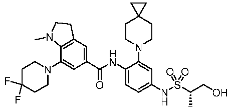 | 618 |
| **302** | 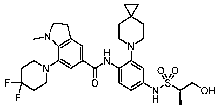 | 618 | **303** | 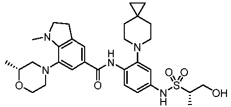 | 598 |
| **304** | 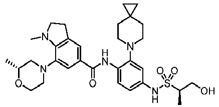 | 598 | **305** | 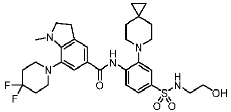 | 604 |
| **306** | 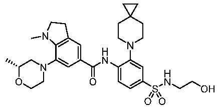 | 584 | **307** | 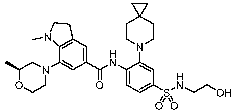 | 584 |
| **308** | 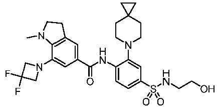 | 576 | **309** | 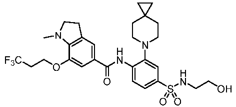 | 597 |
| **310** | 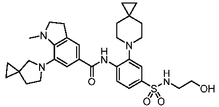 | 580 | **311** | 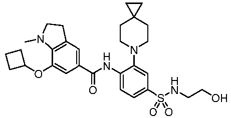 | 555 |
| **312** | 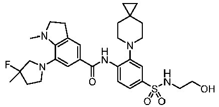 | 586 | **313** | 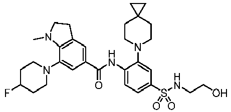 | 586 |
| **314** | 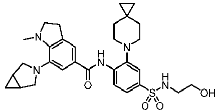 | 566 | **315** | 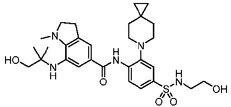 | 572 |
| **316** | 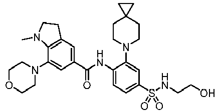 | 570 | **317** | 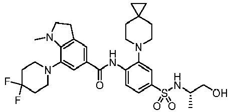 | 618 |
| **318** | 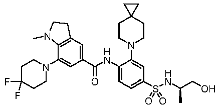 | 618 | **319** | 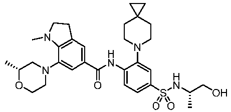 | 598 |
| **320** | 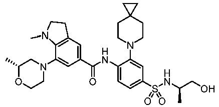 | 598 | **321** | 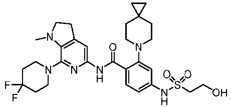 | 605 |
| **322** | 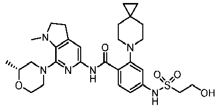 | 585 | **323** | 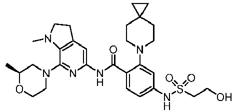 | 585 |
| **324** | 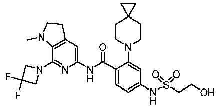 | 577 | **325** | 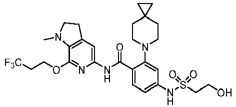 | 598 |
| **326** | 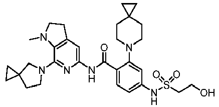 | 581 | **327** | 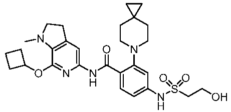 | 556 |
| **328** | 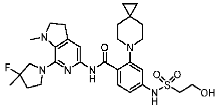 | 587 | **329** | 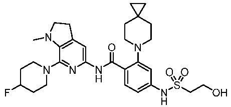 | 587 |
| **330** | 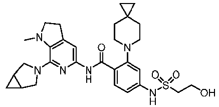 | 567 | **331** | 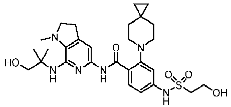 | 573 |
| **332** | 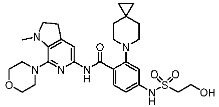 | 571 | **333** | 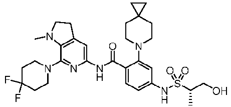 | 619 |
| **334** | 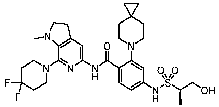 | 619 | **335** | 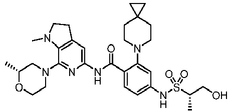 | 599 |
| **336** | 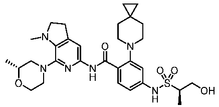 | 599 | **337** | 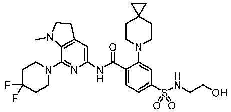 | 605 |
| **338** | 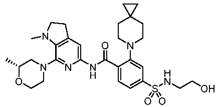 | 585 | **339** | 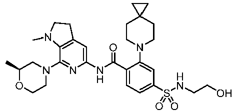 | 585 |
| **340** | 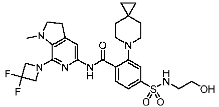 | 577 | **341** | 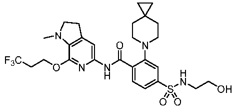 | 598 |
| **342** | 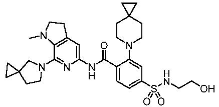 | 581 | **343** | 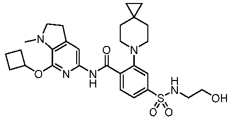 | 556 |
| **344** | 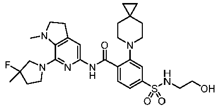 | 587 | **345** | 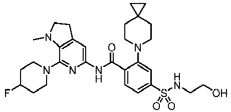 | 587 |
| **346** | 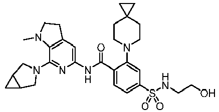 | 567 | **347** | 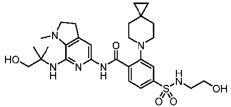 | 573 |
| **348** | 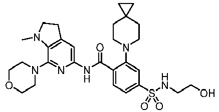 | 571 | **349** | 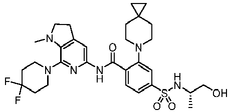 | 619 |
| **350** | 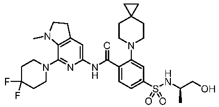 | 619 | **351** | 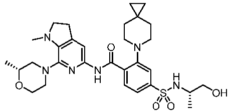 | 599 |
| **352** | 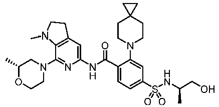 | 599 | **353** | 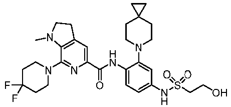 | 605 |
| **354** | 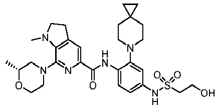 | 585 | **355** | 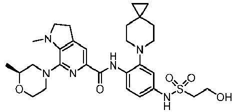 | 585 |
| **356** | 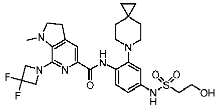 | 577 | **357** | 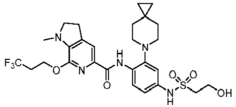 | 598 |
| **358** | 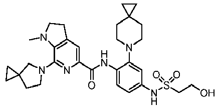 | 581 | **359** | 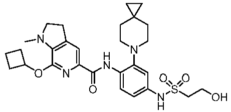 | 556 |
| **360** | 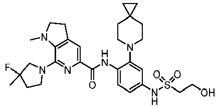 | 587 | **361** | 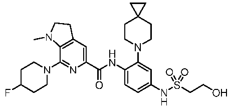 | 587 |
| **362** | 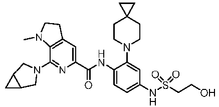 | 567 | **363** | 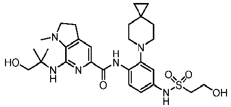 | 573 |
| **364** | 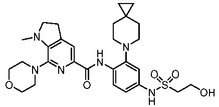 | 571 | **365** | 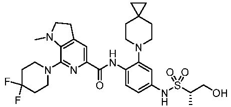 | 619 |
| **366** | 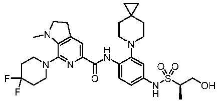 | 619 | **367** | 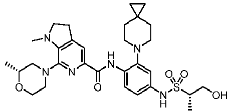 | 599 |
| **368** | 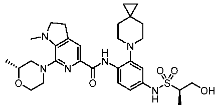 | 599 | **369** | 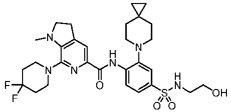 | 605 |
| **370** | 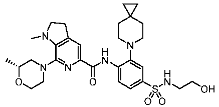 | 585 | **371** | 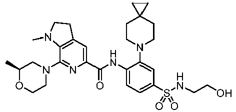 | 585 |
| **372** | 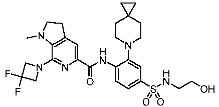 | 577 | **373** | 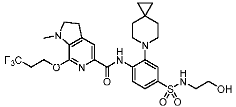 | 598 |
| **374** | 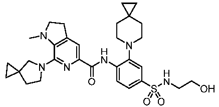 | 581 | **375** | 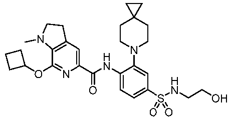 | 556 |
| **376** | 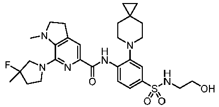 | 587 | **377** | 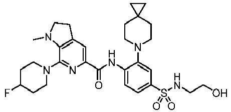 | 587 |
| **378** | 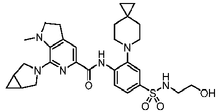 | 567 | **379** | 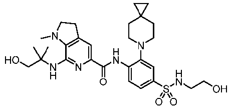 | 573 |
| **380** | 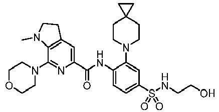 | 571 | **381** | 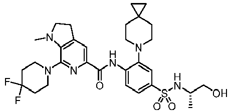 | 619 |
| **382** | 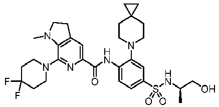 | 619 | **383** | 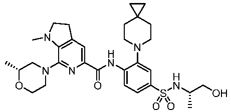 | 599 |
| **384** | 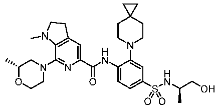 | 599 | **385** | 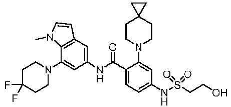 | 602 |
| **386** | 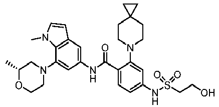 | 582 | **387** | 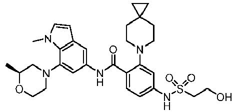 | 582 |
| **388** | 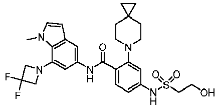 | 574 | **389** | 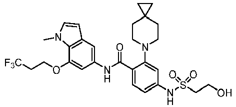 | 595 |
| **390** | 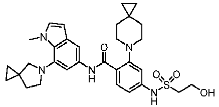 | 578 | **391** | 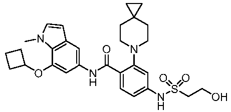 | 553 |
| **392** | 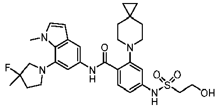 | 584 | **393** | 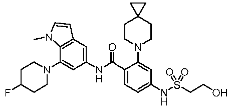 | 584 |
| **394** | 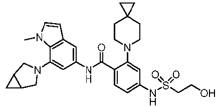 | 564 | **395** | 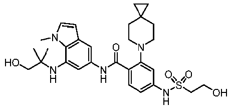 | 570 |
| **396** | 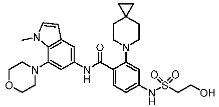 | 568 | **397** | 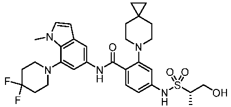 | 616 |
| **398** | 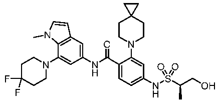 | 616 | **399** | 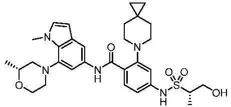 | 596 |
| **400** | 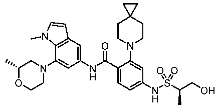 | 596 | **401** | 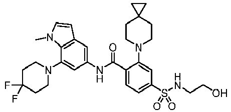 | 602 |
| **402** | 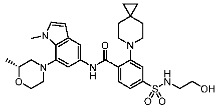 | 582 | **403** | 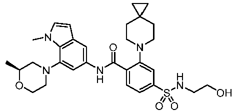 | 582 |
| **404** | 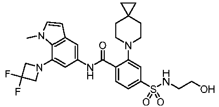 | 574 | **405** | 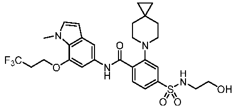 | 595 |
| **406** | 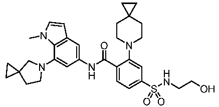 | 578 | **407** | 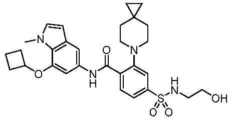 | 553 |
| **408** | 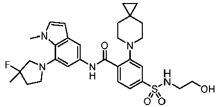 | 584 | **409** | 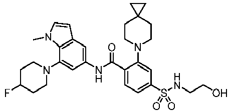 | 584 |
| **410** | 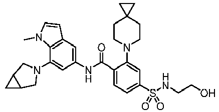 | 564 | **411** | 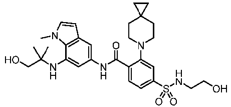 | 570 |
| **412** | 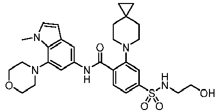 | 568 | **413** | 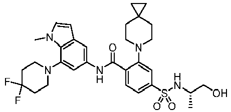 | 616 |
| **414** | 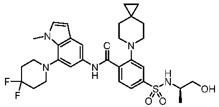 | 616 | **415** | 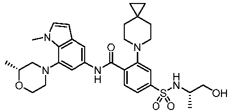 | 596 |
| **416** | 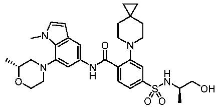 | 596 | **417** | 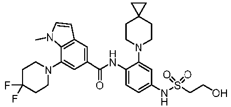 | 602 |
| **418** | 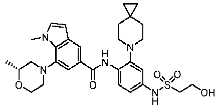 | 582 | **419** | 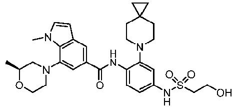 | 582 |
| **420** | 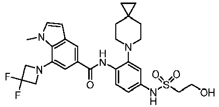 | 574 | **421** | 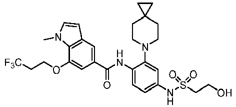 | 595 |
| **422** | 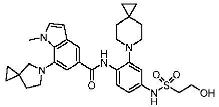 | 578 | **423** | 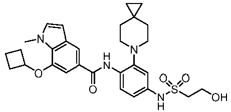 | 553 |
| **424** | 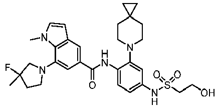 | 584 | **425** | 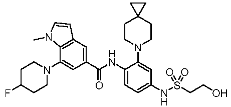 | 584 |
| **426** | 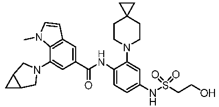 | 564 | **427** | 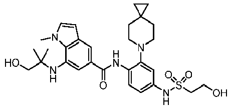 | 570 |
| **428** | 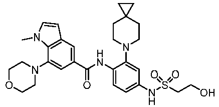 | 568 | **429** | 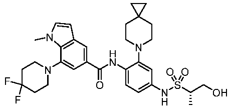 | 616 |
| **430** | 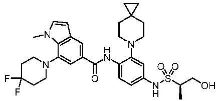 | 616 | **431** | 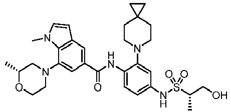 | 596 |
| **432** | 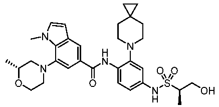 | 596 | **433** | 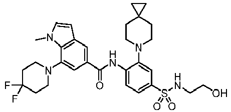 | 602 |
| **434** | 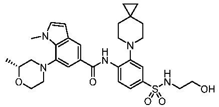 | 582 | **435** | 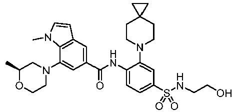 | 582 |
| **436** | 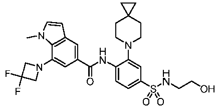 | 574 | **437** | 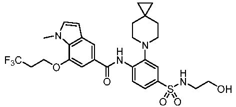 | 595 |
| **438** | 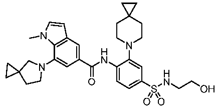 | 578 | **439** | 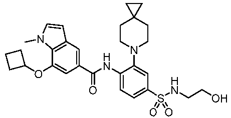 | 553 |
| **440** | 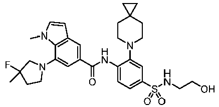 | 584 | **441** | 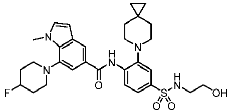 | 584 |
| **442** | 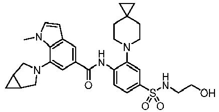 | 564 | **443** | 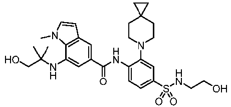 | 570 |
| **444** | 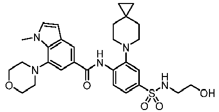 | 568 | **445** | 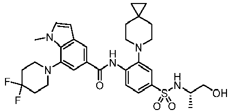 | 616 |
| **446** | 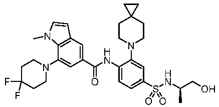 | 616 | **447** | 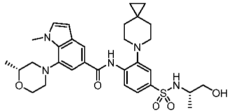 | 596 |
| **448** | 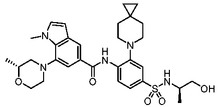 | 596 | **449** | 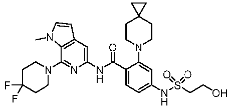 | 603 |
| **450** | 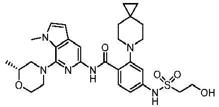 | 583 | **451** | 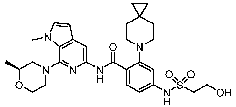 | 583 |
| **452** | 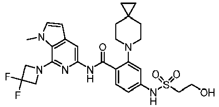 | 575 | **453** | 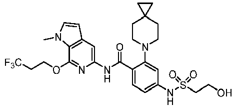 | 596 |
| **454** | 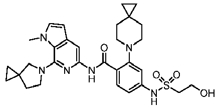 | 579 | **455** | 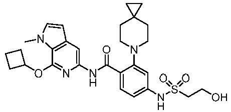 | 554 |
| **456** | 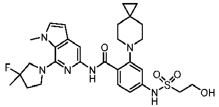 | 585 | **457** | 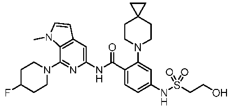 | 585 |
| **458** | 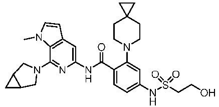 | 565 | **459** | 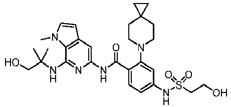 | 571 |
| **460** | 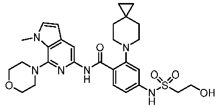 | 569 | **461** | 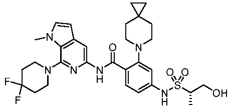 | 617 |
| **462** | 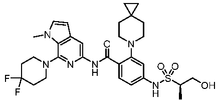 | 617 | **463** | 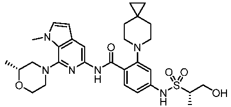 | 597 |
| **464** | 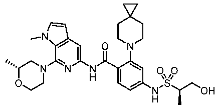 | 597 | **465** | 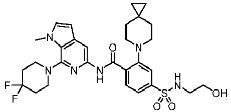 | 603 |
| **466** | 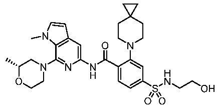 | 583 | **467** | 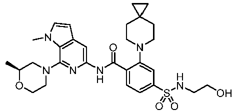 | 583 |
| **468** | 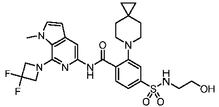 | 575 | **469** | 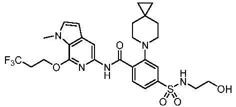 | 596 |
| **470** | 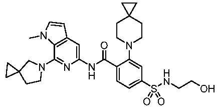 | 579 | **471** | 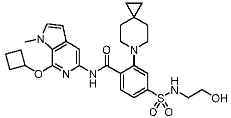 | 554 |
| **472** | 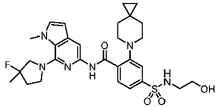 | 585 | **473** | 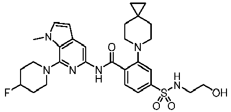 | 585 |
| **474** | 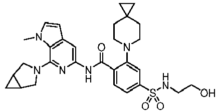 | 565 | **475** | 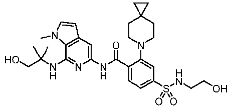 | 571 |
| **476** | 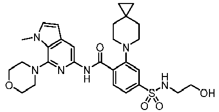 | 569 | **477** | 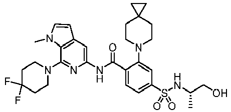 | 617 |
| **478** | 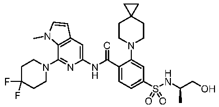 | 617 | **479** | 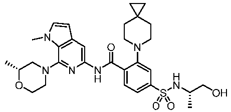 | 597 |
| **480** | 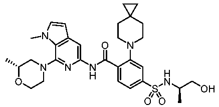 | 597 | **481** | 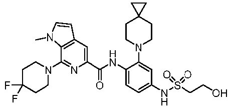 | 603 |
| **482** | 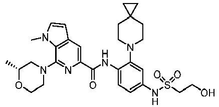 | 583 | **483** | 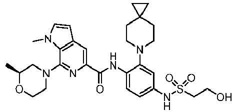 | 583 |
| **484** | 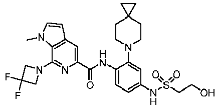 | 575 | **485** | 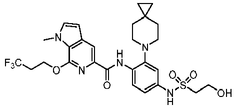 | 596 |
| **486** | 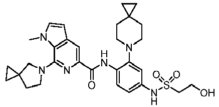 | 579 | **487** | 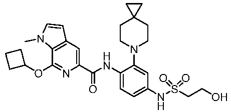 | 554 |
| **488** | 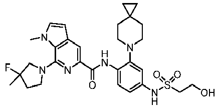 | 585 | **489** | 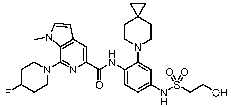 | 585 |
| **490** | 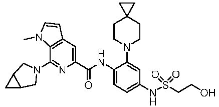 | 565 | **491** | 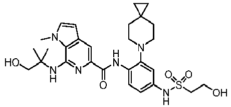 | 571 |
| **492** | 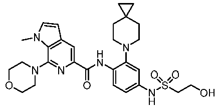 | 569 | **493** | 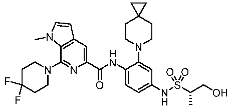 | 617 |
| **494** | 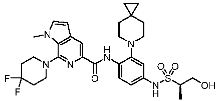 | 617 | **495** | 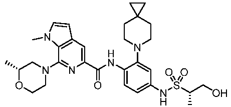 | 597 |
| **496** | 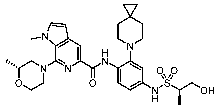 | 597 | **497** | 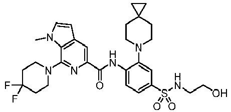 | 603 |
| **498** | 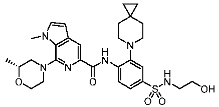 | 583 | **499** | 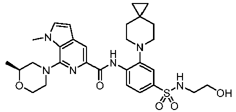 | 583 |
| **500** | 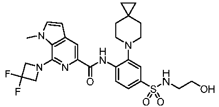 | 575 | **501** | 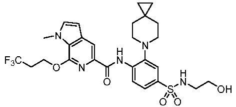 | 596 |
| **502** | 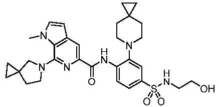 | 579 | **503** | 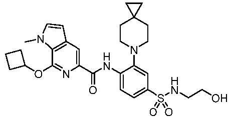 | 554 |
| **504** | 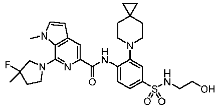 | 585 | **505** | 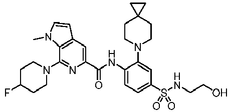 | 585 |
| **506** | 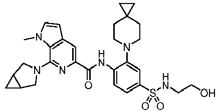 | 565 | **507** | 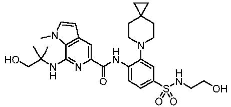 | 571 |
| **508** | 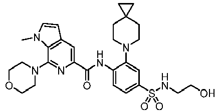 | 569 | **509** | 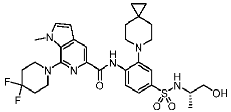 | 617 |
| **510** | 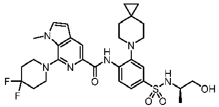 | 617 | **511** | 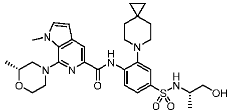 | 597 |
| **512** | 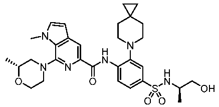 | 597 | **513** | 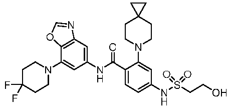 | 590 |
| **514** | 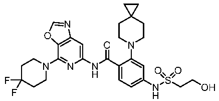 | 591 | **515** | 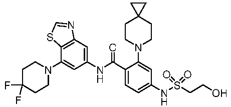 | 606 |
| **516** | 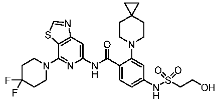 | 607 | **517** | 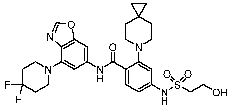 | 590 |
| **518** | 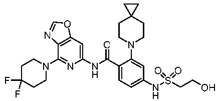 | 591 | **519** | 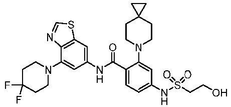 | 606 |
| **520** | 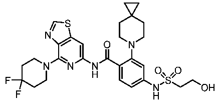 | 607 | **521** | 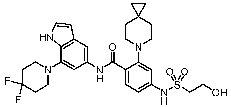 | 588 |
| **522** | 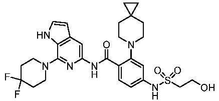 | 589 | **523** | 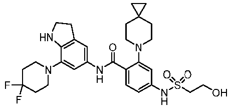 | 590 |
| **524** | 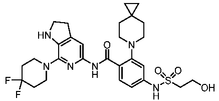 | 591 | **525** | 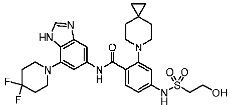 | 589 |
| **526** | 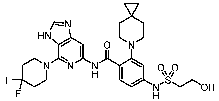 | 590 | **527** | 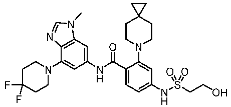 | 603 |
| **528** | 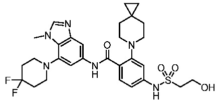 | 603 | **529** | 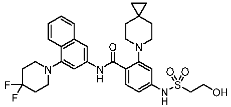 | 599 |
| **530** | 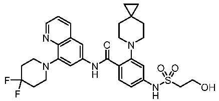 | 600 | **531** | 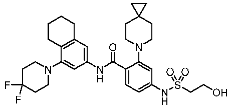 | 603 |
| **532** | 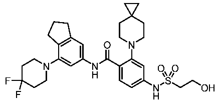 | 589 | **533** | 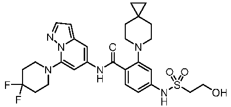 | 589 |
| **534** | 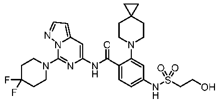 | 590 | **535** | 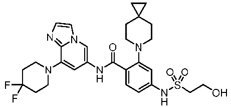 | 589 |
| **536** | 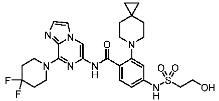 | 590 | **537** | 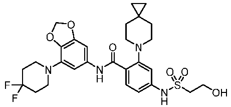 | 593 |
| **538** | 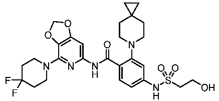 | 594 | **539** | 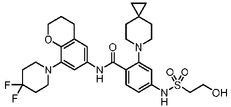 | 605 |
| **540** | 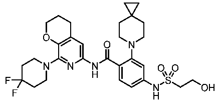 | 606 | **541** | 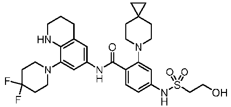 | 604 |
| **542** | 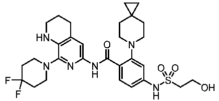 | 605 | **543** | 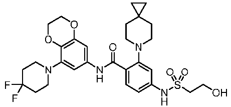 | 607 |
| **544** | 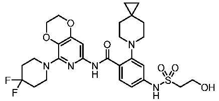 | 608 | **545** | 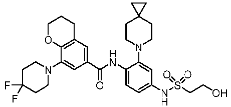 | 605 |
| **546** | 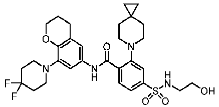 | 605 | **547** | 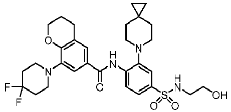 | 605 |
| **548** | 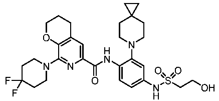 | 606 | **549** | 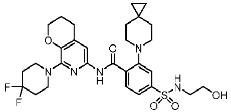 | 606 |
| **550** | 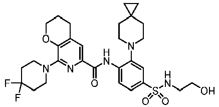 | 606 | **551** | 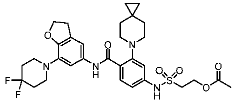 | 633 |
| **552** | 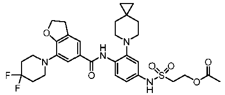 | 633 | **553** | 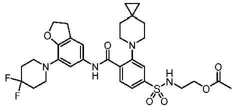 | 633 |
| **554** | 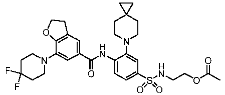 | 633 | **555** | 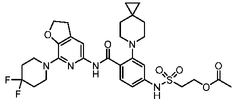 | 634 |
| **556** | 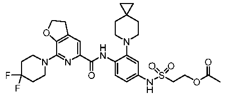 | 634 | **557** | 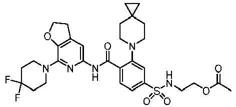 | 634 |
| **558** | 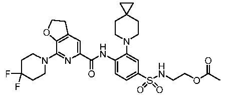 | 634 | **559** | 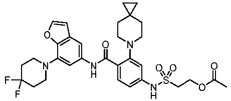 | 631 |
| **560** | 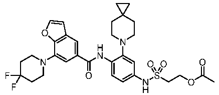 | 631 | **561** | 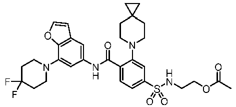 | 631 |
| **562** | 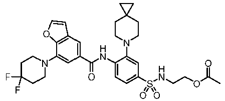 | 631 | **563** | 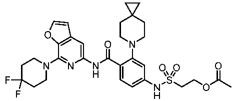 | 632 |
| **564** | 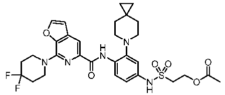 | 632 | **565** | 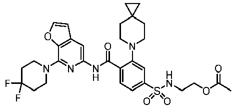 | 632 |
| **566** | 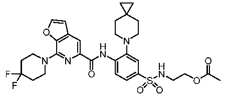 | 632 | **567** | 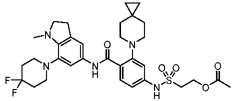 | 646 |
| **568** | 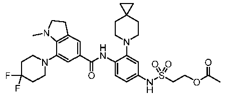 | 646 | **569** | 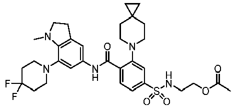 | 646 |
| **570** | 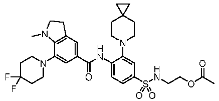 | 646 | **571** | 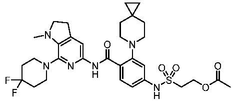 | 647 |
| **572** | 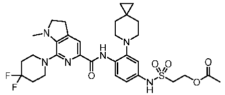 | 647 | **573** | 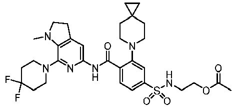 | 647 |
| **574** | 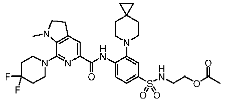 | 647 | **575** | 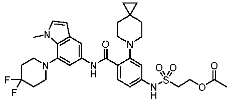 | 644 |
| **576** | 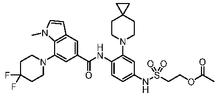 | 644 | **577** | 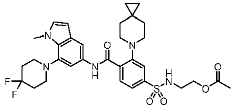 | 644 |
| **578** | 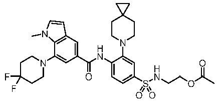 | 644 | **579** | 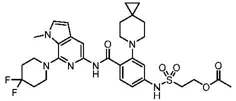 | 645 |
| **580** | 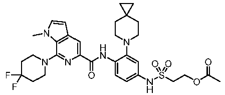 | 645 | **581** | 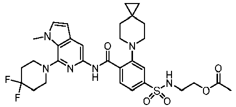 | 645 |
| **582** | 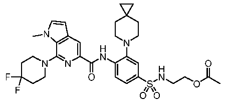 | 645 | **583** | 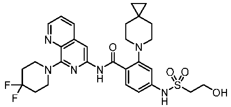 | 601 |
| **584** | 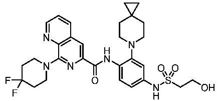 | 601 | **585** | 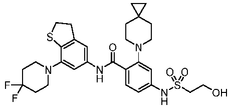 | 607 |
| **586** | 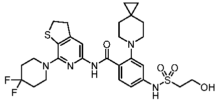 | 608 | **587** | 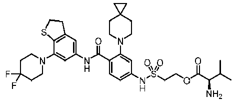 | 706 |
| **588** | 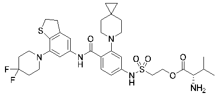 | 706 | **589** | 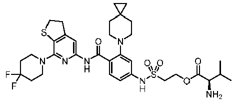 | 707 |
| **590** | 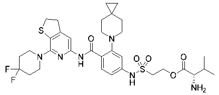 | 707 | **591** | 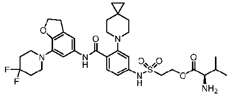 | 690 |
| **592** | 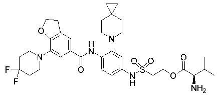 | 690 | **593** | 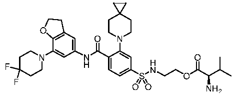 | 690 |
| **594** | 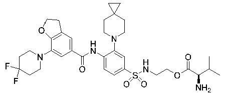 | 690 | **595** | 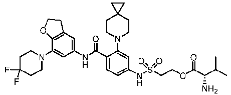 | 690 |
| **596** | 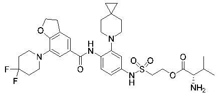 | 690 | **597** | 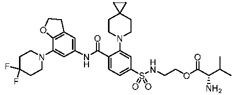 | 690 |
| **598** | 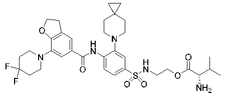 | 690 | **599** | 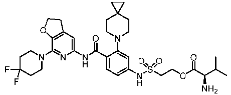 | 691 |
| **600** | 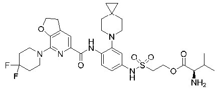 | 691 | **601** | 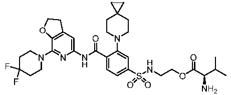 | 691 |
| **602** | 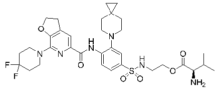 | 691 | **603** | 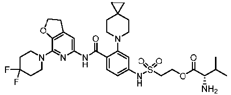 | 691 |
| **604** | 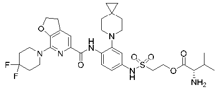 | 691 | **605** | 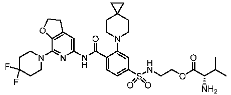 | 691 |
| **606** | 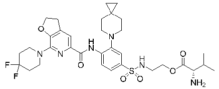 | 691 | **607** | 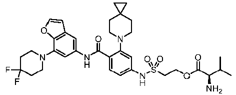 | 688 |
| **608** | 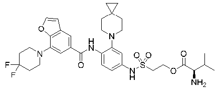 | 688 | **609** | 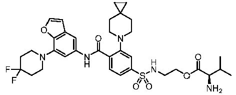 | 688 |
| **610** | 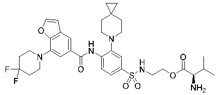 | 688 | **611** | 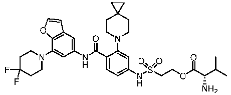 | 688 |
| **612** | 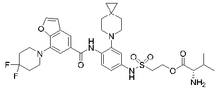 | 688 | **613** | 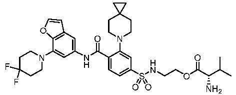 | 688 |
| **614** | 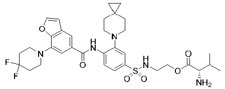 | 688 | **615** | 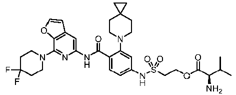 | 689 |
| **616** | 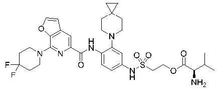 | 689 | **617** | 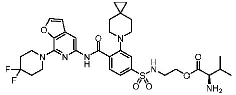 | 689 |
| **618** | 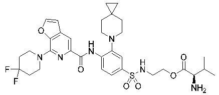 | 689 | **619** | 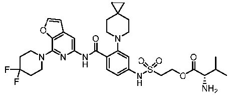 | 689 |
| **620** | 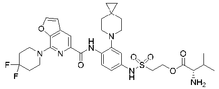 | 689 | **621** | 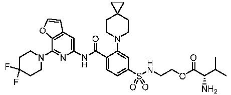 | 689 |
| **622** | 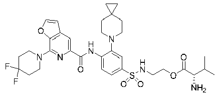 | 689 | **623** | 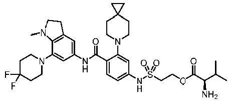 | 703 |
| **624** | 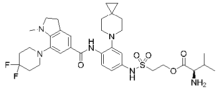 | 703 | **625** | 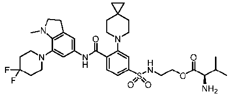 | 703 |
| **626** | 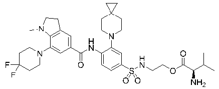 | 703 | **627** | 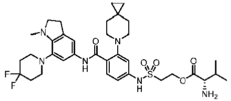 | 703 |
| **628** | 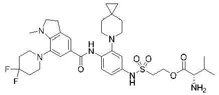 | 703 | **629** | 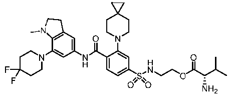 | 703 |
| **630** | 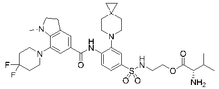 | 703 | **631** | 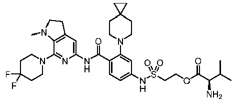 | 704 |
| **632** | 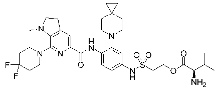 | 704 | **633** | 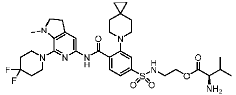 | 704 |
| **634** | 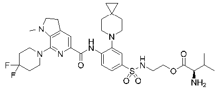 | 704 | **635** | 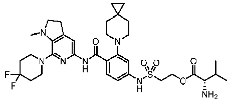 | 704 |
| **636** | 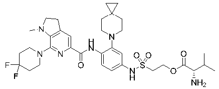 | 704 | **637** | 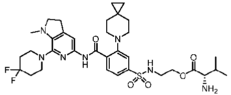 | 704 |
| **638** | 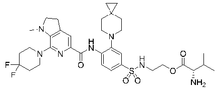 | 704 | **639** | 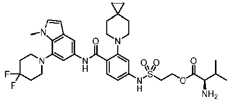 | 701 |
| **640** | 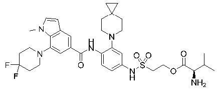 | 701 | **641** | 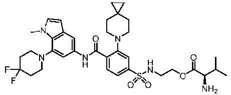 | 701 |
| **642** | 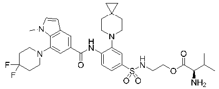 | 701 | **643** | 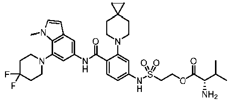 | 701 |
| **644** | 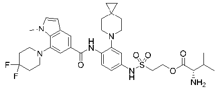 | 701 | **645** | 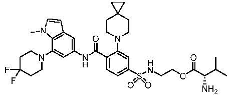 | 701 |
| **646** | 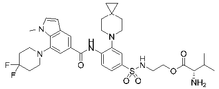 | 701 | **647** | 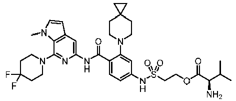 | 702 |
| **648** | 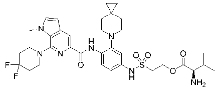 | 702 | **649** | 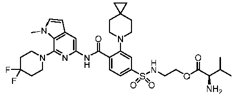 | 702 |
| **650** | 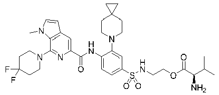 | 702 | **651** | 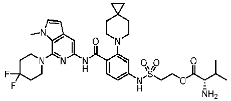 | 702 |
| **652** | 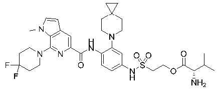 | 702 | **653** | 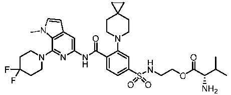 | 702 |
| **654** | 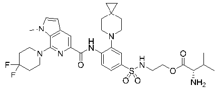 | 702 | **655** | 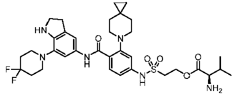 | 689 |
| **656** | 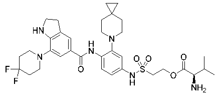 | 689 | **657** | 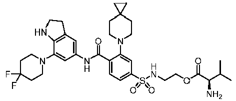 | 689 |
| **658** | 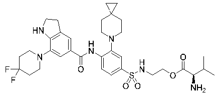 | 689 | **659** | 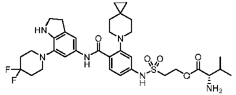 | 689 |
| **660** | 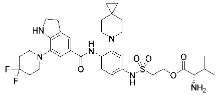 | 689 | **661** | 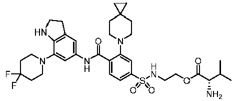 | 689 |
| **662** | 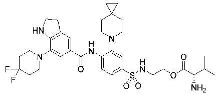 | 689 | **663** | 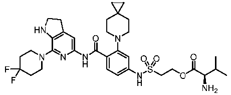 | 690 |
| **664** | 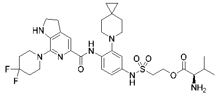 | 690 | **665** | 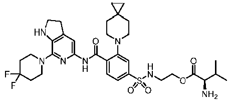 | 690 |
| **666** | 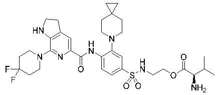 | 690 | **667** | 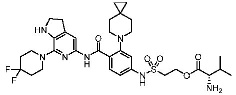 | 690 |
| **668** | 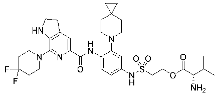 | 690 | **669** | 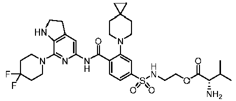 | 690 |
| **670** | 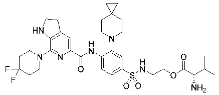 | 690 | **671** | 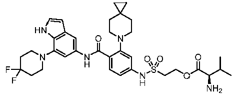 | 687 |
| **672** | 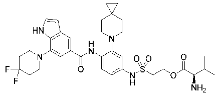 | 687 | **673** | 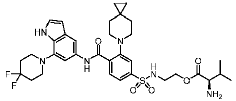 | 687 |
| **674** | 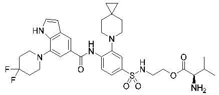 | 687 | **675** | 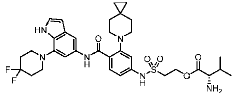 | 687 |
| **676** | 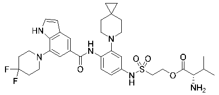 | 687 | **677** | 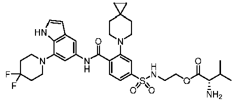 | 687 |
| **678** | 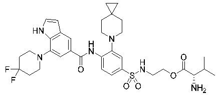 | 687 | **679** | 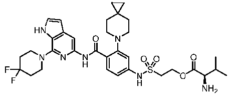 | 688 |
| **680** | 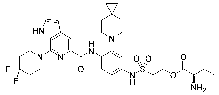 | 688 | **681** | 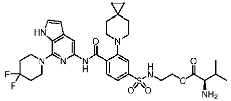 | 688 |
| **682** | 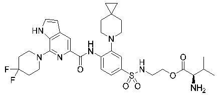 | 688 | **683** | 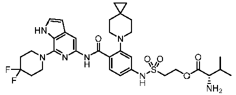 | 688 |
| **684** | 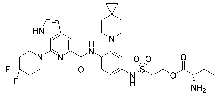 | 688 | **685** | 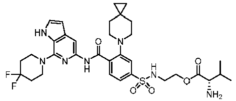 | 688 |
| **686** | 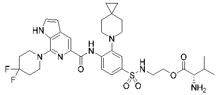 | 688 | **687** | 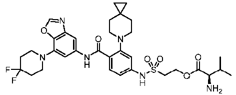 | 689 |
| **688** | 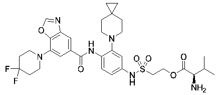 | 689 | **689** | 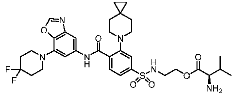 | 689 |
| **690** | 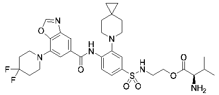 | 689 | **691** | 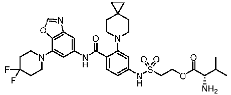 | 689 |
| **692** | 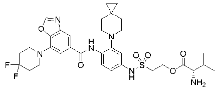 | 689 | **693** | 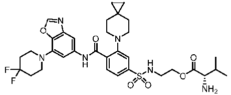 | 689 |
| **694** | 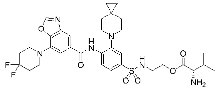 | 689 | **695** | 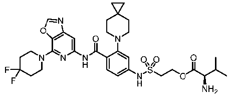 | 690 |
| **696** | 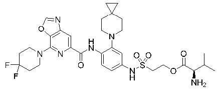 | 690 | **697** | 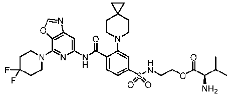 | 690 |
| **698** | 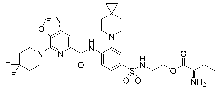 | 690 | **699** | 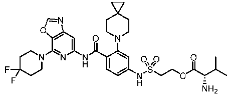 | 690 |
| **700** | 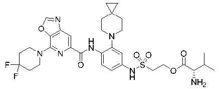 | 690 | **701** | 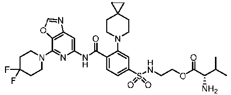 | 690 |
| **702** | 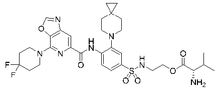 | 690 | **703** | 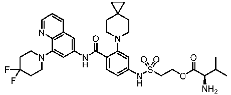 | 699 |
| **704** | 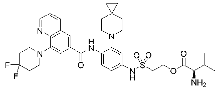 | 699 | **705** | 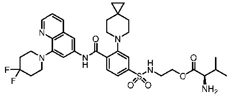 | 699 |
| **706** | 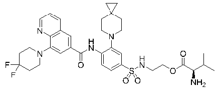 | 699 | **707** | 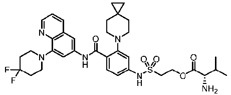 | 699 |
| **708** | 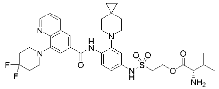 | 699 | **709** | 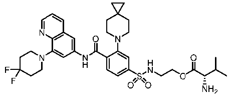 | 699 |
| **710** | 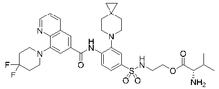 | 699 | **711** | 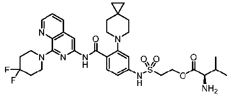 | 700 |
| **712** | 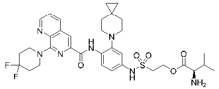 | 700 | **713** | 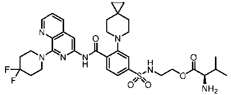 | 700 |
| **714** | 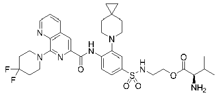 | 700 | **715** | 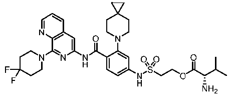 | 700 |
| **716** | 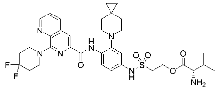 | 700 | **717** | 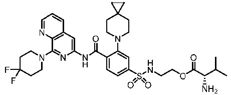 | 700 |
| **718** | 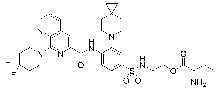 | 700 | **719** | 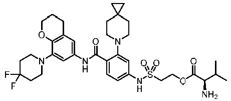 | 704 |
| **720** | 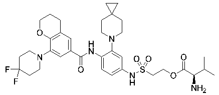 | 704 | **721** | 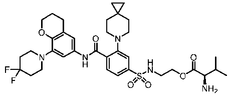 | 704 |
| **722** | 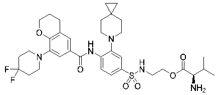 | 704 | **723** | 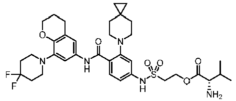 | 704 |
| **724** | 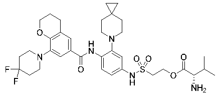 | 704 | **725** | 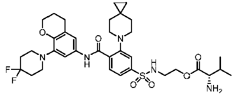 | 704 |
| **726** | 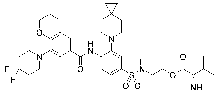 | 704 | **727** | 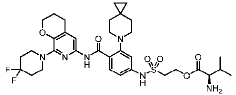 | 705 |
| **728** | 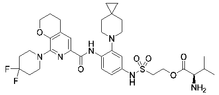 | 705 | **729** | 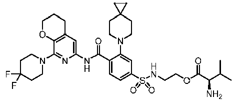 | 705 |
| **730** | 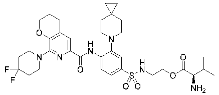 | 705 | **731** | 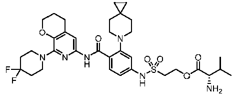 | 705 |
| **732** | 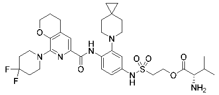 | 705 | **733** | 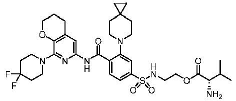 | 705 |
| **734** | 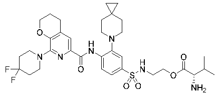 | 705 | **735** | 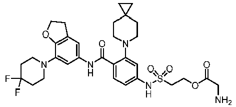 | 648 |
| **736** | 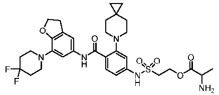 | 662 | **737** | 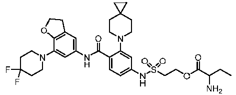 | 676 |
| **738** | 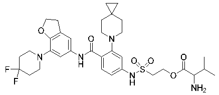 | 690 | **739** | 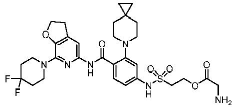 | 649 |
| **740** | 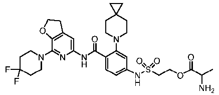 | 663 | **741** | 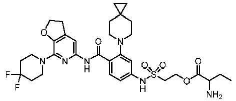 | 677 |
| **742** | 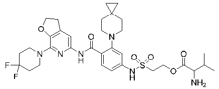 | 691 | **743** | 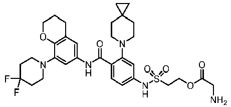 | 662 |
| **744** | 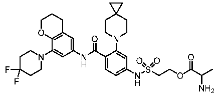 | 676 | **745** | 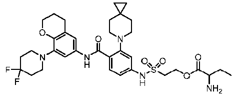 | 690 |
| **746** | 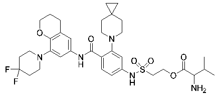 | 704 | **747** | 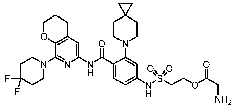 | 663 |
| **748** | 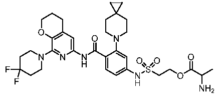 | 677 | **749** | 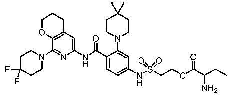 | 691 |
| **750** | 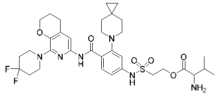 | 705 | **751** | 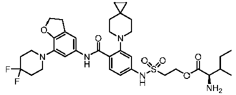 | 704 |
| **752** | 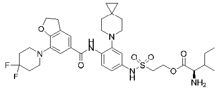 | 704 | **753** | 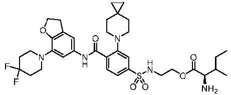 | 704 |
| **754** | 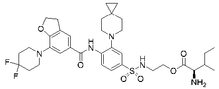 | 704 | **755** | 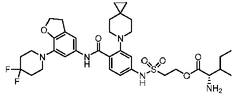 | 704 |
| **756** | 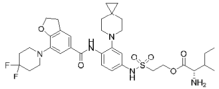 | 704 | **757** | 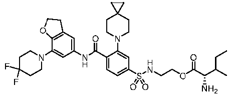 | 704 |
| **758** | 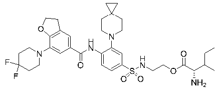 | 704 | **759** | 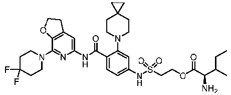 | 705 |
| **760** | 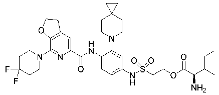 | 705 | **761** | 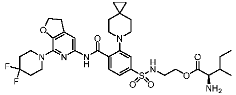 | 705 |
| **762** | 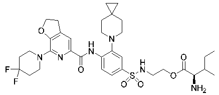 | 705 | **763** | 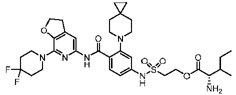 | 705 |
| **764** | 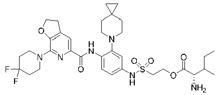 | 705 | **765** | 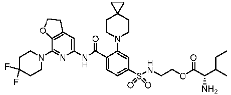 | 705 |
| **766** | 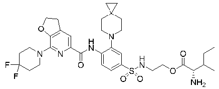 | 705 | **767** | 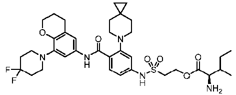 | 718 |
| **768** | 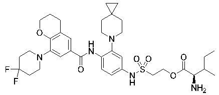 | 718 | **769** | 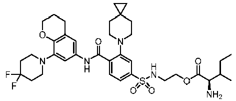 | 718 |
| **770** | 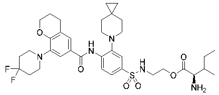 | 718 | **771** | 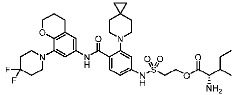 | 718 |
| **772** | 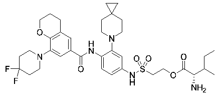 | 718 | **773** | 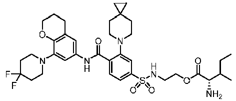 | 718 |
| **774** | 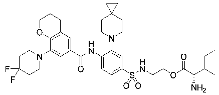 | 718 | **775** | 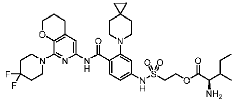 | 719 |
| **776** | 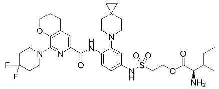 | 719 | **777** | 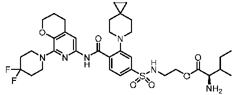 | 719 |
| **778** | 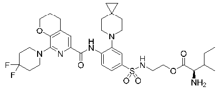 | 719 | **779** | 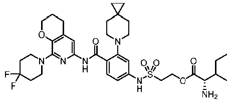 | 719 |
| **780** | 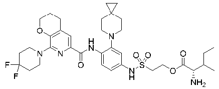 | 719 | **781** | 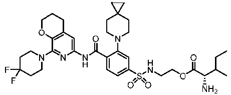 | 719 |
| **782** | 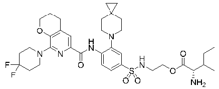 | 719 | **783** | 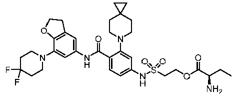 | 676 |
| **784** | 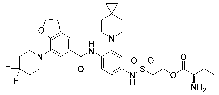 | 676 | **785** | 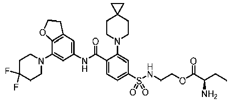 | 676 |
| **786** | 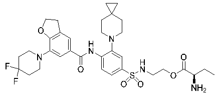 | 676 | **787** | 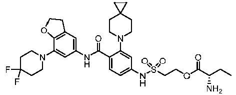 | 676 |
| **788** | 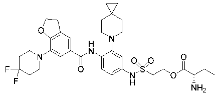 | 676 | **789** | 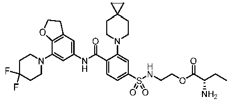 | 676 |
| **790** | 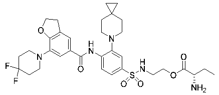 | 676 | **791** | 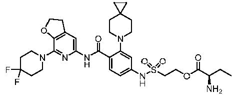 | 677 |
| **792** | 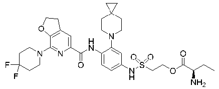 | 677 | **793** | 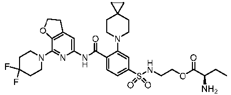 | 677 |
| **794** | 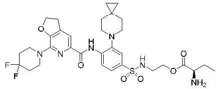 | 677 | **795** | 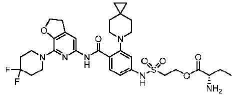 | 677 |
| **796** | 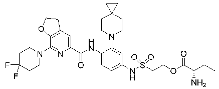 | 677 | **797** | 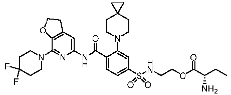 | 677 |
| **798** | 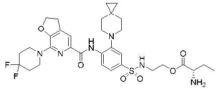 | 677 | **799** | 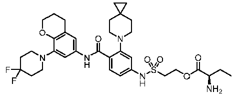 | 690 |
| **800** | 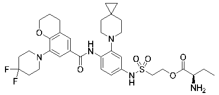 | 690 | **801** | 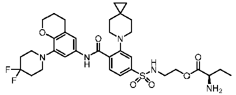 | 690 |
| **802** | 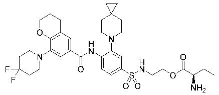 | 690 | **803** | 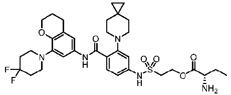 | 690 |
| **804** | 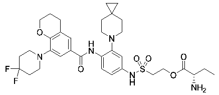 | 690 | **805** | 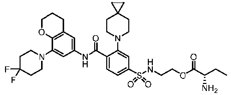 | 690 |
| **806** | 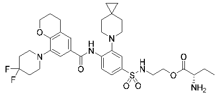 | 690 | **807** | 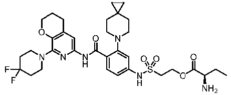 | 691 |
| **808** | 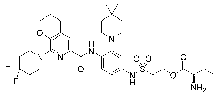 | 691 | **809** | 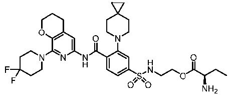 | 691 |
| **810** | 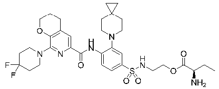 | 691 | **811** | 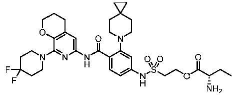 | 691 |
| **812** | 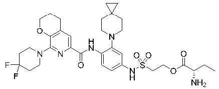 | 691 | **813** | 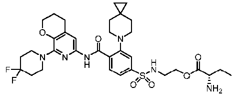 | 691 |
| **814** | 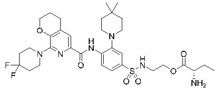 | 691 | | | |

### Biological Example 1. Assay for In Vitro Inhibition of Enzymatic Activity of KIF18A by Compounds of the Present Invention

KIF18A enzyme assay: The enzymatic activity of KIF18A after treatment with the compounds was measured by an assay of microtubule-stimulated ATPase activity. ADP generated from the ATPase reaction was measured in this assay. The compounds were serially diluted 2-fold in DMSO over a range of 22 concentration points. Recombinant human KIF18A (1-467His-tagged) protein was expressed using a baculovirus system. Concentrations of KIF18A protein, microtubules, and ATP in the reaction were optimized for a standardized homogenous enzyme assay using an ADP-Glo kinase/ATPase assay kit. A reaction buffer [(15 mM Tris, pH 7.5), 10 mM MgCl₂, 0.01% PluronicF-68, 1 µM paclitaxel, and 30 µg/mL pig microtubules] was prepared. The compounds and KIF18A protein (30 nM) were added to the prepared reaction buffer, and the reaction mixture was incubated at room temperature for 15 min, followed by addition of ATP (Km, 75 µM). The resulting reaction mixture was incubated at room temperature for another 15 min. 5 µL of ADP-Glo reagent and 2.5 µL of the reaction mixture were mixed and the resulting mixture was incubated at room temperature for 40 min. 10 µL of ADP-Glo detection reagent was added and the mixture was incubated at room temperature for 40 min. Luminescence was measured using a microplate reader and compared with that of the DMSO group, and then the inhibition percentages and IC₅₀ values of the compounds were calculated. The results are shown in Table 2 below.

**Table 2. The inhibitory activity of the compounds of the present invention against KIF18A (IC50, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | +++ | **2** | +++ | **3** | +++ | **4** | +++ |
| **5** | +++ | **6** | +++ | **7** | +++ | **8** | +++ |
| **9** | +++ | **10** | +++ | **11** | +++ | **12** | +++ |
| **13** | +++ | **14** | +++ | **15** | +++ | **16** | +++ |
| **17** | +++ | **18** | +++ | **19** | +++ | **20** | +++ |
| **21** | +++ | **22** | +++ | **23** | +++ | **24** | +++ |
| **25** | +++ | **26** | +++ | **27** | +++ | **28** | +++ |
| **29** | +++ | **30** | +++ | **31** | +++ | **32** | +++ |
| **33** | +++ | **34** | +++ | **35** | +++ | **36** | +++ |
| **37** | +++ | **38** | +++ | **39** | +++ | **40** | +++ |
| **41** | +++ | **42** | +++ | **43** | +++ | **44** | +++ |
| **45** | +++ | **46** | +++ | **47** | +++ | **48** | +++ |
| **49** | +++ | **50** | +++ | **51** | +++ | **52** | +++ |
| **53** | +++ | **54** | +++ | **55** | +++ | **56** | +++ |
| **57** | +++ | **58** | +++ | **59** | +++ | **60** | +++ |
| **61** | +++ | **62** | +++ | **63** | +++ | **64** | +++ |
| **65** | +++ | **66** | +++ | **67** | +++ | **68** | +++ |
| **69** | +++ | **70** | +++ | **71** | +++ | **72** | +++ |
| **73** | +++ | **74** | +++ | **75** | +++ | **76** | +++ |
| **77** | +++ | **78** | +++ | **79** | +++ | **80** | +++ |
| **81** | +++ | **82** | +++ | **83** | +++ | **84** | +++ |
| **85** | +++ | **86** | +++ | **87** | +++ | **88** | +++ |
| **89** | +++ | **90** | +++ | **91** | +++ | **92** | +++ |
| **93** | +++ | **94** | +++ | **95** | +++ | **96** | +++ |
| **97** | +++ | **98** | +++ | **99** | +++ | **100** | +++ |
| **101** | +++ | **102** | +++ | **103** | +++ | **104** | +++ |
| **105** | +++ | **106** | +++ | **107** | +++ | **108** | +++ |
| **109** | +++ | **110** | +++ | **111** | +++ | **112** | +++ |
| **113** | +++ | **114** | +++ | **115** | +++ | **116** | +++ |
| **117** | +++ | **118** | +++ | **119** | +++ | **120** | +++ |
| **121** | +++ | **122** | +++ | **123** | +++ | **124** | +++ |
| **125** | +++ | **126** | +++ | **127** | +++ | **128** | +++ |
| **129** | +++ | **130** | +++ | **131** | +++ | **132** | +++ |
| **133** | +++ | **134** | +++ | **135** | +++ | **136** | +++ |
| **137** | +++ | **138** | +++ | **139** | +++ | **140** | +++ |
| **141** | +++ | **142** | +++ | **143** | +++ | **144** | +++ |
| **145** | +++ | **146** | +++ | **147** | +++ | **148** | +++ |
| **149** | +++ | **150** | +++ | **151** | +++ | **152** | +++ |
| **153** | +++ | **154** | +++ | **155** | +++ | **156** | +++ |
| **157** | +++ | **158** | +++ | **159** | +++ | **160** | +++ |
| **161** | +++ | **162** | +++ | **163** | +++ | **164** | +++ |
| **165** | +++ | **166** | +++ | **167** | +++ | **168** | +++ |
| **169** | +++ | **170** | +++ | **171** | +++ | **172** | +++ |
| **173** | +++ | **174** | +++ | **175** | +++ | **176** | +++ |
| **177** | +++ | **178** | +++ | **179** | +++ | **180** | +++ |
| **181** | +++ | **182** | +++ | **183** | +++ | **184** | +++ |
| **185** | +++ | **186** | +++ | **187** | +++ | **188** | +++ |
| **189** | +++ | **190** | +++ | **191** | +++ | **192** | +++ |
| **193** | +++ | **194** | +++ | **195** | +++ | **196** | +++ |
| **197** | +++ | **198** | +++ | **199** | +++ | **200** | +++ |
| **201** | +++ | **202** | +++ | **203** | +++ | **204** | +++ |
| **205** | +++ | **206** | +++ | **207** | +++ | **208** | +++ |
| **209** | +++ | **210** | +++ | **211** | +++ | **212** | +++ |
| **213** | +++ | **214** | +++ | **215** | +++ | **216** | +++ |
| **217** | +++ | **218** | +++ | **219** | +++ | **220** | +++ |
| **221** | +++ | **222** | +++ | **223** | +++ | **224** | +++ |
| **225** | +++ | **226** | +++ | **227** | +++ | **228** | +++ |
| **229** | +++ | **230** | +++ | **231** | +++ | **232** | +++ |
| **233** | +++ | **234** | +++ | **235** | +++ | **236** | +++ |
| **237** | +++ | **238** | +++ | **239** | +++ | **240** | +++ |
| **241** | +++ | **242** | +++ | **243** | +++ | **244** | +++ |
| **245** | +++ | **246** | +++ | **247** | +++ | **248** | +++ |
| **249** | +++ | **250** | +++ | **251** | +++ | **252** | +++ |
| **253** | +++ | **254** | +++ | **255** | +++ | **256** | +++ |
| **257** | +++ | **258** | +++ | **259** | +++ | **260** | +++ |
| **261** | +++ | **262** | +++ | **263** | +++ | **264** | +++ |
| **265** | +++ | **266** | +++ | **267** | +++ | **268** | +++ |
| **269** | +++ | **270** | +++ | **271** | +++ | **272** | +++ |
| **273** | +++ | **274** | +++ | **275** | +++ | **276** | +++ |
| **277** | +++ | **278** | +++ | **279** | +++ | **280** | +++ |
| **281** | +++ | **282** | +++ | **283** | +++ | **284** | +++ |
| **285** | +++ | **286** | +++ | **287** | +++ | **288** | +++ |
| **289** | +++ | **290** | +++ | **291** | +++ | **292** | +++ |
| **293** | +++ | **294** | +++ | **295** | +++ | **296** | +++ |
| **297** | +++ | **298** | +++ | **299** | +++ | **300** | +++ |
| **301** | +++ | **302** | +++ | **303** | +++ | **304** | +++ |
| **305** | +++ | **306** | +++ | **307** | +++ | **308** | +++ |
| **309** | +++ | **310** | +++ | **311** | +++ | **312** | +++ |
| **313** | +++ | **314** | +++ | **315** | +++ | **316** | +++ |
| **317** | +++ | **318** | +++ | **319** | +++ | **320** | +++ |
| **321** | +++ | **322** | +++ | **323** | +++ | **324** | +++ |
| **325** | +++ | **326** | +++ | **327** | +++ | **328** | +++ |
| **329** | +++ | **330** | +++ | **331** | +++ | **332** | +++ |
| **333** | +++ | **334** | +++ | **335** | +++ | **336** | +++ |
| **337** | +++ | **338** | +++ | **339** | +++ | **340** | +++ |
| **341** | +++ | **342** | +++ | **343** | +++ | **344** | +++ |
| **345** | +++ | **346** | +++ | **347** | +++ | **348** | +++ |
| **349** | +++ | **350** | +++ | **351** | +++ | **352** | +++ |
| **353** | +++ | **354** | +++ | **355** | +++ | **356** | +++ |
| **357** | +++ | **358** | +++ | **359** | +++ | **360** | +++ |
| **361** | +++ | **362** | +++ | **363** | +++ | **364** | +++ |
| **365** | +++ | **366** | +++ | **367** | +++ | **368** | +++ |
| **369** | +++ | **370** | +++ | **371** | +++ | **372** | +++ |
| **373** | +++ | **374** | +++ | **375** | +++ | **376** | +++ |
| **377** | +++ | **378** | +++ | **379** | +++ | **380** | +++ |
| **381** | +++ | **382** | +++ | **383** | +++ | **384** | +++ |
| **385** | +++ | **386** | +++ | **387** | +++ | **388** | +++ |
| **389** | +++ | **390** | +++ | **391** | +++ | **392** | +++ |
| **393** | +++ | **394** | +++ | **395** | +++ | **396** | +++ |
| **397** | +++ | **398** | +++ | **399** | +++ | **400** | +++ |
| **401** | +++ | **402** | +++ | **403** | +++ | **404** | +++ |
| **405** | +++ | **406** | +++ | **407** | +++ | **408** | +++ |
| **409** | +++ | **410** | +++ | **411** | +++ | **412** | +++ |
| **413** | +++ | **414** | +++ | **415** | +++ | **416** | +++ |
| **417** | +++ | **418** | +++ | **419** | +++ | **420** | +++ |
| **421** | +++ | **422** | +++ | **423** | +++ | **424** | +++ |
| **425** | +++ | **426** | +++ | **427** | +++ | **428** | +++ |
| **429** | +++ | **430** | +++ | **431** | +++ | **432** | +++ |
| **433** | +++ | **434** | +++ | **435** | +++ | **436** | +++ |
| **437** | +++ | **438** | +++ | **439** | +++ | **440** | +++ |
| **441** | +++ | **442** | +++ | **443** | +++ | **444** | +++ |
| **445** | +++ | **446** | +++ | **447** | +++ | **448** | +++ |
| **449** | +++ | **450** | +++ | **451** | +++ | **452** | +++ |
| **453** | +++ | **454** | +++ | **455** | +++ | **456** | +++ |
| **457** | +++ | **458** | +++ | **459** | +++ | **460** | +++ |
| **461** | +++ | **462** | +++ | **463** | +++ | **464** | +++ |
| **465** | +++ | **466** | +++ | **467** | +++ | **468** | +++ |
| **469** | +++ | **470** | +++ | **471** | +++ | **472** | +++ |
| **473** | +++ | **474** | +++ | **475** | +++ | **476** | +++ |
| **477** | +++ | **478** | +++ | **479** | +++ | **480** | +++ |
| **481** | +++ | **482** | +++ | **483** | +++ | **484** | +++ |
| **485** | +++ | **486** | +++ | **487** | +++ | **488** | +++ |
| **489** | +++ | **490** | +++ | **491** | +++ | **492** | +++ |
| **493** | +++ | **494** | +++ | **495** | +++ | **496** | +++ |
| **497** | +++ | **498** | +++ | **499** | +++ | **500** | +++ |
| **501** | +++ | **502** | +++ | **503** | +++ | **504** | +++ |
| **505** | +++ | **506** | +++ | **507** | +++ | **508** | +++ |
| **509** | +++ | **510** | +++ | **511** | +++ | **512** | +++ |
| **513** | +++ | **514** | +++ | **515** | +++ | **516** | +++ |
| **517** | +++ | **518** | +++ | **519** | +++ | **520** | +++ |
| **521** | +++ | **522** | +++ | **523** | +++ | **524** | +++ |
| **525** | +++ | **526** | +++ | **527** | +++ | **528** | +++ |
| **529** | ++ | **530** | +++ | **531** | ++ | **532** | +++ |
| **533** | +++ | **534** | +++ | **535** | +++ | **536** | +++ |
| **537** | +++ | **538** | +++ | **539** | +++ | **540** | +++ |
| **541** | +++ | **542** | +++ | **543** | +++ | **544** | +++ |
| **545** | +++ | **546** | +++ | **547** | +++ | **548** | +++ |
| **549** | +++ | **550** | +++ | | | | |

| | | | | | | | |
|---|---|---|---|---|---|---|---|
| +++ means that IC₅₀ is less than or equal to 100 nM. ++ means that IC ₅₀ is 100 nM to 500 nM. + means that IC₅₀ is greater than 500 nM. | | | | | | | |

As can be seen from the data in Table 2, the compounds of the present invention have good inhibitory activity against the enzymatic activity of KIF18A.

### Biological Example 2. In Vitro Anti-Proliferative Activity of Compounds of the Present Invention Against HT-29 Cells

HT-29 cells were seeded on a 384-well plate at 3000 cells/well. After overnight adherence culture, DMSO or the compounds serially diluted in a 1:5 ratio from 5 µM were added. The viability was assessed 72 h after dosing by measuring the intracellular ATP content. The inhibition percentages of viable cells by the compounds were calculated by comparing with the DMSO group, and the IC ₅₀ values were also calculated. The results are shown in Table 3 below.

**Table 3. The anti-proliferative activity of the compounds of the present invention against HT-29 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | 9 | **4** | 63 | **5** | 88 | **7** | 73 |
| **33** | 52 | **49** | 61 | **65** | 8 | **66** | 85 |
| **68** | 51 | **69** | 71 | **71** | 56 | **81** | 51 |
| **97** | 57 | **113** | 81 | **129** | 40 | **513** | 71 |
| **522** | 69 | **530** | 36 | **537** | 95 | **539** | 43 |
| **583** | 38 | **584** | 70 | **AMG650** | 89 | | |

The reference compound AMG650 is compound 4 in WO2020132648A1.

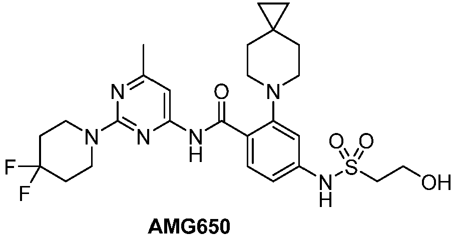

As can be seen from the data in Table 3, some of the compounds of the present invention have stronger anti-proliferative activities against HT-29 cells than AMG650.

### Biological Example 3. In Vitro Anti-Proliferative Activity of Compounds of the Present Invention Against HCT116 Cells

HCT116 cells were seeded on a 384-well plate at 3000 cells/well. After overnight adherence culture, DMSO or the compounds serially diluted in a 1:5 ratio from 5 µM were added. The viability was assessed 72 h after dosing by measuring the intracellular ATP content. The inhibition percentages of viable cells by the compounds were calculated by comparing with the DMSO group, and the IC ₅₀ values were also calculated. The results are shown in Table 4 below.

**Table 4. The anti-proliferative activity of the compounds of the present invention against HCT116 cells (IC₅₀, nM)**

| **Compound** | **IC₅₀** | **Compound** | **IC₅₀** | **Compound** | **IC₅₀** | **Compound** | **IC₅₀** |
|---|---|---|---|---|---|---|---|
| **1** | >10000 | **2** | >10000 | **3** | >10000 | **4** | >10000 |
| **5** | >10000 | **6** | >10000 | **7** | >10000 | **33** | >10000 |
| **49** | >10000 | **65** | >10000 | **66** | >10000 | **67** | >10000 |
| **68** | >10000 | **69** | >10000 | **70** | >10000 | **71** | >10000 |
| **81** | >10000 | **97** | >10000 | **113** | >10000 | **129** | >10000 |
| **385** | >10000 | **513** | >10000 | **522** | 8187 | **530** | >10000 |
| **537** | >10000 | **539** | >10000 | **583** | >10000 | **584** | >10000 |
| **AMG650** | >10000 | | | | | | |

As can be seen from the data in Table 4, none of the compounds of the present invention has anti-proliferative activity against HCT116 cells.

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A compound of general formula (1) or an isomer thereof, a crystalline form thereof, a pharmaceutically acceptable salt thereof, a hydrate thereof, or a solvate thereof: 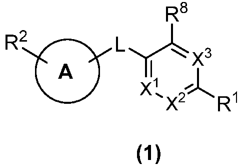 wherein in general formula (1):
X¹ is -CR⁵ = or N;
X² is -CR⁶ = or N;
X³ is -CR⁷ = or N;
ring A is 7-10 membered cycloalkylene, 7-10 membered heterocycloalkylene, 7-10 membered heteroarylene, or 10-membered arylene, wherein the 7-10 membered cycloalkylene, 7-10 membered heterocycloalkylene, 7-10 membered heteroarylene, or 10-membered arylene may be optionally substituted with 0, 1, 2, or 3 groups selected from H, halogen, -C₁₋₄ hydrocarbyl, -C₁₋₄ halohydrocarbyl, or -O-C₁₋₄ hydrocarbyl;
L is -(C=O)-NR³-**** or -NR³-(C=O)-****; **** represents attachment to a 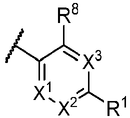 end;
R¹ is -CN or -Z-R¹⁰, wherein Z is a chemical bond, -C₀₋₄ hydrocarbylene-, -NR¹¹-, -NR¹¹SO₂-, -SO₂NR¹¹-, - NR¹¹-S(=O)(=NH)-**, -S(=O)(=NH)-, -S-, -S(=O)-, -SOz-, -C₀₋₄ hydrocarbylene-O-**, -(C=O)-, - (C=O)NR¹¹-, -C(=N-OH)-, or -NR¹¹(C=O)-**; or the group -Z-R¹⁰ is -N=S(=O)-(R¹⁰)₂, wherein the two R¹⁰ may be combined with the sulfur atom to which they are each attached to form a saturated or partially saturated 3-, 4-, 5-, or 6-membered monocyclic ring containing 0, 1, 2, or 3 N atoms and 0, 1 or 2 atoms selected from O and S; ** represents attachment to an R¹⁰ end;
R² is halogen or a group -Y-R¹², wherein Y is a chemical bond, -C₀₋₄ hydrocarbylene-, -N(C₀₋₁ hydrocarbyl)-C₀₋₄ hydrocarbylene-***, -C(=O)NR^{a} (C₁₋₄ hydrocarbyl)-***, -O-C₀₋₄ hydrocarbylene-***, -S-, -S(=O)-, - SO₂-, -SO₂NR¹²-***, or -S(=O)(=NH)-***; *** represents attachment to an R¹² end;
R³ is H or C₁₋₆ hydrocarbyl;
R⁵ is H, halogen, C₁₋₈ alkyl, or C₁₋₄ haloalkyl;
R⁶ is H, halogen, C₁₋₈ alkyl, C₁₋₄ haloalkyl, -OH, -O-R^{6a}, or -O-R^{6b};
R⁷ is H, halogen, C₁₋₈ hydrocarbyl, or C₁₋₄ halohydrocarbyl;
R⁸ is selected from the group consisting of: 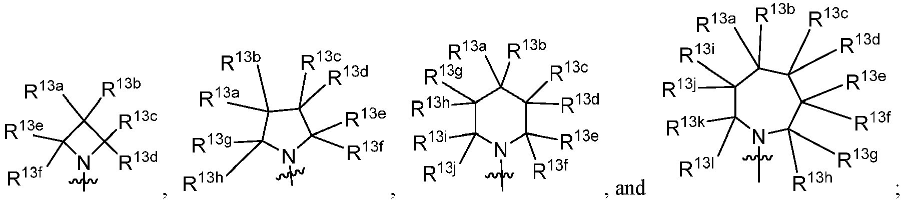
R^{13a}, R^{13b}, R^{13c}, R^{13d}, R^{13e}, R^{13f}, R^{13g}, R^{13h}, R¹³ⁱ, R^{13j}, R^{13k}, and R^{13l} are each independently H, halogen, R^{13m}, or R¹³ⁿ; or each of the pairs of R^{13a}/R^{13b}, R^{13c}/R^{13d}, R^{13e}/R^{13f}, R^{13g}/R^{13h}, R¹³ⁱ/R^{13j}, and R^{13k}/R^{13l} may independently form, with the carbon atom to which they are each attached, a saturated or partially saturated 3-, 4-, 5-, or 6-membered monocyclic ring spiro-linked to R⁸ ring, wherein the 3-, 4-, 5-, or 6-membered monocyclic ring contains 0, 1, 2, or 3 N atoms and 0, 1, or 2 atoms selected from O and S, and further, the 3-, 4-, 5-, or 6-membered monocyclic ring is substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -NR^{a}R^{a}, and oxo;
R¹⁰ is H, R^{10a}, R^{10b}, or R^{10c};
R¹¹ is H, R^{11a}, or R^{11b};
R¹² is R^{12a} or R^{12b};
R^{6a}, R^{10a}, R^{11a}, R^{12a}, or R^{13m}, in each case, is independently selected from a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0, 1 or 2 atoms selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -C(=O)R^{b}, - C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)R^{b}, -C₁₋₆ hydrocarbyl C(=O)NR^{a}R^{a}, -C₁₋₆ hydrocarbyl C(=O)OR^{a}, R⁴, and oxo;
R^{6b}, R^{10b}, R^{11b}, R^{12b}, or R¹³ⁿ, in each case, is independently selected from C₁₋₆ hydrocarbyl, wherein the hydrocarbyl may be optionally substituted with 0, 1, 2, 3, 4, or 5 groups selected from F, Cl, Br, -R^{a}, -OR^{a}, - OC₁₋₄ halohydrocarbyl, and CN;
R^{10c}, in each case, is independently selected from C₁₋₆ hydrocarbyl, wherein the hydrocarbyl may be optionally substituted with 0, 1, 2, 3, 4, or 5 groups selected from F, Cl, Br, -R^{a}, -R^{c}, -OR^{a}, -OC₁₋₄ halohydrocarbyl, and CN;
R4, in each case, is independently selected from the group consisting of: a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OR^{a}, -OC₁₋₄ halohydrocarbyl, CN, -C(=O)Rb, - C(=O)OR^{a}, -C(=O)NR^{a}R^{a}, -C(=NR^{a})NR^{a}R^{a}, -OC(=O)R^{b}, -OC(=O)NR^{a}R^{a}, -OC₂₋₆ hydrocarbyl NR^{a}R^{a}, -OC₂₋₆ hydrocarbyl OR^{a}, -SR^{a}, -S(=O)R^{b}, -S(=O)₂R^{b}, -S(=O)₂NR^{a}R^{a}, -NR^{a}R^{a}, -N(R^{a})C(=O)R^{b}, -N(R^{a})C(=O)OR^{b}, - N(R^{a})C(=O)NR^{a}R^{a}, -N(R^{a})C(=NR^{a})NR^{a}R^{a}, -N(R^{a})S(=O)₂R^{b}, -N(R^{a})S(=O)₂NR^{a}R^{a}, -NR^{a}C₂₋₆ hydrocarbyl NR^{a}R^{a}, -NR^{a}C₂₋₆ hydrocarbyl OR^{a}, -C₁₋₆ hydrocarbyl NR^{a}R^{a}, -C₁₋₆ hydrocarbyl OR^{a}, -C₁₋₆ hydrocarbyl N(R^{a})C(=O)R^{b}, -C₁₋₆ hydrocarbyl OC(=O)Rb, -C₁₋₆ hydrocarbyl C(=O)NR^{a}R^{a}, -C₁₋₆ hydrocarbyl C(=O)OR^{a}, and oxo;
R^{a}, in each case, is independently H or R^{b};
R^{b}, in each case, is independently C₁₋₆ hydrocarbyl, phenyl, or benzyl, wherein the hydrocarbyl may be optionally substituted with 0, 1, 2, or 3 groups selected from halogen, -OH, -OC₁₋₄ hydrocarbyl, -NH₂, -NHC₁₋₄ hydrocarbyl, -OC(=O)C₁₋₄ hydrocarbyl, or -N(C₁₋₄ hydrocarbyl) C₁₋₄ hydrocarbyl; and the phenyl and benzyl may be each independently and optionally substituted with 0, 1, 2, or 3 groups selected from halogen, C₁₋₄ hydrocarbyl, C₁₋₃ halohydrocarbyl, -OH, -OC₁₋₄ hydrocarbyl, -NH₂, -NHC₁₋₄ hydrocarbyl, -OC(=O)C₁₋₄ hydrocarbyl, or -N(C₁₋₄ hydrocarbyl) C₁₋₄ hydrocarbyl;
R^{c}, in each case, is independently -OC(=O)C₁₋₅ hydrocarbyl, wherein the hydrocarbyl may be optionally substituted with 1, 2, or 3 groups selected from -OH or -NH₂.

2. The compound or the isomer thereof, the crystalline form thereof, the pharmaceutically acceptable salt thereof, the hydrate thereof, or the solvate thereof according to claim 1, wherein the general formula (1) has the following structure:

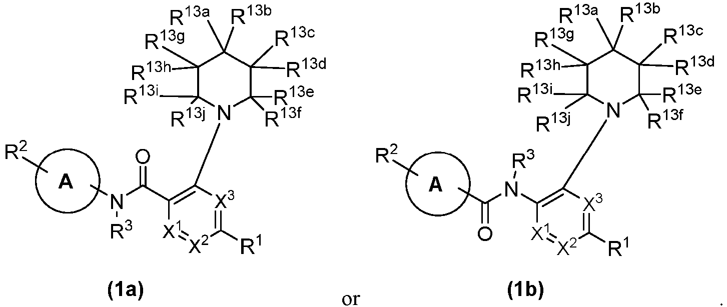

3. The compound or the isomer thereof, the crystalline form thereof, the pharmaceutically acceptable salt thereof, the hydrate thereof, or the solvate thereof according to any one of claims 1-2, wherein in the general formula (1), ring A is 9-10 membered cycloalkylene, 9-10 membered heterocycloalkylene, 9-10 membered heteroarylene, or 10-membered arylene, wherein the 9-10 membered cycloalkylene, 9-10 membered heterocycloalkylene, 9-10 membered heteroarylene, or 10-membered arylene may be optionally substituted with 0, 1, 2, or 3 groups selected from H, F, Cl, Br, -C₁₋₄ hydrocarbyl, -C₁₋₄ halohydrocarbyl, or -O-C₁₋₄ hydrocarbyl.

4. The compound or the isomer thereof, the crystalline form thereof, the pharmaceutically acceptable salt thereof, the hydrate thereof, or the solvate thereof according to any one of claims 1-3, wherein in the general formula (1), ring A is: 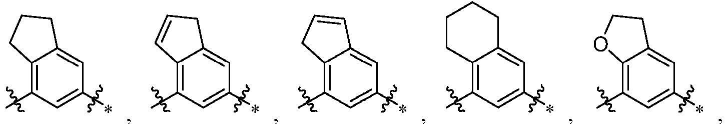 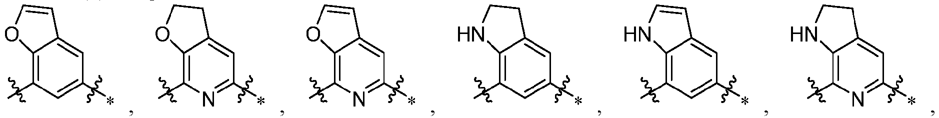 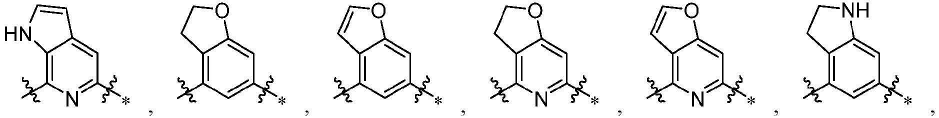 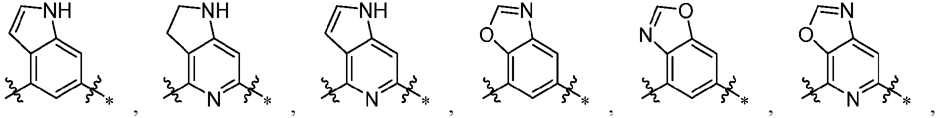 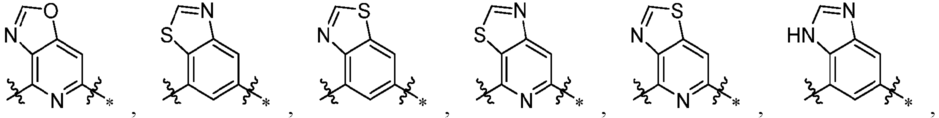 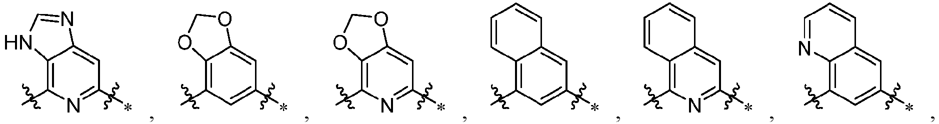 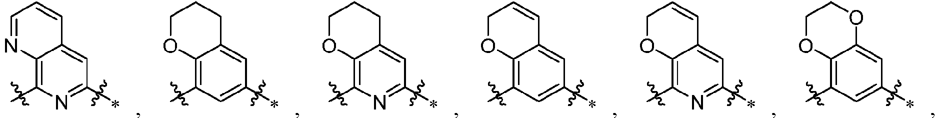 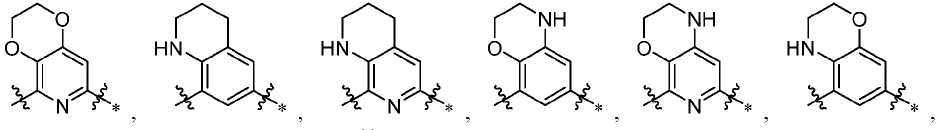 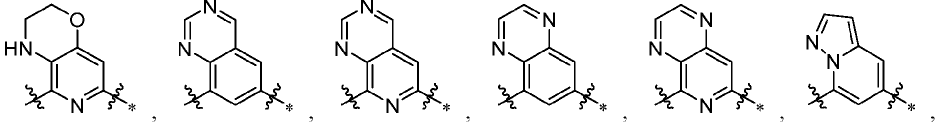 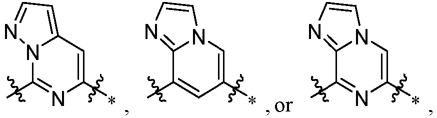 wherein * represents attachment to an L end; and the groups described above may be optionally substituted with 0, 1, 2, or 3 groups selected from H, F, Cl, Br, -CH₃, - CH₂CH₃, -CF₃, -CH₂CF₃, -OCH₃, or -OCH₂CH₃.

5. The compound or the isomer thereof, the crystalline form thereof, the pharmaceutically acceptable salt thereof, the hydrate thereof, or the solvate thereof according to any one of claims 1-3, wherein in the general formula (1), ring A is: 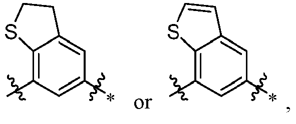 wherein * represents attachment to an L end; and the groups described above may be optionally substituted with 0, 1, 2, or 3 groups selected from H, F, Cl, Br, - CH₃, -CH₂CH₃, -CF₃, -CH₂CF₃, -OCH₃, or -OCH₂CH₃.

6. The compound or the isomer thereof, the crystalline form thereof, the pharmaceutically acceptable salt thereof, the hydrate thereof, or the solvate thereof according to any one of claims 1-5, wherein in the general formula (1), R³ is H, methyl, or ethyl, preferably H.

7. The compound or the isomer thereof, the crystalline form thereof, the pharmaceutically acceptable salt thereof, the hydrate thereof, or the solvate thereof according to claim 1, wherein in the general formula (1), R^{13c}, R'^{3a}, R^{13e}, R^{13f}, R^{13g}, R^{13h}, R¹³ⁱ, R^{13j}, R^{13k}, and R^{13l} are each independently H, halogen, C₁₋₆ hydrocarbyl, or C₁₋₄ halohydrocarbyl; and R^{13a} and R^{13b} in a pair of R^{13a}/R^{13b} may be combined with the carbon atom to which they are each attached to form a saturated 3-, 4-, or 5-membered monocyclic ring spiro-linked to R⁸ ring, wherein the monocyclic ring contains 0, 1, 2, or 3 N atoms and 0, 1, or 2 atoms selected from O and S; preferably, R^{13c}, R^{13d}, R^{13e}, R^{13f}, R^{13g}, R^{13h}, R¹³ⁱ, R^{13j}, R^{13k}, and R^{13l} are each independently H, methyl, or ethyl; and R^{13a} and R^{13b} in the pair of R^{13a}/R^{13b} may be combined with the carbon atom to which they are each attached to form a cyclopropyl, cyclobutyl, or cyclopentyl ring spiro-linked to R⁸ ring.

8. The compound or the isomer thereof, the crystalline form thereof, the pharmaceutically acceptable salt thereof, the hydrate thereof, or the solvate thereof according to any one of claims 1-7, wherein in the general formula (1), the structural unit 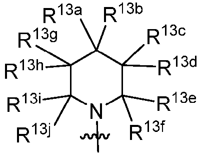 is: 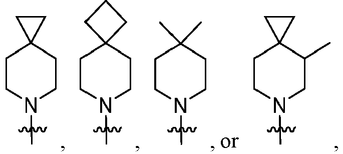 preferably

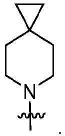

9. The compound or the isomer thereof, the crystalline form thereof, the pharmaceutically acceptable salt thereof, the hydrate thereof, or the solvate thereof according to any one of claims 1-8, wherein in the general formula (1), Z is a chemical bond, -NH-, -NHSO₂-, -SO₂NH-, -S(=O)(=NH)-, -S-, -S(=O)-, -SO₂-, -(C=O)-, - (C=O)NH-, or -NH(C=O)-.

10. The compound or the isomer thereof, the crystalline form thereof, the pharmaceutically acceptable salt thereof, the hydrate thereof, or the solvate thereof according to any one of claims 1-9, wherein, in the general formula (1), R¹⁰ is selected from: (a) H; or (b) C₁₋₆ hydrocarbyl, wherein the hydrocarbyl may be optionally substituted with 0, 1, 2, or 3 groups selected from F, Cl, Br, -OH, or -OCH₃; or (c) a group such that when the group -Z-R¹⁰ is -N=S(=O)-(R¹⁰)₂, the two R¹⁰ may be combined with the sulfur atom to which they are each attached to form a saturated, partially saturated, or unsaturated 3-, 4-, 5-, 6-, or 7-membered monocyclic ring containing 0, 1, 2, or 3 N atoms and 0 or 1 atom selected from O and S, wherein the monocyclic ring is substituted with 0, 1, 2, or 3 groups selected from F, Cl, Br, -C₁₋₆ hydrocarbyl, -C₁₋₄ halohydrocarbyl, -C₁₋₆ hydrocarbylene OH, -OH, -OCH₃, -NH₂, or oxo; or (d) C₁₋₆ hydrocarbyl, wherein the C₁₋₆ hydrocarbyl may be optionally substituted with 1, 2, or 3 groups selected from -OC(=O)C₁₋₅ hydrocarbyl, wherein the C₁₋₅ hydrocarbyl may be optionally substituted with 1 or 2 groups selected from -OH or -NH₂; and the C₁₋₆ hydrocarbyl may be optionally substituted with 0, 1, 2, or 3 groups selected from F, Cl, Br, -OH, or -OCH₃.

11. The compound or the isomer thereof, the crystalline form thereof, the pharmaceutically acceptable salt thereof, the hydrate thereof, or the solvate thereof according to any one of claims 1-10, wherein in the general formula (1), R¹ is -CN or a group -Z-R¹⁰, wherein Z is a chemical bond, -NH-, -NHSOz-, -SOzNH-, - S(=O)(=NH)-, -S-, -S(=O)-, -SOz-, -(C=O)-, -(C=O)NH-, or -NH(C=O)-; and R¹⁰ is selected from:
(a) H;
(b) cyclopropyl, cyclobutyl, cyclopentyl, cyclohexyl, oxiranyl, oxetanyl, tetrahydrofuranyl, azetidinyl, imidazolyl, morpholinyl, pyrrolidinyl, piperazinyl, 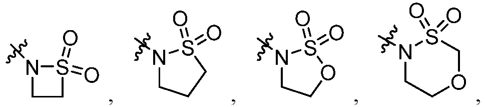 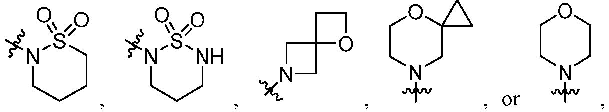 wherein each of the rings may be independently and optionally substituted with 0, 1, 2, or 3 groups selected from OH, F, methyl, -CH₂OH, - C(=O)OCH₃, -C(=O)OC(CH₃)₃, NH₂, CN, and oxo; preferably oxetanyl or cyclopropyl;
(c) C₁₋₆ hydrocarbyl substituted with 0, 1, 2, or 3 OH, F, -C(=O)OCH₃, -NH₂, -NH(CH₃), or -N(CH₃)₂; preferably C₁₋₆ hydrocarbyl substituted with 0, 1, 2, or 3 OH groups; and more preferably C1-6 hydrocarbyl substituted with 1 OH group; or
(d) C₁₋₆ hydrocarbyl, wherein the C₁₋₆ hydrocarbyl may be optionally substituted with 1, 2 or 3 groups selected from 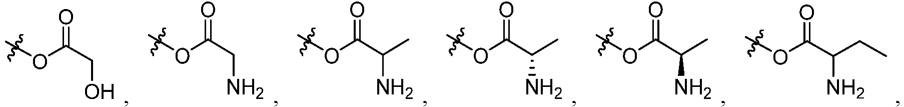 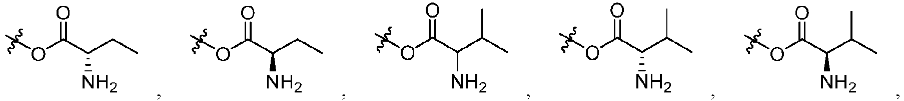 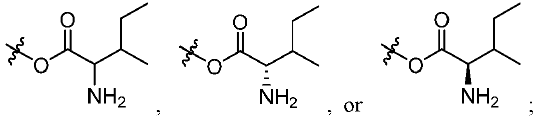 and the C₁₋₆ hydrocarbyl may be optionally substituted with 0, 1, 2, or 3 groups selected from F, Cl, Br, -OH, or -OCH₃.

12. The compound or the isomer thereof, the crystalline form thereof, the pharmaceutically acceptable salt thereof, the hydrate thereof, or the solvate thereof according to any one of claims 1-11, wherein in the general formula (1), the group -Z-R¹⁰ is -N=S(=O)-(R¹⁰)₂, wherein the two R¹⁰ may be combined with the sulfur atom to which they are each attached to form a saturated or partially saturated 3-, 4-, 5-, or 6-membered monocyclic ring containing 0, 1, 2, or 3 N atoms and 0, 1 or 2 atoms selected from O and S; preferably, the group -Z-R¹⁰ is selected from

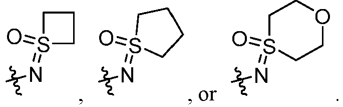

13. The compound or the isomer thereof, the crystalline form thereof, the pharmaceutically acceptable salt thereof, the hydrate thereof, or the solvate thereof according to any one of claims 1-11, wherein in the general formula (1), R¹ is a group -Z-R¹⁰, wherein Z is -NHSOz- or -SO₂NH-; and R¹⁰ is oxetanyl or cyclopropyl; or R¹⁰ is C₁₋₆ hydrocarbyl substituted with 0, 1, 2, or 3 OH groups; or R¹⁰ is C₁₋₆ hydrocarbyl, wherein the C₁₋₆ hydrocarbyl may be optionally substituted with 1, 2, or 3 groups selected from

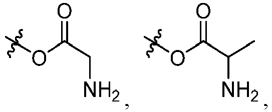

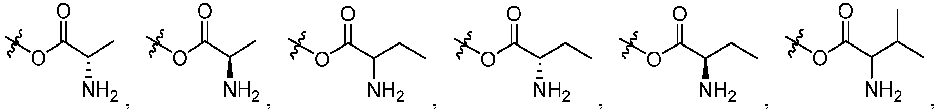

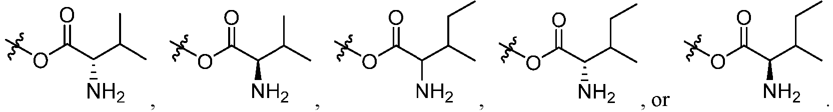

14. The compound or the isomer thereof, the crystalline form thereof, the pharmaceutically acceptable salt thereof, the hydrate thereof, or the solvate thereof according to any one of claims 1-9, wherein in the general formula (1), R¹⁰ is selected from C₁₋₆ hydrocarbyl, wherein the hydrocarbyl may be optionally substituted with 0, 1, 2, or 3 groups selected from 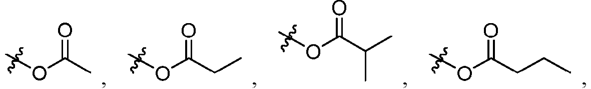 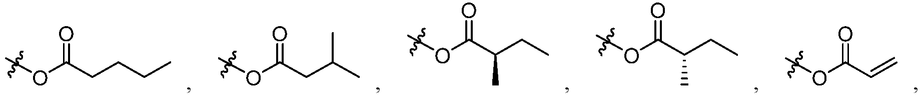 or 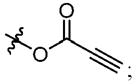 preferably 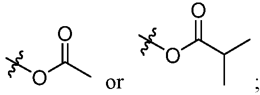 Z is -NHSOz- or -SOzNH-; Z is preferably -NHSO₂-.

15. The compound or the isomer thereof, the crystalline form thereof, the pharmaceutically acceptable salt thereof, the hydrate thereof, or the solvate thereof according to any one of claims 1-9, wherein in the general formula (1), R¹⁰ is selected from C₁₋₆ hydrocarbyl, wherein the hydrocarbyl may be optionally substituted with 1, 2 or 3 groups selected from 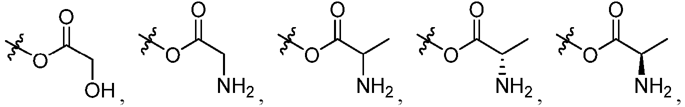 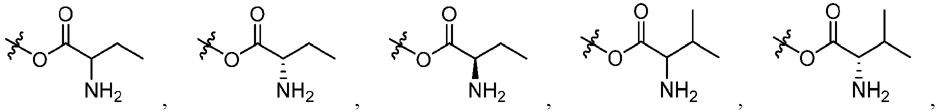 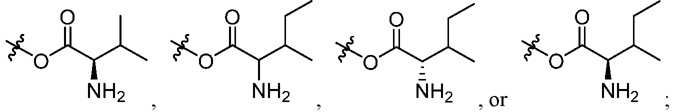 Z is -NHSOz- or -SO₂NH-.

16. The compound or the isomer thereof, the crystalline form thereof, the pharmaceutically acceptable salt thereof, the hydrate thereof, or the solvate thereof according to any one of claims 1-15, wherein in the general formula (1), R² is halogen or a group -Y-R¹², wherein Y is a chemical bond, -NH-, -NH-(CH₂)₀₋₄-, or -O-(CH₂)₀₋₄-; and R¹² is a saturated, partially saturated, or unsaturated 3-, 4-, 5-, 6-, or 7-membered monocyclic ring or 4-, 5-, 6-, 7-, 8-, 9-, 10-, 11-, or 12-membered bicyclic ring containing 0, 1, 2, or 3 N atoms and 0 or 1 atom selected from O and S, wherein the monocyclic ring and bicyclic ring may be each independently and optionally substituted with 0, 1, 2, or 3 groups selected from F, Cl, Br, C₁₋₆ hydrocarbyl, C₁₋₄ halohydrocarbyl, -OH, -OC₁₋₄ halohydrocarbyl, CN, R¹⁴, and oxo; or R¹² is C₁₋₆ hydrocarbyl, wherein the hydrocarbyl may be optionally substituted with 0, 1, 2, 3, 4, or 5 groups selected from F, Cl, Br, -OH, -OC₁₋₄ halohydrocarbyl, or CN.

17. The compound or the isomer thereof, the crystalline form thereof, the pharmaceutically acceptable salt thereof, the hydrate thereof, or the solvate thereof according to any one of claims 1-16, wherein in the general formula (1), R² is a saturated 5- or 6-membered monocyclic ring, wherein each of the rings contains 0, 1 or 2 N atoms and 0 or 1 O atom, and each of the rings is substituted with 0, 1, 2, or 3 groups selected from F, Cl, Br, C₁₋₆ hydrocarbyl, C₁₋₄ halohydrocarbyl, -OH, -OC₁₋₄ halohydrocarbyl, CN, R¹⁴, and oxo.

18. The compound or the isomer thereof, the crystalline form thereof, the pharmaceutically acceptable salt thereof, the hydrate thereof, or the solvate thereof according to any one of claims 1-17, wherein in the general formula (1), R² is: (a) halogen; (b) a group -Y-R¹², wherein Y is a chemical bond; and R¹² is morpholinyl, piperidinyl, azetidinyl, pyrrolidinyl, cyclopropyl, cyclobutyl, cyclopentyl, cyclohexyl, piperazinyl, tetrahydrofuranyl, 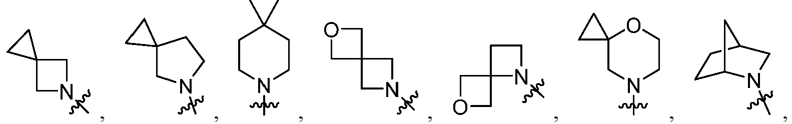 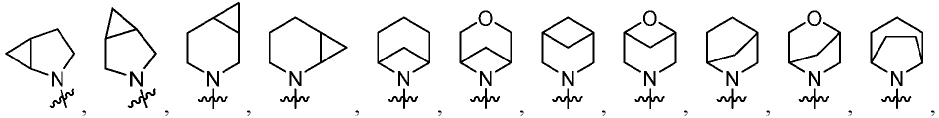 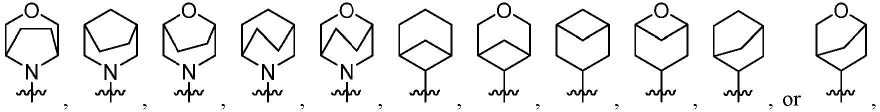 wherein each of the rings is substituted with 0, 1, 2, or 3 groups selected from F, Cl, Br, methyl, CF₃, -OH, -OCHF₂, CN, and oxo; or (c) a group -Y-R¹², wherein Y is -NH-, -O-, -O-(CHz)-, -O-(CHz)-(CHz)-, or -O-(CHz)-(CHz)-(CH₂)-, and R¹² is 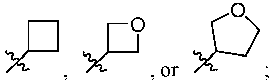 or R¹² is C₁₋₆ hydrocarbyl, wherein the hydrocarbyl may be optionally substituted with 0, 1, 2, 3, 4, or 5 groups selected from F, Cl, Br, methyl, CF3, -OH, or CN.

19. The compound or the isomer thereof, the crystalline form thereof, the pharmaceutically acceptable salt thereof, the hydrate thereof, or the solvate thereof according to any one of claims 1-18, wherein in the general formula (1), R² is morpholinyl or piperidinyl, wherein the morpholinyl and piperidinyl may be optionally substituted with 0, 1, 2, or 3 groups selected from F, Cl, Br, methyl, CF₃, -OH, -OCHF₂, and CN.

20. The compound or the isomer thereof, the crystalline form thereof, the pharmaceutically acceptable salt thereof, the hydrate thereof, or the solvate thereof according to claim 19, wherein in the general formula (1), R² is piperidinyl substituted with 1, 2, or 3 fluorine groups.

21. The compound or the isomer thereof, the crystalline form thereof, the pharmaceutically acceptable salt thereof, the hydrate thereof, or the solvate thereof according to any one of claims 1-18, wherein in the general formula (1), R² is:

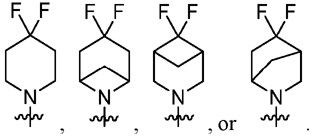

22. The compound or the isomer thereof, the crystalline form thereof, the pharmaceutically acceptable salt thereof, the hydrate thereof, or the solvate thereof according to claim 19, wherein in the general formula (1), R² is morpholinyl substituted with 1, 2 or 3 methyl groups.

23. The compound or the isomer thereof, the crystalline form thereof, the pharmaceutically acceptable salt thereof, the hydrate thereof, or the solvate thereof according to any one of claims 1-18, wherein in the general formula (1), R² is

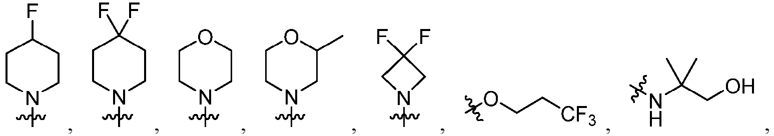

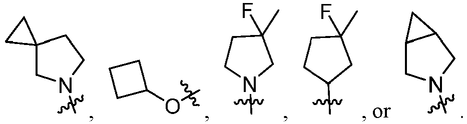

24. The compound or the isomer thereof, the crystalline form thereof, the pharmaceutically acceptable salt thereof, the hydrate thereof, or the solvate thereof according to any one of claims 1-10, wherein in the general formula (1), R¹⁰ is selected from cyclopropyl, cyclobutyl, cyclopentyl, oxetanyl, azetidinyl, tetrahydrofuranyl, or 1,3,4-oxathiazinanyl.

25. The compound or the isomer thereof, the crystalline form thereof, the pharmaceutically acceptable salt thereof, the hydrate thereof, or the solvate thereof according to any one of claims 1-24, wherein in the general formula (1), R⁵ is H or F, preferably H.

26. The compound or the isomer thereof, the crystalline form thereof, the pharmaceutically acceptable salt thereof, the hydrate thereof, or the solvate thereof according to any one of claims 1-25, wherein in the general formula (1), R⁶ is H or F, preferably H.

27. The compound or the isomer thereof, the crystalline form thereof, the pharmaceutically acceptable salt thereof, the hydrate thereof, or the solvate thereof according to any one of claims 1-26, wherein in the general formula (1), R⁷ is H.

28. The compound or the isomer thereof, the crystalline form thereof, the pharmaceutically acceptable salt thereof, the hydrate thereof, or the solvate thereof according to any one of claims 1-27, wherein the compound has one of the following structures:

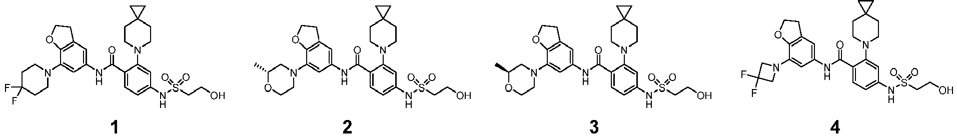

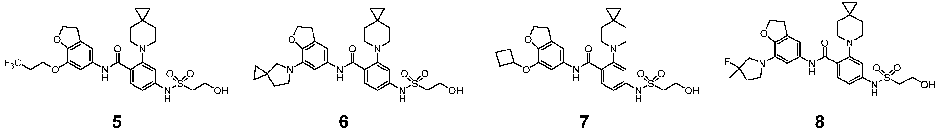

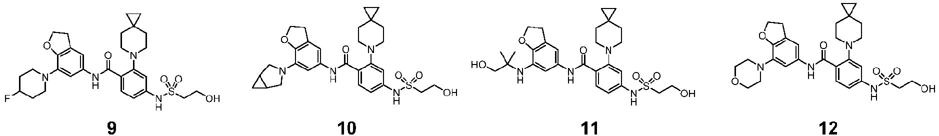

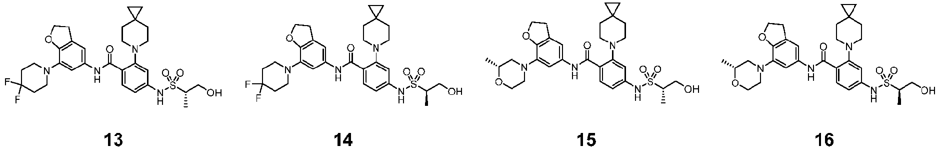

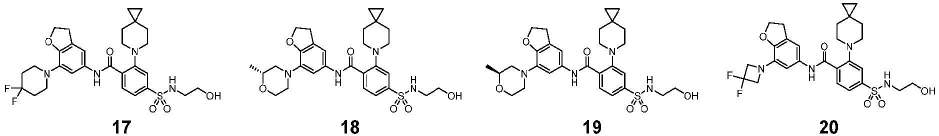

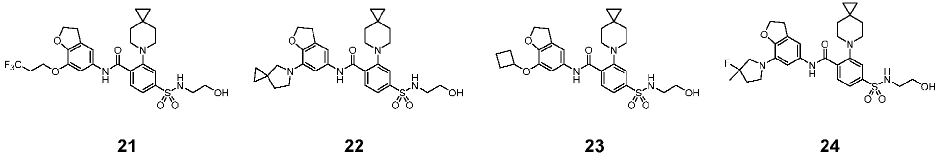

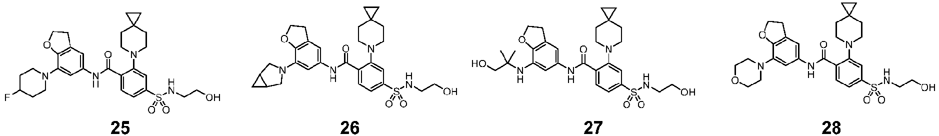

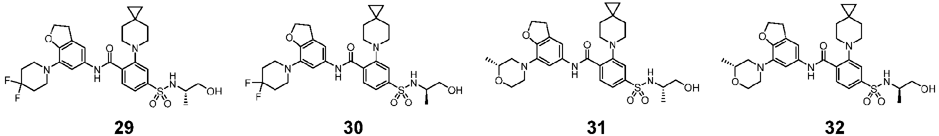

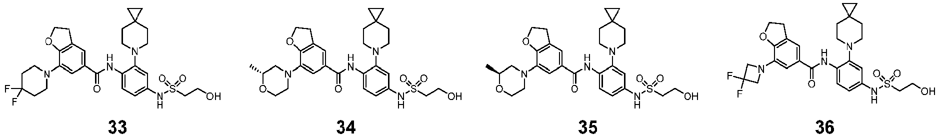

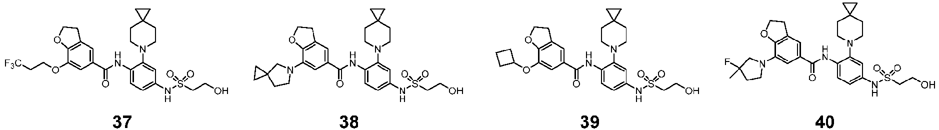

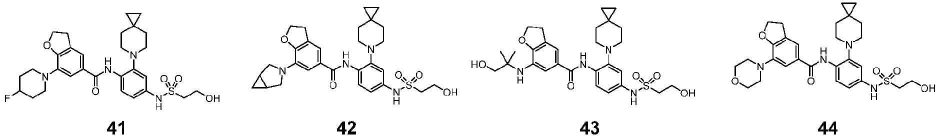

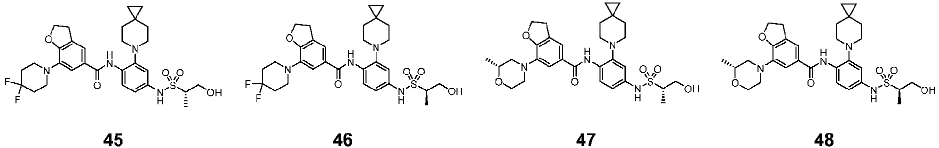

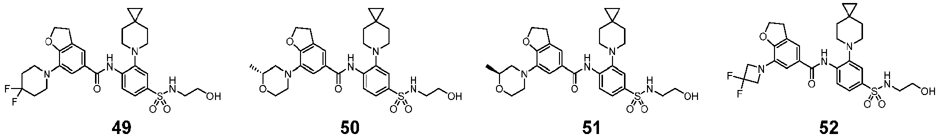

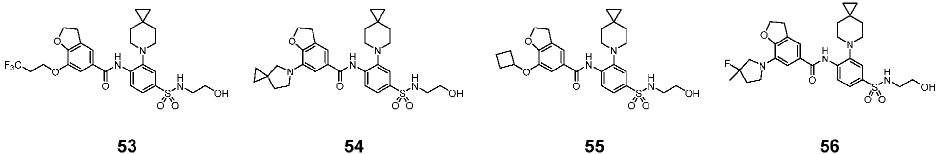

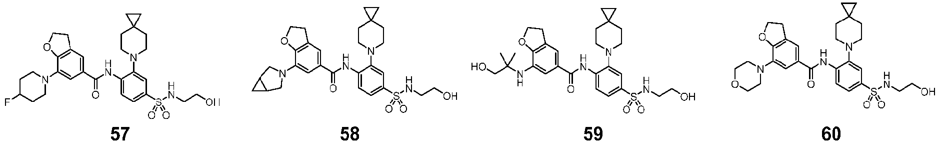

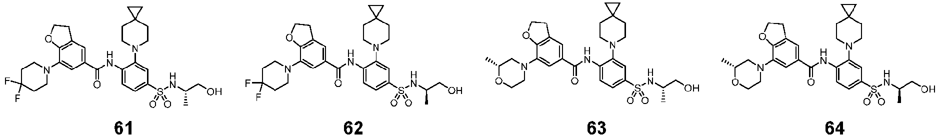

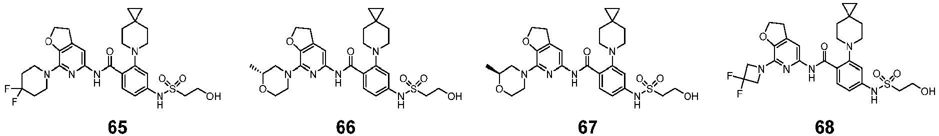

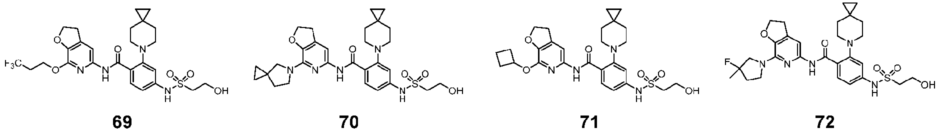

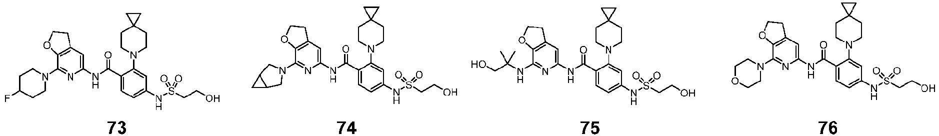

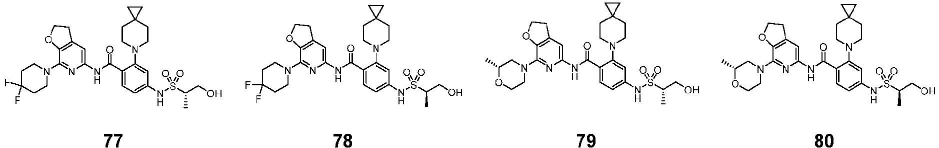

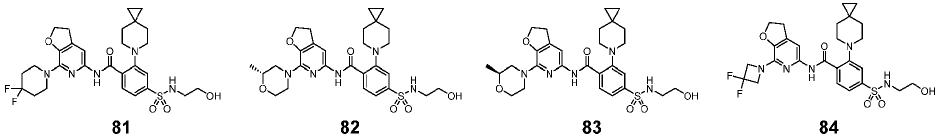

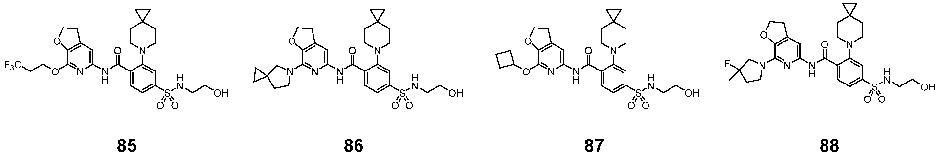

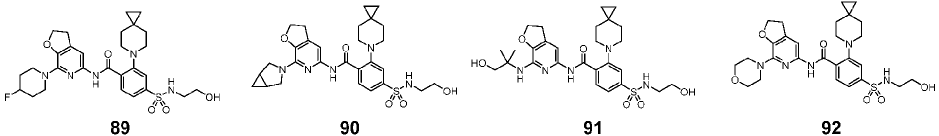

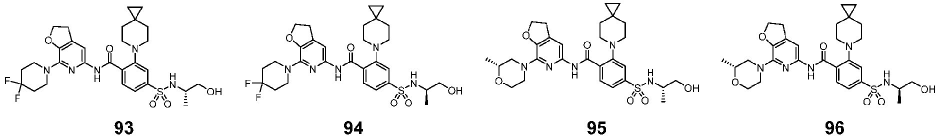

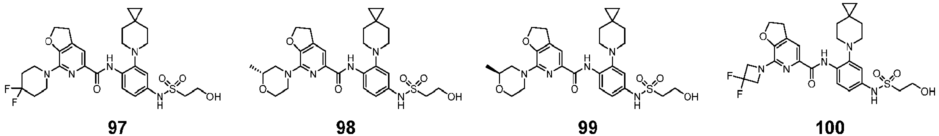

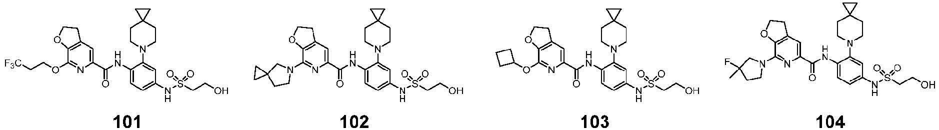

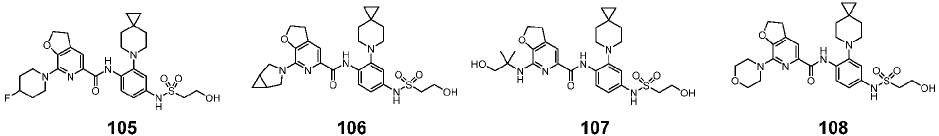

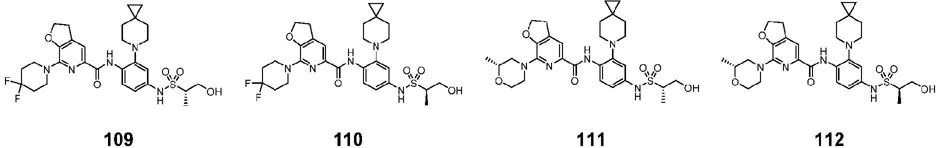

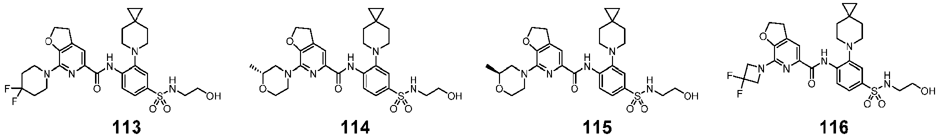

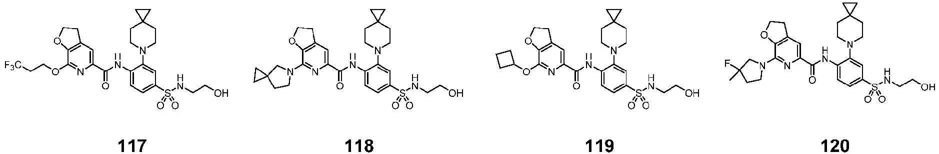

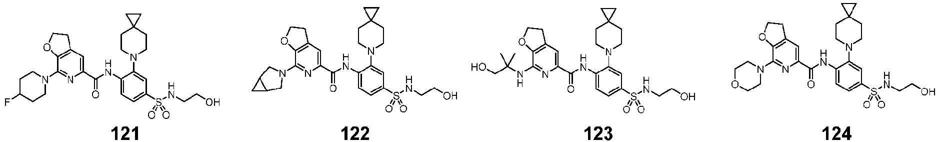

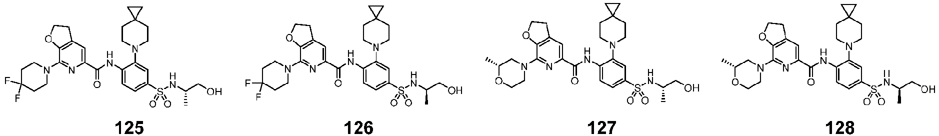

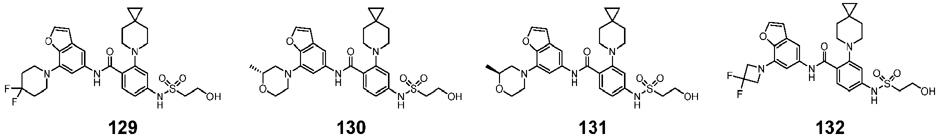

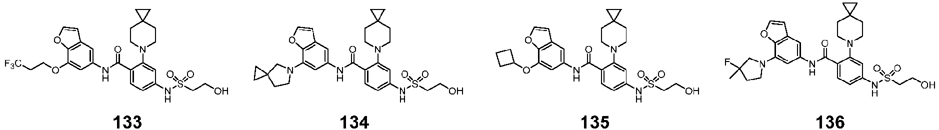

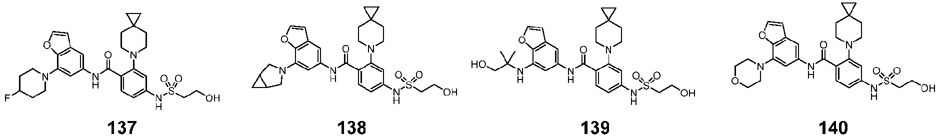

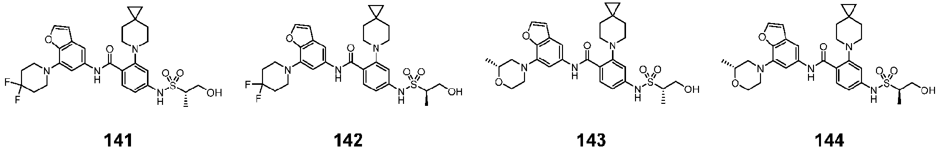

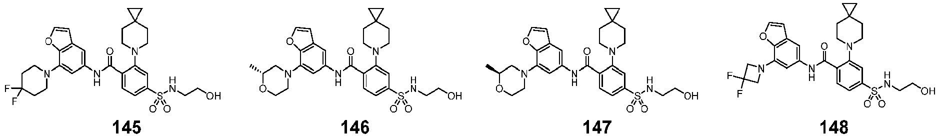

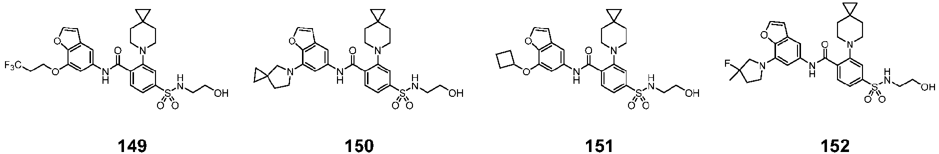

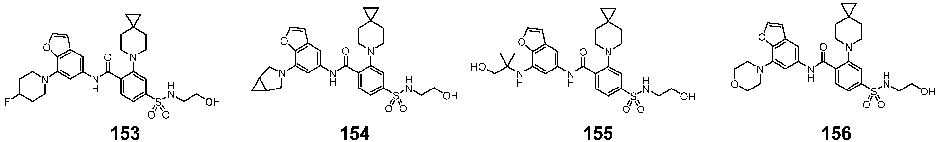

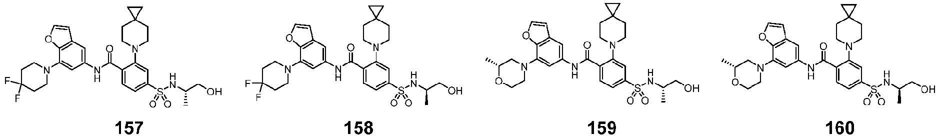

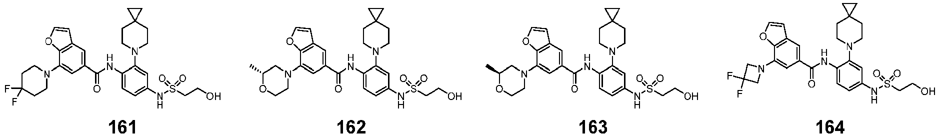

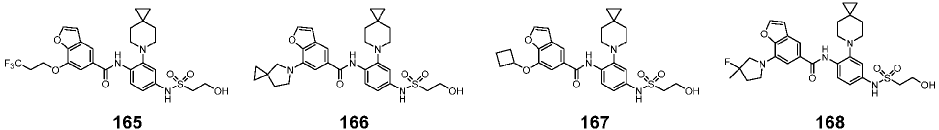

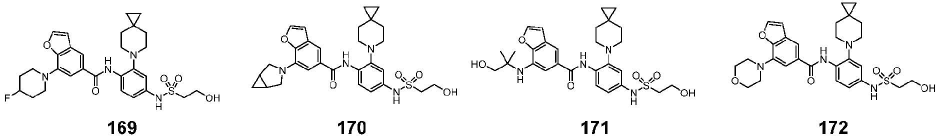

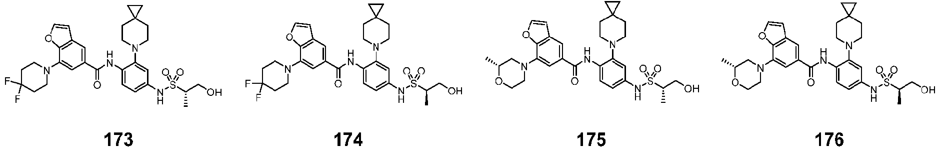

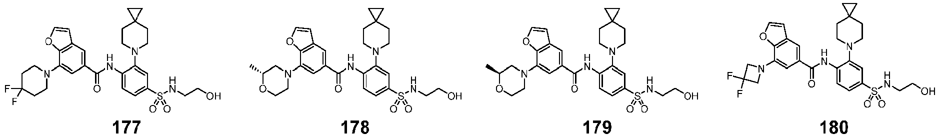

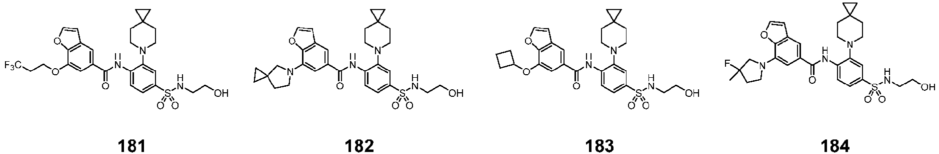

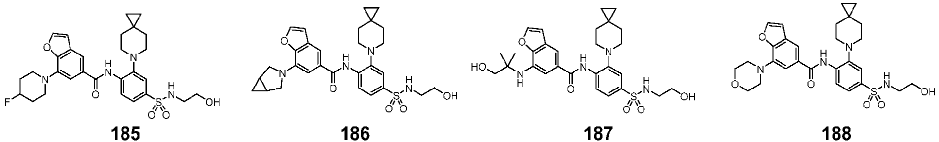

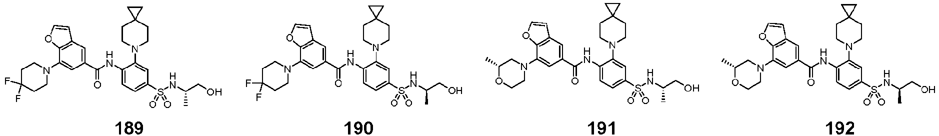

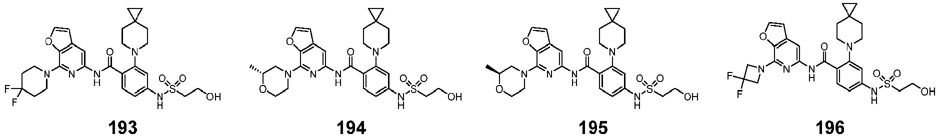

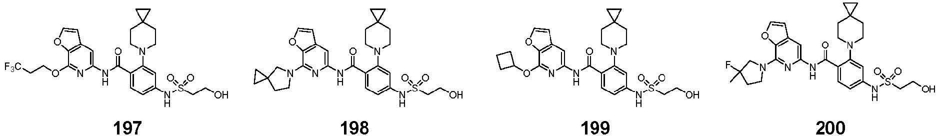

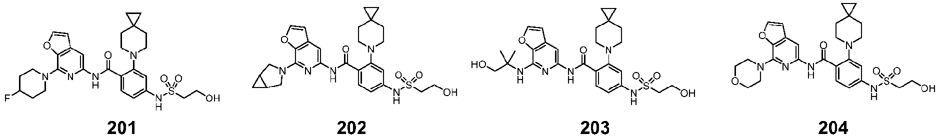

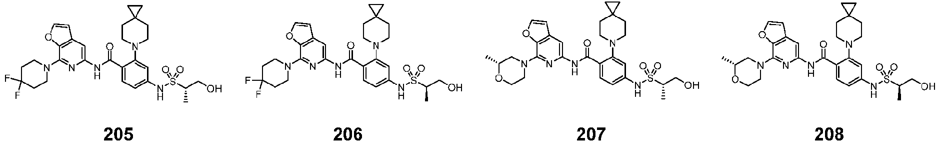

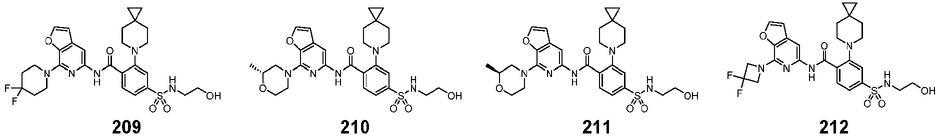

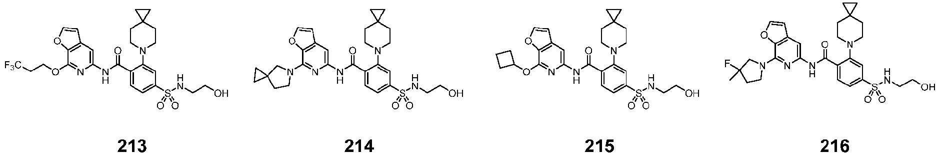

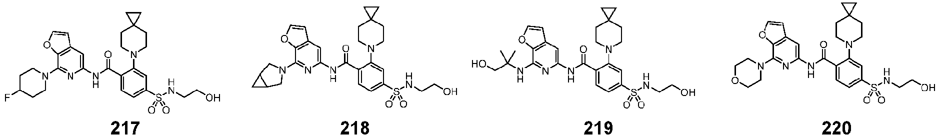

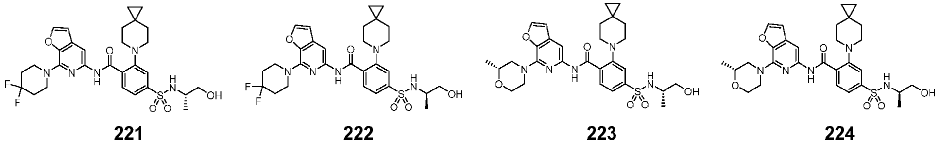

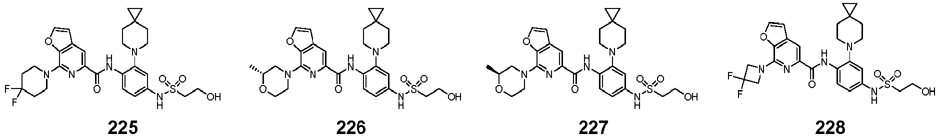

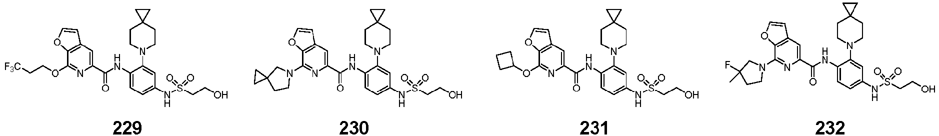

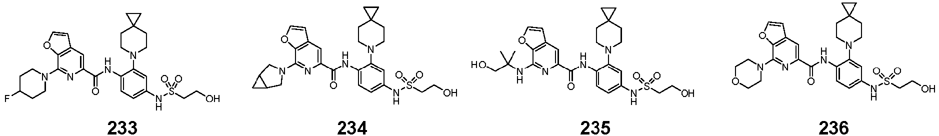

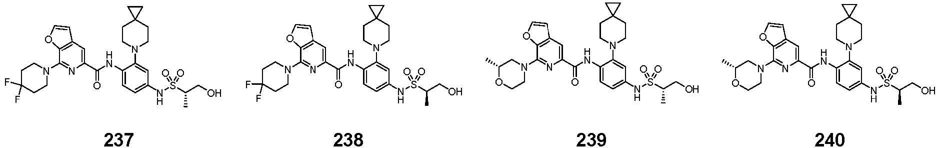

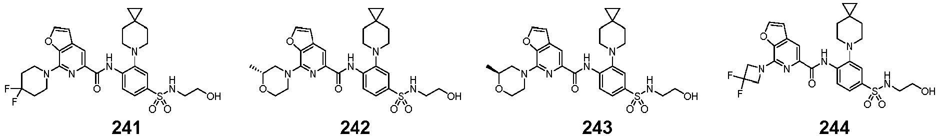

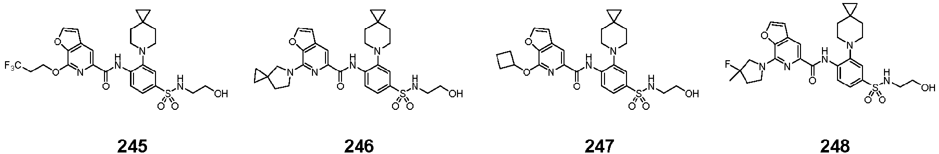

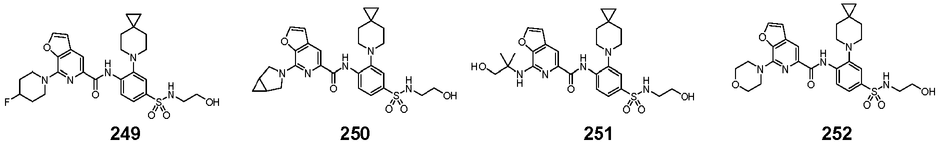

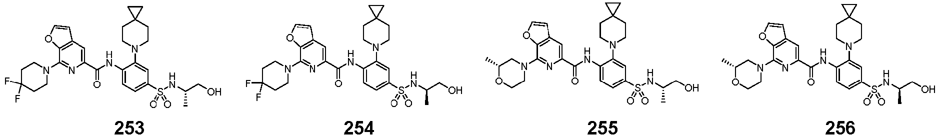

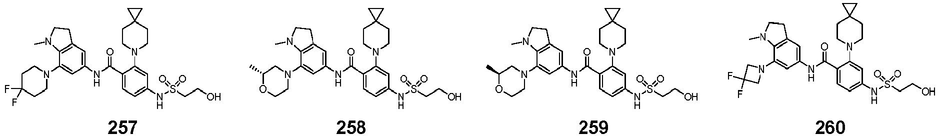

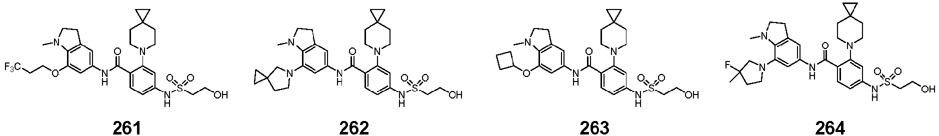

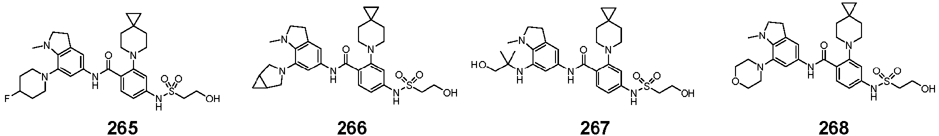

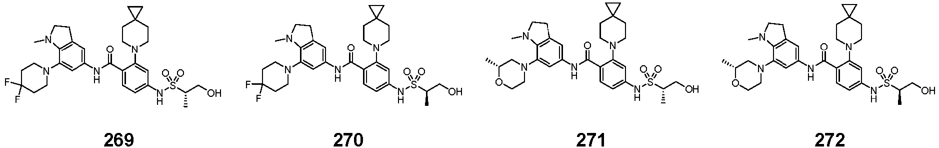

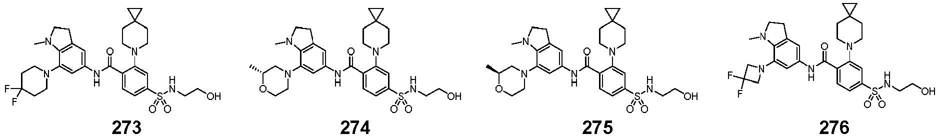

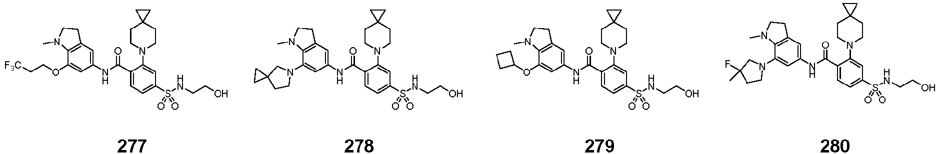

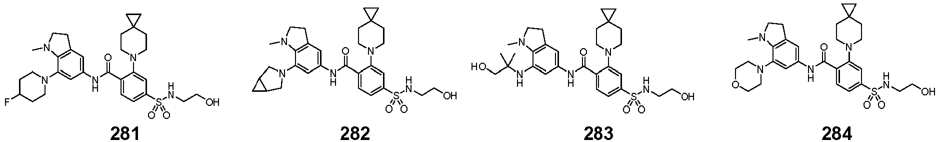

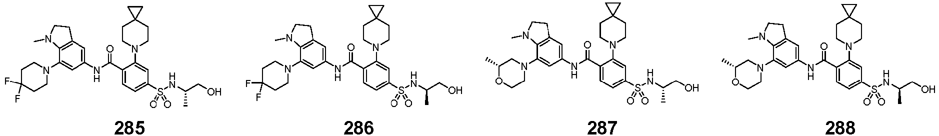

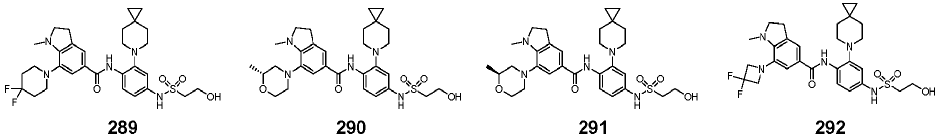

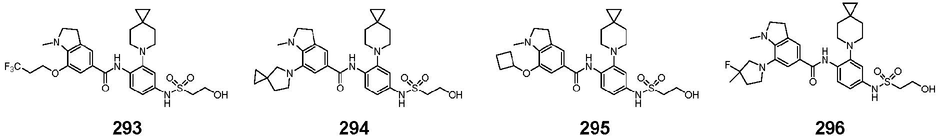

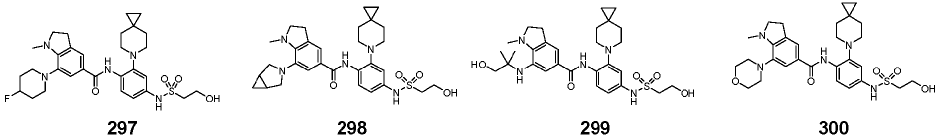

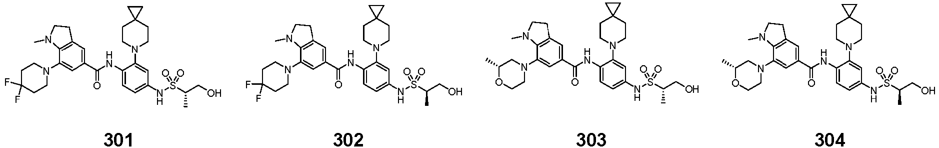

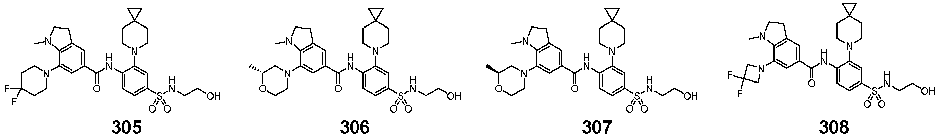

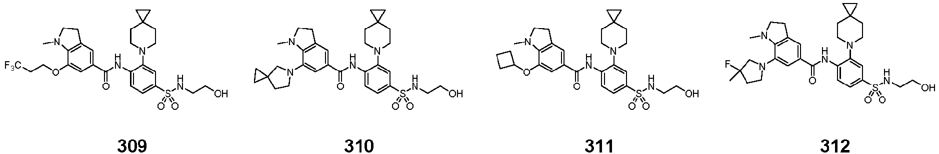

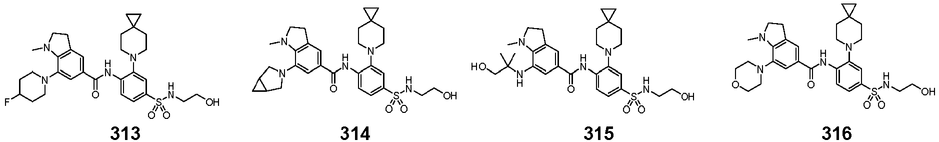

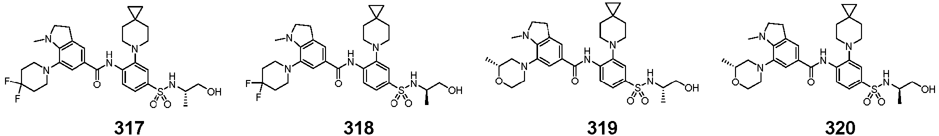

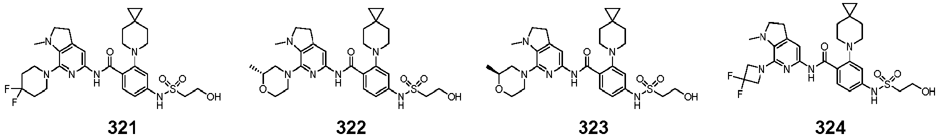

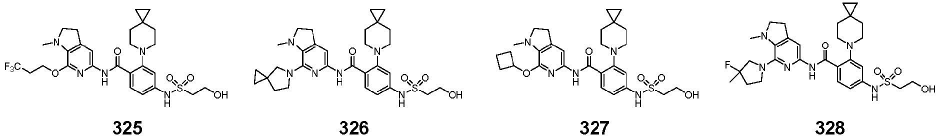

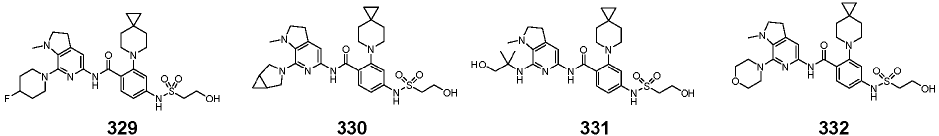

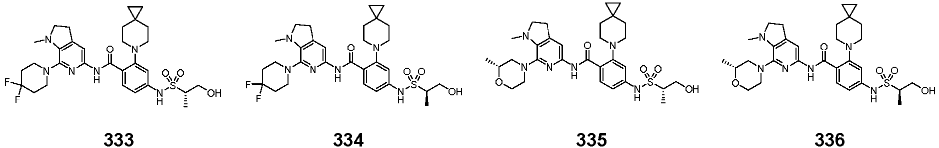

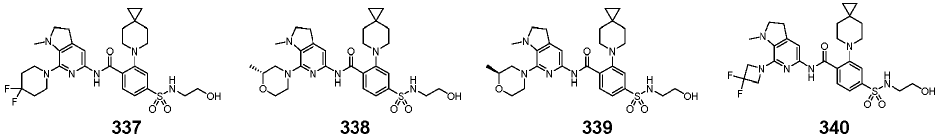

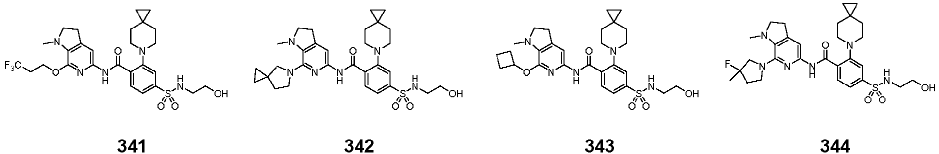

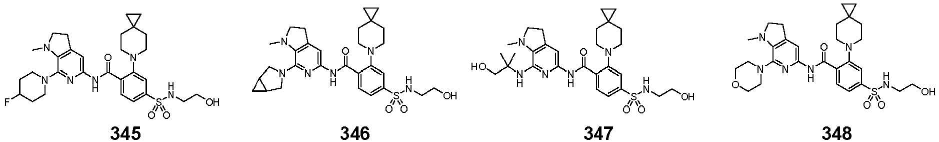

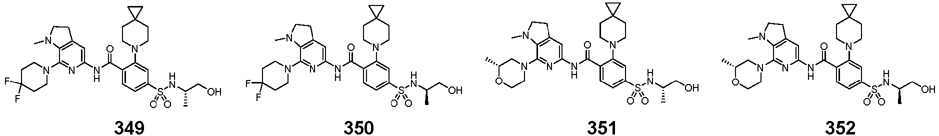

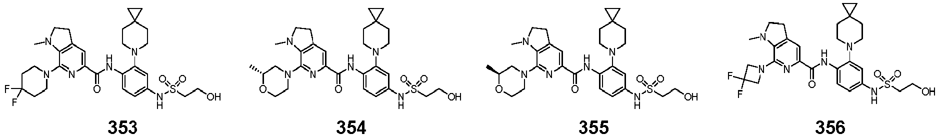

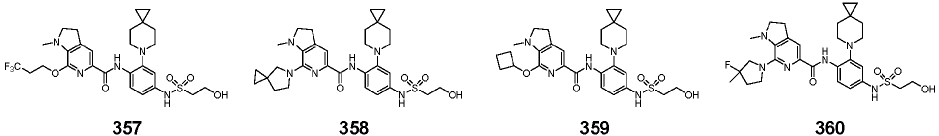

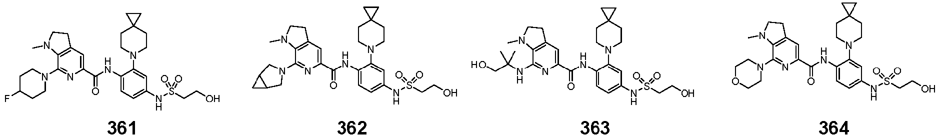

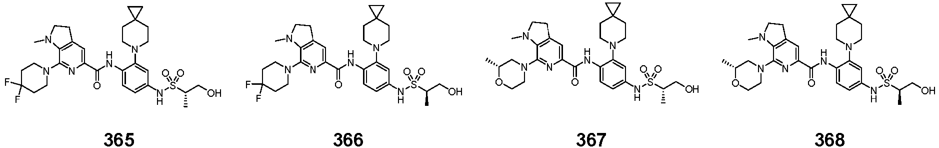

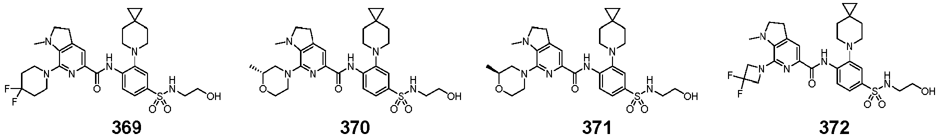

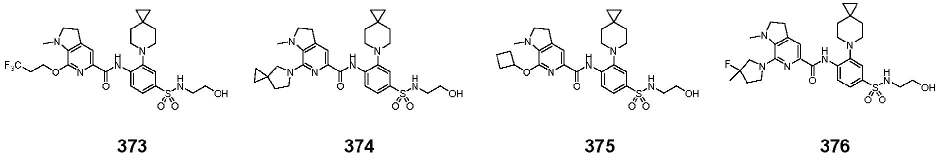

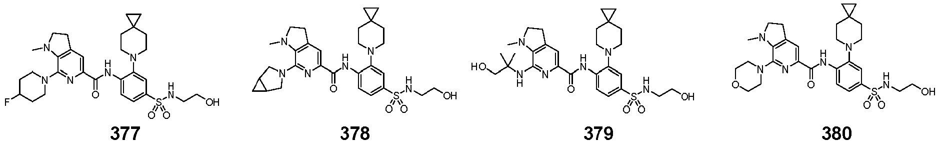

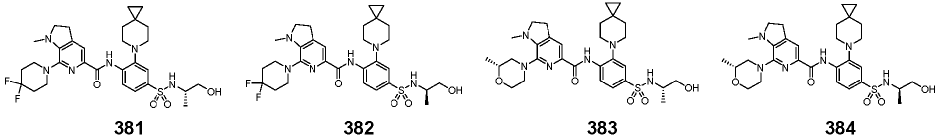

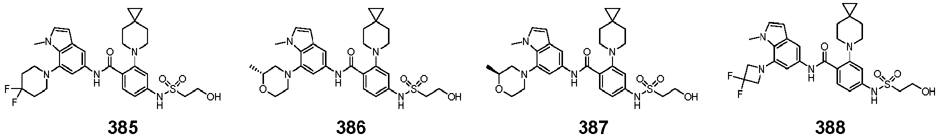

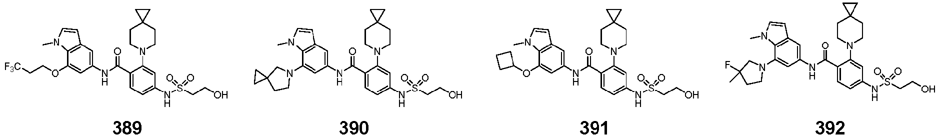

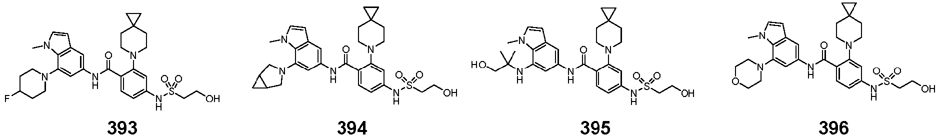

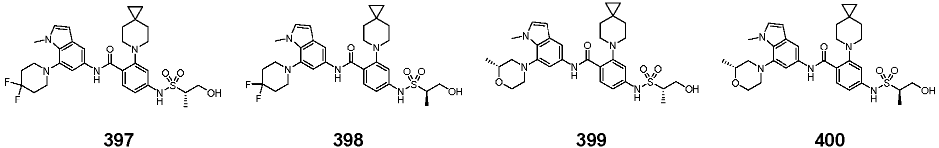

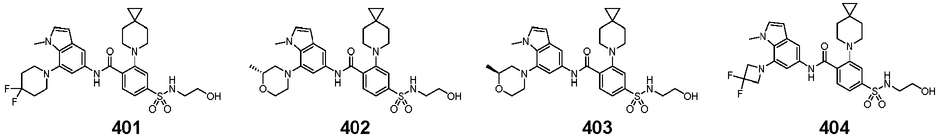

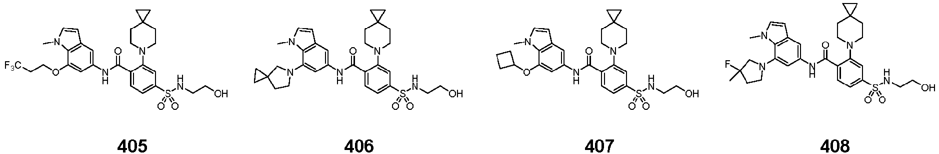

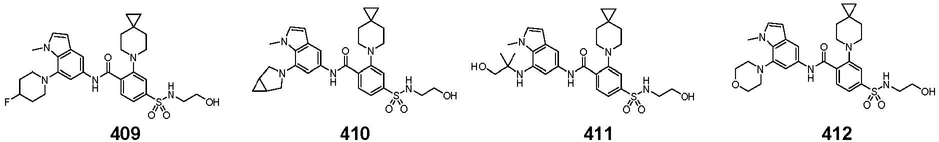

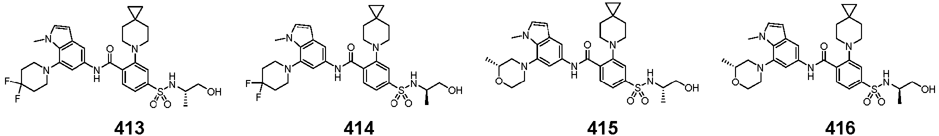

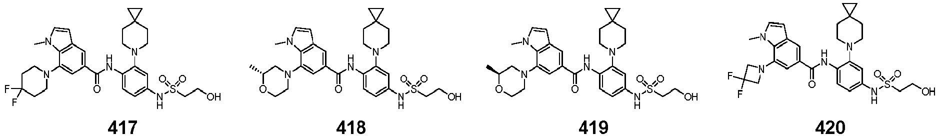

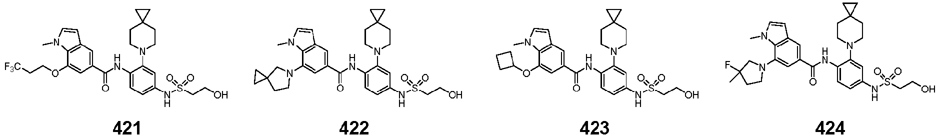

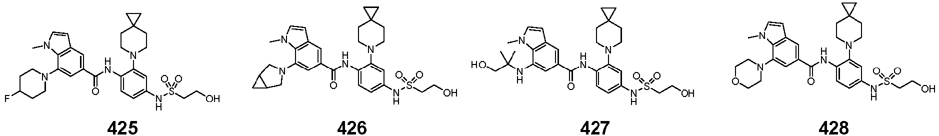

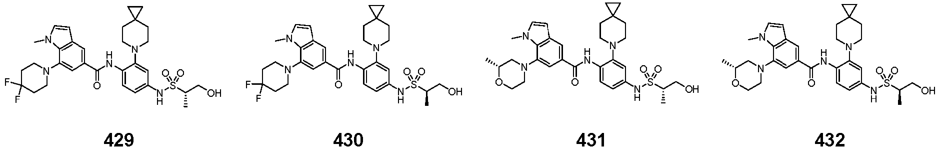

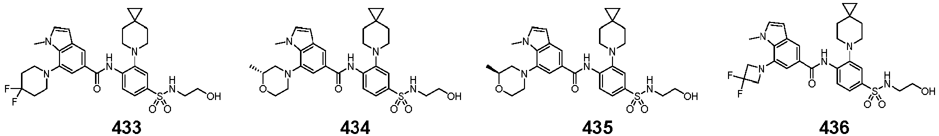

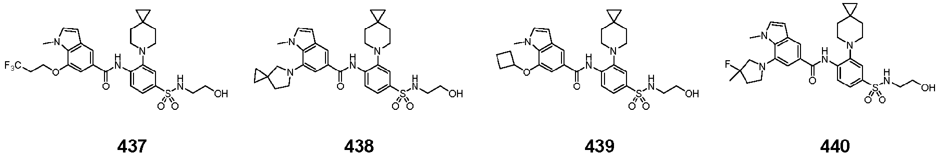

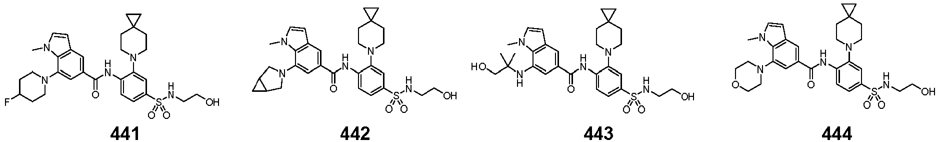

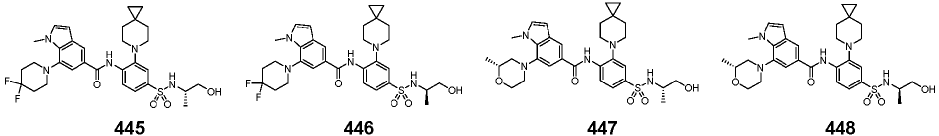

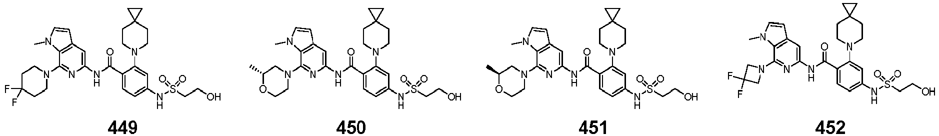

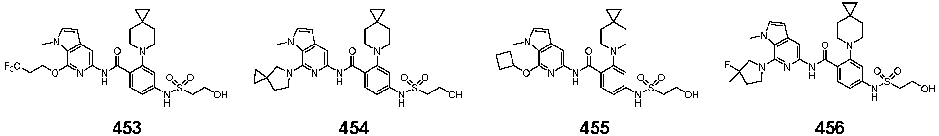

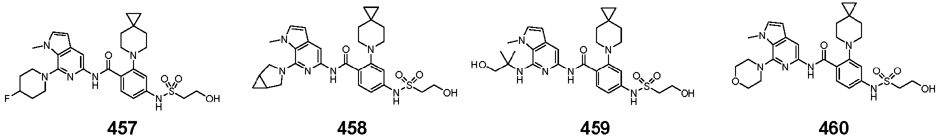

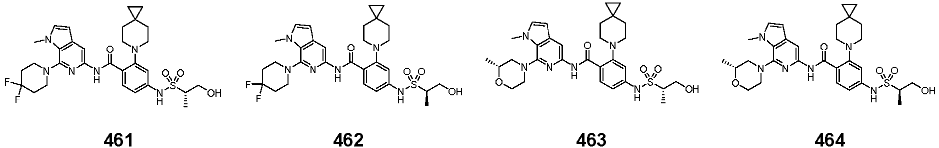

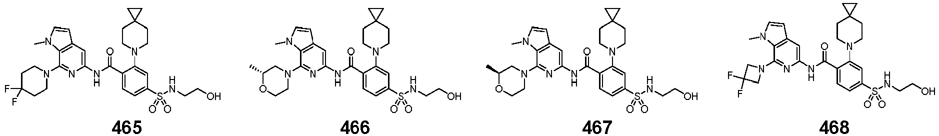

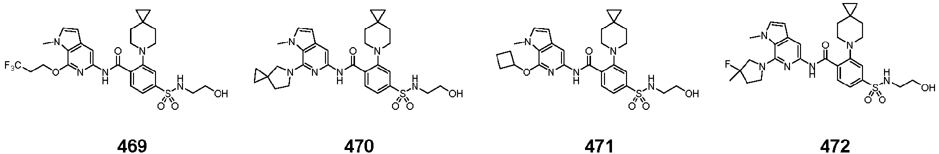

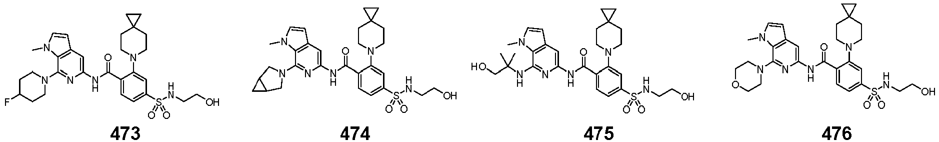

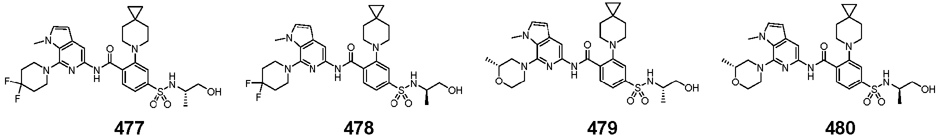

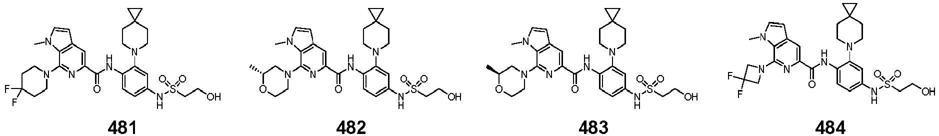

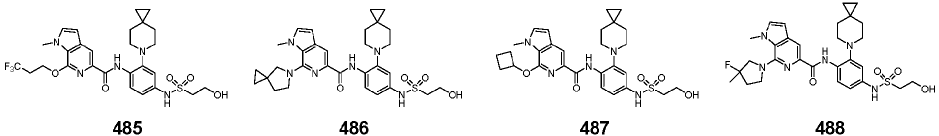

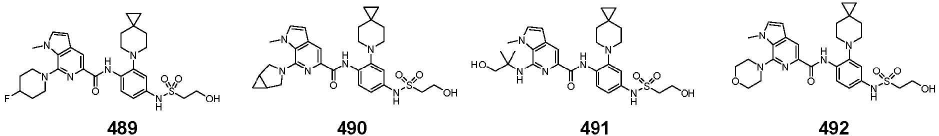

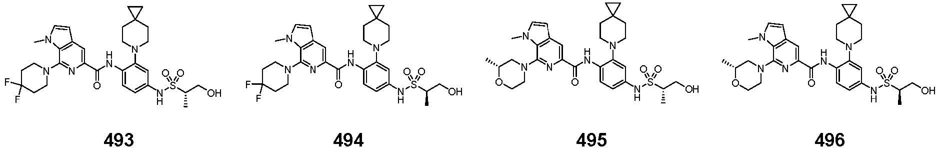

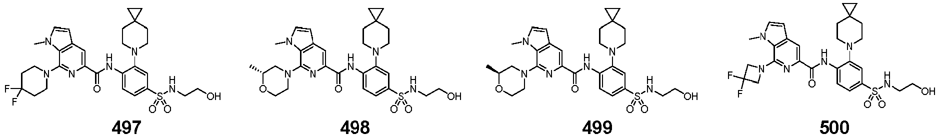

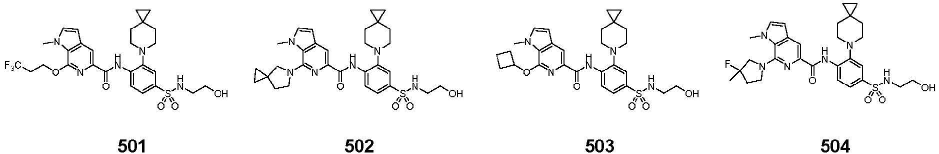

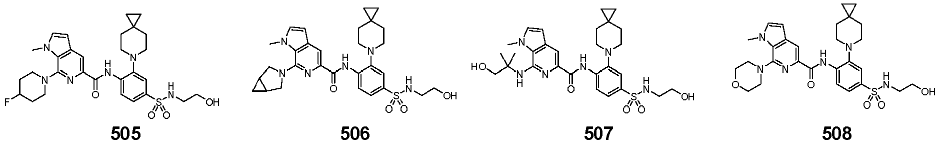

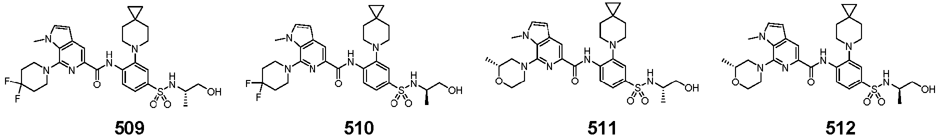

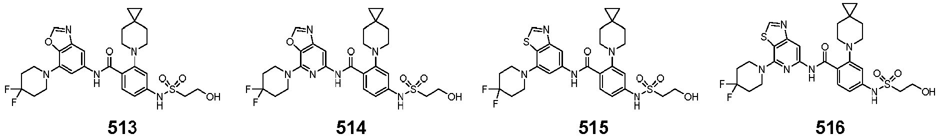

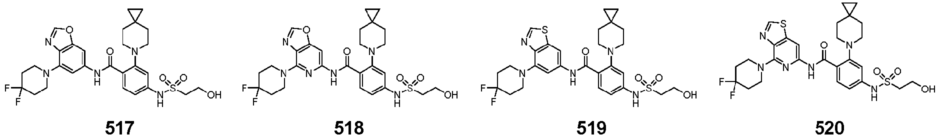

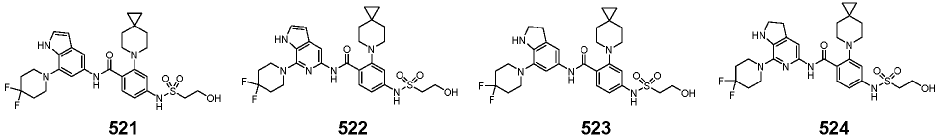

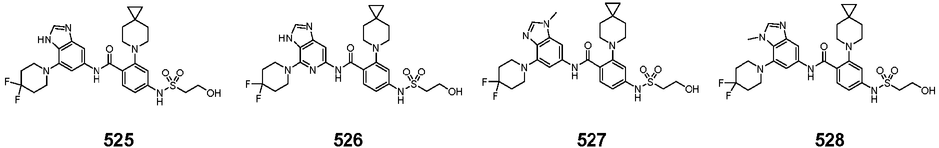

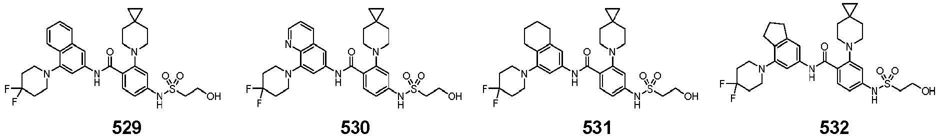

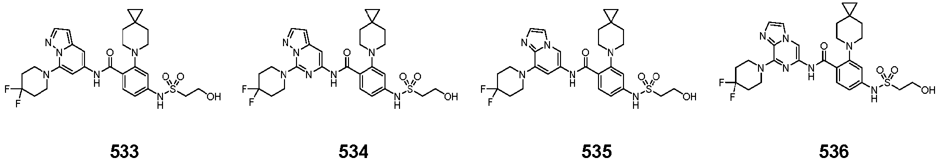

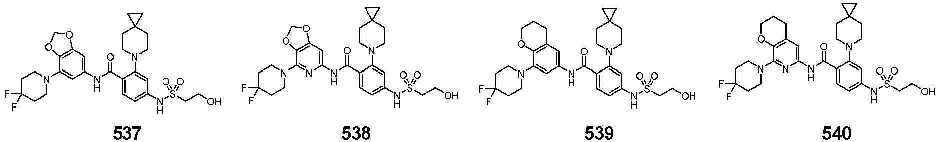

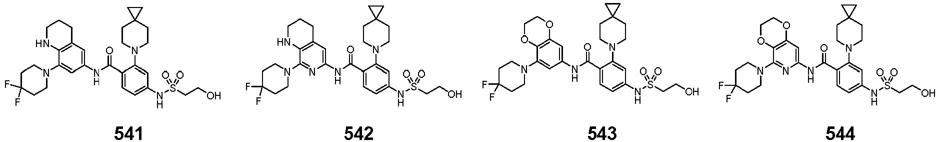

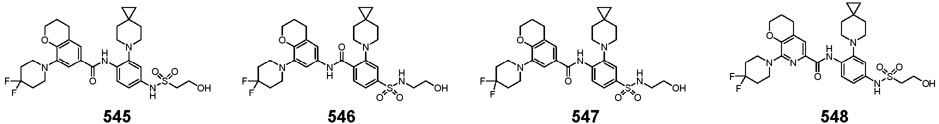

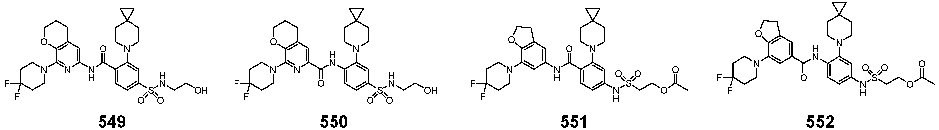

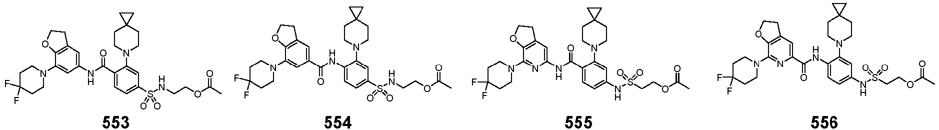

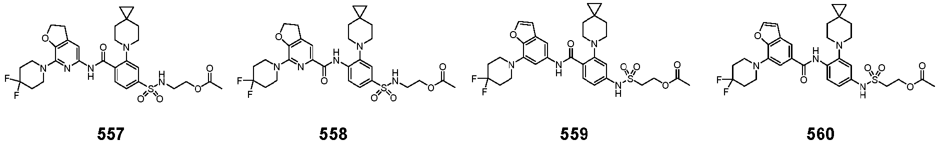

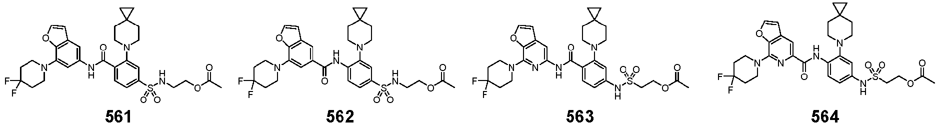

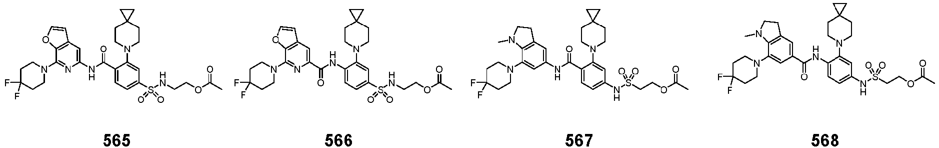

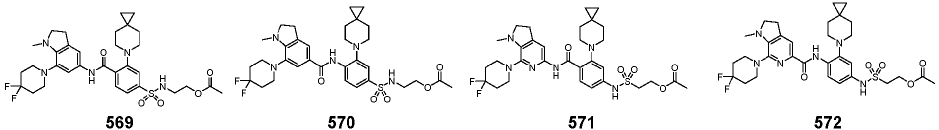

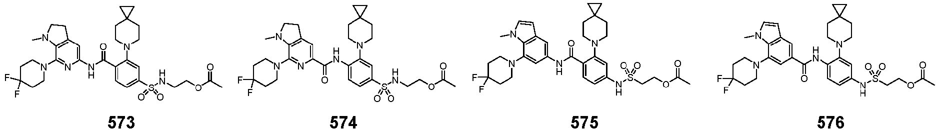

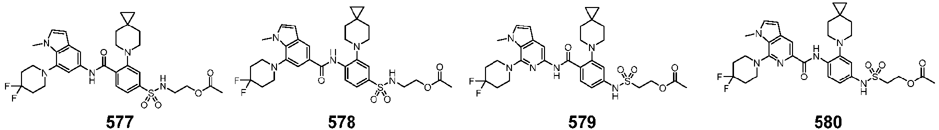

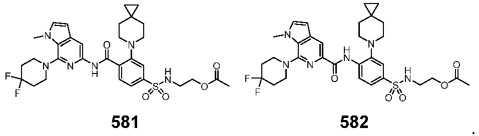

29. The compound or the isomer thereof, the crystalline form thereof, the pharmaceutically acceptable salt thereof, the hydrate thereof, or the solvate thereof according to any one of claims 1-27, wherein the compound has one of the following structures:

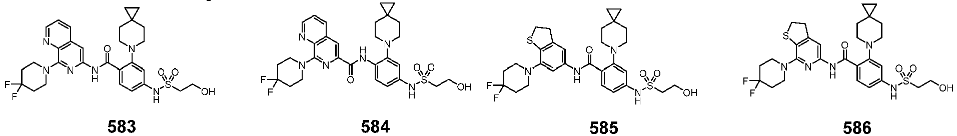

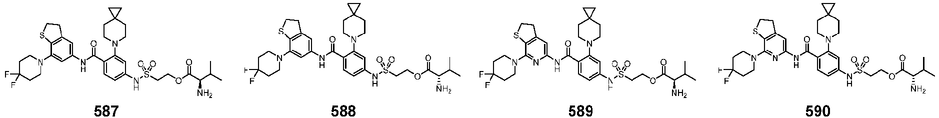

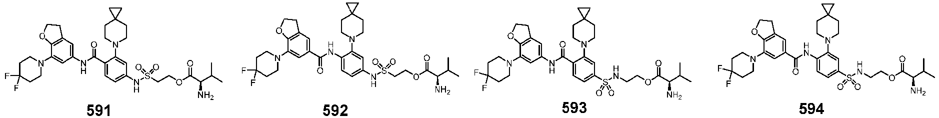

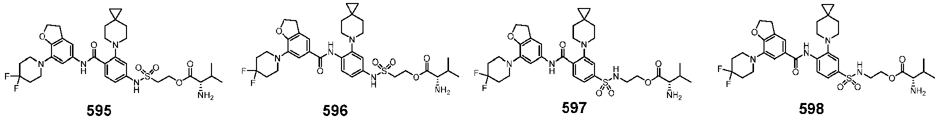

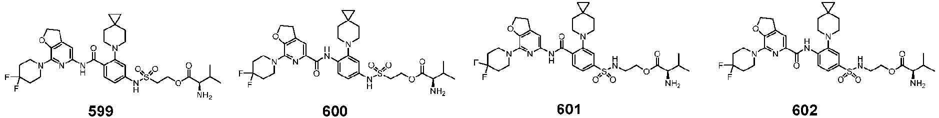

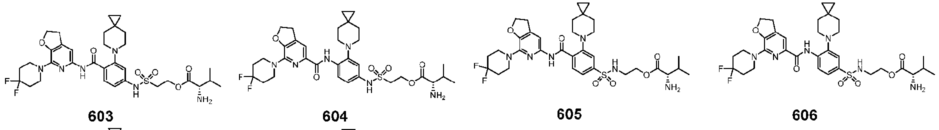

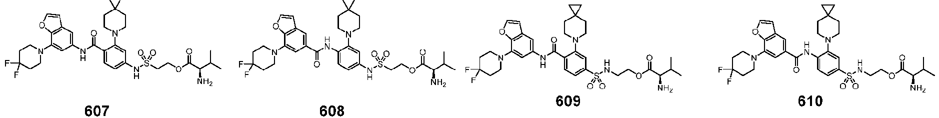

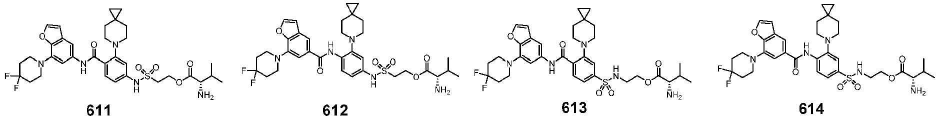

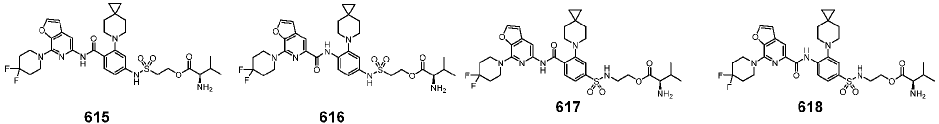

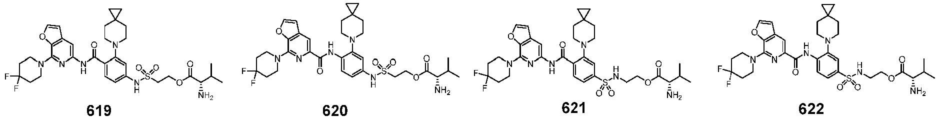

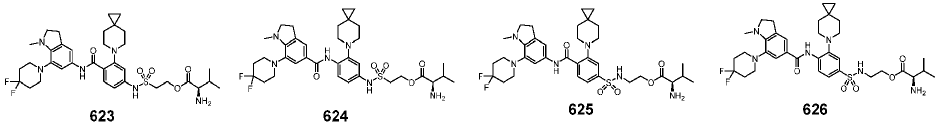

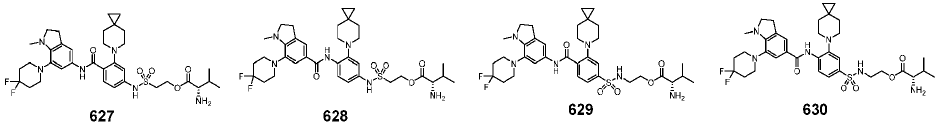

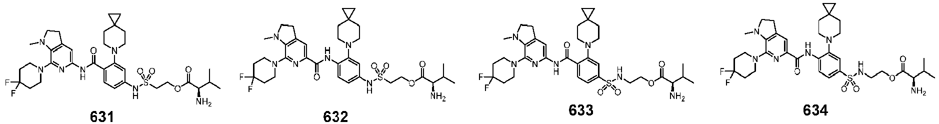

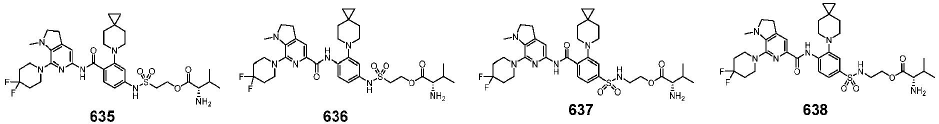

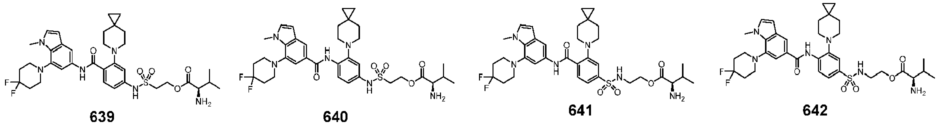

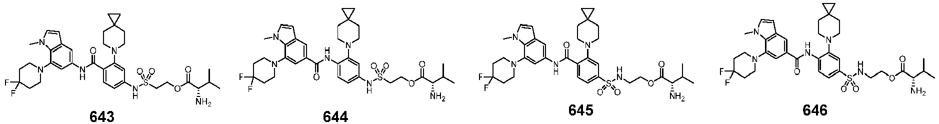

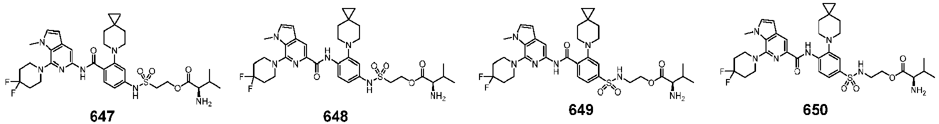

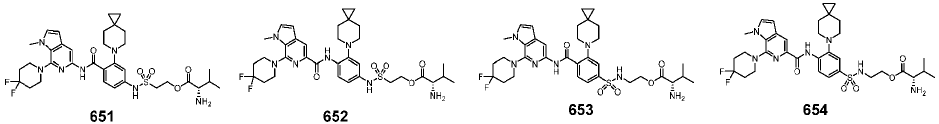

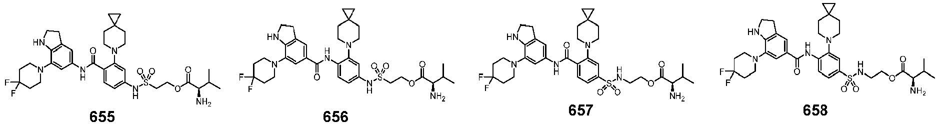

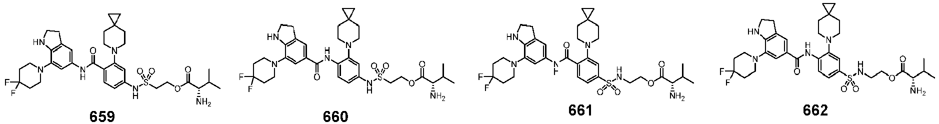

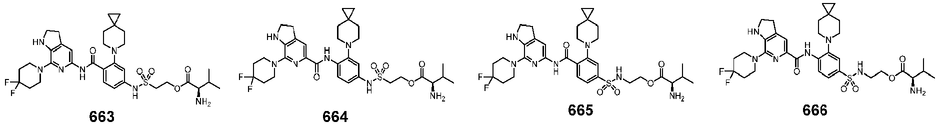

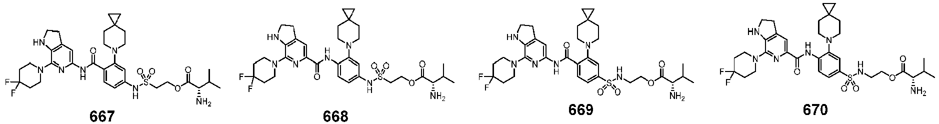

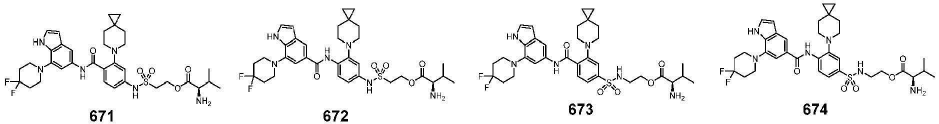

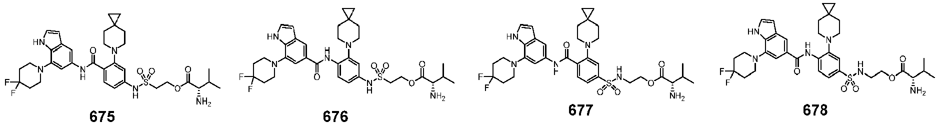

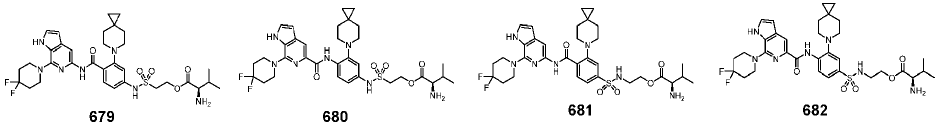

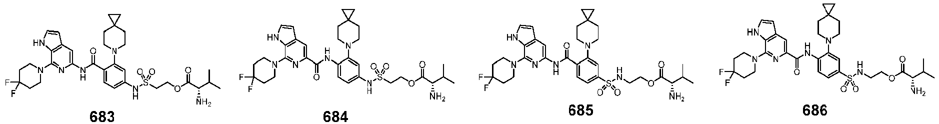

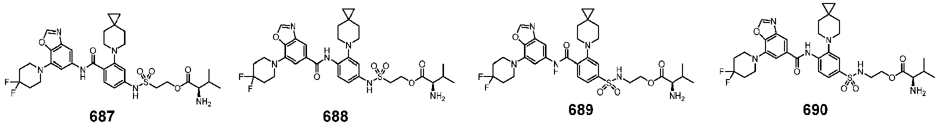

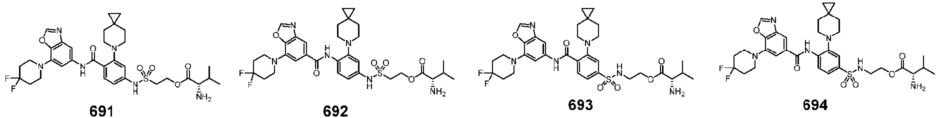

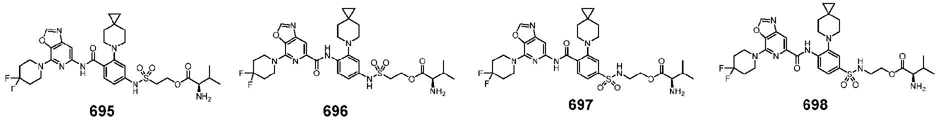

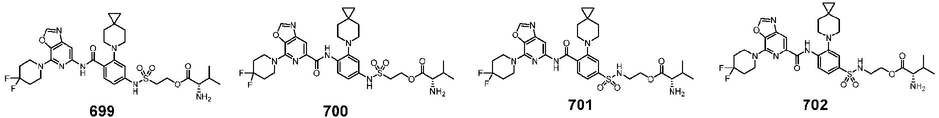

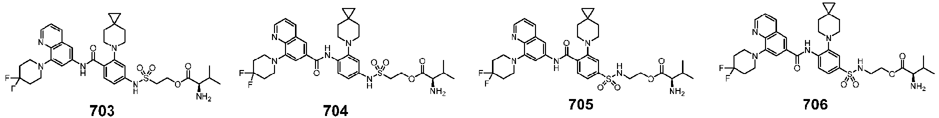

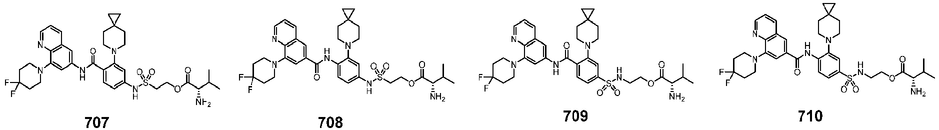

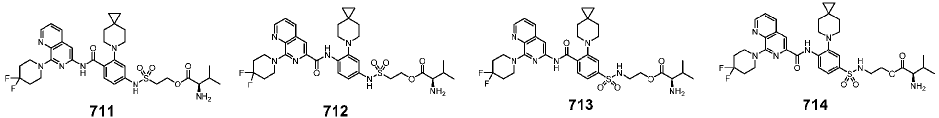

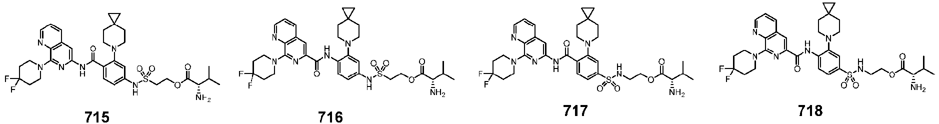

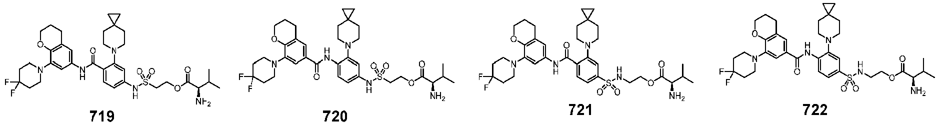

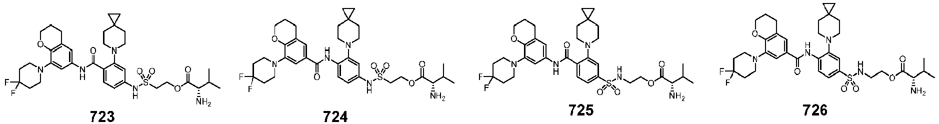

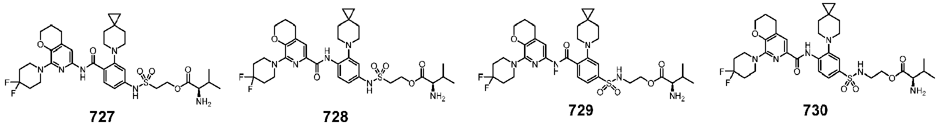

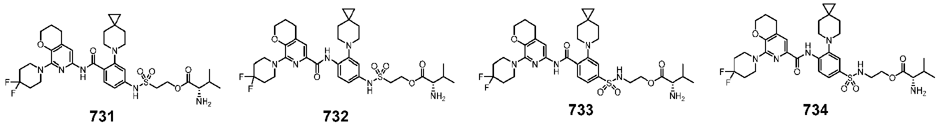

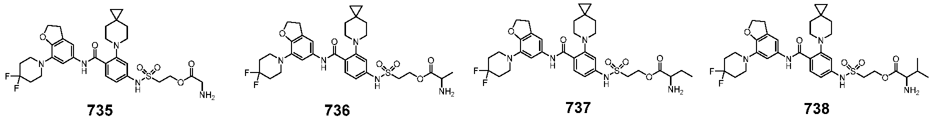

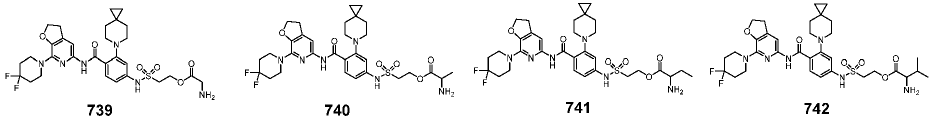

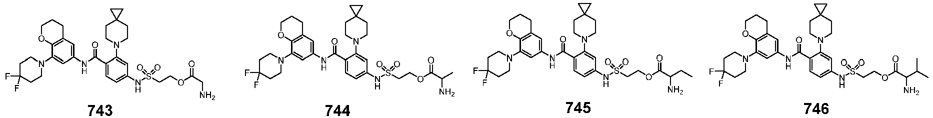

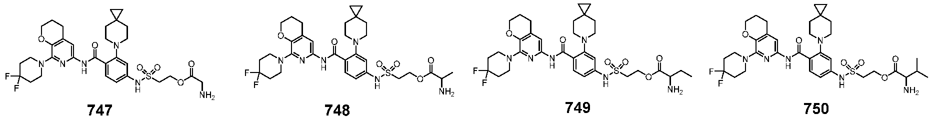

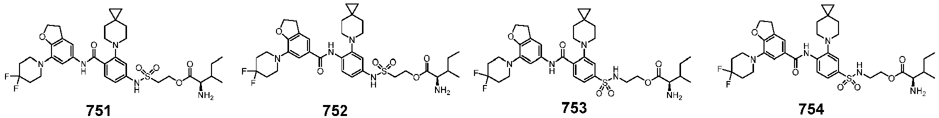

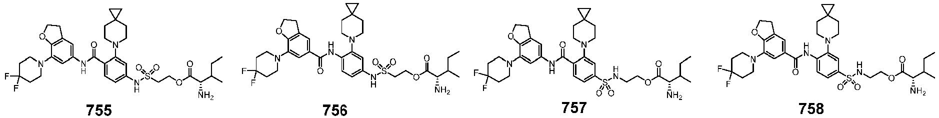

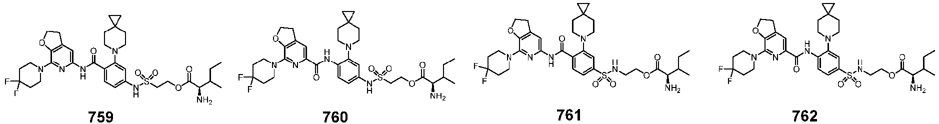

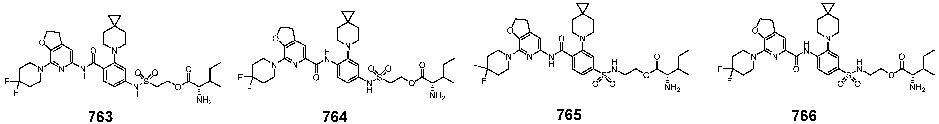

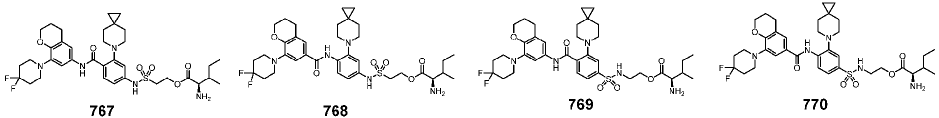

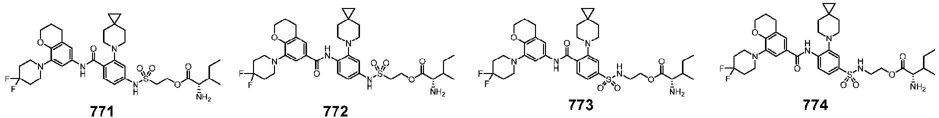

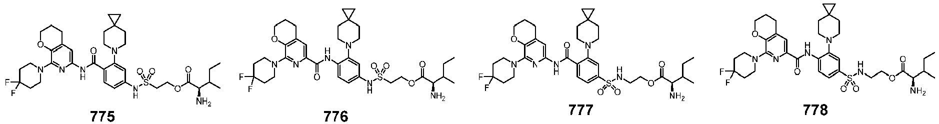

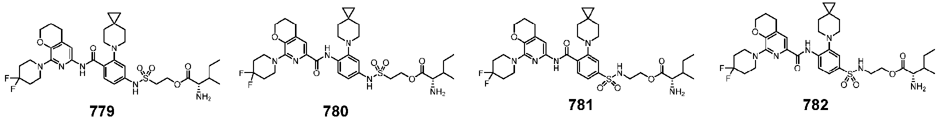

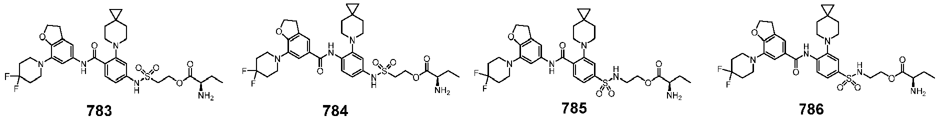

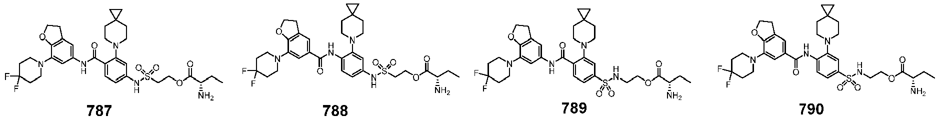

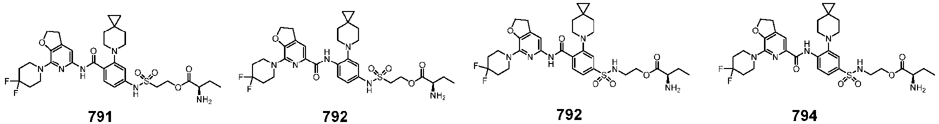

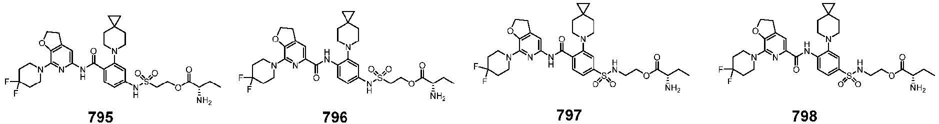

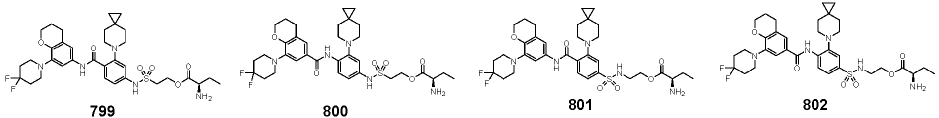

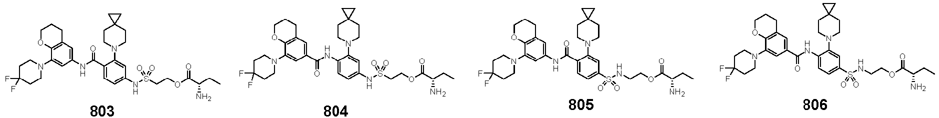

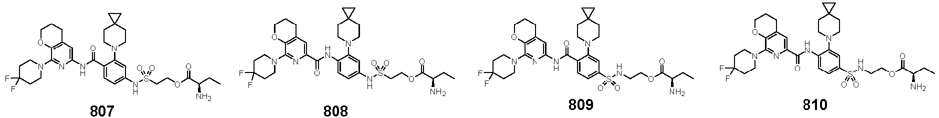

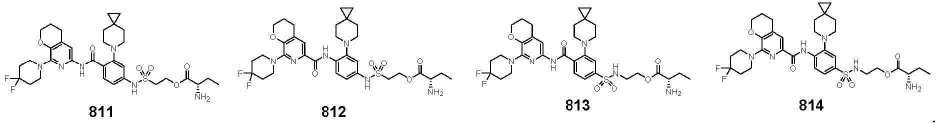

30. A pharmaceutical composition, comprising a pharmaceutically acceptable excipient or carrier, and the compound or the isomer thereof, the crystalline form thereof, the pharmaceutically acceptable salt thereof, the hydrate thereof, or the solvate thereof according to any one of claims 1-29 as an active ingredient.

31. Use of the compound or the isomer thereof, the crystalline form thereof, the pharmaceutically acceptable salt thereof, the hydrate thereof, or the solvate thereof according to any one of claims 1-29, or the pharmaceutical composition according to claim 30 in preparing a medicament for treating a related disease mediated by KIF18A protein.

32. The disease according to claim 31, wherein the disease is cancer, and the cancer is a hematologic cancer or a solid tumor.
